# EUROPEAN PATENT SPECIFICATION

(11) **EP 2 280 704 B1**
(45) Date of publication and mention of the grant of the patent: **13.05.2015**
(21) Application number: 09727673.7
(22) Date of filing: 30.03.2009
(51) Int. Cl.: A61K 31/401, A61K 31/4523, A61K 31/4985, A61K 31/506

(54) **OXYMETHYLENE ARYL COMPOUNDS AND USES THEREOF**
OXYMETHYLEN-ARYL-VERBINDUNGEN UND IHRE VERWENDUNGEN
COMPOSÉS D'OXYMÉTHYLÈNE ARYLIQUE ET UTILISATIONS DE CEUX-CI

(30) Priority: 31.03.2008 US 41196 P
(43) Date of publication of application: 09.02.2011
(73) Proprietor: Cymabay Therapeutics, Inc., Newark, CA 94560 (US)
(72) Inventor: WILSON, Maria, E., Hayward, CA 94545 (US); JOHNSON, Jeffrey, Hayward, CA 94545 (US); CLEMENS, L., Edward, Hayward, CA 94545 (US); ZHAO, Zuchun, Hayward, CA 94545 (US); CHEN, Xin, Hayward, CA 94545 (US)
(74) Representative: Gibson, Mark
(86) International application number: PCT/US2009/038847
(87) International publication number: WO 2009/123992

(56) References cited:
- WO-A-2006/076231
- WO-A-2007/003960
- WO-A-2007/003961
- WO-A-2007/039177
- WO-A-2008/083238
- WO-A-2008/109702
- US-A- 5 817 677

## Description

### BACKGROUND OF THE INVENTION

Diabetes mellitus can be divided into two clinical syndromes, Type I and Type II diabetes mellitus. Type I diabetes, or insulin-dependent diabetes mellitus, is a chronic autoimmune disease characterized by the extensive loss of beta cells in the pancreatic islets of Langerhans (hereinafter referred to as "pancreatic islet cells" or "islet cells"), which produce insulin. As these cells are progressively destroyed, the amount of secreted insulin decreases, eventually leading to hyperglycemia (abnormally high level of glucose in the blood) when the amount secreted drops below the level required for euglycemia (normal blood glucose level). Although the exact trigger for this immune response is not known, patients with Type I diabetes have high levels of antibodies against pancreatic beta cells (hereinafter "beta cells"). However, not all patients with high levels of these antibodies develop Type I diabetes.

Type II diabetes, or non-insulin-dependent diabetes mellitus, develops when muscle, fat and liver cells fail to respond normally to insulin. This failure to respond (called insulin resistance) may be due to reduced numbers of insulin receptors on these cells, or a dysfunction of signaling pathways within the cells, or both. The beta cells initially compensate for this insulin resistance by increasing their insulin output. Over time, these cells become unable to produce enough insulin to maintain normal glucose levels, indicating progression to Type II diabetes (Kahn SE, Am. J. Med. (2000) 108 Suppl 6a, 2S-8S).

The fasting hyperglycemia that characterizes Type II diabetes occurs as a consequence of the combined lesions of insulin resistance and beta cell dysfunction. The beta cell defect has two components: the first component, an elevation of basal insulin release (occurring in the presence of low, non-stimulatory glucose concentrations), is observed in obese, insulin-resistant pre-diabetic stages as well as in Type II diabetes. The second component is a failure to increase insulin release above the already elevated basal output in response to a hyperglycemic challenge. This lesion is absent in pre-diabetes and appears to define the transition from normo-glycemic insulin-resistant states to frank diabetes. There is currently no cure for diabetes. Conventional treatments for diabetes are very limited, and focus on attempting to control blood glucose levels in order to minimize or delay complications. Current treatments target either insulin resistance (metformin, thiazolidinediones ("TZDs")), or insulin release from the beta cell (sulphonylureas, exenatide). Sulphonylureas, and other compounds that act by depolarizing the beta cell, have the side effect of hypoglycemia since they cause insulin secretion independent of circulating glucose levels. One approved drug, Byetta® (exenatide) stimulates insulin secretion only in the presence of high glucose, but is not orally available and must be injected. Januvia^{™} (sitagliptin) is another recently approved drug that increases blood levels of incretin hormones, which can increase insulin secretion, reduce glucagon secretion and have other less well characterized effects. However, Januvia^{™} and other dipeptidyl peptidases IV (DPP IV) inhibitors may also influence the tissue levels of other hormones and peptides, and the long-term consequences of this broader effect have not been fully investigated. There is an unmet need for oral drugs that stimulate insulin secretion in a glucose dependent manner.

Progressive insulin resistance and loss of insulin secreting pancreatic β-cells are primary characteristics of Type II diabetes. Normally, a decline in the insulin sensitivity of muscle and fat is compensated for by increases in insulin secretion from the beta cell. However, loss of beta cell function and mass results in insulin insufficiency and diabetes (Kahn BB, Cell 92:593-596, 1998; Cavaghan MK, et al., J. Clin. Invest. 106:329-333. 2000; Saltiel AR, Cell 104:517-529, 2001; Prentki M and Nolan CJ. J Clin Invest. 116:1802-1812. (2006); and Kahn SE. J. Clin. Endocrinol. Metab. 86:4047-4058, 2001). Hyperglycemia further accelerates the decline in beta cell function (UKPDS Group, J.A.M.A. 281:2005-2012, 1999; Levy J, et al., Diabetes Med. 15:290-296, 1998; and Zhou YP, et al., J Biol Chem 278:51316-23, 2003). Several of the genes in which allelic variation is associated with an increased risk of Type II diabetes are expressed selectively in the beta cell (Bell GI and Polonsky KS, Nature 414:788-791 (2001); Saxena R, et al., Science. (2007) Apr 26; [Epub ahead of print]; and Valgerdur Steinthorsdottir, et al., Nature Genetics (2007) Apr 26; [Epub ahead of print]).

Insulin secretion from the beta cells of pancreatic islets is elicited by increased levels of blood glucose. Glucose is taken up into the beta cell primarily by the beta cell and liver selective transporter GLUT2 (Thorens B. Mol Membr Biol. 2001 Oct-Dec;18(4):265-73). Once inside the cell, glucose is phosphorylated by glucokinase, which is the primary glucose sensor in the beta cell since it catalyzes the irreversible rate limiting step for glucose metabolism (Matschinsky FM. Curr Diab Rep. 2005 Jun;5(3):171-6). The rate of glucose-6-phosphate production by glucokinase is dependent on the concentration of glucose around the beta cell, and therefore this enzyme allows for a direct relationship between level of glucose in the blood and the overall rate of glucose oxidation by the cell. Mutations in glucokinase produce abnormalities in glucose dependent insulin secretion in humans giving further evidence that this hexokinase family member plays a key role in the islet response to glucose (Gloyn AL, et al., J Biol Chem. 2005 Apr 8;280(14):14105-13. Epub 2005 Jan 25). Small molecule activators of glucokinase enhance insulin secretion and may provide a route for therapeutic exploitation of the role of this enzyme (Guertin KR and Grimsby J. Curr Med Chem. 2006;13(15):1839-43; and Matschinsky FM, et al., Diabetes 2006 Jan;55(1):1-12) in diabetes. Glucose metabolism via glycolysis and mitochondrial oxidative phosphorylation ultimately results in ATP production, and the amount of ATP produced in a beta cell is directly related to the concentration of glucose to which the beta cell is exposed.

Elevated ratios of ATP to ADP that occur in the presence of higher glucose result in the closure of the Kir6.2 channel via interaction with the SUR1 subunit of the channel complex. Closure of these channels on the plasma membrane of the beta cell results in depolarization of the membrane and subsequent activation of voltage dependent calcium channels (VDCCs) (Ashcroft FM, and Gribble FM, Diabetologia 42:903-919, 1999; and Seino S, Annu Rev Physiol. 61:337-362, 1999). Calcium ion entry as well as release of calcium from intracellular stores triggers exocytosis of insulin granules, resulting is secretion of insulin into the blood stream. Agents which close the Kir6.2 channel such as sulphonylureas and metaglitinides (Rendell M. Drugs 2004;64(12):1339-58; and Blickle JF, Diabetes Metab. 2006 Apr;32(2):113-20) also cause membrane depolarization, and therefore these agents stimulate insulin secretion in a glucose independent fashion. Potassium channel openers, such as diazoxide, inhibit insulin secretion by preventing elevated ATP/ADP ratios from closing the Kir6.2 channel (Hansen JB. Curr Med Chem. 2006;13(4):361-76). Calcium channel blockers, such as verapamil and nifedipine, can also inhibit insulin secretion (Henquin, J. C. (2004) Diabetes 53, S48-S58). Although sulfonylureas and metaglitinides are effective glucose lowering agents in the clinic, they act independently of blood glucose levels. Because they act independently of glucose levels, these drugs may result in hypoglycemia.

Glucose dependent insulin secretion from the beta cell is dependent on numerous neurotransmitters and blood-borne hormones, as well as local, intra-islet factors. CNS activation of the vagal innervation of the islet can lead to the release of small molecules such as acetylcholine and peptides such as vasoactive intestinal polypeptide (VIP), gastrin releasing peptide (GRP) and Pituitary Adenylate Cyclase Activating Peptide (PACAP). Acetylcholine activation of phospholipase C through the G_{αq}-coupled GPCR M3 muscarinic receptor leads to release of Ca++ from intracellular stores (Gilon P, and Henquin JC. Endocr Rev. 2001 Oct;22(5):565-604). Cholinergic agonists also lead to a subtle Na+ - dependent plasma membrane depolarization that can work in concert with glucose-initiated depolarization to enhance insulin release (Gilon P, and Henquin JC. Endocr Rev. 2001 Oct;22(5):565-604). VIP and PACAP each bind to an overlapping set of G_{α}-coupled GPCRs (PAC1, VIPR1, and VIPR2) on the beta cell that lead to stimulation of adenylate cyclase and an increase in intracellular cAMP (Filipsson K, et al., Diabetes, 2001 Sep;50(9):1959-69; Yamada H, et al., Regul Pept. 2004 Dec 15;123(1-3):147-53; and Qader SS, et al., Am J Physiol Endocrinol Metab. 2007 May;292(5):E1447-55).

Elevation of beta cell cAMP has a substantial potentiating effect on insulin secretion in the presence of stimulatory levels of glucose *(see* below). Unfortunately, many potentiators of glucose-stimulated insulin secretion also have effects outside of the islet which limit their ability to be used as diabetes therapeutics. For example, the best available selective muscarinic agonists which stimulate insulin secretion also stimulate multiple undesirable responses in multiple tissues (Rhoades RA and Tanner GA, eds. (2003) Medical Physiology, 2nd ed. Lippincott, Williams and Wilkins. ISBN 0-7817-1936-4). Likewise, VIP and PACAP receptors are present in multiple organ systems and mediate effects on the reproductive, immune and other diverse systems that make them less attractive as specific enhancers of glucose dependent insulin secretion.

Incretin hormones such as Glucagon-Like Peptide 1 (GLP-1) and Glucose-dependent Insulinotropic Polypeptide (GIP, also known as Gastric Inhibitory Polypeptide) also bind to specific G*alpha*ₛ-coupled GPCRs receptors on the surface of islet cells, including beta cells, and raise intracellular cAMP (Drucker DJ, J Clin Invest. 2007 Jan;117(1):24-32). Although the receptors for these hormones are present in other cells and tissues, the overall sum of effects of these peptides appear to be beneficial to control of glucose metabolism in the organism (Hansotia T, et al., J Clin Invest. 2007 Jan;117(1):143-52. Epub 2006 Dec 21). GIP and GLP-1 are produced and secreted from intestinal K and L cells, respectively, and these peptide hormones are released in response to meals by both direct action of nutrients in the gut lumen and neural stimulation resulting from food ingestion. GIP and GLP-1 have short half-lives in human circulation due to the action of the protease dipeptidyl-peptidase IV (DPP IV), and inhibitors of this protease can lower blood glucose due to their ability to raise the levels of active forms of the incretin peptides. The glucose lowering that can be obtained with DPP IV inhibitors, however, is somewhat limited since these drugs are dependent on the endogenous release of the incretin hormones. Peptides (e.g., exenatide (Byetta®)) and peptide-conjugates that bind to the GIP or GLP-1 receptors but are resistant to serum protease cleavage can also lower blood glucose substantially (Gonzalez C, et al., Expert Opin Investig Drugs 2006 Aug;15(8):887-95), but these incretin mimetics must be injected and tend to induce a high rate of nausea and therefore are not ideal therapies for general use in the Type II diabetic population. The clinical success of DPP IV inhibitors and incretin mimetics, though far from ideal, do point to the potential utility of compounds that increase incretin activity in the blood or directly stimulate cAMP in the beta cell. Some studies have indicated that beta cell responsiveness to GIP is diminished in Type II diabetes (Nauck MA, et al., J. Clin. Invest. 91:301-307 (1993); and Elahi D, et al., Regul. Pept. 51:63-74 (1994)). Restoration of this responsiveness (Meneilly GS, et al., Diabetes Care. 1993 Jan;16(1):110-4) maybe a promising way to improve beta cell function *in vivo.*

Since increased incretin activity has a positive effect on glucose dependent insulin secretion and perhaps other mechanisms that lead to lower blood glucose, it is also of interest to explore therapeutic approaches to increasing incretin release from intestinal K and L cells. GLP-1 secretion appears to be attenuated in Type II diabetes (Vilsboll T, et al., Diabetes 50:609-613), so improving incretin release may ameliorate this component of metabolic dysregulation. Nutrients such as glucose and fat in the gut lumen prompt incretin secretion by interaction with apical receptors (Vilsboll T, et al., Diabetes 50:609-613). GLP-1 and GIP release can also result from neural stimulation; acetylcholine and GRP can enhance incretin release in a manner perhaps analogous to the effects of these neurotransmitters on the beta cell in regard to insulin secretion (Brubaker P, Ann N Y Acad Sci. 2006 Jul;1070:10-26; and Reimann F, et al., Diabetes 2006 Dec; 55 (Supplement 2):S78-S85). Somatostatin, leptin and free fatty acids also appear to modulate incretin secretion (Brubaker P, Ann N Y Acad Sci. 2006 Jul;1070:10-26; and Reimann, F. et al., Diabetes. 2006 Dec;55(Supplement 2):S78-S85). To date, however, there does not appear to be a way to selectively impact these pathways to promote incretin secretion for therapeutic benefit. There is a need for oral drugs that stimulate incretin secretion in the treatment of diabetes.

Incretins can also increase the rate of beta cell proliferation and decrease the apoptotic rates of beta cells in animal models (Farilla L, et al., Endocrinology 2002 Nov;143(11):4397-408) and human islets *in vitro* (Farilla L, et al., Endocrinology 2003 Dec;144(12):5149-58). The net result of these changes is an increase in beta cell number and islet mass, and this should provide for increased insulin secretory capacity, which is another desired aim of anti-diabetic therapies. GLP-1 has also been shown to protect islets from the destructive effects of agents such as streptozotocin by blocking apoptosis (Li Y, et al., J Biol Chem. 2003 Jan 3;278(1):471-8). Cyclin D1, a key regulator of progression through the cell cycle, is up-regulated by GLP-1, and other agents that increase cAMP and PKA activity also have a similar effect (Friedrichsen BN, et al., J Endocrinol. 2006 Mar;188(3):481-92; and Kim MJ, et al., J Endocrinol. 2006 Mar;188(3):623-33). Increased transcription of the cyclin D1 gene occurs in response to PKA phosphorylation of CREB (cAMP-response element binding) transcription factors (Hussain MA, et al., Mol Cell Biol. 2006 Oct;26(20):7747-59). There is a need for oral drugs that increase beta cell number and islet mass in the treatment of diabetes.

Beta cell cAMP levels may also be raised by inhibiting the degradation of this second messenger by phosphodiesterases to AMP (Furman B, and Pyne N, Curr Opin Investig Drugs 2006 Oct;7(10):898-905). There are several different cAMP phosphodiesterases in the beta cell, and many of these have been shown to serve as a brake on glucose-dependent insulin secretion. Inhibitors of cAMP phosphodiesterases have been shown to increase insulin secretion *in vitro* and *in vivo,* including PDE1C, PDE3B, PDE10, (Han P, et al., J Biol Chem. 1999 Aug 6;274(32):22337-44; Harndahl L, et al., J Biol Chem. 2002 Oct 4;277(40):37446-55; Walz HA, et al., J Endocrinol. 2006 Jun;189(3):629-41; Choi YH, et al., J Clin Invest. 2006 Dec;116(12):3240-51; and Cantin LD, et al., Bioorg Med Chem Lett. 2007 May 15;17(10):2869-73) but so far, no PDEs have been found to have the cell type selectivity necessary to avoid undesirable effects. However, this remains an area of active investigation due to the potential for amplification of the effects of incretins and other agents that stimulate adenylate cyclase.

There appear to be multiple mechanisms by which cAMP elevation in the beta cell can enhance glucose dependent insulin secretion. Classically, many of the intracellular effects of cAMP are mediated by the cAMP-dependent protein kinase (protein kinase A, PKA) (Hatakeyama H, et al., J Physiol. 2006 Jan 15;570(Pt 2):271-82). PKA consists of a complex of two regulatory and two catalytic domains; binding of cAMP to the catalytic domains releases the catalytic domains and results in increased protein phosphorylation activity. One of the downstream effects of this kinase activity is enhanced efficiency of insulin exocytosis (Gromada J, et al., Diabetes 1998 Jan;47(1):57-65). Another cAMP binding protein is Epac, a guanine nucleotide exchange factor (GEF) (Kashima Y, et al., J Biol Chem. 2001 Dec 7;276(49):46046-53. Epub 2001 Oct 11; and Shibasaki T, et al., J Biol Chem. 2004 Feb 27;279(9):7956-61), which mediates a cAMP-dependent, but PKA-independent, increase in insulin exocytosis. Epac activated by cAMP may also enhance of release of intracellular Ca++ (Holz GG, Diabetes 2004 Jan;53(1):5-13). The effects of cAMP on insulin secretion are dependent on elevated glucose levels, so raising cAMP in the pancreatic beta cell is an important goal for therapeutics of Type II diabetes.

Agents that raise intracellular cAMP levels in the beta cell increase insulin secretion in a glucose dependent manner (MiuraY and Matsui H, Am. J. Physiol Endocrinol. Metab (2003) 285, E1001-E1009). One mechanism for raising cAMP is by the action of G-protein coupled cell surface receptors, which stimulate the enzyme adenylate cyclase to produce more cAMP. The GLP-1 receptor, which is the target of exenatide, is an example of such a receptor (Thorens B, et al., Diabetes (1993) 42, 1678-1682). There is a need for oral drugs that increase intracellular levels of cAMP in the treatment of diabetes.

DPP IV inhibitors are inhibitors of dipeptidyl peptidase-4. DPP IV is a prolyl protease that preferentially cleaves peptides after a proline amino acid residue. DPP IV is believed to degrade GLP-1. DPP IV inhibitors have been shown to prevent N-terminal degradation of GLP-1, and lowered blood glucose in preclinical studies. In addition, mice with a targeted disruption of the DPP IV gene had increased plasma levels of GLP-1 and GIP. Approved DPP IV inhibitors for treatment of diabetes include sitagliptin (Januvia^{™}) and vildagliptin (Galvus^{™}). Saxagliptin (BMS-477118) is another DPP IV inhibitor currently in clinical trials.

### BRIEF SUMMARY OF THE INVENTION

An unexpected finding of the inventors is that GPR119 (G-protein coupled receptor 119) agonists and DPP IV inhibitors are useful when both are administered to a diabetic subject. In one embodiment, this disclosure provides a method of treating diabetes comprising administering to a patient in need thereof a compound of Formula (I) and a DPP IV inhibitor. Formula (I) is 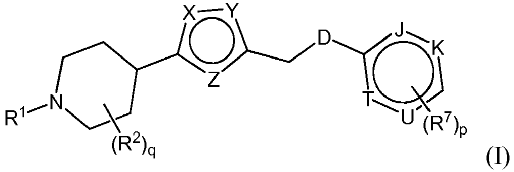 wherein,
D is selected from the group consisting of O, S, and NR⁸;
X, Y, and Z are independently selected from the group consisting of O, N, NR⁸, S, and CR³ and at least one of X, Y, and Z is O, N, NR⁸, or S;
J, K, T, and U are each independently selected from the group consisting of C, CH, and N;
the subscript p is an integer of from 0 to 4;
the subscript q is an integer of from 0 to 4;
R¹ is a member selected from the group consisting of H, C₁₋₁₀alkyl, C₁₋₁₀substituted alkyl, C₃₋₇cycloalkyl, C₂₋₁₀alkenyl, C₂₋₁₀alkynyl, -X¹-COR^{a}, -X¹-CO₂R^{a}, -X¹-CONR^{a}R^{b}, -SO₂R^{a}, a 4- to 7-membered heterocyclo group, aryl and a 5- to 10-membered heteroaryl group, wherein each of said cycloalkyl group, heterocyclo group, aryl group and heteroaryl group is optionally substituted with from 1 to 4 substituents independently selected from halo, C₁₋₁₀alkyl, C₁₋₁₀substituted alkyl, C₃₋₇cycloalkyl, C₂₋₁₀ alkenyl, C₂₋₁₀alkynyl, aryl, heteroaryl, -CN, -NR^{a}COR^{b}, -NR^{a}CONR^{a}R^{b}, -NO₂, -OR^{a}, -NR^{a}R^{b}, -COR^{a}, -CO₂R^{a}, -CONR^{a}R^{b}, -S(O)ₘR^{a}, -NR^{a}S(O)₂R^{b}, and -SO₂NR^{a}R^{b}, or optionally R^{a} and R^{b} are combined to form a 4-, 5- or 6-membered ring, and X¹ is selected from the group consisting of a bond, C₂₋₆alkene, C₂₋₆alkyne, -C(O)-, and -C(O)-(CH₂)₁₋₄-, wherein the aliphatic portions of X¹ are optionally substituted with one to three members selected from halogen, C₁₋₄alkyl, C₁₋₄substituted alkyl and C₁₋₄haloalkyl;
each R² is a member independently selected from the group consisting of halogen, C₁₋₅ alkyl, C₁₋₅substituted alkyl, C₃₋₇cycloalkyl, -COR^{a}, -CO₂R^{a}, -CONR^{a}R^{b}, -OR^{a}, -NR^{a}R^{b}, -NR^{a}COR^{b}, -SOR^{a}R^{b}, -SO₂R^{a} and -SO₂NR^{a}R^{b}, and wherein when the subscript q is 2 and R² is alkyl or substituted alkyl, the two R² members can optionally cyclize to form a ring;
R³ is a member selected from the group consisting of hydrogen, halogen, C₁₋₄alkyl, and C₁₋₄haloalkyl;
each R⁷ is independently selected from the group consisting of halo, C₁₋₁₀alkyl, C₁₋₁₀ substituted alkyl, C₃₋₇cycloalkyl, C₂₋₁₀alkenyl, C₂₋₁₀alkynyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -NR^{a}CONR^{a}R^{b}, -S(O)ₘR^{a}, -NR^{a}S(O)ₘR^{b}, -SO₂NR^{a}R^{b}, a 4- to 7-membered heterocyclo group, aryl and a 5- to 10-membered heteroaryl group, wherein each of said heterocyclo groups, said aryl and heteroaryl groups are optionally substituted with from one to four substituents independently selected from halo, oxo, C₁₋₄ alkyl, C₁₋₄ haloalkyl, C₃₋₇ cycloalkyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -NR^{a}CONR^{a}R^{b}, -S(O)ₘR^{a}, -NR^{a}SO₂R^{b}, and -SO₂NR^{a}R^{b} and wherein the subscript m is an integer of from 0 to 2, or optionally R^{a} and R^{b} are combined to form a 4-, 5- or 6-membered ring;
R⁸ is a member independently selected from the group consisting of hydrogen, C₁₋₄alkyl, and C₁₋₄haloalkyl;
and each R^{a} and R^{b} is independently selected from the group consisting of hydrogen, C₁₋₁₀ alkyl, C₁₋₁₀haloalkyl, C₃₋₁₀cycloalkyl, heterocyclyl, C₂₋₁₀alkenyl, C₂₋₁₀alkynyl, aryl, 5- to 6-membered heteroaryl and arylC₁₋₄alkyl; and wherein the aliphatic portions of each of said R^{a} and R^{b} is optionally substituted with from one to three members selected from the group consisting of halo, -ORⁿ, -OCORⁿ, -OC(O)N(Rⁿ)₂, -SRⁿ, -S(O)Rⁿ, -S(O)₂Rⁿ, -S(O)₂N(Rⁿ)₂, -NRⁿS(O)₂Rⁿ, -C(O)N(Rⁿ)₂, -C(O)Rⁿ, -NRⁿC(O)Rⁿ, -NRⁿC(O)N(Rⁿ)₂, -CO₂Rⁿ, -NRⁿCO₂Rⁿ, -CN, -NO₂, -N(Rⁿ)₂ and -NRⁿS(O)₂N(Rⁿ)₂, wherein each Rⁿ is independently hydrogen or an unsubstituted C₁₋₆ alkyl;
and wherein the aryl and heteroaryl portions are optionally substituted with from one to three members selected from halogen, -OR^{m}, -OC(O)N(R^{m})₂, -SR^{m}, -S(O)_{R}^{m}, -S(O)₂R^{m}, -S(O)₂N(R^{m})₂, -NR^{m}S(O)₂R^{m}, -C(O)N(R^{m})₂, -C(O)R^{m}, -NR^{m}C(O)R^{m}, -NR^{m}C(O)N(R^{m})₂, -CO₂R^{m}, -NR^{m}CO₂R^{m}, -CN, -NO₂, -N(R^{m})₂ and -NR^{m}S(O)₂N(R^{m})₂, wherein each R^{m} is independently hydrogen or an unsubstituted C₁₋₆ alkyl; or a pharmaceutically acceptable salt or ester therof; and wherein the molecular weight of said compound is less than 1200.

Example 1 shows the glucose lowering effect of administering both a compound of Formula (I) and sitagliptin, a DPP IV inhibitor. Example 2 shows the glucose lowering effect of administering both a compound of Formula (I) and vildagliptin, another DPP IV inhibitor. Example 3 shows the reduction of plasma insulin levels in DIO rats observed when a compound of Formula (I) and vildagliptin were co-administered. Example 4 shows the stimulation of incretin secretion in mice observed when a compound of Formula (I) and sitagliptin were administered. Similarly, Example 5 shows the stimulation of incretin secretion in DIO rats when a compound of Formula (I) and vildagliptin were co-administered. Example 6 shows the stimulation of incretin secretion in C57BL/6J mice when both a compound of Formula (I) and sitagliptin were administered.

An aspect of this disclosure provides methods of lowering blood levels of glucose in a subject by administering to a patient in need thereof a compound of Formula (I) and a DPP IV inhibitor.

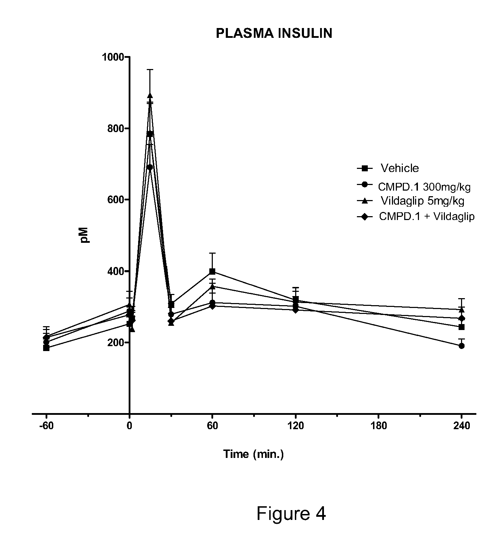

Another aspect of this disclosure provides methods of lowering blood levels of insulin in a subject by administering to a patient in need thereof a compound of Formula (I) and a DPP IV inhibitor. Figure 4 shows the plasma insulin levels of diet induce obesity (DIO) rats when the DIO rats are treated with a compound of Formula (I) and vildaglitpin.

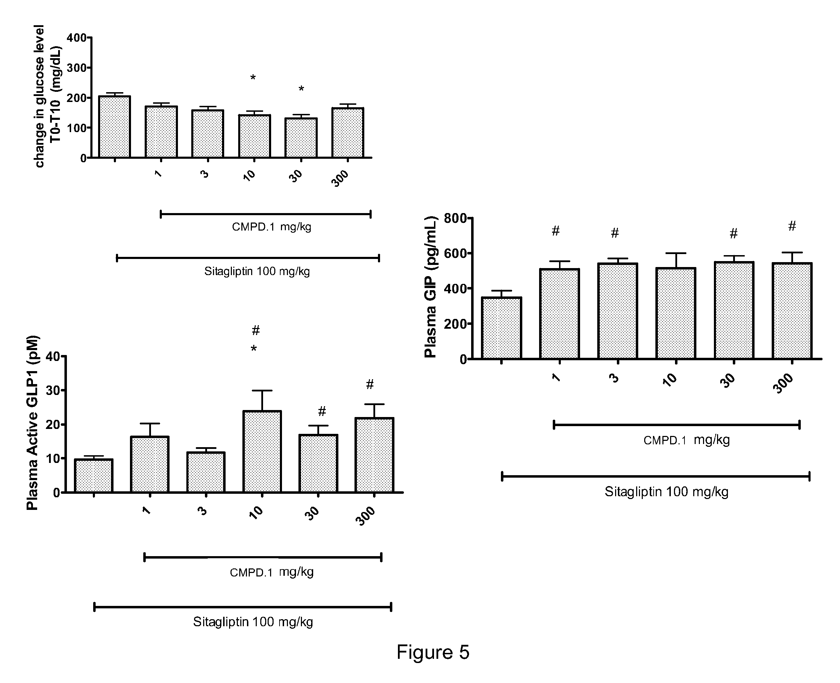

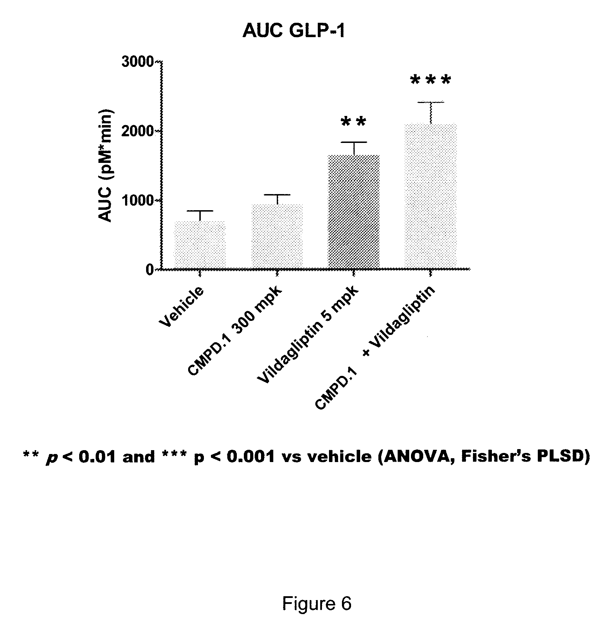

In another aspect, this disclosure provides methods of increasing blood levels of incretins in a subject by administering to a patient in need thereof a compound of Formula (I) and a DPP IV inhibitor. The incretins are GLP-1 and GIP. Figure 5 and 5a show increasing blood levels of GLP-1 and GIP in mice (Fig. 5) andGLP-1 in DIO rats (Fig. 5a) when the animals are treated with a compound of Formula (I) and sitagliptin or vildaglitpin. Figure 6 shows the increases in blood levels of GLP-1 and GIP in mice and DIO rats following an oral glucose challenge.

Yet another aspect of this disclosure provides methods of lowering blood triglyceride levels in a patient by administering to a patient in need thereof a compound of Formula (I) and a DPP IV inhibitor.

A further aspect of this disclosure provides methods of lowing gastric emptying in a patient by administering to a patient in need thereof a compound of Formula (I) and a DPP IV inhibitor.

Another aspect of this disclosure provides methods of increasing insulin production in the islet cells of a patient by administering to a patient in need thereof a compound of Formula (I) and a DPP IV inhibitor.

In yet another aspect, this disclosure provides methods of preserving islet function in a subject by administering to a patient in need thereof a compound of Formula (I) and a DPP IV inhibitor.

### BRIEF DESCRIPTION OF THE FIGURES

Figure 1 shows the data obtained from experiments as described in Example 1. Briefly, Figure 1 shows that blood glucose levels are lowered in response to administration of a compound of Formula (I) and a DPP IV inhibitor.
Figure 2 shows the data obtained from experiments as described in Example 2. Briefly, Figure 2 shows that blood glucose levels are lowered in response to administration of a compound of Formula (I) and a DPP IV inhibitor in DIO rats.
Figure 3 shows the data obtained from experiments as described in Example 2. Briefly, Figure 3 shows that blood glucose levels are lowered in response to administration of a compound of Formula (I) and a DPP IV inhibitor.
Figure 4 shows the data obtained from experiments as described in Example 3. Briefly, Figure 4 shows that plasma insulin levels are lowered in response to administration of a compound of Formula (I) and a DPP IV inhibitor.
Figure 5 shows the data obtained from experiments as described in Example 4. Briefly, Figure 5 show that plasma levels of active GLP-1 are increased in response to administration of a compound of Formula (I) and a DPP IV inhibitor.
Figure 6 shows the data obtained from experiments as described in Example 5. Briefly, Figure 6 shows that plasma GLP-1 levels are increased in response to administration of a compound of Formula (I) and a DPP IV inhibitor.
Figure 7 shows the data obtained from experiments as described in Example 6. Briefly, Figure 7 shows that plasma GLP-1 levels are increased in response to administration of a compound of Formula (I) and a DPP IV inhibitor.

### DETAILED DESCRIPTION OF THE INVENTION

### Abbreviations and Definitions

Unless otherwise stated, the following terms used in the specification and claims have the meanings given below:
"Alkyl" refers to monovalent saturated aliphatic hydrocarbyl groups having from 1 to 10 carbon atoms and, in some embodiments, from 1 to 6 carbon atoms. "Cᵤ₋ᵥalkyl" refers to alkyl groups having from u to v carbon atoms. This term includes, by way of example, linear and branched hydrocarbyl groups such as methyl (CH₃-), ethyl (CH₃CH₂-), *n*-propyl (CH₃CH₂CH₂-), isopropyl ((CH₃)₂CH-), *n*-butyl (CH₃CH₂CH₂CH₂-), isobutyl ((CH₃)₂CHCH₂-), *sec*-butyl ((CH₃)(CH₃CH₂)CH-), *t*-butyl ((CH₃)₃C-), *n*-pentyl (CH₃CH₂CH₂CH₂CH₂-), and neopentyl ((CH₃)₃CCH₂-).
"Substituted alkyl" refers to an alkyl group having from 1 to 5 and, in some embodiments, 1 to 3 or 1 to 2 substituents selected from the group consisting of alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, azido, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, guanidino, substituted guanidino, halo, hydroxy, hydroxyamino, alkoxyamino, hydrazino, substituted hydrazino, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, spirocycloalkyl, SO₃H, substituted sulfonyl, sulfonyloxy, thioacyl, thiocyanate, thiol, alkylthio, and substituted alkylthio, wherein said substituents are as defined herein.
"Alkylidene" or "alkylene" refers to divalent saturated aliphatic hydrocarbyl groups having from 1 to 10 carbon atoms and, in some embodiments, from 1 to 6 carbon atoms. "(Cᵤ₋ᵥ)alkylene" refers to alkylene groups having from u to v carbon atoms. The alkylidene and alkylene groups include branched and straight chain hydrocarbyl groups. For example "(C₁₋₆)alkylene" is meant to include methylene, ethylene, propylene, 2-methypropylene, pentylene, and the like.
"Substituted alkylidene" or "substituted alkylene" refers to an alkylidene group having from 1 to 5 and, in some embodiments, 1 to 3 or 1 to 2 substituents selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, azido, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, guanidino, substituted guanidino, halo, hydroxy, hydroxyamino, alkoxyamino, hydrazino, substituted hydrazino, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, oxo, thione, spirocycloalkyl, SO₃H, substituted sulfonyl, sulfonyloxy, thioacyl, thiocyanate, thiol, alkylthio, and substituted alkylthio, wherein said substituents are as defined herein.
"Alkenyl" refers to a linear or branched hydrocarbyl group having from 2 to 10 carbon atoms and in some embodiments from 2 to 6 carbon atoms or 2 to 4 carbon atoms and having at least 1 site of vinyl unsaturation (>C=C<). For example, (Cᵤ₋ᵥ)alkenyl refers to alkenyl groups having from u to v carbon atoms and is meant to include for example, ethenyl, propenyl, 1,3-butadienyl, and the like.
"Substituted alkenyl" refers to alkenyl groups having from 1 to 3 substituents and, in some embodiments, 1 to 2 substituents, selected from the group consisting of alkoxy, substituted alkoxy, acyl, acylamino, acyloxy, alkyl, substituted alkyl, alkynyl, substituted alkynyl, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, sulfonyloxy, thioacyl, thiol, alkylthio, and substituted alkylthio, wherein said substituents are defined as herein and with the proviso that any hydroxy or thiol substitution is not attached to an acetylenic carbon atom.
"Alkynyl" refers to a linear monovalent hydrocarbon radical or a branched monovalent hydrocarbon radical containing at least one triple bond. The term "alkynyl" is also meant to include those hydrocarbyl groups having one triple bond and one double bond. For example, (C₂-C₆)alkynyl is meant to include ethynyl, propynyl, and the like.
"Substituted alkynyl" refers to alkynyl groups having from 1 to 3 substituents and, in some embodiments, from 1 to 2 substituents, selected from the group consisting of alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, cycloalkenyl, substituted cycloalkenyl, cycloalkenyloxy, substituted cycloalkenyloxy, cycloalkenylthio, substituted cycloalkenylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, sulfonyloxy, thioacyl, thiol, alkylthio, and substituted alkylthio, wherein said substituents are defined herein and with the proviso that any hydroxy or thiol substitution is not attached to an acetylenic carbon atom.
"Alkoxy" refers to the group -O-alkyl wherein alkyl is defined herein. Alkoxy includes, by way of example, methoxy, ethoxy, *n*-propoxy, isopropoxy, *n*-butoxy, *t*-butoxy, *sec-*butoxy, and *n*-pentoxy.
"Substituted alkoxy" refers to the group -O-(substituted alkyl) wherein substituted alkyl is as defined herein.
"Acyl" refers to the groups H-C(O)-, alkyl-C(O)-, substituted alkyl-C(O)-, alkenyl-C(O)-, substituted alkenyl-C(O)-, alkynyl-C(O)-, substituted alkynyl-C(O)-, cycloalkyl-C(O)-, substituted cycloalkyl-C(O)-, aryl-C(O)-, substituted aryl-C(O)-, substituted hydrazino-C(O)-, heteroaryl-C(O)-, substituted heteroaryl-C(O)-, heterocyclic-C(O)-, and substituted heterocyclic-C(O)-, wherein alkyl, substituted alkyl, alkenyl, substituted alkenyl, alkynyl, substituted alkynyl, cycloalkyl, substituted cycloalkyl, aryl, substituted aryl, substituted hydrazino, heteroaryl, substituted heteroaryl, heterocyclic and substituted heterocyclic are as defined herein. Acyl includes the "acetyl" group CH₃C(O)-.
"Acylamino" refers to the groups -NR²⁰C(O)H, -NR²⁰C(O)alkyl, -NR²⁰C(O)substituted alkyl, -NR²⁰C(O)cycloalkyl, -NR²⁰C(O)substituted cycloalkyl, -NR²⁰C(O)alkenyl, -NR²⁰C(O)substituted alkenyl, -NR²⁰C(O)alkynyl, -NR²⁰C(O)substituted alkynyl, -NR²⁰C(O)aryl, -NR²⁰C(O)substituted aryl, -NR²⁰C(O)heteroaryl, -NR²⁰C(O)substituted heteroaryl, -NR²⁰C(O)heterocyclic, and -NR²⁰C(O)substituted heterocyclic wherein R²⁰ is hydrogen or alkyl and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.
"Acyloxy" refers to the groups H-C(O)O-, alkyl-C(O)O-, substituted alkyl-C(O)O-, alkenyl-C(O)O-, substituted alkenyl-C(O)O-, alkynyl-C(O)O-, substituted alkynyl-C(O)O-, aryl-C(O)O-, substituted aryl-C(O)O-, cycloalkyl-C(O)O-, substituted cycloalkyl-C(O)O-, heteroaryl-C(O)O-, substituted heteroaryl-C(O)O-, heterocyclic-C(O)O-, and substituted heterocyclic-C(O)O- wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic, and substituted heterocyclic are as defined herein.
"Amino" refers to the group -NH₂.
"Substituted amino" refers to the group -NR²¹R²² where R²¹ and R²² are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, substituted heterocyclic, -SO₂-alkyl, -SO₂-substituted alkyl, -SO₂-alkenyl, -SO₂-substituted alkenyl, -SO₂-cycloalkyl, -SO₂-substituted cylcoalkyl, -SO₂-aryl, -SO₂-substituted aryl, -SO₂-heteroaryl, -SO₂-substituted heteroaryl, -SO₂-heterocyclyl, and -SO₂-substituted heterocyclyl and wherein R²¹ and R²² are optionally joined together with the nitrogen bound thereto to form a heterocyclyl or substituted heterocyclyl group, provided that R²¹ and R²² are both not hydrogen, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic, and substituted heterocyclic are as defined herein. When R²¹ is hydrogen and R²² is alkyl, the substituted amino group is sometimes referred to herein as alkylamino. When R²¹ and R²² are alkyl, the substituted amino group is sometimes referred to herein as dialkylamino. When referring to a monosubstituted amino, it is meant that either R²¹ or R²² is hydrogen but not both. When referring to a disubstituted amino, it is meant that neither R²¹ nor R²² are hydrogen.
"Hydroxyamino" refers to the group -NHOH.
"Alkoxyamino" refers to the group -NHO-alkyl wherein alkyl is defined herein.
"Aminocarbonyl" refers to the group -C(O)NR²³R²⁴ where R²³ and R²⁴ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, substituted heterocyclic, hydroxy, alkoxy, substituted alkoxy, amino, substituted amino, and acylamino, and where R²³ and R²⁴ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.
"Aminothiocarbonyl" refers to the group -C(S)NR²³R²⁴ where R²³ and R²⁴ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where R²³ and R²⁴ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.
"Aminocarbonylamino" refers to the group -NR²⁰C(O)NR²¹R²⁴ where R²⁰ is hydrogen or alkyl and R²³ and R²⁴ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where R²³ and R²⁴ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.
"Aminothiocarbonylamino" refers to the group -NR²⁰C(S)NR²³R²⁴ where R²⁰ is hydrogen or alkyl and R²³ and R²⁴ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where R²³ and R²⁴ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.
"Aminocarbonyloxy" refers to the group -O-C(O)NR²³R²⁴ where R²³ and R²⁴ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where R²³ and R²⁴ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.
"Aminosulfonyl" refers to the group -SO₂NR²³R²⁴ where R²³ and R²⁴ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where R²³ and R²⁴ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.
"Aminosulfonyloxy" refers to the group -O-SO₂NR²³R²⁴ where R²³ and R²⁴ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where R²³ and R²⁴ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.
"Aminosulfonylamino" refers to the group -NR²⁰-SO₂NR²³R²⁴ where R²⁰ is hydrogen or alkyl and R²³ and R²⁴ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where R²³ and R²⁴ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.
"Amidino" refers to the group -C(=NR²⁵)NR²³R²⁴ where R²⁵,R²³, and R²⁴ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic and where R²³ and R²⁴ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.
"Aryl" refers to an aromatic group of from 6 to 14 carbon atoms and no ring heteroatoms and having a single ring (*e.g*., phenyl) or multiple condensed (fused) rings (*e.g*., naphthyl or anthryl). For multiple ring systems, including fused, bridged, and spiro ring systems having aromatic and non-aromatic rings that have no ring heteroatoms, the term "Aryl" or "Ar" applies when the point of attachment is at an aromatic carbon atom (*e.g*., 5,6,7,8 tetrahydronaphthalene-2-yl is an aryl group as its point of attachment is at the 2-position of the aromatic phenyl ring).
"Substituted aryl" refers to aryl groups which are substituted with 1 to 8 and, in some embodiments, 1 to 5, 1 to 3 or 1 to 2 substituents selected from the group consisting of alkyl, substituted alkyl, alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, azido, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, guanidino, substituted guanidino, halo, hydroxy, hydroxyamino, alkoxyamino, hydrazino, substituted hydrazino, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, sulfonyloxy, thioacyl, thiocyanate, thiol, alkylthio, and substituted alkylthio, wherein said substituents are defined herein.
"Arylalkyl" or "Aryl(C₁-C_{z})alkyl" refers to the radical -R^{u}R^{v} where R^{u} is an alkylene group (having eight or fewer main chain carbon atoms) and R^{v} is an aryl group as defined herein. Thus, "arylalkyl" refers to groups such as, for example, benzyl, and phenylethyl, and the like. Similarly, "Arylalkenyl" means a radical -R^{u}R^{v} where R^{u} is an alkenylene group (an alkylene group having one or two double bonds) and R^{v} is an aryl group as defined herein, *e.g*., styrenyl, 3-phenyl-2-propenyl, and the like.
"Aryloxy" refers to the group -O-aryl, where aryl is as defined herein, that includes, by way of example, phenoxy and naphthoxy.
"Substituted aryloxy" refers to the group -O-(substituted aryl) where substituted aryl is as defined herein.
"Arylthio" refers to the group -S-aryl, where aryl is as defined herein.
"Substituted arylthio" refers to the group -S-(substituted aryl), where substituted aryl is as defined herein.
"Azido" refers to the group -N₃.
"Hydrazino" refers to the group -NHNH₂.
"Substituted hydrazino" refers to the group -NR²⁶NR²⁷R²⁸ where R²⁶, R²⁷, and R²⁸ are independently selected from the group consisting of hydrogen, alkyl, substituted alkyl, alkenyl, substituted alkenyl, alkynyl, substituted alkynyl, aryl, substituted aryl, carboxyl ester, cycloalkyl, substituted cycloalkyl, heteroaryl, substituted heteroaryl, heterocyclic, substituted heterocyclic, -SO₂-alkyl -SO₂-substituted alkyl, -SO₂-alkenyl, -SO₂-substituted alkenyl, -SO₂-cycloalkyl, -SO₂-substituted cylcoalkyl, -SO₂-aryl, -SO₂-substituted aryl, -SO₂-heteroaryl, -SO₂-substituted heteroaryl, -SO₂-heterocyclic, and -SO₂-substituted heterocyclic and wherein R²⁷ and R²⁸ are optionally joined, together with the nitrogen bound thereto to form a heterocyclic or substituted heterocyclic group, provided that R²⁷ and R²⁸ are both not hydrogen, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.
"Cyano" or "carbonitrile" refers to the group -CN.
"Carbonyl" refers to the divalent group -C(O)- which is equivalent to -C(=O)-.
"Carboxyl" or "carboxy" refers to -COOH or salts thereof.
"Carboxyl ester" or "carboxy ester" refers to the groups -C(O)O-alkyl, -C(O)O-substituted alkyl, -C(O)O-alkenyl, -C(O)O-substituted alkenyl, -C(O)O-alkynyl, -C(O)O-substituted alkynyl, -C(O)O-aryl, -C(O)O-substituted aryl, -C(O)O-cycloalkyl, -C(O)O-substituted cycloalkyl, -C(O)O-heteroaryl, -C(O)O-substituted heteroaryl, -C(O)O-heterocyclic, and -C(O)O-substituted heterocyclic wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic, and substituted heterocyclic are as defined herein.
"(Carboxyl ester)amino" refers to the group -NR²⁰-C(O)O-alkyl, -NR²⁰-C(O)O-substituted alkyl, -NR²⁰-C(O)O-alkenyl, -NR²⁰-C(O)O-substituted alkenyl, -NR²⁰-C(O)O-alkynyl, -NR²⁰-C(O)O-substituted alkynyl, -NR²⁰-C(O)O-aryl, -NR²⁰-C(O)O-substituted aryl, -NR²⁰-C(O)O-cycloalkyl, -NR²⁰-C(O)O-substituted cycloalkyl, -NR²⁰-C(O)O-heteroaryl, -NR²⁰-C(O)O-substituted heteroaryl, -NR²⁰-C(O)O-heterocyclic, and -NR²⁰-C(O)O-substituted heterocyclic wherein R²⁰ is alkyl or hydrogen, and wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic, and substituted heterocyclic are as defined herein.
"(Carboxyl ester)oxy" refers to the group -O-C(O)O-alkyl, -O-C(O)O-substituted alkyl, -O-C(O)O-alkenyl, -O-C(O)O-substituted alkenyl, -O-C(O)O-alkynyl, -O-C(O)O-substituted alkynyl, -O-C(O)O-aryl, -O-C(O)O-substituted aryl, -O-C(O)O-cycloalkyl, -O-C(O)O-substituted cycloalkyl, -O-C(O)O-heteroaryl, -O-C(O)O-substituted heteroaryl, -O-C(O)O-heterocyclic, and -O-C(O)O-substituted heterocyclic wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.
"Cycloalkyl" refers to a saturated or partially saturated cyclic group of from 3 to 14 carbon atoms and no ring heteroatoms and having a single ring or multiple rings including fused, bridged, and spiro ring systems. For multiple ring systems having aromatic and non-aromatic rings that have no ring heteroatoms, the term "cycloalkyl" applies when the point of attachment is at a non-aromatic carbon atom (*e.g*., 5,6,7,8,-tetrahydronaphthalene-5-yl). The term "cycloalkyl" includes cycloalkenyl groups. Examples of cycloalkyl groups include, for instance, adamantyl, cyclopropyl, cyclobutyl, cyclopentyl, cyclooctyl, and cyclohexenyl. "Cᵤ₋ᵥcycloalkyl" refers to cycloalkyl groups having u to v carbon atoms as ring members. "Cᵤ₋ᵥcycloalkenyl" refers to cycloalkenyl groups having u to v carbon atoms as ring members.
"Cycloalkenyl" refers to a partially saturated cycloalkyl ring having at least one site of >C=C< ring unsaturation.
"Substituted cycloalkyl" refers to a cycloalkyl group, as defined herein, having from 1 to 8, or 1 to 5, or in some embodiments 1 to 3 substituents selected from the group consisting of oxo, thione, alkyl, substituted alkyl, alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, azido, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, guanidino, substituted guanidino, halo, hydroxy, hydroxyamino, alkoxyamino, hydrazino, substituted hydrazino, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, sulfonyloxy, thioacyl, thiocyanate, thiol, alkylthio, and substituted alkylthio, wherein said substituents are as defined herein. The term "substituted cycloalkyl" includes substituted cycloalkenyl groups.
"Cycloalkyloxy" refers to -O-cycloalkyl wherein cycloalkyl is as defined herein.
"Substituted cycloalkyloxy" refers to -O-(substituted cycloalkyl) wherein substituted cycloalkyl is as defined herein.
"Cycloalkylthio" refers to -S-cycloalkyl wherein substituted cycloalkyl is as defined herein.
"Substituted cycloalkylthio" refers to -S-(substituted cycloalkyl) wherein substituted cycloalkyl is as defined herein.
"Guanidino" refers to the group -NHC(=NH)NH₂.
"Substituted guanidino" refers to -NR²⁹C(=NR²⁹)N(R²⁹)₂ where each R²⁹ is independently selected from the group consisting of hydrogen, alkyl, substituted alkyl, aryl, substituted aryl, heteroaryl, substituted heteroaryl, heterocyclyl, and substituted heterocyclyl and two R²⁹ groups attached to a common guanidino nitrogen atom are optionally joined together with the nitrogen bound thereto to form a heterocyclic or substituted heterocyclic group, provided that at least one R²⁹ is not hydrogen, and wherein said substituents are as defined herein.
"Halo" or "halogen" refers to fluoro, chloro, bromo and iodo.
"Haloalkyl" refers to substitution of alkyl groups with 1 to 5 or in some embodiments 1 to 3 halo groups, *e.g*., -CH₂Cl, -CH₂F, -CH₂Br, -CFClBr, -CH₂CH₂Cl, -CH₂CH₂F, -CF₃, -CH₂CF₃, -CH₂CCl₃, and the like, and further includes those alkyl groups such as perfluoroalkyl in which all hydrogen atoms are replaced by fluorine atoms.
"Haloalkoxy" refers to substitution of alkoxy groups with 1 to 5 or in some embodiments 1 to 3 halo groups, e.g., -OCH₂Cl, -OCH₂F, -OCH₂CH₂Br, -OCH₂CH₂Cl, -OCF₃, and the like.
"Hydroxy" or "hydroxyl" refers to the group -OH.
"Heteroalkyl" means an alkyl radical as defined herein with one, two or three substituents independently selected from cyano, -OR^{w}, -NR^{x}R^{y}, and -S(O)ₙR^{z} (where n is an integer from 0 to 2), with the understanding that the point of attachment of the heteroalkyl radical is through a carbon atom of the heteroalkyl radical. R^{w} is hydrogen, alkyl, cycloalkyl, cycloalkyl-alkyl, aryl, arylalkyl, alkoxycarbonyl, aryloxycarbonyl, carboxamido, or mono- or di-alkylcarbamoyl. R^{x} is hydrogen, alkyl, cycloalkyl, cycloalkyl-alkyl, aryl or arylalkyl. R^{y} is hydrogen, alkyl, cycloalkyl, cycloalkyl-alkyl, aryl, arylalkyl, alkoxycarbonyl, aryloxycarbonyl, carboxamido, mono- or di-alkylcarbamoyl or alkylsulfonyl. R^{z} is hydrogen (provided that n is 0), alkyl, cycloalkyl, cycloalkyl-alkyl, aryl, arylalkyl, amino, mono-alkylamino, di-alkylamino, or hydroxyalkyl. Representative examples include, for example, 2-hydroxyethyl, 2,3-dihydroxypropyl, 2-methoxyethyl, benzyloxymethyl, 2-cyanoethyl, and 2-methylsulfonyl-ethyl. For each of the above, R^{w}, R^{x} , R^{y}, and R^{z} can be further substituted by amino, fluorine, alkylamino, di-alkylamino, OH or alkoxy. Additionally, the prefix indicating the number of carbon atoms (*e.g*., C₁-C₁₀) refers to the total number of carbon atoms in the portion of the heteroalkyl group exclusive of the cyano, -OR^{w}, -NR^{x}R^{y}, or -S(O)ₙR^{z} portions.
"Heteroaryl" refers to an aromatic group of from 1 to 14 carbon atoms and 1 to 6 heteroatoms selected from the group consisting of oxygen, nitrogen, and sulfur and includes a 5 to 18 member ring or ring system that includes a single ring (*e.g*., imidazolyl) or multiple rings (*e.g*., benzimidazol-2-yl and benzimidazol-6-yl). For multiple ring systems, including fused, bridged, and spiro ring systems having aromatic and non-aromatic rings, the term "heteroaryl" applies if there is at least one ring heteroatom and the point of attachment is at an atom of an aromatic ring (*e.g*., 1,2,3,4-tetrahydroquinolin-6-yl and 5,6,7,8-tetrahydroquinolin-3-yl). In one embodiment, the nitrogen and/or the sulfur ring atom(s) of the heteroaryl group are optionally oxidized to provide for the N-oxide (N→O), sulfinyl, or sulfonyl moieties. More specifically the term heteroaryl includes, but is not limited to, pyridyl, furanyl, thienyl, thiazolyl, isothiazolyl, triazolyl, imidazolyl, isoxazolyl, pyrrolyl, pyrazolyl, pyridazinyl, pyrimidinyl, benzofuranyl, tetrahydrobenzofuranyl, isobenzofuranyl, benzothiazolyl, benzoisothiazolyl, benzotriazolyl, indolyl, isoindolyl, benzoxazolyl, quinolyl, tetrahydroquinolinyl, isoquinolyl, quinazolinonyl, benzimidazolyl, benzisoxazolyl, or benzothienyl.
"Substituted heteroaryl" refers to heteroaryl groups that are substituted with from 1 to 8, or in some embodiements 1 to 5, or 1 to 3, or 1 to 2 substituents selected from the group consisting of the substituents defined for substituted aryl.
"Heteroaryloxy" refers to -O-heteroaryl wherein heteroaryl is as defined herein.
"Substituted heteroaryloxy" refers to the group -O-(substituted heteroaryl) wherein heteroaryl is as defined herein.
"Heteroarylthio" refers to the group -S-heteroaryl wherein heteroaryl is as defined herein.
"Substituted heteroarylthio" refers to the group -S-(substituted heteroaryl) wherein heteroaryl is as defined herein.
"Heterocycle" or "heterocyclic" or "heterocyclo" or "heterocycloalkyl" or "heterocyclyl" refers to a saturated or partially saturated cyclic group having from 1 to 14 carbon atoms and from 1 to 6 heteroatoms selected from the group consisting of nitrogen, sulfur, or oxygen and includes single ring and multiple ring systems including fused, bridged, and spiro ring systems. For multiple ring systems having aromatic and/or non-aromatic rings, the term "heterocyclic", "heterocycle", "heterocyclo", "heterocycloalkyl" or "heterocyclyl" applies when there is at least one ring heteroatom and the point of attachment is at an atom of a non-aromatic ring (*e.g*., 1,2,3,4-tetrahydroquinoline-3-yl, 5,6,7,8-tetrahydroquinoline-6-yl, and decahydroquinolin-6-yl). In one embodiment, the nitrogen and/or sulfur atom(s) of the heterocyclic group are optionally oxidized to provide for the N-oxide, sulfinyl, and sulfonyl moieties. More specifically the heterocyclyl includes, but is not limited to, tetrahydropyranyl, piperidinyl, N-methylpiperidin-3-yl, piperazinyl, N-methylpyrrolidin-3-yl, 3-pyrrolidinyl, 2-pyrrolidon-1-yl, morpholinyl, and pyrrolidinyl. A prefix indicating the number of carbon atoms (*e.*g., C₃-C₁₀) refers to the total number of carbon atoms in the portion of the heterocyclyl group exclusive of the number of heteroatoms.
"Substituted heterocycle" or "substituted heterocyclic" or "substituted heterocyclo" or "substituted heterocycloalkyl" or "substituted heterocyclyl" refers to heterocyclic groups, as defined herein, that are substituted with from 1 to 5 or in some embodiments 1 to 3 of the substituents as defined for substituted cycloalkyl.
"Heterocyclyloxy" refers to the group -O-heterocyclyl wherein heterocyclyl is as defined herein.
"Substituted heterocyclyloxy" refers to the group -O-(substituted heterocyclyl) wherein heterocyclyl is as defined herein.
"Heterocyclylthio" refers to the group -S-heterocycyl wherein heterocyclyl is as defined herein.
"Substituted heterocyclylthio" refers to the group -S-(substituted heterocycyl) wherein heterocyclyl is as defined herein.

Examples of heterocycle and heteroaryl groups include, but are not limited to, azetidine, pyrrole, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholinyl, thiomorpholinyl (also referred to as thiamorpholinyl), 1,1-dioxothiomorpholinyl, piperidinyl, pyrrolidine, and tetrahydrofuranyl.

"Nitro" refers to the group -NO₂.

"Oxo" refers to the atom (=O).

"Oxide" refers to products resulting from the oxidation of one or more heteroatoms. Examples include N-oxides, sulfoxides, and sulfones.

"Spirocycloalkyl" refers to a 3 to 10 member cyclic substituent formed by replacement of two hydrogen atoms at a common carbon atom with an alkylene group having 2 to 9 carbon atoms, as exemplified by the following structure wherein the methylene group shown below attached to bonds marked with wavy lines is substituted with a spirocycloalkyl group:

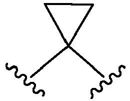

"Sulfonyl" refers to the divalent group -S(O)₂-.

"Substituted sulfonyl" refers to the group -SO₂-alkyl, -SO₂-substituted alkyl, -SO₂-alkenyl, -SO₂-substituted alkenyl, -SO₂-alkynyl, -SO₂-substituted alkynyl, -SO₂-cycloalkyl, -SO₂-substituted cylcoalkyl, -SO₂-aryl, -SO₂-substituted aryl, -SO₂-heteroaryl, -SO₂-substituted heteroaryl, -SO₂-heterocyclic, -SO₂-substituted heterocyclic, wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein. Substituted sulfonyl includes groups such as methyl-SO₂-, phenyl-SO₂-, and 4-methylphenyl-SO₂-.

"Sulfonyloxy" refers to the group -OSO₂-alkyl, -OSO₂-substituted alkyl, -OSO₂-alkenyl, -OSO₂-substituted alkenyl, -OSO₂-cycloalkyl, -OSO₂-substituted cylcoalkyl, -OSO₂-aryl, -OSO₂-substituted aryl, -OSO₂-heteroaryl, -OSO₂-substituted heteroaryl, -OSO₂-heterocyclic, -OSO₂-substituted heterocyclic, wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Thioacyl" refers to the groups H-C(S)-, alkyl-C(S)-, substituted alkyl-C(S)-, alkenyl-C(S)-, substituted alkenyl-C(S)-, alkynyl-C(S)-, substituted alkynyl-C(S)-, cycloalkyl-C(S)-, substituted cycloalkyl-C(S)-, aryl-C(S)-, substituted aryl-C(S)-, heteroaryl-C(S)-, substituted heteroaryl-C(S)-, heterocyclic-C(S)-, and substituted heterocyclic-C(S)-, wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein.

"Thiol" refers to the group -SH.

"Alkylthio" refers to the group -S-alkyl wherein alkyl is as defined herein.

"Substituted alkylthio" refers to the group -S-(substituted alkyl) wherein substituted alkyl is as defined herein.

"Thiocarbonyl" refers to the divalent group -C(S)- which is equivalent to -C(=S)-.

"Thione" refers to the atom (=S).

"Thiocyanate" refers to the group -SCN.

"Compound" and "compounds" as used herein refers to a compound encompassed by the generic formulae disclosed herein, any subgenus of those generic formulae, and any forms of the compounds within the generic and subgeneric formulae, such as an oxide, ester, prodrug, pharmaceutically acceptable salt, or solvate. Unless specified otherwise, the term further includes the racemates, stereoisomers, and tautomers of the compound or compounds.

"Racemates" refers to a mixture of enantiomers.

"Solvate" or "solvates" of a compound refer to those compounds, where compounds are as defined above, that are bound to a stoichiometric or non-stoichiometric amount of a solvent. Solvates of a compound includes solvates of all forms of the compound such as the oxide, ester, prodrug, or pharmaceutically acceptable salt of the disclosed generic and subgeneric formulae. Preferred solvents are volatile, non-toxic, and/or acceptable for administration to humans.

"Stereoisomer" or "stereoisomers" refer to compounds that differ in the chirality of one or more stereocenters. Stereoisomers include enantiomers and diastereomers. The compounds of this invention may exist in stereoisomeric form if they possess one or more asymmetric centers or a double bond with asymmetric substitution and, therefore, can be produced as individual stereoisomers or as mixtures. Unless otherwise indicated, the description is intended to include individual stereoisomers as well as mixtures. The methods for the determination of stereochemistry and the separation of stereoisomers are well-known in the art *(see* discussion in Chapter 4 of Advanced Organic Chemistry, 4th edition J. March, John Wiley and Sons, New York, 1992).

"Tautomer" refers to alternate forms of a compound that differ in the position of a proton, such as enol-keto and imine-enamine tautomers, or the tautomeric forms of heteroaryl groups containing a ring atom attached to both a ring -NH- moiety and a ring =N- moiety such as pyrazoles, imidazoles, benzimidazoles, triazoles, and tetrazoles.

"Prodrug" refers to any derivative of a compound of the embodiments that is capable of directly or indirectly providing a compound of the embodiments or an active metabolite or residue thereof when administered to a patient. Prodrugs of a compound of the present invention are prepared by modifying functional groups present in the compound in such a way that the modifications may be cleaved *in vivo* to release the parent compound, or an active metabolite. For example, prodrugs include compounds wherein a hydroxy, amino, or sulfhydryl group in a compound I is bonded to any group that may be cleaved *in vivo* to regenerate the free hydroxyl, amino, or sulfhydryl group, respectively. Particularly favored derivatives and prodrugs are those that increase the bioavailability of the compounds of the embodiments when such compounds are administered to a patient (*e.g*., by allowing an orally administered compound to be more readily absorbed into the blood) or which enhance delivery of the parent compound to a biological compartment (*e.g*., the brain or lymphatic system) relative to the parent species. Prodrugs include ester, amide, and carbamate (*e.g*., N, N-dimethylaminocarbonyl) forms of hydroxy functional groups of compounds of the invention. Examples of ester prodrugs include formate, acetate, propionate, butyrate, acrylate, and ethylsuccinate derivatives. An general overview of prodrugs is provided in T Higuchi and V Stella, Pro-drugs as Novel Delivery Systems, Vol. 14 of the A.C.S. Symposium Series, and in Edward B Roche, ed., Bioreversible Carriers in Drug Design, American Pharmaceutical Association and Pergamon Press, 1987, both of which are incorporated herein by reference.

"Pharmaceutically acceptable salt" refers to pharmaceutically acceptable salts derived from a variety of organic and inorganic counter ions well known in the art and includes, by way of example only, sodium, potassium, calcium, magnesium, ammonium, and tetraalkylammonium. When the molecule contains a basic functionality, acid addition salts of organic or inorganic acids, such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, oxalic acid, 4-toluenesulfonic acid, camphorsulfonic acid, methanesulfonic acid,4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like. Salts can also be formed when an acidic proton present in the parent compound is either replaced by a metal ion, *e.g*., an alkali metal ion, an alkaline earth ion, or an aluminum ion; or coordinates with an organic base such as ethanolamine, diethanolamine, triethanolamine, trimethylamine, N-methylglucamine, and the like. Pharmaceutically acceptable salts are suitable for administration in a patient and possess desirable pharmacological properties. Suitable salts further include those described in P. Heinrich Stahl, Camille G. Wermuth (Eds.), Handbook of Pharmaceutical Salts Properties, Selection, and Use; 2002.

Unless indicated otherwise, the nomenclature of substituents that are not explicitly defined herein are arrived at by naming the terminal portion of the functionality followed by the adjacent functionality toward the point of attachment. For example, the substituent "arylalkyloxycabonyl" refers to the group (aryl)-(alkyl)-O-C(O)-.

It is understood that in all substituted groups defined above, polymers arrived at by defining substituents with further substituents to themselves (*e.g*., substituted aryl having a substituted aryl group as a substituent which is itself substituted with a substituted aryl group, which is further substituted by a substituted aryl group, etc.) are not intended for inclusion herein. In such cases, the maximum number of such substitutions is three. For example, serial substitutions of substituted aryl groups with two other substituted aryl groups are limited to -substituted aryl-(substituted aryl)-substituted aryl.

Similarly, it is understood that the above definitions are not intended to include impermissible substitution patterns (*e.g*., methyl substituted with 5 fluoro groups). Such impermissible substitution patterns are well known to the skilled artisan.

The terms "optional" or "optionally" as used throughout the specification means that the subsequently described event or circumstance may but need not occur, and that the description includes instances where the event or circumstance occurs and instances in which it does not. For example, "heterocyclo group optionally mono- or di- substituted with an alkyl group" means that the alkyl may but need not be present, and the description includes situations where the heterocyclo group is mono- or disubstituted with an alkyl group and situations where the heterocyclo group is not substituted with the alkyl group.

Turning next to the compositions of the invention, the term "pharmaceutically acceptable carrier or excipient" means a carrier or excipient that is useful in preparing a pharmaceutical composition that is generally safe, possesses acceptable toxicities. Acceptable carriers or excipients include those that are acceptable for veterinary use as well as human pharmaceutical use. A "pharmaceutically acceptable carrier or excipient" as used in the specification and claims includes both one and more than one such carrier or excipient.

With reference to the methods of the present disclosure the following terms are used with the noted meanings:

The terms "treating" or "treatment" of a disease includes:
(1) preventing or reducing the risk of developing the disease, *i.e*., causing the clinical symptoms of the disease not to develop in a mammal that may be exposed to or predisposed to the disease but does not yet experience or display symptoms of the disease,
(2) inhibiting the disease, *i.e*., arresting or reducing the development of the disease or its clinical symptoms, or
(3) relieving the disease, *i.e*., causing regression of the disease or its clinical symptoms.

A preferred embodiment of the disclosure is treatment of a disease that consists of relieving the disease.

The term "diagnosing" refers to determining the presence or absence of a particular disease or condition. Additionally, the term refers to determining the level or severity of a particular disease or condition, as well as monitoring of the disease or condition to determine its response to a particular therapeutic regimen.

The term "therapeutically effective amount" means the amount of the subject compound that will elicit the biological or medical response of a tissue, system, animal or human that is being sought by the researcher, veterinarian, medical doctor or other clinician. "A therapeutically effective amount" includes the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

"Patient" refers to mammals and includes humans and non-human mammals. Examples of patients include, but are not limited to mice, rats, hamsters, guinea pigs, pigs, rabbits, cats, dogs, goats, sheep, cows, and humans.

The term "mammal" includes, without limitation, humans, domestic animals (*e.g*., dogs or cats), farm animals (cows, horses, or pigs), and laboratory animals (mice, rats, hamsters, guinea pigs, pigs, rabbits, dogs, or monkeys).

The term "insulin resistance" can be defined generally as a disorder of glucose metabolism. More specifically, insulin resistance can be defined as the diminished ability of insulin to exert its biological action across a broad range of concentrations producing less than the expected biologic effect (*see, e.g.,* Reaven GM, J. Basic & Clin. Phys. & Pharm. (1998) 9:387-406 and Flie J, Ann Rev. Med. (1983) 34:145-60). Insulin resistant persons have a diminished ability to properly metabolize glucose and respond poorly, if at all, to insulin therapy. Manifestations of insulin resistance include insufficient insulin activation of glucose uptake, oxidation and storage in muscle and inadequate insulin repression of lipolysis in adipose tissue and of glucose production and secretion in liver. Insulin resistance can cause or contribute to polycystic ovarian syndrome, impaired glucose tolerance, gestational diabetes, metabolic syndrome, hypertension, obesity, atherosclerosis and a variety of other disorders. Eventually, the insulin resistant individuals can progress to a point where a diabetic state is reached.

The term "diabetes mellitus" or "diabetes" means a disease or condition that is generally characterized by metabolic defects in production and utilization of glucose that result in the failure to maintain appropriate blood sugar levels in the body. The result of these defects is elevated blood glucose, referred to as "hyperglycemia." Two major forms of diabetes are Type I diabetes and Type II diabetes. As described above, Type I diabetes is generally the result of an absolute deficiency of insulin, the hormone that regulates glucose utilization. Type II diabetes often occurs in the face of normal, or even elevated levels of insulin and can result from the inability of tissues to respond appropriately to insulin. Most Type II diabetic patients are insulin resistant and have a relative deficiency of insulin, in that insulin secretion can not compensate for the resistance of peripheral tissues to respond to insulin. In addition, many Type II diabetics are obese. Other types of disorders of glucose homeostasis include impaired glucose tolerance, which is a metabolic stage intermediate between normal glucose homeostasis and diabetes, and gestational diabetes mellitus, which is glucose intolerance in pregnancy in women with no previous history of Type I or Type II diabetes.

The term "metabolic syndrome" refers to a cluster of metabolic abnormalities including abdominal obesity, insulin resistance, glucose intolerance, diabetes, hypertension and dyslipidemia. These abnormalities are known to be associated with an increased risk of vascular events.

The term "abdominal obesity" is defined by a cutoff point of waist circumference ≥ 102 cm in men and ≥ 80 cm in women, as recommended by the third report of the national cholesterol education program expert panel on detection, evaluation, and treatment of high blood cholesterol in adults (NCEP/ATP Panel III).

The guidelines for diagnosis of Type II diabetes, impaired glucose tolerance, and gestational diabetes have been outlined by the American Diabetes Association (*see, e.g.,* The Expert Committee on the Diagnosis and Classification of Diabetes Mellitus, Diabetes Care, (1999) Vol 2 (Suppl 1):SS-19).

The term "secretagogue" means a substance or compound that stimulates secretion. For example, an insulin secretagogue is a substance or compound that stimulates secretion of insulin.

The term "symptom" of diabetes, includes, but is not limited to, polyuria, polydipsia, and polyphagia, as used herein, incorporating their common usage. For example, "polyuria" means the passage of a large volume of urine during a given period; "polydipsia" means chronic, excessive thirst; and "polyphagia" means excessive eating. Other symptoms of diabetes include, *e.g*., increased susceptibility to certain infections (especially fungal and staphylococcal infections), nausea, and ketoacidosis (enhanced production of ketone bodies in the blood).

The term "complication" of diabetes includes, but is not limited to, microvascular complications and macro vascular complications. Microvascular complications are those complications that generally result in small blood vessel damage. These complications include, *e.g*., retinopathy (the impairment or loss of vision due to blood vessel damage in the eyes); neuropathy (nerve damage and foot problems due to blood vessel damage to the nervous system); and nephropathy (kidney disease due to blood vessel damage in the kidneys). Macrovascular complications are those complications that generally result from large blood vessel damage. These complications include, *e.g*., cardiovascular disease and peripheral vascular disease. Cardiovascular disease refers to diseases of blood vessels of the heart. See, *e.g.,* Kaplan RM, et al., "Cardiovascular diseases" in Health and Human Behavior, pp. 206-242 (McGraw-Hill, New York 1993). Cardiovascular disease is generally one of several forms, including, *e.g*., hypertension (also referred to as high blood pressure), coronary heart disease, stroke, and rheumatic heart disease. Peripheral vascular disease refers to diseases of any of the blood vessels outside of the heart. It is often a narrowing of the blood vessels that carry blood to leg and arm muscles.

The term "atherosclerosis" encompasses vascular diseases and conditions that are recognized and understood by physicians practicing in the relevant fields of medicine. Atherosclerotic cardiovascular disease, coronary heart disease (also known as coronary artery disease or ischemic heart disease), cerebrovascular disease and peripheral vessel disease are all clinical manifestations of atherosclerosis and are therefore encompassed by the terms "atherosclerosis" and "atherosclerotic disease".

The term "antihyperlipidemic" refers to the lowering of excessive lipid concentrations in blood to desired levels.

The term "modulate" refers to the treating, prevention, suppression, enhancement or induction of a function or condition. For example, compounds can modulate Type II diabetes by increasing insulin in a human, thereby suppressing hyperglycemia.

The term "triglyceride(s)" ("TGs"), as used herein, incorporates its common usage. TGs consist of three fatty acid molecules esterified to a glycerol molecule. TGs serve to store fatty acids that are used by muscle cells for energy production or are taken up and stored in adipose tissue.

Because cholesterol and TGs are water insoluble, they must be packaged in special molecular complexes known as "lipoproteins" in order to be transported in the plasma. Lipoproteins can accumulate in the plasma due to overproduction and/or deficient removal. There are at least five distinct lipoproteins differing in size, composition, density, and function. In the cells of the small intestine, dietary lipids are packaged into large lipoprotein complexes called "chylomicrons", which have a high TG and low-cholesterol content. In the liver, TG and cholesterol esters are packaged and released into plasma as TG-rich lipoprotein called very low density lipoprotein ("VLDL"), whose primary function is the endogenous transport of TGs made in the liver or released by adipose tissue. Through enzymatic action, VLDL can be either reduced and taken up by the liver, or transformed into intermediate density lipoprotein ("IDL"). IDL, is in turn, either taken up by the liver, or is further modified to form low density lipoprotein ("LDL"). LDL is either taken up and broken down by the liver, or is taken up by extrahepatic tissue. High density lipoprotein ("HDL") helps remove cholesterol from peripheral tissues in a process called reverse cholesterol transport.

The term "dyslipidemia" refers to abnormal levels of lipoproteins in blood plasma including both depressed and/or elevated levels of lipoproteins (*e.g*., elevated levels of LDL and/or VLDL, and depressed levels of HDL).

The term "hyperlipidemia" includes, but is not limited to, the following:
(1) *Familiar Hyperchylomicronemia,* a rare genetic disorder that causes a deficiency in an enzyme, LP lipase, that breaks down fat molecules. The LP lipase deficiency can cause the accumulation of large quantities of fat or lipoproteins in the blood;
(2) *Familiar Hypercholesterolemia,* a relatively common genetic disorder caused where the underlying defect is a series of mutations in the LDL receptor gene that result in malfunctioning LDL receptors and/or absence of the LDL receptors. This brings about ineffective clearance of LDL by the LDL receptors resulting in elevated LDL and total cholesterol levels in the plasma;
(3) *Familiar Combined Hyperlipidemia,* also known as multiple lipoprotein-type hyperlipidemia is an inherited disorder where patients and their affected first-degree relatives can at various times manifest high cholesterol and high triglycerides. Levels of HDL cholesterol are often moderately decreased;
(4) *Familiar Defective Apolipoprotein B-100* is a relatively common autosomal dominant genetic abnormality. The defect is caused by a single nucleotide mutation that produces a substitution of glutamine for arginine, which can cause reduced affinity of LDL particles for the LDL receptor. Consequently, this can cause high plasma LDL and total cholesterol levels;
*(5) Familiar Dysbetaliproteinermia,* also referred to as Type III Hyperlipoproteinemia, is an uncommon inherited disorder resulting in moderate to severe elevations of serum TG and cholesterol levels with abnormal apolipoprotein E function. HDL levels are usually normal; and
(6) *Familiar Hypertriglyceridemia,* is a common inherited disorder in which the concentration of plasma VLDL is elevated. This can cause mild to moderately elevated TG levels (and usually not cholesterol levels) and can often be associated with low plasma HDL levels.

Risk factors for hyperlipidemia include, but are not limited to, the following: (1) disease risk factors, such as a history of Type I diabetes, Type II diabetes, Cushing's syndrome, hypothyroidism and certain types of renal failure; (2) drug risk factors, which include, birth control pills; hormones, such as estrogen, and corticosteroids; certain diuretics; and various β blockers; (3) dietary risk factors include dietary fat intake per total calories greater than 40%; saturated fat intake per total calories greater than 10%; cholesterol intake greater than 300 mg per day; habitual and excessive alcohol use; and obesity.

The terms "obese" and "obesity" refers to, according to the World Health Organization, a Body Mass Index ("BMI") greater than 27.8 kg/m² for men and 27.3 kg/m² for women (BMI equals weight (kg)/height (m²). Obesity is linked to a variety of medical conditions including diabetes and hyperlipidemia. Obesity is also a known risk factor for the development of Type II diabetes (*see, e.g.,* Barrett-Conner E, Epidemol. Rev. (1989) 11:172-181; and Knowler, et al., Am. J. Clin. Nutr. (1991) 53:1543-1551).

The term "pancreas" refers to a gland organ in the digestive and endocrine system of vertebrates, including mammals. The pancreas secretes both digestive enzymes and hormones such as insulin, GLP-1 and GIP as well as other hormones.

The term "islet" or "islet of Langerhans" refers to endocrine cells of the pancreas that are grouped together in islets and secrete insulin and other hormones.

The term "beta cell" refers to cells found in the islet of Langerhans that secrete insulin, amylin, and other hormones.

The term "endocrine cell" refers to cells that secrete hormones into the blood stream. Endocrine cells are found various glands and organ systems of the body including the pancreas, intestines, and other organs.

The term "L cell" refers to gut endocrine cells that produce GLP-1.

The term "K cell" refers to gut endocrine cells that produce GIP.

The term "incretin" refers to a group of hormones that increases insulin secretion in response to food intake. Incretins include GLP-1 and GIP.

The term "insulin" refers to a polypeptide hormone that regulates glucose metabolism. Insulin binds to insulin receptors in insulin sensitive cells and mediates glucose uptake. Insulin is used to treat Type I diabetes and may be used to treat Type II diabetes.

The term "GLP-1" or "glucagon-like peptide" is a peptide hormone primarily produced by L cells. GLP-1 increases insulin secretion, decrease glucagon secretion, increase beta cell mass and insulin gene expression, inhibits acid secretion and gastric emptying in the stomach, and decreases food intake by increasing satiety.

The term "GIP" or "gastric inhibitory peptide" or "glucose dependent insulinotropic polypeptide" refers to a peptide hormone produced primarily by K cells. GIP stimulates insulin secretion. GIP also has significant effects on lipid metabolism.

The term "cAMP" or "cyclic AMP" or "cyclic adenosine monophosphate" refers to an intracellular signaling molecule involved in many biological processes, including glucose and lipid metabolism.

The term "agonist" refers to a compound that binds to a receptor and triggers a response in a cell. An agonist mimics the effect of an endogenous ligand, a hormone for example, and produces a physiological response similar to that produced by the endogenous ligand.

The term "partial agonist" refers to a compound that binds to a receptor and triggers a partial response in a cell. A partial agonist produces only a partial physiological response of the endogenous ligand.

The present invention derives from the discovery of compounds that act as agonists of IC-GPCR2 (Seq. ID 1) using a cell-based screen. A stable CHO cell line expressing IC-GPCR2 under the control of the CMV promoter was used and cAMP levels were measured in the cells using a homogeneous time resolved fluorescence assay. With a parental CHO cell line as a control, increased cAMP levels could be measured and compounds identified that, like exenatide, raise cAMP in cells (*see In Vitro* Activity Table in Biological Example 1). Since elevated intracellular cAMP levels in the beta cell increase insulin secretion in a glucose dependant manner (*see* Biological Examples 2 and 3), the present invention is useful for the treatment of, *inter alia,* Type II diabetes and other diseases associated with poor glycemic control. The novel agonists described in this invention are orally active *(see* Biological Example 3), providing a significant differentiating feature to exenatide. Additionally, the islet specific expression of the receptor for the novel agonists of the present invention (*see* Biological Example 4) also make the present invention useful for the diagnosis of, *inter alia,* diabetes and other diseases associated with beta cell health.

In one embodiment, this disclosure provides methods of treating diabetes by administering a compound of Formula (I) and a DPP IV inhibitor.

Turning now to the compounds of Formula (I), it is represented by the following:

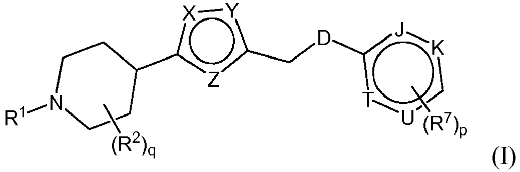

Wherein the letters X, Y, and Z are each independently selected from the group consisting of O, N, NR⁸, S, and C(R³) and at least one of X, Y, and Z is O, N, NR⁸, or S; J, K, T, and U are each independently selected from the group consisting of C, CH, and N; the subscript p is an integer of from 0 to 4; and the subscript q is an integer of from 0 to 4.

In Formula (I), R¹ is a member selected from the group consisting of H, C₁₋₁₀alkyl, C₁₋₁₀substituted alkyl, C₃₋₇cycloalkyl, C₂₋₁₀alkenyl, C₂₋₁₀alkynyl, -X¹-COR^{a}, -X¹-CO₂R^{a}, -X¹-CONR^{a}R^{b}, -SO₂R^{a}, a 4- to 7-membered heterocyclo group, aryl and a 5- to 10-membered heteroaryl group, wherein each of said cycloalkyl group, heterocyclo group, aryl group and heteroaryl group is optionally substituted with from 1 to 4 substituents independently selected from halo, C₁₋₁₀alkyl, C₁₋₁₀substituted alkyl, C₃₋₇cycloalkyl, C₂₋₁₀ alkenyl, C₂₋₁₀alkynyl, aryl, heteroaryl, -CN, -NR^{a}COR^{b}, -NR^{a}CONR^{a}R^{b}, -NO₂, -OR^{a}, -NR^{a}R^{b}, -COR^{a}, -CO₂R^{a}, -CONR^{a}R^{b}, -S(O)ₘR^{a}, -NR^{a}S(O)₂R^{b}, and -SO₂NR^{a}R^{b}, or optionally R^{a} and R^{b} are combined to form a 4-, 5- or 6-membered ring, and X¹ is selected from the group consisting of a bond, C₂₋₆alkene, C₂₋₆alkyne, -C(O)-, and -C(O)-(CH₂)₁₋₄-, wherein the aliphatic portions of X¹ are optionally substituted with one to three members selected from halogen, C₁₋₄alkyl, C₁₋₄substituted alkyl and C₁₋₄haloalkyl.

Turning next to R², each R² is a member independently selected from the group consisting of halogen, C₁₋₅ alkyl, C₁₋₅ substituted alkyl, C₃₋₇cycloalkyl, -COR^{a}, -CO₂R^{a}, -CONR^{a}R^{b}, -OR^{a}, -NR^{a}R^{b}, -NR^{a}COR^{b}, -SOR^{a}R^{b}, -SO₂R^{a} and -SO₂NR^{a}R^{b}, and wherein when the subscript q is 2 and R² is alkyl or substituted alkyl, the two R² members can optionally cyclize to form a ring.

R³ is a member selected from the group consisting of hydrogen, halogen, C₁₋₄alkyl, and C₁₋₄haloalkyl.

Each R⁷ of Formula (I) is independently selected from the group consisting of halo, C₁₋₁₀alkyl, C₁₋₁₀substituted alkyl, C₃₋₇cycloalkyl, C₂₋₁₀alkenyl, C₂₋₁₀alkynyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -NR^{a}CONR^{a}R^{b}, -S(O)ₘR^{a}, -NR^{a}S(O)ₘR^{b}, -SO₂NR^{a}R^{b}, a 4- to 7- membered heterocyclo group, aryl and a 5- to 10-membered heteroaryl group, wherein each of said heterocyclo groups, said aryl and heteroaryl groups are optionally substituted with from one to four substituents independently selected from halo, oxo, C₁₋₄ alkyl, C₁₋₄ haloalkyl, C₃₋₇ cycloalkyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -NR^{a}CONR^{a}R^{b}, -S(O)ₘR^{a}, -NR^{a}SO₂R^{b}, and -SO₂NR^{a}R^{b} and wherein the subscript m is an integer of from 0 to 2, or optionally R^{a} and R^{b} are combined to form a 4-, 5- or 6-membered ring.

R⁸ is a member independently selected from the group consisting of hydrogen, C₁₋₄alkyl, and C₁₋₄haloalkyl.

For each of the above groups, each R^{a} and R^{b} is independently selected from the group consisting of hydrogen, C₁₋₁₀ alkyl, C₁₋₁₀haloalkyl, C₃₋₁₀cycloalkyl, heterocyclyl, C₂₋₁₀alkenyl, C₂₋₁₀alkynyl, aryl, 5- to 6-membered heteroaryl and arylC₁₋₄alkyl; and wherein the aliphatic portions of each of said R^{a} and R^{b} is optionally substituted with from one to three members selected from the group consisting of halo, -ORⁿ, -OCORⁿ, -OC(O)N(Rⁿ)₂, -SRⁿ, -S(O)Rⁿ, -S(O)₂Rⁿ, -S(O)₂N(Rⁿ)₂, -NRⁿS(O)₂Rⁿ, -C(O)N(Rⁿ)₂, -C(O)Rⁿ, -NRⁿC(O)Rⁿ, -NRⁿC(O)N(Rⁿ)₂, -CO₂Rⁿ, -NRⁿCO₂Rⁿ, -CN, -NO₂, -N(Rⁿ)₂ and -NRⁿS(O)₂N(Rⁿ)₂, wherein each Rⁿ is independently hydrogen or an unsubstituted C₁₋₆ alkyl; and wherein the aryl and heteroaryl portions are optionally substituted with from one to three members selected from halogen, -OR^{m}, -OC(O)N(R^{m})₂, -SR^{m}, -S(O)_{R}^{m}, -S(O)₂R^{m}, -S(O)₂N(R^{m})₂, -NR^{m}S(O)₂R^{m}, -C(O)N(R^{m})₂, -C(O)R^{m}, -NR^{m}C(O)R^{m}, -NR^{m}C(O)N(R^{m})₂, -CO₂R^{m}, -NR^{m}CO₂R^{m}, -CN, -NO₂, -N(R^{m})₂ and -NR^{m}S(O)₂N(R^{m})₂, wherein each R^{m} is independently hydrogen or an unsubstituted C₁₋₆ alkyl.

The compounds provided herein also include any pharmaceutically acceptable salts of the compounds as well as any isotopically labeled isomers thereof. In general, the compounds useful in the methods described herein are those compound of the formula above, wherein the molecular weight of the compound is less than 1200, more preferably less than about 1000, still more preferably less than about 800 and still more preferably from about 200 to about 600.

In one embodiment, a preferred R¹ group is selected from the group consisting of -X¹-COR^{a}, -X¹-CO₂R^{a}, -X¹-CONR^{a}R^{b}, -SO₂R^{a}, aryl, heteroaryl, substituted aryl and substituted heteroaryl. When R¹ is an aromatic substituent, R¹ is preferably selected from the group consisting of pyridyl, substituted pyridyl, pyrimidinyl, substituted pyrimidinyl, pyrazinyl, substituted pyrazinyl, pyridazinyl, substituted pyridazinyl, phenyl, substituted phenyl, imidazolyl, triazolyl, substituted triazolyl, substituted imidazolyl, oxazolyl, substituted oxazolyl, thiazolyl, substituted thiazolyl, oxadiazolyl, substituted oxadiazolyl, tetrazolyl, and substituted tetrazolyl.

When R¹ is an aromatic substituent, *e.g.*, aryl or heteroaryl, R¹ can be substituted with from one to three substituents selected from the group consisting of C₁₋₁₀alkyl, C₁₋₁₀haloalkyl, C₃₋₇cycloalkyl, aryl, heteroaryl, -NO₂, -OR^{a}, -NR^{a}R^{b}, -CO₂R^{a}, -CONR^{a}R^{b}, -S(O)ₘR^{a}, -NR^{a}S(O)₂R^{b}, and -SO₂NR^{a}R^{b}.

In one embodiment, a preferred R² is a member independently selected from the group consisting of halo, C₁₋₅alkyl, C₁₋₅haloalkyl, and the subscript q is an integer of from 0 to 2.

In another preferred embodiment, D is O. In compounds of Formula (I), when D is O, a preferred R¹ group is selected from the group consisting of -X¹-COR^{a}, -X¹-CO₂R^{a}, -X¹-CONR^{a}R^{b}, -SO₂R^{a}, aryl, heteroaryl, substituted aryl and substituted heteroaryl. When R¹ is an aromatic substituent, R¹ is preferably selected from the group consisting of pyridyl, substituted pyridyl, pyrimidinyl, substituted pyrimidinyl, pyrazinyl, substituted pyrazinyl, pyridazinyl, substituted pyridazinyl, phenyl, substituted phenyl, imidazolyl, triazolyl, substituted triazolyl, substituted imidazolyl, oxazolyl, substituted oxazolyl, thiazolyl, substituted thiazolyl, oxadiazolyl, substituted oxadiazolyl, tetrazolyl, and substituted tetrazolyl.

Additionally, when D is O, and R¹ is an aromatic substituent, e.g., aryl or heteroaryl, R¹ can be substituted with from one to three substituents selected from the group consisting of C₁₋₁₀alkyl, C₁₋₁₀haloalkyl, C₃₋₇cycloalkyl, aryl, heteroaryl, -NO₂, -OR^{a}, -NR^{a}R^{b}, -CO₂R^{a}, -CONR^{a}R^{b}, -S(O)ₘR^{a}, -NR^{a}S(O)₂R^{b}, and -SO₂NR^{a}R^{b}.

Yet another embodiment of this disclosure is a compound of Formula (I) wherein J, K, T, and U are all C or CH. In this embodiment, a preferred R¹ group is selected from the group consisting of -X¹-COR^{a}, -X¹-CO₂R^{a}, -X¹-CONR^{a}R^{b}, -SO₂R^{a}, aryl, heteroaryl, substituted aryl and substituted heteroaryl. When R¹ is an aromatic substituent, R¹ is preferably selected from the group consisting of pyridyl, substituted pyridyl, pyrimidinyl, substituted pyrimidinyl, pyrazinyl, substituted pyrazinyl, pyridazinyl, substituted pyridazinyl, phenyl, substituted phenyl, imidazolyl, triazolyl, substituted triazolyl, substituted imidazolyl, oxazolyl, substituted oxazolyl, thiazolyl, substituted thiazolyl, oxadiazolyl, substituted oxadiazolyl, tetrazolyl, and substituted tetrazolyl. Further, when J, K, T, and U are all C or CH, and R¹ is an aromatic substituent, *e.g.*, aryl or heteroaryl, R¹ can be substituted with from one to three substituents selected from the group consisting of C₁₋₁₀alkyl, C₁₋₁₀haloalkyl, C₃₋₇cycloalkyl, aryl, heteroaryl, -NO₂, -OR^{a}, -NR^{a}R^{b}, -CO_{2R}^{a}, -CONR^{a}R^{b}, -S(O)ₘR^{a}, -NR^{a}S(O)₂R^{b}, and -SO₂NR^{a}R^{b}.

One embodiment of this disclosure comprises compounds of Formula (I) wherein the subscript p is an integer of from 1 to 3 and each R⁷ is independently selected from the group consisting of halo, C₁₋₁₀alkyl, C₁₋₁₀haloalkyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -S(O)ₘR^{a}, -NR^{a}S(O)ₘR^{b}, -SO₂NR^{a}R^{b}, a 4- to 7-membered heterocyclo group, aryl and a 5- to 10-membered heteroaryl group, wherein each of said heterocyclo groups, said aryl and heteroaryl groups are optionally substituted with from one to four substituents independently selected from halo, oxo, C₁₋₄ alkyl, C₁₋₄ haloalkyl, C₃₋₇ cycloalkyl, -CN, -NO₂, -OR^{a}, -NR^{a}R^{b}, -CO₂R^{a}, -CONR^{a}R^{b}, -NR^{a}COR^{b}, -NR^{a}CO₂R^{b}, -S(O)ₘR^{a}, -NR^{a}SO₂R^{b}, and -SO₂NR^{a}R^{b} and wherein the subscript m is an integer of from 0 to 2.

Yet another aspect of this disclosure provides compounds of Formula (I) wherein J, K, T, and U are all C or CH. A preferred R¹ group is selected from the group consisting of -X¹-COR^{a}, -X¹-CO₂R^{a}, -X¹-CONR^{a}R^{b}, -SO₂R^{a}, aryl, heteroaryl, substituted aryl and substituted heteroaryl. When R¹ is an aromatic substituent, R¹ is preferably selected from the group consisting of pyridyl, substituted pyridyl, pyrimidinyl, substituted pyrimidinyl, pyrazinyl, substituted pyrazinyl, pyridazinyl, substituted pyridazinyl, phenyl, substituted phenyl, imidazolyl, triazolyl, substituted triazolyl, substituted imidazolyl, oxazolyl, substituted oxazolyl, thiazolyl, substituted thiazolyl, oxadiazolyl, substituted oxadiazolyl, tetrazolyl, and substituted tetrazolyl; and the subscript p is an integer of from 1 to 3 and each R⁷ is independently selected from the group consisting of halo, C₁₋₁₀alkyl, C₁₋₁₀haloalkyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -S(O)ₘR^{a}, -NR^{a}S(O)ₘR^{b}, -SO₂NR^{a}R^{b}, a 4- to 7-membered heterocyclo group, aryl and a 5- to 10-membered heteroaryl group, wherein each of said heterocyclo groups, said aryl and heteroaryl groups are optionally substituted with from one to four substituents independently selected from halo, oxo, C₁₋₄ alkyl, C₁₋₄ haloalkyl, C₃₋₇ cycloalkyl, -CN, -NO₂, -OR^{a}, -NR^{a}R^{b}, -CO₂R^{a}, -CONR^{a}R^{b}, -NR^{a}COR^{b}, -NR^{a}CO₂R^{b}, -S(O)ₘR^{a}, -NR^{a}SO₂R^{b}, and -SO₂NR^{a}R^{b} and wherein the subscript m is an integer of from 0 to 2. Optionally, R¹ is substituted with from one to three substituents selected from the group consisting of C₁₋₁₀alkyl, C₁₋₁₀haloalkyl, C₃₋₇cycloalkyl, aryl, heteroaryl, -NO₂, -OR^{a}, -NR^{a}R^{b}, -CO₂R^{a}, -CONR^{a}R^{b}, -S(O)ₘR^{a}, -NR^{a}S(O)₂R^{b}, and -SO₂NR^{a}R^{b}.

A further embodiment of the compounds of the disclosure are compounds of Formula (I), wherein at least one of J, K, T, and U is N. In this embodiment, D is O, S, or NR⁸.

A preferred embodiment of Formula (I) provides compounds wherein at least one of J, K, T, and U is N and D is O.

In compounds of Formula (I) when at least one of J, K, T, and U is N and D is O, a preferred R¹ group is selected from the group consisting of -X¹-COR^{a}, -X¹-CO₂R^{a}, -X¹-CONR^{a}R^{b}, -SO₂R^{a}, aryl, heteroaryl, substituted aryl and substituted heteroaryl. When R¹ is an aromatic substituent, R¹ is preferably selected from the group consisting of pyridyl, substituted pyridyl, pyrimidinyl, substituted pyrimidinyl, pyrazinyl, substituted pyrazinyl, pyridazinyl, substituted pyridazinyl, phenyl, substituted phenyl, imidazolyl, substituted imidazolyl, triazolyl, substituted triazolyl, oxazolyl, substituted oxazolyl, thiazolyl, substituted thiazolyl, oxadiazolyl, substituted oxadiazolyl, tetrazolyl, and substituted tetrazolyl; and the subscript p is an integer of from 1 to 3 and each R⁷ is independently selected from the group consisting of halo, C₁₋₁₀alkyl, C₁₋₁₀haloalkyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -S(O)ₘR^{a}, -NR^{a}S(O)ₘR^{b}, -SO₂NR^{a}R^{b}, a 4- to 7-membered heterocyclo group, aryl and a 5-to 10-membered heteroaryl group, wherein each of said heterocyclo groups, said aryl and heteroaryl groups are optionally substituted with from one to four substituents independently selected from halo, oxo, C₁₋₄ alkyl, C₁₋₄ haloalkyl, C₃₋₇ cycloalkyl, -CN, -NO₂, -OR^{a}, -NR^{a}R^{b}, -CO₂R^{a}, -CONR^{a}R^{b}, -NR^{a}COR^{b}, -NR^{a}CO₂R^{b}, -S(O)ₘR^{a}, -NR^{a}SO₂R^{b}, and -SO₂NR^{a}R^{b} and wherein the subscript m is an integer of from 0 to 2. Optionally, R¹ is substituted with from one to three substituents selected from the group consisting of C₁₋₁₀alkyl, C₁₋₁₀haloalkyl, C₃₋₇cycloalkyl, aryl, heteroaryl, -NO₂, -OR^{a}, -NR^{a}R^{b}, -C0₂R^{a}, -CONR^{a}R^{b}, -S(O)ₘR^{a}, -NR^{a}S(O)₂R^{b}, and -SO₂NR^{a}R^{b}.

One preferred embodiment provides compounds of Formula (I) wherein when at least one of J, K, T, and U is N and D is O, and R¹ is as described in the above paragraph, the subscript p is an integer of from 1 to 3 and each R⁷ is independently selected from the group consisting of halo, C₁₋₁₀alkyl, C₁₋₁₀haloalkyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -S(O)ₘR^{a}, -NR^{a}S(O)ₘR^{b}, -SO₂NR^{a}R^{b}, a 4- to 7-membered heterocyclo group, aryl and a 5- to 10-membered heteroaryl group, wherein each of said heterocyclo groups, said aryl and heteroaryl groups are optionally substituted with from one to four substituents independently selected from halo, oxo, C₁₋₄ alkyl, C₁₋₄ haloalkyl, C₃₋₇ cycloalkyl, -CN, -NO₂, -OR^{a}, -NR^{a}R^{b}, -CO₂R^{a}, -CONR^{a}R^{b}, -NR^{a}COR^{b}, -NR^{a}CO₂R^{b}, -S(O)ₘR^{a}, -NR^{a}SO₂R^{b}, and -SO₂NR^{a}R^{b} and wherein the subscript m is an integer of from 0 to 2.

Yet another preferred compound of Formula (I) provides compounds wherein J, T, and U are all C or CH, and D is O, S, or NR⁸.

An even more preferred compound of Formula (I) provides compounds wherein J, T, and U are all C or CH, and D is O.

For compounds of Formula (I) when J, T, and U are all C or CH, and D is O, the R⁷ group is a member independently selected from the group consisting of halo, C₁₋₁₀ alkyl, C₁₋₁₀ haloalkyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -S(O)ₘR^{a}, -NR^{a}S(O)ₘR^{b}, -SO₂NR^{a}R^{b}, a 4- to 5-membered heterocyclo group, and a 5- to 6-membered heteroaryl group and wherein the subscript m is an integer of from 0 to 2. Preferred R⁷ groups are independently selected from the group consisting of halo, C₁₋₅alkyl, C₁₋₅haloalkyl, -SOR^{a}, -SO₂R^{a}, and 5-membered heteroaryl group. Even more preferred R⁷ groups are independently selected from the group consisting of fluoro, chloro, methyl, ethyl, -CF₃, -SO₂CH₃, imidazolyl, triazolyl, and tetrazolyl and wherein the subscript p is integer of from 1 to 2.

In Formula (I), when J, T, and U are all C or CH, and D is O, preferred compounds are compounds wherein the R⁷ group is a member independently selected from the group consisting of halo, C₁₋₁₀ alkyl, C₁₋₁₀ haloalkyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -S(O)ₘR^{a}, -NR^{a}S(O)ₘR^{b}, -SO₂NR^{a}R^{b}, a 4- to 5-membered heterocyclo group, and a 5- to 6-membered heteroaryl group and wherein the subscript m is an integer of from 0 to 2, and each R² is a member independently selected from the group consisting of halo, C₁₋₅alkyl, C₁₋₅haloalkyl, and the subscript q is an integer of from 0 to 2. Preferred R⁷ groups are independently selected from the group consisting of halo, C₁₋₅alkyl, C₁₋₅haloalkyl, -SOR^{a}, -SO₂R^{a}, and 5-membered heteroaryl group. Even more preferred R⁷ groups are independently selected from the group consisting of fluoro, chloro, methyl, ethyl, -CF₃, -SO₂C₁₋₃alkyl, imidazolyl, triazolyl, and tetrazolyl and wherein the subscript p is integer of from 1 to 2.

Another embodiment of the disclosure provides compounds of Formula (I) wherein when J, T, and U are all C or CH, and D is O, the R⁷ group is a member as described above, and R¹ is selected from the group consisting of -X¹-COR^{a}, -X¹CO₂R^{a}, -X¹-CONR^{a}R^{b}, -SO₂R^{a}, aryl, heteroaryl, substituted aryl and substituted heteroaryl. A preferred R¹ group is selected from the group consisting of is aryl, heteroaryl, substituted aryl and substituted heteroaryl. Even more preferred are compounds wherein R¹ is selected from the group consisting of pyridyl, substituted pyridyl, pyrimidinyl, substituted pyrimidinyl, pyrazinyl, substituted pyrazinyl, pyridazinyl, substituted pyridazinyl, phenyl, substituted phenyl, imidazolyl, triazolyl, substituted triazolyl, substituted imidazolyl, oxazolyl, substituted oxazolyl, thiazolyl, substituted thiazolyl, oxadiazolyl, substituted oxadiazolyl, tetrazolyl, and substituted tetrazolyl. Yet even more preferred are compounds wherein R¹ is selected from the group consisting of pyrimidinyl, substituted pyrimidinyl, oxadiazolyl, substituted oxadiazolyl, and -X¹-CO₂R^{a} and wherein X¹ is a bond.

Additional preferred compounds of the disclosure are compounds wherein, J, T, and U are all C or CH; and D is O, X is S, Y is C, Z is N; R¹ is selected from the group consisting of pyrimidinyl, substituted pyrimidinyl, pyridyl, and substituted pyridyl, each R⁷ is independently selected from the group consisting of fluoro and tetrazolyl.

Compounds of Formula (I) are shown in the example section herein. Preferred compounds of Formula (I) are the compounds of examples 1-210. The compounds of Formula (I) to which the invention according to the claims is directed are the compounds of examples 52, 76, 77, 95, 148, 162, 170, 171, 182, 184, 185, and 195.

In particular, a preferred compound of Formula (I) is 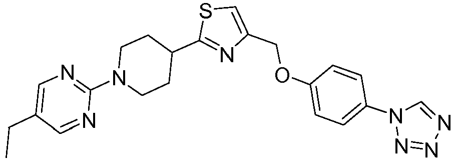 or a pharmaceutically acceptable salt thereof.

The compounds of Formula (I) are synthesized according to the procedures set forth in co-owned and co-pending applications USSN 11/964,461 and PCT/US2007/088978. One of skill in the art can readily synthesize compounds of Formula (I) as taught in these patent applications.

In one aspect, this disclosure provides method of treating a disease or condition selected from the group consisting of Type I diabetes, Type II diabetes and metabolic syndrome. The method comprises administering to a subject in need of such treatment an effective amount of a compound of Formula (I) and a DPP IV inhibitor.

This disclosure provides method of treating diabetes comprising administering to a subject in need thereof a compound of Formula (I) and a DPP IV inhibitor. Formula (I) is 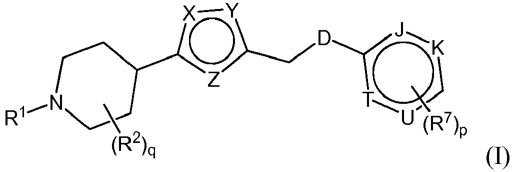 wherein,
D is selected from the group consisting of O, S, and NR⁸;
X, Y, and Z are independently selected from the group consisting of O, N, NR⁸, S, and CR³ and at least one of X, Y, and Z is O, N, NR⁸, or S;
J, K, T, and U are each independently selected from the group consisting of C, CH, and N;
the subscript p is an integer of from 0 to 4;
the subscript q is an integer of from 0 to 4;
R¹ is a member selected from the group consisting of H, C₁₋₁₀alkyl, C₁₋₁₀substituted alkyl, C₃₋₇cycloalkyl, C₂₋₁₀alkenyl, C₂₋₁₀alkynyl, -X¹-COR^{a}, -X¹-CO₂R^{a}, -X¹-CONR^{a}R^{b}, -SO₂R^{a}, a 4- to 7-membered heterocyclo group, aryl and a 5- to 10-membered heteroaryl group, wherein each of said cycloalkyl group, heterocyclo group, aryl group and heteroaryl group is optionally substituted with from 1 to 4 substituents independently selected from halo, C₁₋₁₀alkyl, C₁₋₁₀substituted alkyl, C₃₋₇cycloalkyl, C₂₋₁₀ alkenyl, C₂₋₁₀alkynyl, aryl, heteroaryl, -CN, -NR^{a}COR^{b}, -NR^{a}CONR^{a}R^{b}, -NO₂, -OR^{a}, -NR^{a}R^{b}, -COR^{a}, -CO₂R^{a}, -CONR^{a}R^{b}, -S(O)ₘR^{a}, -NR^{a}S(O)₂R^{b}, and -SO₂NR^{a}R^{b}, or optionally R^{a} and R^{b} are combined to form a 4-, 5- or 6-membered ring, and X¹ is selected from the group consisting of a bond, C₂₋₆alkene, C₂₋₆alkyne, -C(O)-, and -C(O)-(CH₂)₁₋₄-, wherein the aliphatic portions of X¹ are optionally substituted with one to three members selected from halogen, C₁₋₄alkyl, C₁₋₄substituted alkyl and C₁₋₄haloalkyl;
each R² is a member independently selected from the group consisting of halogen, C₁₋₅ alkyl, C₁₋₅substituted alkyl, C₃₋₇cycloalkyl, -COR^{a}, -CO₂R^{a}, -CONR^{a}R^{b}, -OR^{a}, -NR^{a}R^{b}, -NR^{a}COR^{b}, -SOR^{a}R^{b}, -SO₂R^{a} and -SO₂NR^{a}R^{b}, and wherein when the subscript q is 2 and R² is alkyl or substituted alkyl, the two R² members can optionally cyclize to form a ring;
R³ is a member selected from the group consisting of hydrogen, halogen, C₁₋₄alkyl, and C₁₋₄haloalkyl;
each R⁷ is independently selected from the group consisting of halo, C₁₋₁₀alkyl, C₁₋₁₀ substituted alkyl, C₃₋₇cycloalkyl, C₂₋₁₀alkenyl, C₂₋₁₀alkynyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -NR^{a}CONR^{a}R^{b}, -S(O)ₘR^{a}, -NR^{a}S(O)ₘR^{b}, -SO₂NR^{a}R^{b}, a 4- to 7-membered heterocyclo group, aryl and a 5- to 10-membered heteroaryl group, wherein each of said heterocyclo groups, said aryl and heteroaryl groups are optionally substituted with from one to four substituents independently selected from halo, oxo, C₁₋₄ alkyl, C₁₋₄ haloalkyl, C₃₋₇ cycloalkyl, -CN, -NO₂, -OR^{a}, -NR^{a}R^{b}, -COR^{a}, -CO₂R^{a}, -CONR^{a}R^{b}, -NR^{a}COR^{b}, -NR^{a}CO₂R^{b}, -NR^{a}CONR^{a}R^{b}, -S(O)ₘR^{a}, -NR^{a}SO₂R^{b}, and -SO₂NR^{a}R^{b} and wherein the subscript m is an integer of from 0 to 2, or optionally R^{a} and R^{b} are combined to form a 4-, 5- or 6-membered ring;
R⁸ is a member independently selected from the group consisting of hydrogen, C₁₋₄alkyl, and C₁₋₄haloalkyl;
and each R^{a} and R^{b} is independently selected from the group consisting of hydrogen, C₁₋₁₀ alkyl, C₁₋₁₀haloalkyl, C₃₋₁₀cycloalkyl, heterocyclyl, C₂₋₁₀alkenyl, C₂₋₁₀alkynyl, aryl, 5- to 6-membered heteroaryl and arylC₁₋₄alkyl; and wherein the aliphatic portions of each of said R^{a} and R^{b} is optionally substituted with from one to three members selected from the group consisting of halo, -ORⁿ, -OCORⁿ, -OC(O)N(Rⁿ)₂, -SRⁿ, -S(O)Rⁿ, -S(O)₂Rⁿ, -S(O)₂N(Rⁿ)₂, -NRⁿS(O)₂Rⁿ, -C(O)N(Rⁿ)₂, -C(O)Rⁿ, -NRⁿC(O)Rⁿ, -NRⁿC(O)N(Rⁿ)₂, -CO₂Rⁿ, -NRⁿCO₂Rⁿ, -CN, -NO₂, -N(Rⁿ)₂ and -NRⁿS(O)₂N(Rⁿ)₂, wherein each Rⁿ is independently hydrogen or an unsubstituted C₁₋₆ alkyl;
and wherein the aryl and heteroaryl portions are optionally substituted with from one to three members selected from halogen, -OR^{m}, -OC(O)N(R^{m})₂, -SR^{m}, -S(O)_{R}^{m}, -S(O)₂R^{m}, -S(O)₂N(R^{m})₂, -NR^{m}S(O)₂R^{m}, -C(O)N(R^{m})₂, -C(O)R^{m}, -NR^{m}C(O)R^{m}, -NR^{m}C(O)N(R^{m})₂, -CO₂R^{m}, -NR^{m}CO₂R^{m}, -CN, -NO₂, -N(R^{m})₂ and -NR^{m}S(O)₂N(R^{m})₂, wherein each R^{m} is independently hydrogen or an unsubstituted C₁₋₆ alkyl; or a pharmaceutically acceptable salt or ester thereof; and wherein the molecular weight of said compound is less than 1200.

Preferred compounds of Formula (I) are the compounds of examples 1-210. The compounds of Formula (I) to which the invention according to the claims is directed are the compounds of examples 52, 76, 77, 95, 148, 162, 170, 171, 182, 184, 185, and 195.

A more preferred compound is 5-Ethyl-2-{4-[4-(4-tetrazol-1-yl-phenoxymethyl)-thiazol-2-yl]-piperidin-1-yl}-pyrimidine or a pharmaceutically acceptable salt thereof. The structure of the compound is shown below.

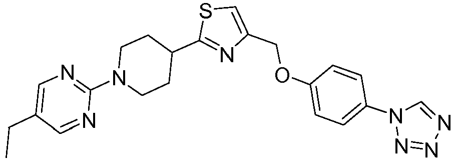

In one aspect, this disclosure provides a method of treating diabetes comprising administering a compound of Formula (I) and a DPP IV inhibitor.

The DPP IV inhibitors useful in the present invention are sitagliptin (Merck), vildagliptin (Novartis), BMS-477118 (saxagliptin) (Bristol-Myers Squibb), R1438 (amino-methylpyridine) (Roche), NVP DPP728 (Novartis), PSN9301 (Prosidion), P32/98 (isoleucine thiozolidide) (Probiodrug), GSK823093C (Denagliptin) (Glaxo Smithkline), SYR-322 (Alogliptin) (Takeda), NN-7201 (NovoNordisk), ALS2-0426 (Alantos). (Green BD, Flatt PR, Bailey CJ, Dipeptidyl peptidase IB (DPP IV) inhibitors: a newly emerging drug class for the treatment of Type II diabetes, Diabetes Vasc Dis Res 2006, 3:159-165) Preferred DPP IV inhibitors are sitagliptin, vildagliptin, Denagliptin, saxagliptin, and alogliptin). Even more preferred CPP4 inhibitors are sitagliptin and vildagliptin.

The compound of Formula (I) and DPP IV inhibitor are administered in a single dosage or in separate dosages. The single dosage is administered once a day or multiple times a day. When the compound of Formula (I) and DPP IV inhibitor are administered is separate dosages, the dosages can be administered once a day or multiple times a day.

In one embodiment, when the compound of Formula (I) and the DPP IV inhibitor are administered in a single dosage, the compound of Formula (I) and DPP IV inhibitor are formulated as a medicament into a single pill, single table, or a single capsule. When the compound of Formula (I) and DPP IV inhibitor are administered in separate dosages, the compound of Formula (I) is formulated as a medicament into a pill, tablet or capsule and the DPP IV inhibitor is formulated into a separate pill or capsule.

When the compound of Formula (I) and DPP IV inhibitor are administered in separate dosages, the compound of Formula (I) can be administered first and the DPP IV inhibitor can be administered next, following administration of the compound of Formula (I). Alternatively, the DPP IV inhibitor can be administered first and the compound of Formula (I) can be administered next, following administration of the DPP IV inhibitor. The time between the sequential first administration and the second administration can be varied by a skilled practitioner. In one embodiment, the first administration (a compound of Formula (I) or DPP IV inhibitor), is followed immediately by the second administration (a compound of Formula (I) or DPP IV inhibitor). In another embodiment, the second administration is within 2 minutes, 5 minutes, 10 minutes, 15 minutes, 30 minutes, or 60 minutes, 1 hour, 2 hours, 3 hours, 4 hours, 5 hours, 6 hours, 7 hours, 8 hours, 9 hours, 10 hours, 11 hours, or 12 hours following the first administration. Yet another embodiment provides for the administration to a patient a compound for Formula (I) and/or DPP IV inhibitor in the morning followed by administration to the previously treated patient a compound of Formula (I) and/or DPP IV inhibitor in the evening.

Another aspect of this disclosure provides methods of lowering blood levels of glucose in a subject by administering a compound of Formula (I) and a DPP IV inhibitor. The method comprises administering an effective amount of a compound of Formula (I) and DPP IV inhibitor to the mammal. The method further comprises steps to measure blood glucose levels before and after administration of a compound of Formula (I) and DPP IV inhibitor. Blood glucose levels are easily measured by numerous commercially available glucose monitoring devices that measure blood glucose from samples of blood or urine, or as taught herein. Blood glucose can also be measured by commercially available glucometers that do not require blood or urine samples.

Another aspect of this disclosure provides methods of lowering blood levels of insulin in a subject by administering a compound of Formula (I) and a DPP IV inhibitor. The method comprises administering an effective amount of a compound of Formula (I) and DPP IV inhibitor to the mammal. The method further comprises steps to measure blood insulin levels before and after administration of a compound of Formula (I) and a DPP IV inhibitor. Blood insulin levels are easily measured by well-known insulin monitoring assays that measure insulin from samples of blood or urine, or as taught herein.

In another aspect, this disclosure provides methods of increasing blood levels of incretins in a subject by administering a compound of Formula (I) and a DPP IV inhibitor. The incretins are GLP-1 and GIP. The method comprises administering an effective amount of a compound of Formula (I) and DPP IV inhibitor to the mammal. The method further comprises steps to measure blood incretin levels before and after administration of a compound of Formula (I) and a DPP IV inhibitor. Blood incretin levels are easily measured by well-known incretin monitoring assays that, or as taught herein.

Yet another aspect of this disclosure provides methods of lowering blood triglyceride levels in a subject by administering a compound of Formula (I) and a DPP IV inhibitor. The method comprises administering an effective amount of a compound of Formula (I) and DPP IV inhibitor to the mammal. The method further comprises steps to measure blood triglycerides levels before and after administration of a compound of Formula (I) and DPP IV inhibitor. Blood triglyceride levels are easily measured by numerous commercially available devices that measure blood triglyceride levels from samples of blood.

A further aspect of this disclosure provides methods of lowing gastric emptying in a subject by administering a compound of Formula (I) and a DPP IV inhibitor. The method comprises administering an effective amount of a compound of Formula (I) and DPP IV inhibitor to the mammal. The method further comprises steps to measure blood incretin levels before and after administration of a compound of Formula (I) and a DPP IV inhibitor. Blood incretin levels are easily measured by well-known incretin monitoring assays, or as taught herein.

Another aspect of this disclosure provides methods of increasing insulin production in the islet cells of a subject by administering a compound of Formula (I) and a DPP IV inhibitor. The method comprises administering an effective amount of a compound of Formula (I) and DPP IV inhibitor to the mammal. The method further comprises steps to measure insulin production in islet cells or the beta cells of the pancreas before and after administration of a compound of Formula (I) and a DPP IV inhibitor. The insulin production of islets and beta cells are easily measured by well-known assays, or as taught herein.

In yet another aspect, this disclosure provides methods of preserving islet function in a subject by administering a compound of Formula (I) and a DPP IV inhibitor. The method comprises administering an effective amount of a compound of Formula (I) and DPP IV inhibitor to the mammal. The method further comprises steps to measure the function of islets' or beta cell's ability to produce insulin before and after administration of a compound of Formula (I) and a DPP IV inhibitor. The insulin production of islets and beta cells are easily measured by well-known assays, or as taught herein.

### Compositions and Methods of Treatment

In accordance with the present disclosure a therapeutically effective amount of a compound of Formula (I) and DPP IV inhibitor can be used for the preparation of one or more pharmaceutical compositions useful for treating Type II diabetes and/or lowering the plasma level of glucose. In addition, a therapeutically effective amount of a compound of Formula (I) and a DPP IV inhibitor can be used for the preparation of one or more pharmaceutical compositions useful for treating other indications that include diabetes as a component, such as metabolic syndrome, as well as indications that can be improved as a result of increased insulin production (such as the early stages of Type I diabetes).

The compositions of the disclosure can include compounds of Formula (I), and DPP IV inhibitors, pharmaceutically acceptable salts thereof, or a hydrolysable precursor thereof. In general, the compound is mixed with suitable carriers or excipient(s) in a therapeutically effective amount. By a "therapeutically effective dose", "therapeutically effective amount", or, interchangeably, "pharmacologically acceptable dose" or "pharmacologically acceptable amount", it is meant that a sufficient amount of the compound of the present invention and a pharmaceutically acceptable carrier will be present in order to achieve a desired result, *e.g.,* alleviating a symptom or complication of Type II diabetes.

The compounds of Formula (I) and DPP IV inhibitors that are used in the methods of the present disclosure can be incorporated into a variety of formulations for therapeutic administration. More particularly, the compounds of Formula (I) and DPP IV inhibitors can be formulated into pharmaceutical compositions by combination with appropriate, pharmaceutically acceptable carriers or diluents, and can be formulated into preparations in solid, semi-solid, liquid or gaseous forms, such as tablets, capsules, pills, powders, granules, dragees, gels, slurries, ointments, solutions, suppositories, injections, inhalants and aerosols. The compounds of Formula (I) and DPP IV inhibitors can be formulated into a single composition containing a compound or Formula (I) and DPP IV inhibitor. Alternatively, the compound of Formula (I) and DPP IV inhibitor can be formulated into separate pharmaceutical formulations and manufactured into a single pill, tablet or capsule that physically separates the compound of Formula (I) and DPP IV inhibitor. The administration of the compounds can be achieved in various ways, including oral, buccal, rectal, parenteral, intraperitoneal, intradermal, transdermal, and/or intratracheal administration. Moreover, the compound can be administered in a local rather than systemic manner, in a depot or sustained release formulation. In addition, the compounds can be administered in a liposome.

DPP IV inhibitors are commercially available. In particular, sitagliptin is an approved pharmaceutical marketed as Januvia^{™}, and vildagliptin is an approved pharmaceutical marked as Galvus^{™}.

The compounds of Formula (I) and DPP IV inhibitors can be formulated with common excipients, diluents or carriers, and compressed into tablets, or formulated as elixirs or solutions for convenient oral administration, or administered by the intramuscular or intravenous routes. The compounds can be administered transdermally, and can be formulated as sustained release dosage forms and the like.

Suitable formulations for use in the present invention are found in Remington's Pharmaceutical Sciences (Mack Publishing Company (1985) Philadelphia, PA, 17th ed.), which is incorporated herein by reference. Moreover, for a brief review of methods for drug delivery, *see,* Langer, Science (1990) 249:1527-1533. The pharmaceutical compositions described herein can be manufactured in a manner that is known to those of skill in the art, *i.e.,* by means of conventional mixing, dissolving, granulating, dragee-making, levigating, emulsifying, encapsulating, entrapping or lyophilizing processes. The following methods and excipients are merely exemplary and are in no way limiting.

For injection, the compound of Formula (I) and DPP IV inhibitor can be formulated into preparations by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives. Preferably, the compounds of the present invention can be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art.

For oral administration, the compounds of Formula (I) and DPP IV inhibitors can be formulated readily by combining with pharmaceutically acceptable carriers that are well known in the art. Such carriers enable the compounds to be formulated as tablets, pills, dragees, capsules, emulsions, lipophilic and hydrophilic suspensions, liquids, gels, syrups, slurries, suspensions and the like, for oral ingestion by a patient to be treated. Pharmaceutical preparations for oral use can be obtained by mixing the compounds with a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone. If desired, disintegrating agents can be added, such as the crosslinked polyvinyl pyrrolidone, agar, or alginic acid or a salt thereof such as sodium alginate.

Dragee cores are provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations that can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration.

For buccal administration, the compositions can take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, *e.g.*, dichlorodifluoromethane, trichlorofluoromethane, dichlorotetrafluoroethane, carbon dioxide or other suitable gas, or from propellant-free, dry-powder inhalers. In the case of a pressurized aerosol the dosage unit can be determined by providing a valve to deliver a metered amount. Capsules and cartridges of, *e.g.,* gelatin for use in an inhaler or insufflator can be formulated containing a powder mix of the compound and a suitable powder base such as lactose or starch.

The compounds can be formulated for parenteral administration by injection, *e.g.*, by bolus injection or continuous infusion. Formulations for injection can be presented in unit dosage form, *e.g.,* in ampoules or in multidose containers, with an added preservative. The compositions can take such forms as suspensions, solutions or emulsions in oily or aqueous vehicles, and can contain formulator agents such as suspending, stabilizing and/or dispersing agents.

Pharmaceutical formulations for parenteral administration include aqueous solutions of the active compounds in water-soluble form. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions can contain substances that increase the viscosity of the suspension, such as sodium carboxymethyl cellulose, sorbitol, or dextran. Optionally, the suspension can also contain suitable stabilizers or agents that increase the solubility of the compounds to allow for the preparation of highly concentrated solutions. Alternatively, the active ingredient can be in powder form for constitution with a suitable vehicle, *e.g.*, sterile pyrogen-free water, before use.

The compounds can also be formulated in rectal compositions such as suppositories or retention enemas, *e.g.*, containing conventional suppository bases such as cocoa butter, carbowaxes, polyethylene glycols or other glycerides, all of which melt at body temperature, yet are solidified at room temperature.

In addition to the formulations described previously, the compounds can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

Alternatively, other delivery systems for hydrophobic pharmaceutical compounds can be employed. Liposomes and emulsions are well known examples of delivery vehicles or carriers for hydrophobic drugs. In a presently preferred embodiment, long-circulating, *i.e.,* stealth liposomes can be employed. Such liposomes are generally described in Woodle, et al., U.S. Patent No. 5,013,556. The compounds of the present invention can also be administered by controlled release means and/or delivery devices such as those described in U.S. Patent Nos. 3,845,770; 3,916,899; 3,536,809; 3,598,123; and 4,008,719.

Certain organic solvents such as dimethylsulfoxide ("DMSO") also can be employed, although usually at the cost of greater toxicity. Additionally, the compounds can be delivered using a sustained-release system, such as semipermeable matrices of solid hydrophobic polymers containing the therapeutic agent. Various types of sustained-release materials have been established and are well known by those skilled in the art. Sustained-release capsules can, depending on their chemical nature, release the compounds for a few hours up to over 100 days.

The pharmaceutical compositions also can comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Pharmaceutical compositions suitable for use in the present invention include compositions wherein the active ingredients are contained in a therapeutically effective amount. The amount of composition administered will, of course, be dependent on the subject being treated, on the subject's weight, the severity of the affliction, the manner of administration and the judgment of the prescribing physician. Determination of an effective amount is well within the capability of those skilled in the art, especially in light of the detailed disclosure provided herein.

For any compound used in the method of the present disclosure a therapeutically effective dose can be estimated initially from cell culture assays, animal models, or microdosing of human subjects.

Moreover, toxicity and therapeutic efficacy of the compounds described herein can be determined by standard pharmaceutical procedures in cell cultures or experimental animals, *e.g.*, by determining the LD₅₀, (the dose lethal to 50% of the population) and the ED₅₀ (the dose therapeutically effective in 50% of the population). The dose ratio between toxic and therapeutic effect is the therapeutic index and can be expressed as the ratio between LD₅₀ and ED₅₀. Compounds that exhibit high therapeutic indices are preferred. The data obtained from these cell culture assays and animal studies can be used in formulating a dosage range that is not toxic for use in humans. The dosage of such compounds lies preferably within a range of circulating concentrations that include the ED₅₀ with little or no toxicity. The dosage can vary within this range depending upon the dosage form employed and the route of administration utilized. The exact formulation, route of administration and dosage can be chosen by the individual physician in view of the patient's condition. (*see, e.g.,* Fingl, et al., 1975 In: The Pharmacological Basis of Therapeutics, Ch. 1).

The amount of active compound that can be combined with a carrier material to produce a single dosage form will vary depending upon the disease treated, the mammalian species, and the particular mode of administration. However, as a general guide, suitable unit doses for the compounds of the present invention can, for example, preferably contain between 0.1 mg to about 1000 mg of the active compound. A preferred unit dose is between 1 mg to about 500 mg. A more preferred unit dose is between 1 mg to about 300mg. Even more preferred unit dose is between 1 mg to about 100 mg. Such unit doses can be administered more than once a day, for example 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area.

A typical dosage can be one 1 mg to about 100 mg tablet or 1 mg to about 300 mg taken once a day, or, multiple times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treating physician will know how and when to start, interrupt, adjust, or terminate therapy in conjunction with individual patient response.

The dosing of a compound of Formula (I) and DPP IV inhibitor can be dosed at the same time, within several minutes, or separated by hours. By way of example, a compound of Formula (I) and DPP IV inhibitor can be dosed together in the morning, with no further dosing for the remainder of the day. Alternatively, in the morning, a compound of Formula (I) and a DPP IV inhibitor is dosed followed with a second dose of a compound of Formula (I) and/or a DPP IV inhibitor in the evening or after a meal.

It can be necessary to administer dosages of the compound of Formula (I) and/or DPP IV inhibitor once a day or more than once a day, or before or after a meal, as will be apparent to those skilled in the art. Further, it is noted that the clinician or treating physician will know how and when to start, interrupt, adjust, or terminate therapy in conjunction with individual patient response.

In addition, the present disclosure provides for kits with unit doses of the compounds of Formula (I) and/or DPP IV inhibitor, either in oral or injectable doses. In addition to the containers containing the unit doses will be an informational package insert describing the use and attendant benefits of the drugs in treating Type II diabetes, obesity, hyperlipidemia, atherosclerosis and metabolic syndrome, and/or their respective related symptoms, complications and disorders. Preferred compounds and unit doses are those described herein above.

For the compositions, methods and kits provided above, one of skill in the art will understand that preferred compounds for use in each are those compounds that are noted as preferred above.

### EXAMPLES

**General Methods:** All operations involving moisture and/or oxygen sensitive materials were conducted under an atmosphere of dry nitrogen in pre-dried glassware. Unless noted otherwise, materials were obtained from commercially available sources and used without further purification.

Flash chromatography was performed on E. Merck silica gel 60 (240-400 mesh) according to the protocol of Still, Kahn, and Mitra (*J. Org*. *Chem.* (1978) 43, 2923). Thin layer chromatography was performed using precoated plates purchased from E. Merck (silica gel 60 PF₂₅₄, 0.25 mm) and spots were visualized with ultraviolet light followed by an appropriate staining reagent.

Nuclear magnetic resonance ("NMR") spectra were recorded on a Varian Inova-400 resonance spectrometer. ¹H NMR chemical shifts are given in parts per million (δ) downfield from tetramethylsilane ("TMS") using TMS or the residual solvent signal (CHCl₃=δ 7.24, DMSO = δ 2.50) as internal standard. ¹H NMR information is tabulated in the following format: number of protons, multiplicity (s, singlet; d, doublet; t, triplet; q, quartet; m, multiplet), coupling contant(s) (J) in Hertz, and, in selected cases, position assignment. The prefix app is occasionally applied in cases where the true signal multiplicity was unresolved and br indicates the signal in question was broadened.

### Preparation of Intermediate 1: 4-(4-Chloromethyl-thiazol-2-yl)-piperidine-1-carboxylic acid tert-butyl ester

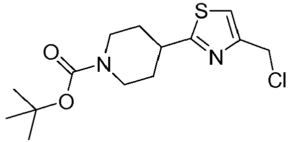

To a solution of 4-thiocarbamoyl-piperidine-1-carboxylic acid *tert*-butyl ester (4.9 g, 20 mmol) in acetone (80 mL) was added 1,3-dichloroacetone (3.3 g, 26 mmol), MgSO₄ (3.6 g, 30 mmol) and MgCO₃ (1.68 g, 20 mmol). The mixture was heated under reflux overnight, cooled and filtered through celite. The solvent was removed *in vacuo* and the residue was redissolved with EtOAc (150 mL). The resulting solution was washed successively with 5% NaHSO₃, saturated NaHCO₃, and brine. After drying (Na₂SO₄), the solvent was removed to afford the desired product. ¹H NMR (CDCl₃): δ 7.20 (1H, s), 4.67 (2H, s), 4.20 (2H, br), 3.16 (1H, m), 2.87 (2H, m), 2.09 (2H, m), 1.72 (2H, m), 1.47 (9H, s).

### Preparation of Intermediate 2: 2-[4-(4-Chloromethyl-thiazol-2-yl)-piperidin-1-yl]-5-ethyl-pyrimidine

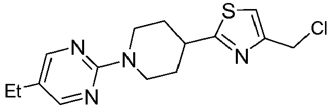

**Intermediate 2** was prepared in a manner analogous to **Intermediate 1** above.

¹H NMR (DMSO-*d₆*): δ 8.45 (2H, d), 7.62 (1H, s), 4.79 (2H, s), 4.61 (2H, m), 3.41 (1H, m), 3.24 (2H, m), 2.52 (2H, q), 2.15 (2H, m), 1.66 (2H, m), 1.17 (3H, m).

### Preparation of Intermediate 3: 4-[4-(4-Methanesulfonyl-phenoxymethyl)-thiazol-2-yl]-piperidine

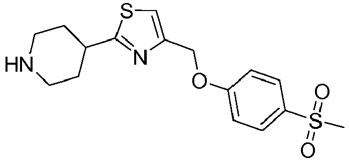

A solution of 4-[4-(4-Methanesulfonyl-phenoxymethyl)-thiazol-2-yl]-piperidine-1-carboxylic acid *tert*-butyl ester (615 mg, 1.36 mmol) in methanol (10 mL) was treated with 10 mL of 4N HCl in dioxane. The resulting solution was stirred at room temperature for 30 minutes. Then all the solvents were removed *in vacuo* to afford the desired product as a HCl salt.

### Preparation of Intermediate 4: 4-[4-(4-Tetrazol-1-yl-phenoxymethyl)-thiazol-2-yl]-piperidine

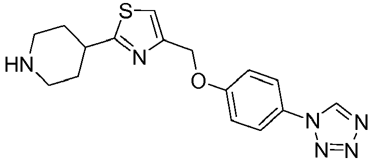

**Intermediate 4** was prepared in a manner anaolgous to **Intermediate 3** above.

¹H NMR (DMSO-d₆): δ 9.98 (1H, s), 7.82 (2H, m), 7.63 (1H, s), 7.28 (2H, m), 5.19 (2H, s), 3.01 (3H, m), 2.54 (3H, m), 1.92 (2H, m), 1.54 (2H, m).

### Preparation of Intermediate 5: 4-[4-(2-Fluoro-4-methanesulfonyl-phenoxymethyl)-thiazol-2-yl]-piperidine

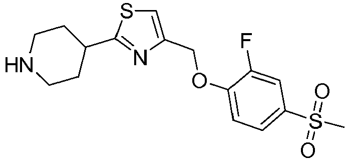

**Intermediate 5** was prepared in a manner analogous to **Intermediate 3** above.

### Preparation of Intermediate 6: 4-[4-(2-Fluoro-4-tetrazol-1-yl-phenoxymethyl)-thiazol-2-yl]-piperidine

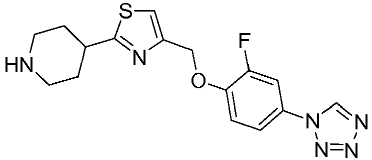

**Intermediate 6** was prepared in a manner analogous to **Intermediate 3** above.

### Preparation of Intermediate 7: 4-[4-(3-Fluoro-4-tetrazol-1-yl-phenoxymethyl)-thiazol-2-yl]-piperidine

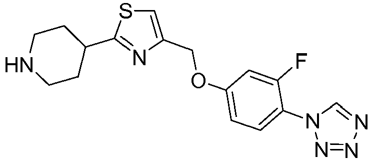

**Intermediate 7** was prepared in a manner analogous to **Intermediate 3** above.

### Preparation of Intermediate 8: 4-[4-(2,6-Difluoro-4-tetrazol-1-yl-phenoxymethyl)-thiazol-2-yl]-piperidine

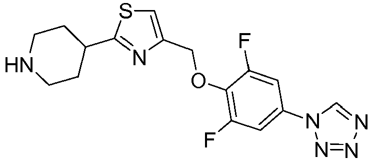

**Intermediate 8** was prepared in a manner analogous to **Intermediate 3** above.

### Preparation of Intermediate 9: 4-[4-(4-Pyrrol-1-yl-phenoxymethyl)-thiazol-2-yl]-piperidine

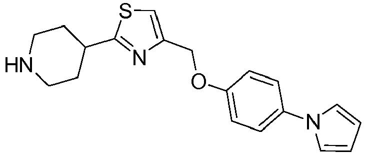

**Intermediate 9** was prepared in a manner analogous to **Intermediate 3** above.

### Preparation of Intermediate 10: (2-Piperidin-4-yl-thiazol-4-ylmethyl)-(4-tetrazol-1-yl-phenyl)-amine

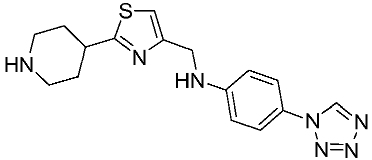

**Intermediate 10** was prepared in a manner analogous to **Intermediate 3** above.

### Preparation of Intermediate 11: 4-[4-(2-Methyl-4-tetrazol-1-yl-phenoxymethyl)-thiazol-2-yl]-piperidine

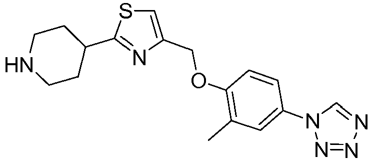

**Intermediate 11** was prepared in a manner analogous to **Intermediate 3** above.

### Preparation of Intermediate 12: 4-[4-(2-Isopropyl-5-methyl-4-tetrazol-1-yl-phenoxymethyl)-thiazol-2-yl]-piperidine

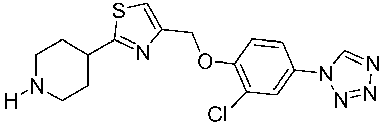

**Intermediate 12** was prepared in a manner analogous to **Intermediate 3** above.

### Preparation of Intermediate 13: 4-[4-(2-Chloro-4-tetrazol-1-yl-phenoxymethyl)-thiazol-2-yl]-piperidine

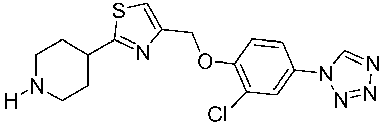

**Intermediate 13** was prepared in a manner analogous to **Intermediate 3** above.

### Preparation of Intermediate 14: 4-(4-Chloromethyl-oxazol-2-yl)-piperidine-1-carboxylic acid tert-butyl ester

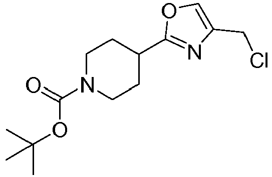

A mixture of 4-(4-Hydroxymethyl-wxazol-2-yl)-piperidine-1-carboxylic acid tert-butyl ester (800 mg, 2.84 mmol) (obtained by the reduction of 4-(4-Ethoxycarbonyl-oxazol-2-yl)-piperidine-1-carboxylic acid tert-butyl ester which was synthesized according to U.S. Patent Publication No. 2006/0135501 A1), TsCl (812 mg, 4.26 mmol) and triethylamine (1mL, 752 mg, 7.44 mmol) in dichloromethane (20 mL) was stirred at room temperature for 5 hours. The resulting solution was washed successively with 5% NaHSO₃, saturated NaHCO₃, and brine. After drying (Na₂SO₄), the solvent was removed to afford the desired product. ¹H NMR (CDCl₃): δ 7.53 (s, 1H), 4.40 (s, 2H), 4.06 (m, 2H), 2.89 (m, 3H), 1.98 (m, 2H), 1.74 (m, 2H), 1.41 (s, 9H).

### Preparation of Intermediate 15: 4-[4-(4-Tetrazol-1-yl-phenoxymethyl)-oxazol-2-yl]-piperidine

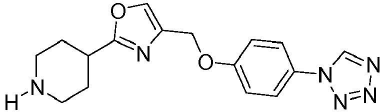

**Intermediate 15** was prepared in a manner analogous to **Intermediate 3** above.

### Preparation of Intermediate 16: 4-[4-(2-Fluoro-4-tetrazol-1-yl-phenoxymethyl)-oxazol-2-yl]-piperidine

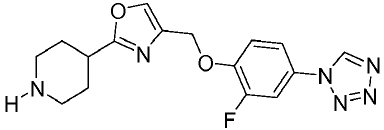

**Intermediate 16** was prepared in a manner analogous to **Intermediate 3** above.

### Preparation of Intermediate 17: 5-(2-Piperidin-4-yl-thiazol-4-ylmethoxy)-2-tetrazol-1-yl-pyridine

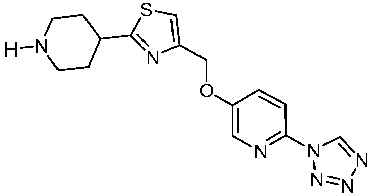

**Intermediate 17** was prepared in a manner analogous to **Intermediate 3** above.

### Preparation of Intermediate 18: (6-Fluoro-pyridin-3-yl)-(2-piperidin-4-yl-thiazol-4-ylmethyl)-amine

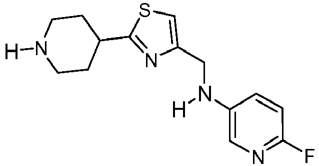

**Intermediate 18** was prepared in a manner analogous to **Intermediate 3** above.

### Preparation of Intermediate 19: 4-[4-(2, 6-Difluoro-4-methanesulfonyl-phenoxymethyl)-thiazol-2-yl]-piperidine

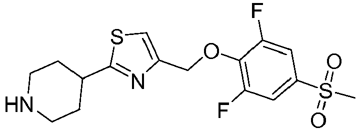

**Intermediate 19** was prepared in a manner analogous to **Intermediate 3** above.

### Preparation of Intermediate 20: 4-[4-(2-Piperidin-4-yl-thiazol-4-ylmethoxy)-phenyl]-morpholine

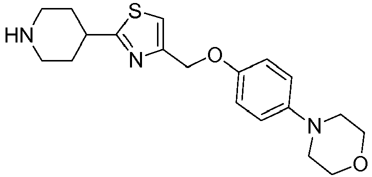

**Intermediate 20** was prepared in a manner analogous to **Intermediate 3** above.

### Preparation of Intermediate 21: 4-[4-(2-Piperidin-4-yl-thiazol-4-ylmethoxy)-phenyl]-morpholine

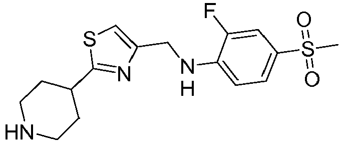

**Intermediate 21** was prepared in a manner analogous to **Intermediate 3** above.

### Preparation of Intermediate 22: 4-(4-Chloromethyl-thiazol-2-yl)-3-methyl-piperidine-1-carboxylic acid tert-butyl ester

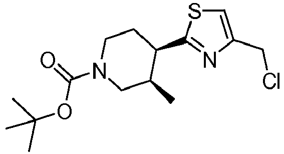

**Intermediate 22** was prepared in a manner analogous to **Intermediate 1** above.

### Preparation of Intermediate 23: 3-Methyl-4-[4-(4-tetrazol-1-yl-phenoxymethyl)-thiazol-2-yl]-piperidine

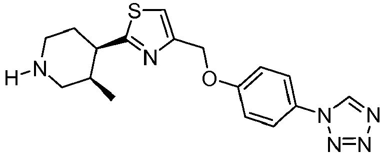

**Intermediate 23** was prepared in a manner analogous to **Intermediate 3** above.

### Preparation of Intermediate 24: 4-[4-(2-Fluoro-4-tetrazol-1-yl-phenoxymethyl)-thiazol-2-yl]-3-methyl-piperidine

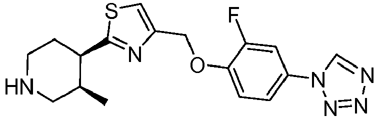

**Intermediate 24** was prepared in a manner analogous to **Intermediate 3** above.

### Preparation of Intermediate 25: 4-[4-(4-Methanesulfonyl-benzyloxymethyl)-thiazol-2-yl]-piperidine

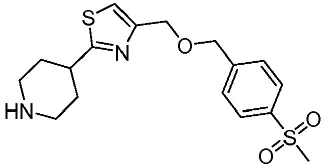

**Intermediate 25** was prepared in a manner analogous to **Intermediate 3** above.

### Example 1

### 4-[4-(4-Methanesulfonyl-phenoxymethyl)-thiazol-2-yl]-piperidine-1-carboxylic acid tert-butyl ester

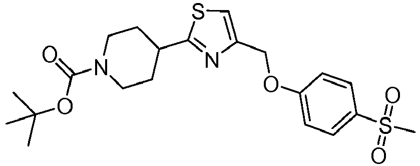

A mixture of 4-(4-Chloromethyl-thiazol-2-yl)-piperidine-1-carboxylic acid *tert*-butyl ester (**Intermediate 1,** 463 mg, 1.46 mmol), 4-methanesulfonyl-phenol (252 mg, 1.46 mmol) and K₂CO₃ (404 mg, 2.92 mmol) in acetone (25 mL) was heated under reflux overnight. After cooling, the solid was filtered through a pad of celite. The filtrate was concentrated *in vacuo*. The residue was purified on silica gel (EtOAc-hexanes, 1:1) to afford the desired product. ¹H NMR (CDCl₃): δ 7.88 (2H, d, *J* = 8.8 Hz), 7.23 (1H, s), 7.12 (2H, d, *J* = 8.8 Hz), 5.24 (2H, s), 4.21 (2H, br), 3.17 (1H, m), 3.04 (3H, s), 2.88 (2H, m), 2.11 (2H, m), 1.73 (2H, m), 1.47 (9H, s).

The compounds in **Examples 2-19** were synthesized from 4-(4-Chloromethyl-thiazol-2-yl)-piperidine-1-carboxylic acid *tert*-butyl ester **(Intermediate 1),** 2-[4-(4-Chloromethyl-thiazol-2-yl)-piperidin-1-yl]-5-ethyl-pyrimidine **(Intermediate 2),** 4-(4-Chloromethyl-oxazol-2-yl)-piperidine-1-carboxylic acid tert-butyl ester **(Intermediate 14)** or with the corresponding phenol, thiophenol, amine or aniline in a similar manner to that described in **Example 1.** One skilled in the art of organic synthesis will appreciate that conditions such as solvent (*e.g.,* DMF, CH₃CN); temperature, base (*e.g.,* NEt₃, K₂CO₃, NaHCO₃, Na₂CO₃, Cs₂CO₃) and concentration can be selected through routine experimentation to optimize yields. Additionally, alternative coupling methods can be used that are well known in the art of organic synthesis.

### Example 2

### 4-[4-(4-Imidazol-1-yl-phenoxymethyl)-thiazol-2-yl]-piperidine-1-carboxylic acid tert-butyl ester

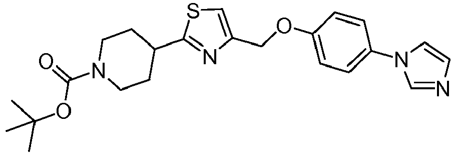

¹H NMR (DMSO-*d₆*): δ 8.12 (1H, s), 7.63 (2H, m), 7.54 (2H, d, *J*= 9.2 Hz), 7.15 (2H, d, *J* = 9.2 Hz), 7.05 (1H, s), 5.15 (2H, s), 3.98 (2H, m), 3.21 (1H, m), 2.87 (2H, m), 2.01 (2H, m), 1.52 (2H, m), 1.39 (9H, s).

### Example 3

### 4-[4-(4-Acetylamino-phenoxymethyl)-thiazol-2-yl]-piperidine-1-carboxylic acid tert-butyl ester

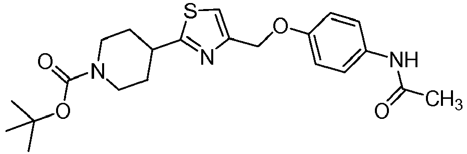

¹H NMR (DMSO-*d₆*): δ 9.77 (1H, s), 7.57 (1H, s), 7.45 (2H, d, *J* = 9.0 Hz), 6.94 (2H, d, *J*= 9.0 Hz), 5.04 (2H, s), 3.98 (2H, m), 3.18 (1H, m), 2.82 (2H, m), 2.02 (2H, m), 1.99 (3H, s), 1.51 (2H, m), 1.39 (9H, s).

### Example 4

### 4-[4-(4-Methoxy-benzenesulfonyloxymethyl)-thiazol-2-yl]-piperidine-1-carboxylic acid tert-butyl ester

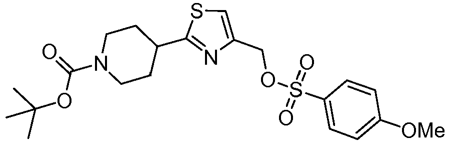

¹H NMR (CDCl₃): δ 7.60 (2H, d, *J* = 9.0 Hz), 7.24 (1H, s), 6.91 (2H, d, *J* = 9.0 Hz), 4.50 (2H, s), 4.10 (2H, m), 3.85 (3H, s), 2.99 (1H, m), 2.82 (2H, m), 1.89~1.92 (2H, m), 1.53~1.57 (2H, m), 1.46 (9H, s).

### Example 5

### 4-[4-(4-[1,2,4]Triazol-1-yl-phenoxymethyl)-thiazol-2-yl]-piperidine-1-carboxylic acid tert-butyl ester

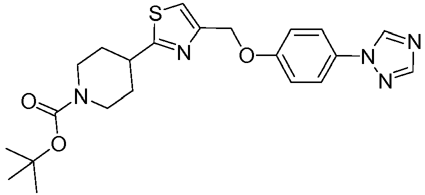

¹H NMR (CDCl₃): δ 8.47 (1H, s), 8.08 (1H, s), 7.58 (2H, d, *J* = 9.2 Hz), 7.24 (1H, s), 7.11 (2H, d, *J* = 9.2 Hz), 5.21 (2H, s), 4.2 (2H, m), 3.18 (1H, m), 2.88 (2H, m), 2.11 (2H, m), 1.74 (2H, m), 1.47 (9H, s).

### Example 6

### 4-{4-[4-(2-Oxo-pyrrolidin-1-yl)-phenoxymethyl]-thiazol-2-yl}-piperidine-1-carboxylic acid tert-butyl ester

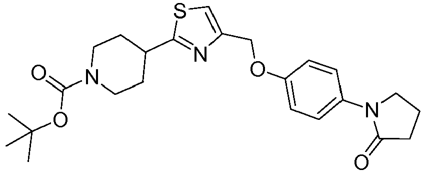

¹H NMR (CDCl₃): δ 7.50 (2H, d), 7.20 (1H, s), 6.98 (2H, d), 5.17 (2H, s), 4.20 (2H, br), 3.81(2H, m), 3.18 (1H, m), 2.88 (2H, m), 2.59 (2H, m), 2.16 (4H, m), 1.73(2H, m), 1.46 (9H, s).

### Example 7

### 4-[4-(4-Tetrazol-1-yl-phenoxymethyl)-thiazol-2-yl]-piperidine-1-carboxylic acid tert-butyl ester

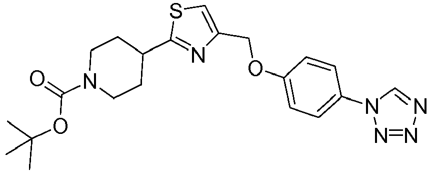

¹H NMR (CDCl₃): δ 8.94 (1H, s), 7.61 (2H, d), 7.25 (1H, s), 7.19 (2H, d), 5.21 (2H, s), 4.20 (2H, br), 3.20 (1H, m), 2.90 (2H, m), 2.16 (2H, m), 1.77 (2H, m), 1.49 (9H, s).

### Example 8

### 4-[4-(4-Methanesulfonyl-phenylsulfanylmethyl)-thiazol-2-yl]-piperidine-1-carboxylic acid tert-butyl ester

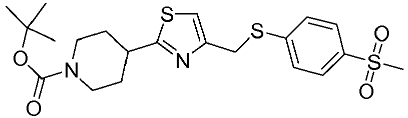

¹H NMR (CDCl₃): δ 7.7 (2H, d, *J* = 9.0 Hz), 7.36 (2H, d, *J* = 9.0 Hz), 7.00 (1H, s), 4.24 (2H, s), 4.3 (2H, m), 3.05 (1H, m), 2.95 (3H, s), 2.78 (2H, m), 1.99 (2H, m), 1.62 (2H, m), 1.38 (9H, s).

### Example 9

### 4-{2-[1-(5-Ethyl-pyrimidin-2-yl)-piperidin-4-yl]-thiazol-4-ylmethoxy}-benzenesulfonamide

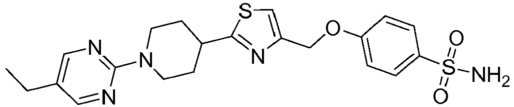

¹H NMR (DMSO-*d₆*): δ 8.24 (2H, s), 7.73 (2H, d), 7.64 (1H, s), 7.20 (4H, m), 5.18 (2H, s), 4.67 (2H, m), 3.38 (1H, m), 3.01 (2H, m), 2.47 (2H, m), 2.08 (2H, m), 1.62 (2H, m), 1.53 (3H, m).

### Example 10

### 2-{4-[4-(2,6-Dichloro-4-methanesulfonyl-phenoxymethyl)-thiazol-2-yl]-piperidin-1-yl}-5-ethyl-pyrimidine

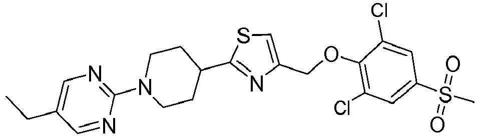

¹H NMR (DMSO-*d₆*): δ 8.23 (2H, s), 7.99 (2H, s), 7.68 (1H, s), 5.20 (2H, s), 4.64 (2H, m), 3.31 (3H, s), 3.30 (1H, m), 3.0 (2H, m), 2.40 (2H, m), 1.98 (2H, m), 1.54 (2H, m), 1.15 (3H, m).

### Example 11

### 5-Ethyl-2-{4-[4-(3-tetrazol-1-yl-phenoxymethyl)-thiazol-2-yl]-piperidin-1-yl}-pyrimidine

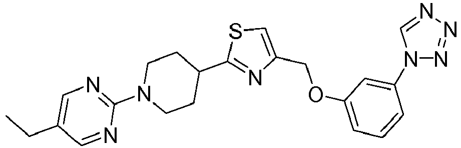

¹H NMR (CDCl₃): δ 9.05 (1H, s), 8.19 (2H, s), 7.55-7.10 (5H, m), 5.24 (2H, s), 4.83 (2H, m), 3.30 (1H, m), 3.04 (2H, m), 2.47 (2H, q, *J*= 7.6 Hz), 2.21 (2H, m), 1.80 (2H, m), 1.19 (3H, t, *J* = 7.6 Hz).

### Example 12

### 5-Ethyl-2-(4-{4-[4-(5-methyl-tetrazol-1-yl)-phenoxymethyl]-thiazol-2-yl}-piperidin-1-yl)-pyrimidine

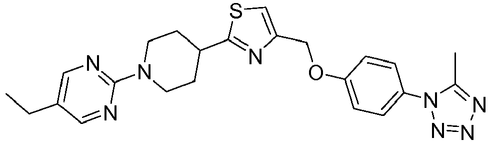

¹H NMR (CDCl₃): δ 8.19 (2H, s), 7.38 (2H, d, *J*= 9.0 Hz), 7.26 (1H, s), 7.17 (2H, d, *J* = 9.0 Hz), 5.24 (2H, s), 4.84 (2H, m), 3.31 (1H, m), 3.05 (2H, m), 2.58 (3H, s), 2.47 (2H, q, *J* = 7.8 Hz), 2.22 (2H, m), 1.82 (2H, m), 1.20 (3H, t, *J* = 7.8 Hz).

### Example 13

### 5-Ethyl-2-{4-[4-(3-methyl-4-methylsulfanyl-phenoxymethyl)-thiazol-2-yl]-piperidin-1-yl} -pyrimidine

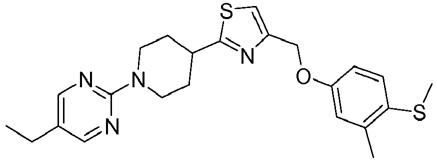

¹H NMR (DMSO-*d₆*): δ 8.23 (2H, s), 7.56 (1H, s), 7.16 (1H, m), 6.90 (1H, m), 6.86 (1H, m), 5.06 (2H, s), 4.67 (2H, m), 3.55 (4H, m), 3.01 (2H, m), 2.48 (3H, s), 2.40 (2H, m), 2.09 (2H, m), 1.57 (2H, m), 1.09 (3H, m).

### Example 14

### 5-Ethyl-2-{4-[4-(4-methanesulfonyl-3-methyl-phenoxymethyl)-thiazol-2-yl]-piperidin-1-yl} -pyrimidine

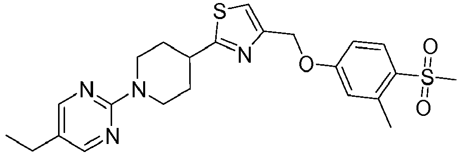

¹H NMR (DMSO-*d₆*): δ 8.13 (2H, s), 7.91 (1H, m), 7.20 (1H, s), 6.85 (2H, m), 5.14 (2H, s), 4.76 (2H, m), 3.23 (1H, m), 2.98 (3H, s), 2.60 (3H, s), 2.42 (2H, m), 2.15 (2H, m), 1.97 (2H, m), 1.76 (2H, m), 1.13 (3H, m).

### Example 15

### 6-{2-[1-(5-Ethyl-pyrimidin-2-yl)-piperidin-4-yl]-thiazol-4-ylmethoxy}-benzo[1,3]oxathiol-2-one

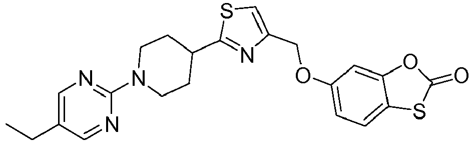

¹H NMR (DMSO-*d₆*): δ 8.23 (2H, s), 7.64 (1H, m), 7.62 (1H, s), 7.30 (1H, m), 7.03 (1H, m), 5.14 (2H, s), 4.64 (2H, m), 3.31 (1H, m), 3.02 (2H, m), 2.40 (2H, q), 2.09 (2H, m), 1.58 (2H, m), 1.12 (3H, t).

### Example 16

### 5-Ethyl-2-{4-[4-(4-trifluoromethylsulfanyl-phenoxymethyl)-thiazol-2-yl]-piperidin-1-yl}-pyrimidine

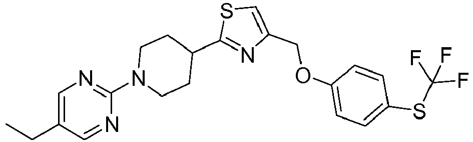

¹H NMR (DMSO-*d₆*): δ 8.23 (2H, s), 7.63 (3H, m), 7.18 (2H, m), 5.17 (2H, s), 4.67 (2H, m), 3.32 (1H, m), 3.01 (2H, m), 2.40 (2H, q), 2.08 (2H, m), 1.59 (2H, m), 1.13 (3H, t).

### Example 17

### 4-[4-(3-Fluoro-4-tetrazol-1-yl-phenoxymethyl)-thiazol-2-yl]-piperidine-1-carboxylic acid tert-butyl ester

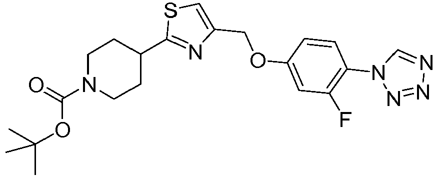

¹H NMR (CDCl₃): δ 9.04 (1H, s), 7.79 (1H, m), 7.29 (1H, s), 7.01 (2H, m), 5.24 (2H, s), 4.22 (2H, m), 3.19 (1H, m), 2.89 (2H, m), 2.11 (2H, m), 1.74 (2H, m), 1.48 (9H, s).

### Example 18

### 4-[4-(2-Fluoro-4-methanesulfonyl-phenoxymethyl)-thiazol-2-yl]-piperidine-1-carboxylic acid tert-butyl ester

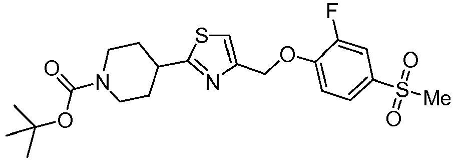

¹H NMR (DMSO-*d₆*): δ 7.79 (1H, m), 7.72 (1H, m), 7.70 (1H, s), 7.57 (1H, m), 5.31 (2H, s), 3.99 (2H, m), 3.21 (3H, s), 3.20 (1H, m), 2.85 (2H, m), 2.02 (2H, m), 1.52 (2H, m), 1.39 (9H, s).

### Example 19

### 4-[4-(2-Fluoro-4-tetrazol-1-yl-phenoxymethyl)-thiazol-2-yl]-piperidine-1-carboxylic acid tert-butyl ester

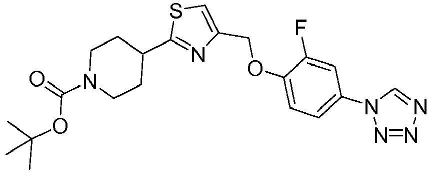

¹H NMR (CDCl₃): δ 8.98 (s, 1H), 7.53 (m, 1H), 7.44 (m, 1H), 7.31 (s, 1H), 7.27 (m, 1H), 5.31 (s, 2H), 4.21 (m, 2H), 3.16 (m, 1H), 2.89 (m, 2H), 2.11 (m, 2H), 1.74 (m, 2H), 1.47 (s, 9H).

### Example 20

### 5-Ethyl-2-{4-[4-(4-trifluoromethanesulfinyl-phenoxymethyl)-thiazol-2-yl]-piperidin-1-yl} -pyrimidine

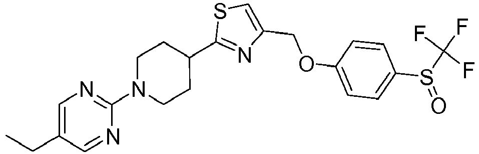

To a solution of 5-Ethyl-2-{4-[4-(4-trifluoromethylsulfanyl-phenoxymethyl)-thiazol-2-yl]-piperidin-1-yl}-pyrimidine (**Example 16**) in DCM at room temperature was added 3-chloro-benzenecarboperoxoic acid (2eq.). The reaction was allowed to stir for 1.5 hours and an additional portion of 3-chloro-benzenecarboperoxoic acid (1eq.) was added to the reaction mixture. The reaction was stirred at room temperature for an additional 4 hours. The organic solution was washed with sodium bicarbonate; the organic layer was isolated, dried over sodium sulfate and filtered. The filtrate was concentrated and the crude product was purified by column chromatography to afford the desired product. ¹H NMR (DMSO-d₆): δ 8.40 (2H, s), 7.58 (2H, d), 7.22 (1H, s), 7.02 (2H, d,), 5.17 (2H, s), 3.74 (2H, m), 3.16 (1H, m), 2.96 (2H, m), 2.57 (2H, m), 2.22 (4H, m), 1.24 (3H, m).

### Example 21

### 4-[4-(4-Methanesulfonyl-benzenesulfonylmethyl)-thiazol-2-yl]-piperidine-1-carboxylic acid tert-butyl ester

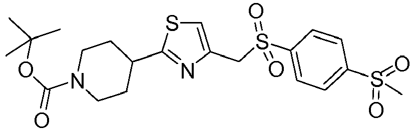

To a solution of 4-[4-(4-Methanesulfonyl-phenylsulfanylmethyl)-thiazol-2-yl]-piperidine-1-carboxylic acid *tert*-butyl ester (**Example 8**, 0.1 g, 0.21 mmol) in CH₂Cl₂ (5 mL) was added *m*CPBA (0.11 g, 0.42 mmol) at room temperature. The resulting mixture was stirred at room temperature for 2 hours and was washed with 5% NaHSO₃, saturated NaHCO₃ and brine. The organic layer was dried with Na₂SO₄ and the solvent was removed *in vacuo*. The residue was purified by flash chromatography on silica gel to afford the desired product. ¹H NMR (CDCl₃): δ 8.03 (2H, d, *J* = 9.0 Hz), 7.88 (2H, d, *J* = 9.0 Hz), 7.29 (1H, s), 4.57 (2H, s), 4.10 (2H, m), 3.07 (3H, s), 2.92 (1H, m), 2.75 (2H, m), 1.85 (2H, m), 1.46 (2H, m), 1.44 (9H, s).

### Example 22

### 4-[4-(4-Methanesulfonyl-phenoxymethyl)-thiazol-2-yl]-piperidine-1-carboxylic acid isopropyl ester

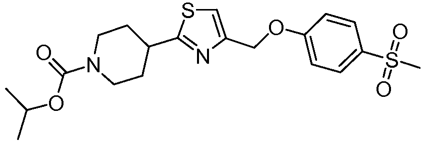

To the HCl salt (**Intermediate 3**, 43 mg, -0.12 mmol) of 4-[4-(4-Methanesulfonyl-phenoxymethyl)-thiazol-2-yl]-piperidine was added 3 mL of THF, followed by isopropyl chloroformate (1.0 M solution in toluene, 0.15 mL, 0.15 mmol) and Et₃N (0.05 mL). The resulting mixture was stirred at room temperature for 2 hours, and then partitioned between EtOAc and H₂O. After concentration of the organic layer *in vacuo*, the residue was purified by silica gel column chromatography with EtOAc/hexanes (40-70%) to give the desired product. ¹H NMR (CDCl₃): δ 7.86 (2H, d, *J*= 9.0 Hz), 7.23 (1H, s), 7.11 (2H, *d, J =* 9.0 Hz), 5.22 (2H, s), 4.92 (1H, m), 4.24 (2H, m), 3.17 (1H, m), 3.03 (3H, s), 2.90 (2H, m), 2.10 (2H, m), 1.72 (2H, m), 1.23 (6H, *d, J=* 6.4 Hz).

The compounds in **Examples 23-46** were synthesized from one **of Intermediates 3-13** or **Intermediates 15-25** with the corresponding sulfonyl chloride, alkyl chloride, alkyl bromide, chloroformate, acid chloride, carbamyl chloride or isocyanate in a manner similar to that described in **Example 22**. One skilled in the art of organic synthesis will appreciate that conditions such as solvent (*e.g.,* DMF, CH₃CN); temperature, base (*e.g.,* NEt₃, K₂CO₃, NaHCO₃, Na₂CO₃, Cs₂CO₃) and concentration can be selected through routine experimentation to optimize yields. Additionally, alternative coupling methods can be used that are well known in the art of organic synthesis.

### Example 23

### 4-[4-(4-Methanesulfonyl-phenoxymethyl)-thiazol-2-yl]-piperidine-1-carboxylic acid benzyl ester

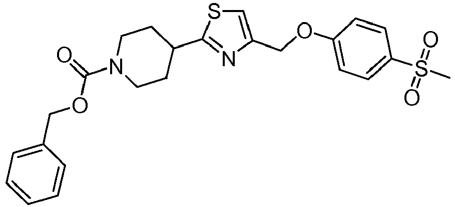

¹H NMR (CDCl₃): δ 7.87 (2H, d, *J* = 9.2 Hz), 7.31-7.37 (5H, m), 7.23 (1H, s), 7.11 (2H, d, *J* = 9.2 Hz), 5.22 (2H, s), 5.14 (2H, s), 4.29 (2H, m), 3.16-3.22 (1H, m), 3.03 (3H, s), 2.96 (2H, m), 2.12 (2H, m), 1.70-1.80 (2H, m).

### Example 24

### 4-[4-(4-Methanesulfonyl-phenoxymethyl)-thiazol-2-yl]-piperidine-1-carboxylic acid isobutyl ester

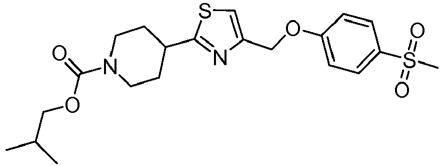

¹H NMR (CDCl₃): δ 7.87 (2H, d, *J* = 9.0 Hz), 7.23 (1H, s), 7.11 (2H, d, *J* = 9.0 Hz), 5.22 (2H, s), 4.25 (2H, m), 3.87 (2H, *d, J=* 6.6 Hz), 3.17 (1H, m), 3.03 (3H, s), 2.94 (2H, m), 2.12 (2H, m), 1.94 (1H, m), 1.75 (2H, m), 0.93 (6H, *d, J =* 6.6 Hz).

### Example 25

### 4-[4-(4-Methanesulfonyl-phenoxymethyl)-thiazol-2-yl]-piperidine-1-carboxylic acid adamantan-1-yl ester

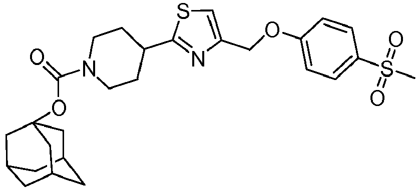

¹H NMR (CDCl₃): δ 7.89 (2H, d, *J* = 8.8 Hz), 7.24 (1H, s), 7.12 (2H, d, *J* = 8.8 Hz), 5.23 (2H, s), 4.21 (2H, m), 3.12-3.20 (1H, m), 3.03 (3H, s), 2.87 (2H, m), 2.05∼2.17 (11H, m), 1.62∼1.79 (8H, m).

### Example 26

### 4-[4-(4-Methanesulfonyl-phenoxymethyl)-thiazol-2-yl]-piperidine-1-carboxylic acid methyl ester

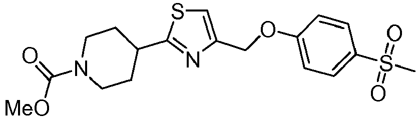

¹H NMR (CDCl₃): δ 7.87 (2H, d, *J* = 9.0 Hz), 7.23 (1H, s), 7.11 (2H, d, *J* = 9.0 Hz), 5.22 (2H, s), 4.24 (2H, m), 3.71 (3H, s), 3.14∼3.17 (1H, m), 3.03 (3H, s), 2.94 (2H, m), 2.12 (2H, m), 1.70∼1.80 (2H, m).

### Example 27

### 4-[4-(4-Methanesulfonyl-phenoxymethyl)-thiazol-2-yl]-piperidine-1-carboxylic acid 4-fluoro-phenyl ester

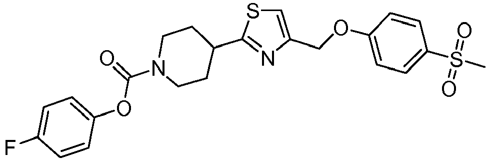

¹H NMR (CDCl₃): δ 7.88 (2H, d, *J* = 8.8 Hz), 7.12 (2H, d, *J* = 8.8 Hz), 7.01-7.09 (5H, m), 5.24 (2H, s), 4.37 (2H, m), 3.23∼3.27 (1H, m), 3.19 (2H, m), 3.04 (3H, s), 2.20 (2H, m), 1.88 (2H, m).

### Example 28

### 4-[4-(4-Methanesulfonyl-phenoxymethyl)-thiazol-2-yl]-piperidine-1-carboxylic acid 4-methoxy-phenyl ester

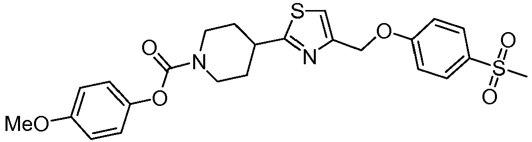

¹H NMR (CDCl₃): δ 7.88 (2H, d, *J=* 8.2 Hz), 7.26 (1H, s), 7.12 (2H, d, *J* = 8.6 Hz), 7.02 (2H, d, *J* = 8.6 Hz), 6.87 (2H, d, *J* = 8.2 Hz), 5.24 (2H, s), 4.38 (2H, m), 3.79 (3H, s), 3.15∼3.28 (3H, m), 3.03 (3H, s). 2.19 (2H, m), 1.87 (2H, m).

### Example 29

### 4-[4-(4-Methanesulfonyl-phenoxymethyl)-thiazol-2-yl]-piperidine-1-carboxylic acid naphthalen-1-yl ester

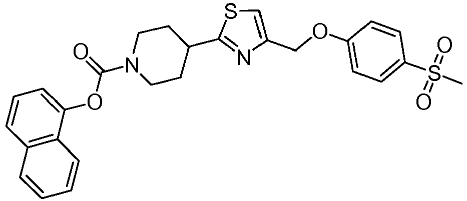

¹H NMR (CDCl₃): δ 7.88 (4H, m), 7.72 (1H, m), 7.49 (3H, m), 7.29 (2H, m), 7.14 (2H, m), 5.26 (2H, s), 4.64 (1H, m), 4.41 (1H, m), 3.34 (2H, m), 3.12 (1H, m), 3.04 (3H, s), 2.27 (2H, m), 2.00 (2H, m).

### Example 30

### 4-[4-(4-Tetrazol-1-yl-phenoxymethyl)-thiazol-2-yl]-piperidine-1-carboxylic acid isobutyl ester

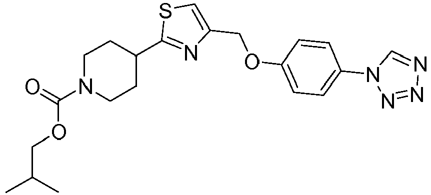

¹H NMR (CDCl₃): δ 8.94 (1H, s), 7.60 (2H, d), 7.24 (1H, s), 7.14 (2H, d,), 5.20 (2H, s), 4.24 (2H, br), 3.85 (2H, d,), 3.18 (1H, m), 2.92 (2H, m), 2.11 (2H, m), 1.91 (1H, m), 1.75 (2H, m), 0.91 (6H, d,).

### Example 31

### 4-[4-(4-Tetrazol-1-yl-phenoxymethyl)-thiazol-2-yl]-piperidine-1-carboxylic acid pentyl ester

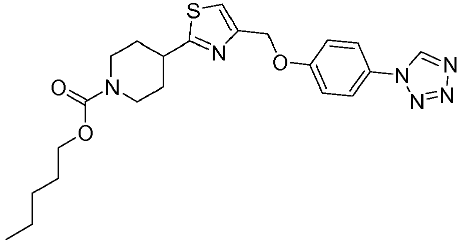

¹H NMR (CDCl₃): δ 8.94 (1H, s), 7.62 (2H, d, *J* = 9.2 Hz), 7.28 (1H, s), 7.18 (2H, d, *J* = 9.2 Hz), 5.24 (2H, s), 4.27 (2H, br), 4.09 (2H, m), 3.21 (1H, m), 2.94 (2H, m), 2.14 (2H, m), 1.78 (2H, m), 1.65 (2H, m), 1.35 (4H, m), 0.91 (3H, m).

### Example 32

### 4-[4-(4-Tetrazol-1-yl-phenoxymethyl)-thiazol-2-yl]-piperidine-1-carboxylic acid 2-fluoro-ethyl ester

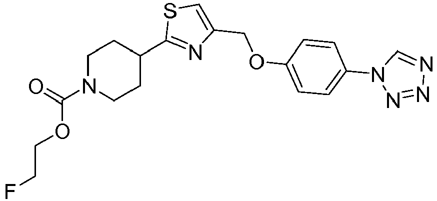

¹H NMR (CDCl₃): δ 8.97 (1H, s), 7.62 (2H, d, *J* = 9.0 Hz), 7.28 (1H, s), 7.17 (2H, d, *J* = 9.0 Hz), 5.24 (2H, s), 4.70-4.30 (6H, m), 3.22 (1H, m), 2.99 (2H, m), 2.15 (2H, m), 1.78 (2H, m).

### Example 33

### 4-[4-(4-Tetrazol-1-yl-phenoxymethyl)-thiazol-2-yl]-piperidine-1-carboxylic acid butyl ester

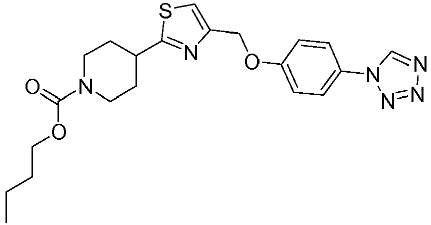

¹H NMR (CDCl₃): δ 9.01 (1H, s), 7.64 (2H, d, *J* = 8.8 Hz), 7.29 (1H, s), 7.17 (2H, d, *J* = 8.8 Hz), 5.24 (2H, s), 4.26 (2H, m), 4.10 (2H, t), 3.21 (1H, m), 2.95 (2H, m), 2.14 (2H, m), 1.78 (2H, m), 1.63 (2H, m), 1.40 (2H, m), 0.95 (3H, *t, J* = 7.4 Hz).

### Example 34

### 4-[4-(4-Tetrazol-1-yl-phenoxymethyl)-thiazol-2-yl]-piperidine-1-carboxylic acid 2,2-dimethyl-propyl ester

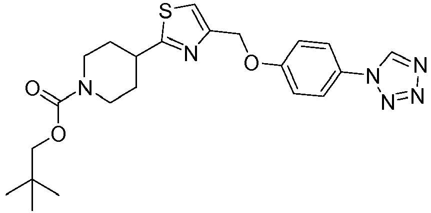

¹H NMR (CDCl₃): δ 9.00 (1H, s), 7.56 (2H, d, *J* = 8.8 Hz), 7.21 (1H, s), 7.08 (2H, d, *J* = 8.8 Hz), 5.14 (2H, s), 4.17 (2H, br), 3.69 (2H, s), 3.13 (1H, m), 2.88 (2H, m), 2.06 (2H, m), 1.73 (2H, m), 0.86 (9H, s).

### Example 35

### 4-[4-(4-Tetrazol-1-yl-phenoxymethyl)-thiazol-2-yl]-piperidine-1-carboxylic acid hexyl ester

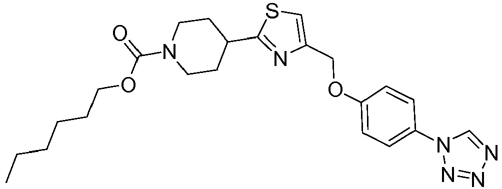

¹H NMR (CDCl₃): δ 9.06 (1H, s), 7.65 (2H, d, *J* = 8.8 Hz), 7.29 (1H, s), 7.18 (2H, d, *J* = 8.8 Hz), 5.24 (2H, s), 4.27 (2H, br), 4.09 (2H, t), 3.21 (1H, m), 2.95 (2H, m), 2.14 (2H, m), 1.78 (2H, m), 1.64 (2H, m), 1.33 (6H, m), 0.89 (3H, m).

### Example 36

### 4-[4-(4-Tetrazol-1-yl-phenoxymethyl)-thiazol-2-yl]-piperidine-1-carboxylic acid 2-ethylhexyl ester

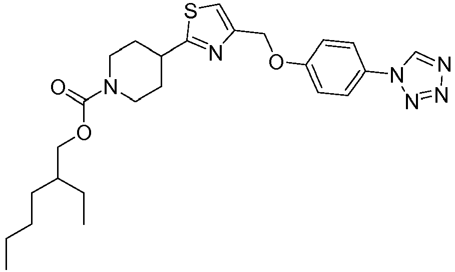

¹H NMR (CDCl₃): δ 8.98 (1H, s), 7.58 (2H, d, *J* = 8.8 Hz), 7.23 (1H, s), 7.10 (2H, d, *J* = 8.8 Hz), 5.17 (2H, s), 4.19 (2H, br), 3.95 (2H, m), 3.15 (1H, m), 2.89 (2H, m), 2.07 (2H, m), 1.69 (2H, m), 1.52 (1H, m), 1.35-1.20 (8H, m), 0.90-0.80 (6H, m).

### Example 37

### 4-[4-(4-Tetrazol-1-yl-phenoxymethyl)-thiazol-2-yl]-piperidine-1-carboxylic acid 2-benzyloxy-ethyl ester

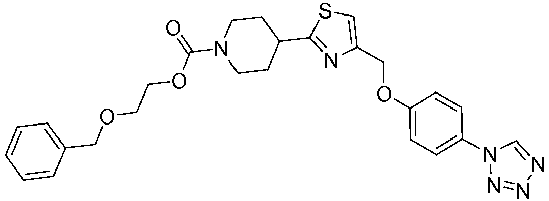

¹H NMR (CDCl₃): δ 8.98 (1H, s), 7.57 (2H, d, *J =* 8.0 Hz), 7.30-7.20 (6H, m), 7.11 (2H, *d*, *J* = 8.0 Hz), 5.17 (2H, s), 4.52 (2H, s), 4.25-4.20 (4H, m), 3.65 (2H, m), 3.15 (1H, m), 2.91 (2H, m), 2.08 (2H, m), 1.73 (2H, m).

### Example 38

### 4-[4-(4-Tetrazol-1-yl-phenoxymethyl)-thiazol-2-yl]-piperidine-1-carboxylic acid 2-isopropyl-5-methyl-cyclohexyl ester

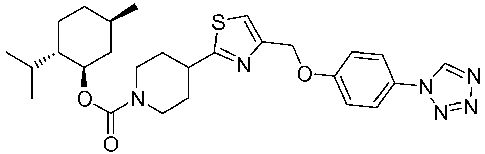

¹H NMR (CDCl₃): δ 8.97 (1H, s), 7.58 (2H, m), 7.23 (1H, s), 7.11 (2H, m), 5.18 (2H, s), 4.21 (2H, br), 3.13 (1H, m), 2.88 (2H, m), 2.05-0.70 (23H, m).

### Example 39

### Adamantan-1-yl-{4-[4-(4-methanesulfonyl-phenoxymethyl)-thiazol-2-yl]-piperidin-1-yl}-methanone

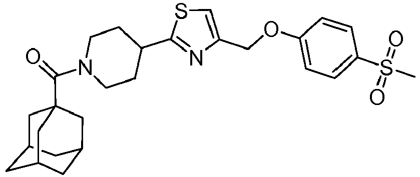

¹H NMR (CDCl₃): δ 7.88 (2H, d, *J* = 8.8 Hz), 7.24 (1H, s), 7.12 (2H, d, *J* = 8.8 Hz), 5.23 (2H, s), 4.61 (2H, m), 3.24-3.30 (1H, m), 3.03 (3H, s), 2.93-3.00 (2H, m), 2.16 (2H, m), 2.02∼2.04 (9H, m), 1.70∼1.80 (8H, m).

### Example 40

### {4-[4-(4-Methanesulfonyl-phenoxymethyl)-thiazol-2-yl]-piperidin-1-yl}-pyridin-3-yl-methanone

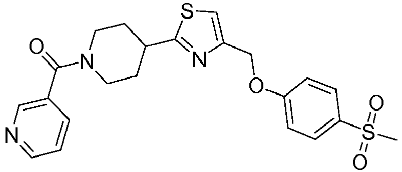

¹H NMR (CDCl₃): δ 8.69 (2H, m), 7.88 (2H, *d, J=* 8.4 Hz), 7.79 (1H, m), 7.38 (1H, m), 7.27 (1H, s), 7.12 (2H, *d, J =* 8.4 Hz), 5.24 (2H, s), 4.79 (2H, br), 3.86 (2H, br), 3.31 (1H, m), 3.04 (3H, s), 2.20 (2H, m), 1.84 (2H, m).

### Example 41

### 3,3-Dimethyl-1-{4-[4-(4-tetrazol-1-yl-phenoxymethyl)-thiazol-2-yl]-piperidin-1-yl}-butan-1-one

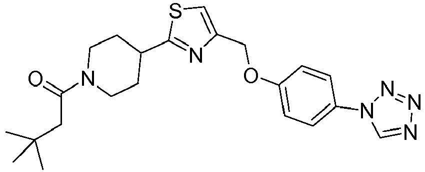

¹H NMR (DMSO-d₆): δ 9.98 (1H, s), 7.81 (2H, d, *J* = 8.8 Hz), 7.66 (1H, s), 7.29 (2H, d, *J =* 8.8 Hz), 5.20 (2H, s), 4.52 (1H, m), 4.10 (1H, m), 3.26 (1H, m), 3.19 (1H, m), 2.70 (1H, m), 2.25 (2H, m), 2.15 (2H, m), 1.50 (2H, m), 0.96 (9H, s).

### Example 42

### Oxo-{4-[4-(4-tetrazol-1-yl-phenoxymethyl)-thiazol-2-yl]-piperidin-1-yl}-acetic acid methyl ester

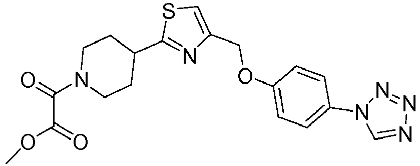

¹H NMR (DMSO-*d₆*): δ 9.98 (1H, s), 7.81 (2H, *d, J=* 8.8 Hz), 7.68 (1H, s), 7.29 (2H, d, *J* = 8.8 Hz), 5.21 (2H, s), 4.32 (1H, m), 3.80 (3H, s), 3.60 (1H, m), 3.32 (1H, m), 2.94 (2H, m), 2.13 (2H, m), 1.57 (2H, m).

### Example 43

### 3-Oxo-3-{4-[4-(4-tetrazol-1-yl-phenoxymethyl)-thiazol-2-yl]-piperidin-1-yl}-propionic acid ethyl ester

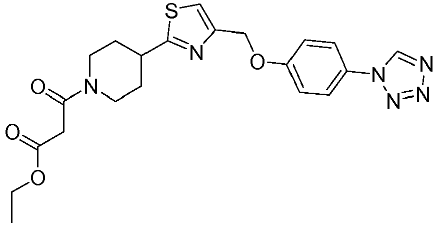

¹H NMR (DMSO-*d₆*): δ 8.94 (1H, s), 7.61 (2H, m), 7.26 (1H, s), 7.15 (2H, m), 5.20 (2H, s), 4.65 (1H, m), 4.17 (2H, q), 3.87 (1H, m), 3.48 (2H, s), 3.26 (2H, m), 2.81 (1H, m), 2.18 (2H, m), 1.78 (2H, m), 1.27 (3H, t).

### Example 44

### (4-Methyl-piperazin-1-yl)-{4-[4-(4-tetrazol-1-yl-phenoxymethyl)-thiazol-2-yl]-piperidin-1-yl}-methanone

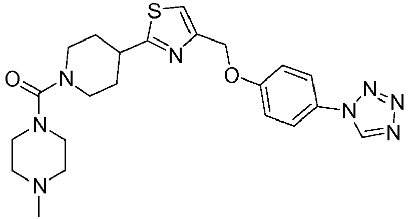

¹H NMR (DMSO-*d₆*): δ 9.98 (1H, s), 7.81 (2H, *d, J=* 8.9 Hz), 7.64 (1H, s), 7.29 (2H, d), 5.20 (2H, s), 3.29 (2H, m), 3.18 (5H, m), 2.95 (2H, d), 2.61 (3H, s), 2.38 (2H, m), 2.03 (4H, m), 1.65 (2H, m).

### Example 45

### 4-[4-(4-Tetrazol-1-yl-phenoxymethyl)-thiazol-2-yl]-piperidine-1-carboxylic acid diethylamide

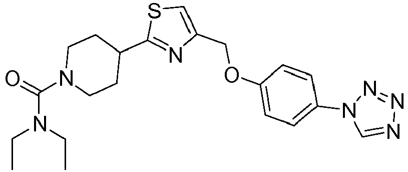

¹H NMR (DMSO-*d₆*): δ 9.98 (1H, s), 7.81 (2H, *d, J=* 8.9 Hz), 7.66 (1H, s), 7.29 (2H, *d, J=* 8.9 Hz), 5.20 (2H, s), 3.55 (2H, m), 3.20 (1H, m), 3.14 (4H, q), 2.81 (2H, m), 2.02 (2H, m), 1.64 (2H, m), 1.02 (6H, *t, J=* 6.8 Hz).

### Example 46

### 4-[4-(4-Tetrazol-1-yl-phenoxymethyl)-thiazol-2-yl]-piperidine-1-carboxylic acid ethylamide

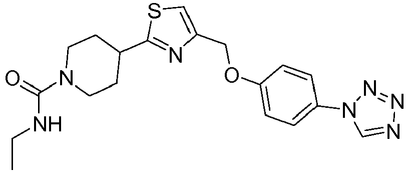

¹H NMR (DMSO-*d₆*): δ 9.98 (1H, s), 7.81 (2H, d, *J* = 8.9 Hz), 7.65 (1H, s), 7.29 (2H, *d,J=* 8.9 Hz), 6.47 (1H, m), 5.20 (2H, s), 4.01 (2H, d), 3.17 (1H, m), 3.04 (2H, m), 2.78 (2H, m), 1.97 (2H, m), 1.52 (2H, m), 0.99 (3H, *t, J=* 6.8 Hz).

### Example 47

### 2- {4-[4-(4-Methanesulfonyl-phenoxymethyl)-thiazol-2-yl]-piperidin-1-yl}-pyrimidine

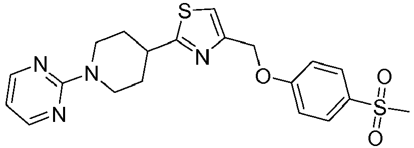

A mixture of 4-[4-(4-methylsulfonyl-phenoxymethyl)-thiazole-2-yl]-piperidine hydrochloride (100 mg, 0.24 mmol), 2-chloropyrimidine (30 mg, 1.1 eq.) and diisopropylethylamine (122 mg, 4 eq.) in *i*-PrOH (5 mL) was heated at 90 °C for 1.5 hours. The solvent was removed *in vacuo*. The residue was purified on silica gel (60% EtOAc in hexanes) to afford the desired product. ¹H NMR (CDCl₃): δ 8.32 (2H, d, *J=* 4.8 Hz), 7.88 (2H, d, *J* = 8.8 Hz), 7.23 (1H, s), 7.12 (2H, d, *J =* 8.8 Hz), 6.49 (1H, t, *J =* 4.8 Hz), 5.24 (2H, s), 4.89 (2H, m), 3.32 (1H, m), 3.06 (2H, m), 3.04 (3H, s), 2.22 (2H, m), 1.81 (2H, m).

The compounds in **Examples 48-77** were synthesized from one **of Intermediates 3-13** or **Intermediates 15-25** with the corresponding substituted 2-chloropyrimidine, 2-iodopyrimidine, 2-chloropyridine, 2-fluoropyridine, 2-methanesulfonyl-pyrimidine, 2-chloropyrazine, 2-chloropyridazine or other suitable heterocycles in a manner similar to that described in **Example 47**. One skilled in the art of organic synthesis will appreciate that conditions such as solvent (such as DMF, CH₃CN); temperature, base (such as NEt₃, K₂CO₃, NaHCO₃, Na₂CO₃, Cs₂CO₃) and concentration can be selected through routine experimentation to optimize yields. Additionally, alternative coupling methods can be used that are well known in the art of organic synthesis.

### Example 48

### 2-{4-[4-(4- Methanesulfonyl-phenoxymethyl)-thiazol-2-yl]-piperidin-1-yl}-4-methoxy-pyrimidine

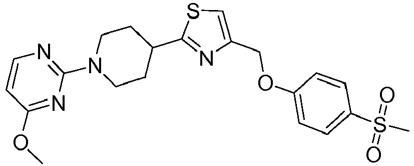

¹H NMR (CDCl₃): δ 8.06 (1H, d, *J* = 6.0 Hz), 7.87 (2H, d, *J* = 8.8 Hz), 7.23 (1H, s), 7.12 (2H, d, *J* = 8.8 Hz), 5.98 (1H, d, *J* = 6.0 Hz), 5.24 (2H, s), 4.88 (2H, m), 3.90 (3H, s), 3.31 (1H, m), 3.04 (5H, m), 2.20 (2H, m), 1.81 (2H, m).

### Example 49

### 2- {4-[4-(4-Methanesulfonyl-phenoxymethyl)-thiazol-2-yl]-piperidin-1-yl}-4-trifluoromethyl-pyrimidine

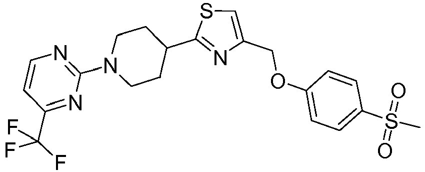

¹H NMR (CDCl₃): δ 8.50 (1H, d, *J* = 4.8 Hz), 7.88 (2H, d, *J* = 8.8 Hz), 7.24 (1H, s), 7.12 (2H, d, *J =* 8.8 Hz), 6.76 (1H, *d, J =* 4.8 Hz), 5.24 (2H, s), 4.92 (2H, m), 3.34 (1H, m), 3.11 (2H, m), 3.04 (3H, s), 2.24 (2H, m), 1.84 (2H, m).

### Example 50

### 2-{4-[4-(4-Methanesulfonyl-phenoxymethyl)-thiazol-2-yl]-piperidin-1-yl}-4,6-dimethyl-pyrimidine

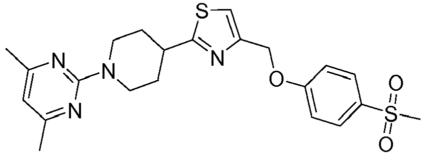

¹H NMR (CDCl₃): δ 7.88 (2H, d, *J* = 8.4 Hz), 7.22 (1H, s), 7.12 (2H, d, *J* = 8.4 Hz), 6.27 (1H, s), 5.24 (2H, s), 4.96 (2H, m), 3.28 (1H, m), 3.04 (3H, s), 2.99 (2H, m), 2.29 (6H, s), 2.19 (2H, m), 1.80 (2H, m).

### Example 51

### 5-Ethyl-2-{4-[4-(4-methanesulfonyl-phenoxymethyl)-thiazol-2-yl]-piperidin-1-yl}-pyrimidine

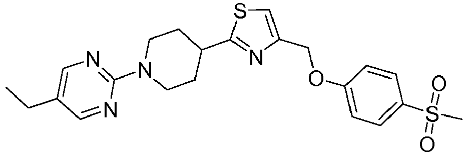

¹H NMR (CDCl₃): δ 8.19 (2H, s), 7.87 (2H, d, *J* = 8.8 Hz), 7.22 (1H, s), 7.12 (2H, d, *J* = 8.8 Hz), 5.24 (2H, s), 4.84 (2H, m), 3.30 (1H, m), 3.04 (2H, m), 3.03 (3H, s), 2.47 (2H, q, *J* = 7.2 Hz), 2.22 (2H, m), 1.81 (2H, m), 1.20 (3H, t, *J* = 7.2 Hz).

### Example 52

### 5-Ethyl-2-{4-[4-(4-tetrazol-1-yl-phenoxymethyl)-thiazol-2-yl]-piperidin-1-yl}-pyrimidine

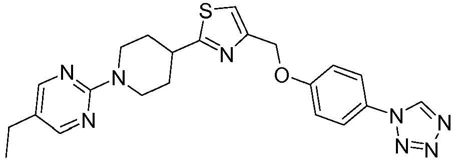

¹H NMR (DMSO-*d₆*): δ 9.98 (1H, s), 8.24 (2H, s), 7.80 (2H, d, *J* = 8.8 Hz), 7.66 (1H, s), 7.28 (2H, d, *J* = 8.8 Hz), 5.20 (2H, s), 4.67 (2H, m), 3.32 (1H, m), 3.01 (2H, m), 2.43 (2H, q, *J* = 7.2 Hz), 2.07 (2H, m), 1.59 (2H, m), 1.11 (3H, t, *J* = 7.2 Hz).

### Example 53

### 5-Fluoro-2-{4-[4-(6-tetrazol-1-yl-pyridin-3-yloxymethyl)-thiazol-2-yl]-piperidin-1-yl}-pyrimidine

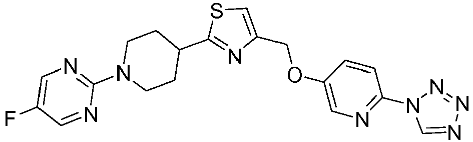

¹H NMR (DMSO-d₆): δ 10.07 (1H, s), 8.43 (2H, s), 8.41 (1H, d, *J* = 3.2 Hz), 7.98 (1H, d, *J* = 9.2 Hz), 7.86 (1H, dd, *J =* 9.2, 3.2 Hz), 7.71 (1H, s), 5.30 (2H, s), 4.58 (2H, m), 3.31 (1H, m), 3.01 (2H, m), 2.10 (2H, m), 1.59 (2H, m).

### Example 54

### 5-Bromo-2-{4-[4-(4-tetrazol-1-yl-phenoxymethyl)-thiazol-2-yl]-piperidin-1-yl}-pyrimidine

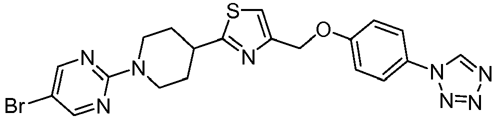

¹H NMR (CDCl₃): δ 8.90 (1H, s), 8.29 (2H, s), 7.60 (2H, d, *J* = 9.0 Hz), 7.25 (1H, s), 7.16 (2H, d, *J* = 9.0 Hz), 5.23 (2H, s), 4.81 (2H, m), 3.31 (1H, m), 3.06 (2H, m), 2.21 (2H, m), 1.79 (2H, m).

### Example 55

### 5-Fluoro-2-{4-[4-(4-tetrazol-1-yl-phenoxymethyl)-thiazol-2-yl]-piperidin-1-yl}-pyrimidine

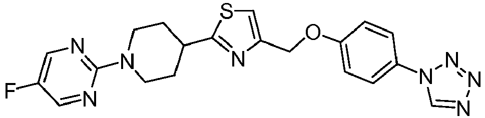

¹H NMR (CDCl₃): δ 8.91 (1H, s), 8.20 (2H, s), 7.60 (2H, d, *J* = 8.6 Hz), 7.25 (1H, s), 7.16 (2H, d, *J* = 8.6 Hz), 5.23 (2H, s), 4.78 (2H, m), 3.31 (1H, m), 3.06 (2H, m), 2.21 (2H, m), 1.83 (2H, m).

### Example 56

### 4,5-Dichloro-2-{4-[4-(4-tetrazol-1-yl-phenoxymethyl)-thiazol-2-yl]-piperidin-1-yl}-pyrimidine

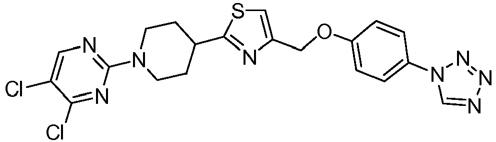

¹H NMR (CDCl₃): δ 8.91 (1H, s), 8.10 (1H, s), 7.61 (2H, d, *J* = 8.8 Hz), 7.27 (1H, s), 7.16 (2H, d, *J* = 8.8 Hz), 5.23 (2H, s), 4.62 (2H, m), 3.34 (1H, m), 3.18 (2H, m), 2.25 (2H, m), 1.98 (2H, m).

### Example 57

### 4-Chloro-5-methyl-2-{4-[4-(4-tetrazol-1-yl-phenoxymethyl)-thiazol-2-yl]-piperidin-1-yl}-pyrimidine

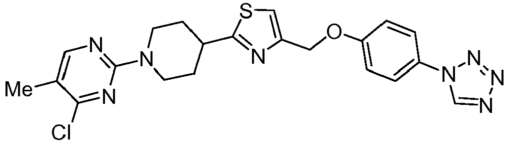

¹H NMR (CDCl₃): δ 8.90 (1H, s), 8.08 (1H, s), 7.60 (2H, d, *J* = 8.8 Hz), 7.24 (1H, s), 7.17 (2H, d, *J* = 8.8 Hz), 5.23 (2H, s), 4.80 (2H, m), 3.30 (1H, m), 3.04 (2H, m), 2.19 (2H, m), 2.16 (3H, s), 1.81 (2H, m).

### Example 58

### 2-Chloro-5-methyl-4-{4-[4-(4-tetrazol-1-yl-phenoxymethyl)-thiazol-2-yl]-piperidin-1-yl}-pyrimidine

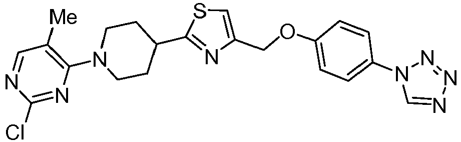

¹H NMR (CDCl₃): δ 8.92 (1H, s), 7.96 (1H, s), 7.60 (2H, *d, J =* 8.8 Hz), 7.27 (1H, s), 7.16 (2H, *d, J =* 8.8 Hz), 5.23 (2H, s), 4.17 (2H, m), 3.31 (1H, m), 3.10 (2H, m), 2.26 (2H, m), 2.21 (3H, s), 1.95 (2H, m).

### Example 59

### 5-(4-Chloro-phenyl)-2-{4-[4-(4-tetrazol-1-yl-phenoxymethyl)-thiazol-2-yl]-piperidin-1-yl} -pyrimidine

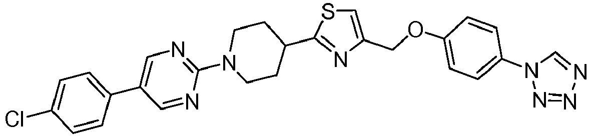

¹H NMR (DMSO-*d₆*): δ 9.97 (1H, s), 8.71 (2H, s), 7.80 (2H, d, *J* = 8.8 Hz), 7.67 (2H, d, *J*= 8.4 Hz), 7.66 (1H, s), 7.48 (2H, d, *J* = 8.4 Hz), 7.28 (2H, d, *J* = 8.8 Hz), 5.21 (2H, s), 4.76 (2H, m), 3.37 (1H, m), 3.13 (2H, m), 2.12 (2H, m), 1.66 (2H, m).

### Example 60

### 5-Chloro-2-{4-[4-(4-tetrazol-1-yl-phenoxymethyl)-thiazol-2-yl]-piperidin-1-yl}-pyrimidine

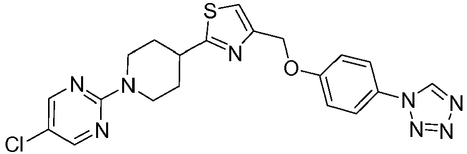

¹H NMR (CDCl₃): δ 8.91 (1H, s), 8.23 (2H, s), 7.61 (2H, d, *J* = 8.8 Hz), 7.26 (1H, s), 7.17 (2H, d, *J* = 8.8 Hz), 5.24 (2H, s), 4.82 (2H, m), 3.32 (1H, m), 3.07 (2H, m), 2.22 (2H, m), 1.81 (2H, m).

### Example 61

### 5-Heptyl-2-{4-[4-(4-methanesulfonyl-phenoxymethyl)-thiazol-2-yl]-piperidin-1-yl}-pyrimidine

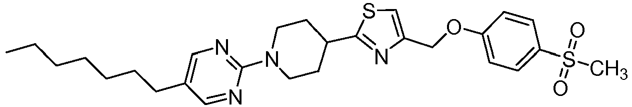

¹H NMR (CDCl₃): δ 8.16 (2H, s), 7.87 (2H, d, *J* = 9.0 Hz), 7.22 (1H, s),7.12 (2H, d, *J* = 9.0 Hz), 5.24 (2H, s), 4.83 (2H, m), 3.29 (1H, m), 3.04 (2H, m), 3.03 (3H, s), 2.42 (2H, t, *J* = 7.4 Hz), 2.21 (2H, m), 1.80 (2H, m), 1.52 (2H, m), 1.28 (8H, m), 0.89 (3H, t).

### Example 62

### 2-{4-[4-(4-Methanesulfonyl-phenoxymethyl)-thiazol-2-yl]-piperidin-1-yl}-5-pentyl-pyrimidine

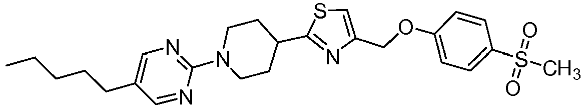

¹H NMR (CDCl₃): δ 8.16 (2H, s), 7.87 (2H, *d, J=* 8.8 Hz), 7.22 (1H, s),7.12 (2H, *d, J=* 8.8 Hz), 5.23 (2H, s), 4.83 (2H, m), 3.29 (1H, m), 3.04 (2H, m), 3.03 (3H, s), 2.42 (2H, *t, J=* 7.6 Hz), 2.21 (2H, m), 1.81 (2H, m), 1.56 (2H, m), 1.32 (4H, m), 0.90 (3H, t).

### Example 63

### 5-Heptyl-2-{4-[4-(4-tetrazol-1-yl-phenoxymethyl)-thiazol-2-yl]-piperidin-1-yl}-pyrimidine

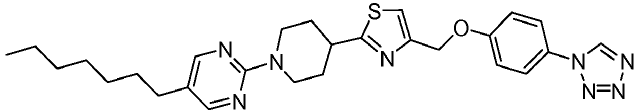

¹H NMR (CDCl₃): δ 8.90 (1H, s), 8.16 (2H, s), 7.60 (2H, d, *J* = 8.8 Hz), 7.24 (1H, s), 7.17 (2H, *d, J =* 8.8 Hz), 5.23 (2H, s), 4.82 (2H, m), 3.29 (1H, m), 3.04 (2H, m), 2.42 (2H, t), 2.20 (2H, m), 1.80 (2H, m), 1.53 (2H, m), 1.28 (8H, m), 0.87 (3H, t).

### Example 64

### 5-Pentyl-2-{4-[4-(4-tetrazol-1-yl-phenoxymethyl)-thiazol-2-yl]-piperidin-1-yl}-pyrimidine

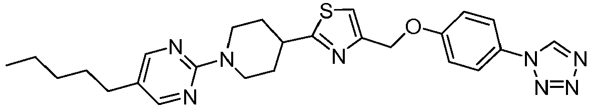

¹H NMR (CDCl₃): δ 8.90 (1H, s), 8.16 (2H, s), 7.60 (2H, d, *J* = 8.8 Hz), 7.24 (1H, s), 7.17 (2H, d, *J* = 8.8 Hz), 5.23 (2H, s), 4.83 (2H, m), 3.30 (1H, m), 3.04 (2H, m), 2.42 (2H, t), 2.20 (2H, m), 1.80 (2H, m), 1.54 (2H, m), 1.30 (4H, m), 0.89 (3H, t).

### Example 65

### 5-Methyl-2-{4-[4-(4-tetrazol-1-yl-phenoxymethyl)-thiazol-2-yl]-piperidin-1-yl}-pyrimidine

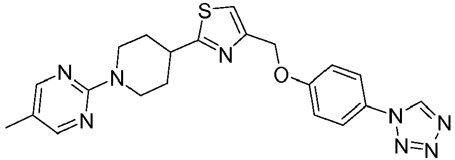

¹H NMR (CDCl₃): δ 8.94 (1H, s), 8.17 (2H, s), 7.62 (2H, d, *J* = 8.8 Hz), 7.25 (1H, s), 7.17 (2H, *d, J =* 8.8 Hz), 5.24 (2H, s), 4.82 (2H, d), 3.30 (1H, m), 3.04 (2H, m), 2.22 (2H, m), 2.13 (3H, s), 1.81 (2H, m).

### Example 66

### 5-(4-Methoxy-phenyl)-2-{4-[4-(4-tetrazol-1-yl-phenoxymethyl)-thiazol-2-yl]-piperidin-1-yl} -pyrimidine

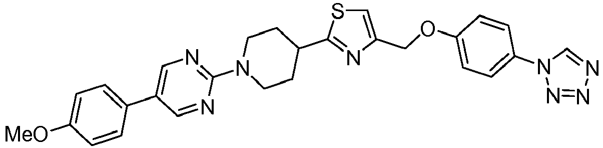

¹H NMR (CDCl₃): δ 8.90 (1H, s), 8.52 (s, 2H), 7.61 (2H, d, *J* = 9.0 Hz), 7.41 (2H, d, *J =* 8.6 Hz), 7.25 (1H, s), 7.17 (2H, d, *J =* 9.0 Hz), 6.99 (2H, d, *J =* 8.6 Hz), 5.24 (2H, s), 4.92 (2H, m), 3.85 (3H, s), 3.34 (1H, m), 3.12 (2H, m), 2.25 (2H, m), 1.85 (2H, m).

### Example 67

### 5-Propyl-2-{4-[4-(4-tetrazol-1-yl-phenoxymethyl)-thiazol-2-yl]-piperidin-1-yl}-pyrimidine

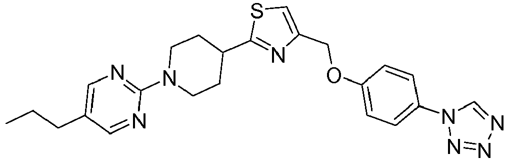

¹H NMR (CDCl₃): δ 8.9 (1H, s), 8.17 (2H, s), 7.61 (2H, d, *J* = 8.8 Hz), 7.24 (1H, s), 7.17 (2H, d, *J* = 8.8 Hz), 5.24 (2H, s), 4.83 (2H, m), 3.31 (1H, m), 3.04 (2H, m), 2.4 (2H, t, *J* = 7.6 Hz), 2.22 (2H, m), 1.81 (2H, m), 1.58 (2H, m), 0.94 (3H, t, *J* = 7.6 Hz).

### Example 68

### 5-Methoxy-2-{4-[4-(4-tetrazol-1-yl-phenoxymethyl)-thiazol-2-yl]-piperidin-1-yl}-pyrimidine

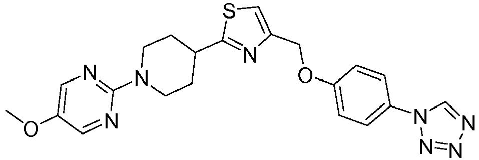

¹H NMR (CDCl₃): δ 8.93 (1H, s), 8.11 (2H, s), 7.61 (2H, d, *J* = 8.8 Hz), 7.25 (1H, s), 7.17 (2H, d, *J* = 8.8 Hz), 5.24 (2H, s), 4.74 (2H, m), 3.81 (3H, s), 3.31 (1H, m), 3.03 (2H, m), 2.22 (2H, m), 1.82 (2H, m).

### Example 69

### 5'-Methyl-4-[4-(4-tetrazol-1-yl-phenoxymethyl)-thiazol-2-yl]-3,4,5,6-tetrahydro-2H-[1,2']bipyridinyl

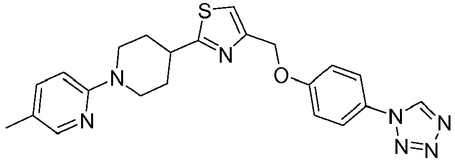

¹H NMR (CDCl₃): δ 8.91 (1H, s), 8.03 (1H, m), 7.61 (2H, m), 7.33 (1H, m), 7.26 (1H, s), 7.18 (2H, m), 6.65 (1H, d, *J =* 8.8 Hz), 5.24 (2H, s), 4.33 (2H, m), 3.25 (1H, m), 2.97 (2H, m), 2.22 (2H, m), 2.21 (3H, s), 1.89 (2H, m).

### Example 70

### 4-[4-(4-Tetrazol-1-yl-phenoxymethyl)-thiazol-2-yl]-5',6"-bis-trifluoromethyl-3,4,5,6-tetrahydro-2H-[1,2';6',2"]terpyridine

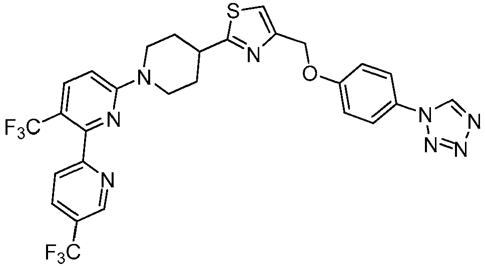

¹H NMR (DMSO-*d₆*): δ 8.81 (1H, m), 8.39 (1H, m), 8.13 (1H, dd, *J* = 8.8, 2.4 Hz), 7.76 (1H, dd, *J* = 8.8, 2.8 Hz), 7.66 (1H, s), 7.59 (2H, m), 7.25 (2H, m), 6.99 (1H, d, *J* = 9 Hz), 6.8 (1H, d, *J* = 9 Hz), 5.19 (2H, s), 4.48 (2H, d), 3.37 (1H, m), 3.10 (2H, m), 2.11(2H, m), 1.65 (2H, m).

### Example 71

### 4-[4-(4-Tetrazol-1-yl-phenoxymethyl)-thiazol-2-yl]-5'-trifluoromethyl-3,4,5,6-tetrahydro-2H-[1,2']bipyridinyl

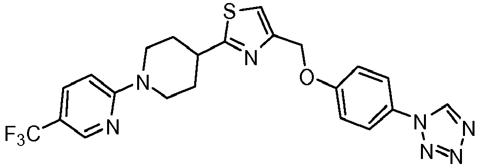

¹H NMR (DMSO-*d₆*): δ 9.98 (1H, s), 8.40 (1H, m), 7.81-7.75 (3H, m), 7.66 (1H, s), 7.28 (2H, d), 6.99 (1H, d, *J* = 8.8 Hz), 5.21 (2H, s), 4.48 (2H, d), 3.37 (1H, m), 3.1 (2H, m), 2.12 (2H, m), 1.65 (2H, m).

### Example 72

### 4-[4-(4-Tetrazol-1-yl-phenoxymethyl)-thiazol-2-yl]-3,4,5,6-tetrahydro-2H-[1,2']bipyridinyl-5'-carbaldehyde

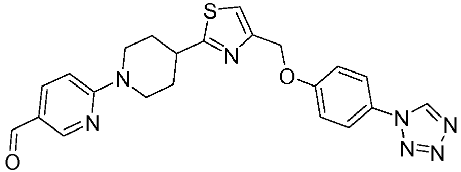

¹H NMR (DMSO-*d₆*): δ 9.98 (1H, s), 9.72 (1H, s), 8.58 (1H, d, *J* = 2.4 Hz), 7.86 (1H, dd, *J* = 9.2, 2 Hz), 7.8 (2H, d, *J* = 8.4 Hz), 7.67 (1H, s), 7.28 (2H, d, *J* = 8.4 Hz), 6.99 (1H, d, *J* = 8.8 Hz), 5.2 (2H, s), 4.58 (2H, d), 3.41 (1H, m), 3.17 (2H, m), 2.13 (2H, m), 1.65 (2H, m).

### Example 73

### 1-(3-Isopropyl-[1,2,4]oxadiazol-5-yl)-4-[4-(4-methanesulfonyl-phenoxymethyl)-thiazol-2-yl]-piperidine

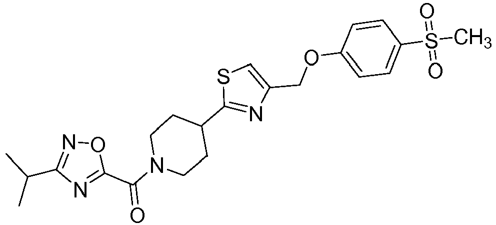

¹H NMR (CDCl₃): δ 7.87 (2H, m), 7.26 (1H, s), 7.11 (2H, m), 5.23 (2H, s), 4.76-4.68 (1H, m), 4.26-4.18 (1H, m), 3.4-3.3 (2H, m), 3.2-3.04 (2H, m), 3.03 (3H, s), 2.32-2.2 (2H, m), 2.00-1.86 (2H, m), 1.36 (6H, d, *J* = 7.2 Hz).

### Example 74

### 2-{4-[4-(4-Methanesulfonyl-phenoxymethyl)-thiazol-2-yl]-piperidin-1-yl}-benzooxazole

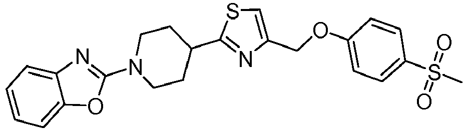

¹H NMR (CDCl₃): δ 7.87 (2H, *d, J=* 8.4 Hz), 7.36 (1H, *d, J=* 7.6 Hz), 7.01-7.19 (6H, m), 5.24 (2H, s), 4.42 (2H, m), 3.30 (3H, m), 3.03 (3H, s), 2.27 (2H, m), 1.95 (2H, m).

### Example 75

### 4-[4-(4-Methanesulfonyl-phenoxymethyl)-thiazol-2-yl]-5'-trifluoromethyl-3,4,5,6-tetrahydro-2H-[1,2']bipyridinyl

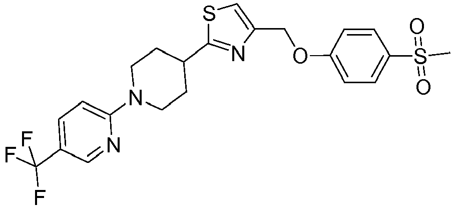

¹H NMR (CDCl₃): δ 8.4 (1H, s), 7.87 (2H, d), 7.63 (1H, m), 7.26 (1H, s), 7.12 (2H, d), 6.69 (1H, d), 5.23 (2H, s), 4.55-4.50 (2H, m), 3.38-3.28 (1H, m), 3.20-3.10 (2H, m), 3.04 (3H, s), 2.30-2.20 (2H, m), 1.90-1.80 (2H, m).

### Example 76

### 5-Ethyl-2-{4-[4-(2-fluoro-4-methanesulfonyl-phenoxymethyl)-thiazol-2-yl]-piperidin-1-yl} -pyrimidine

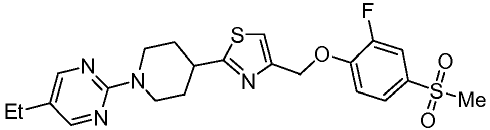

¹H NMR (CDCl₃): δ 8.18 (2H, s), 7.65-7.70 (2H, m), 7.21-7.26 (2H, m), 5.30 (2H, s), 4.81-4.84 (2H, m), 3.25~3.28 (1H, m), 3.03 (3H, s), 3.00-3.07 (2H, m), 2.44 (2H, q), 2.21 (2H, m), 1.77-1.81 (2H, m), 1.19 (3H, t).

### Example 77

### 5-Ethyl-2-{4-[4-(2-fluoro-4-tetrazol-1-yl-phenoxymethyl)-thiazol-2-yl]-piperidin-1-yl}-pyrimidine

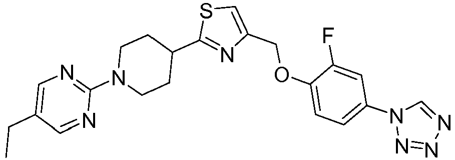

¹H NMR (CDCl₃): δ 8.96 (1H, s), 8.19 (2H, s), 7.55-7.25 (4H, m), 5.31 (2H, s), 4.82 (2H, m), 3.30 (1H, m), 3.04 (2H, m), 2.47 (2H, q), 2.23 (2H, m), 1.81 (2H, m), 1.20 (3H, t).

### Example 78

### 4-[4-(4-Methanesulfonyl-phenoxymethyl)-thiazol-2-yl]-4-methyl-piperidine-1-carboxylic acid tert-butyl ester

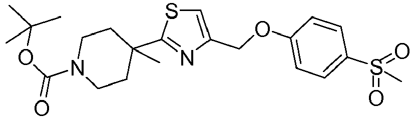

### Step1: 4-Cyano-4-methyl-piperidine-1-carboxylic acid tert-butyl ester

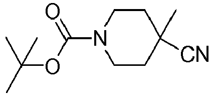

To a solution of 4-cyano-piperidine-1-carboxylic acid *tert*-butyl ester (4.52 g, 20 mmol) in THF (50 mL) was added LHMDS in THF (24 mL, 24 mmol) at 0°C. After stirring at 0°C for 1 hour, MeI (5.7 g) was added. The reaction mixture was kept at 0°C for 2 hours, then partitioned between EtOAc and H₂O. After concentration *in vacuo*, the residue was purified by silica column chromatography with EtOAc/hexanes to give the desired product.

### Step 2: 4-Carbamoyl-4-methyl-piperidine-1-carboxylic acid tert-butyl ester

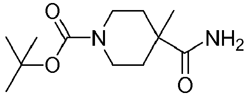

To a solution of 4-cyano-4-methyl-piperidine-1-carboxylic acid *tert*-butyl ester (2.24 g, 10 mmol) in methanol (25 mL) was added DMSO (1mL), aqueous 1N NaOH (12mL, 12 mmol) and H₂O₂ (4 mL) at room temperature. The mixture was heated at 50°C for 3 hours. After cooling to room temperature, the mixture was partitioned between EtOAc and H₂O. The organic layer was washed successively with H₂O and brine. After drying (Na₂SO₄), the solvent was removed to afford the desired product.

### Step 3: 4-Methyl-4-thiocarbamoyl-piperidine-1-carboxylic acid tert-butyl ester

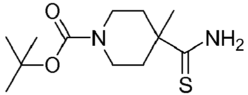

To a solution of 4-carbamoyl-4-methyl-piperidine-1-carboxylic acid *tert*-butyl ester (2.1 g, 8.7 mmol) in THF (30 mL) was added Lawesson's reagent (3.5 g, 8.7 mmol) at room temperature. The mixture was heated at 50°C for 3 hours. After cooling to room temperature, the solvent was removed *in vacuo* and the residue was partitioned between EtOAc and H₂O. The organic layer was washed with saturated NaHCO₃, and brine. After drying (Na₂SO₄), the solvent was removed *in vacuo*, and the residue was purified by silica column chromatography with EtOAc/hexanes to afford the desired product.

### Step 4: 4-(4-Ethoxycarbonyl-thiazol-2-yl)-4-methyl-piperidine-1-carboxylic acid tert-butyl ester

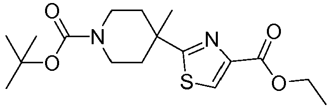

To a solution of 4-methyl-4-thiocarbamoyl-piperidine-1-carboxylic acid *tert*-butyl ester (1 g, 4 mmol) in EtOH (10 mL) was added ethyl bromopyruvate (0.78 g, 4 mmol) at room temperature. The mixture was heated to refluxing for 3 hours. After cooling to room temperature, the solvent was removed *in vacuo*. The residue was dissolved in methylene chloride (15 mL), Et₃N (1 mL) and di-*tert*-butyl dicarbonate (1.3 g) were added to the solution. The mixture was stirred at room temperature overnight. The mixture was washed with H₂O and brine. After drying (Na₂SO₄), the solvent was removed *in vacuo*, and the residue was purified by silica column chromatography with EtOAc/hexanes to afford the desired product.

### Step 5: 4-(4-Hydroxymethyl-thiazol-2-yl)-4-methyl-piperidine-1-carboxylic acid tert-butyl ester

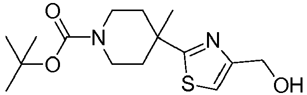

To a solution of 4-(4-ethoxycarbonyl-thiazol-2-yl)-4-methyl-piperidine-1-carboxylic acid *tert*-butyl ester (0.6 g, 1.7 mmol) in anhydrous THF (10 mL) was added LiAlH₄ (0.1 g, 2.6 mmol) at 0 °C. The mixture was kept at 0 °C for 2 hours and the reaction was quenched with EtOH. The solvent was evaporated and the residue was diluted with EtOAc, washed with 1N NaOH, brine. After drying (Na₂SO₄), the solvent was removed *in vacuo*, and the residue was purified by silica column chromatography with EtOAc/hexanes to afford the desired product.

### Step 6: 4-(4-Methanesulfonyloxymethyl-thiazol-2-yl)-4-methyl-piperidine-1-carboxylic acid tert-butyl ester

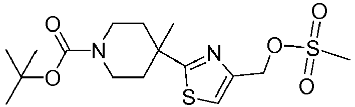

To a solution of 4-(4-hydroxymethyl-thiazol-2-yl)-4-methyl-piperidine-1-carboxylic acid *tert*-butyl ester (0.42 g, 1.3 mmol) in methylene chloride (10mL) was added methanesulfonyl chloride (0.19 g, 1.7 mmol) and triethylamine (0.2 g, 2 mmol) at 0 °C. After stirring at 0°C for 1 hour, the mixture was diluted with EtOAc and washed with H₂O and brine. After drying (Na₂SO₄), the solvent was removed *in vacuo*, and the residue was purified by silica column chromatography with EtOAc/hexanes to afford the desired product.

### Step 7: 4-[4-(4-Methanesulfonyl-phenoxymethyl)-thiazol-2-yl]-4-methyl-piperidine-1-carboxylic acid tert-butyl ester

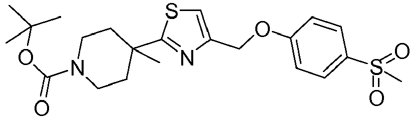

A mixture of 4-(4-methanesulfonyloxymethyl-thiazol-2-yl)-4-methyl-piperidine-1-carboxylic acid *tert*-butyl ester (0.2 g, 0.5 mmol), 4-methanesulfonyl-phenol (86 mg, 0.5 mmol) and Cs₂CO₃ (170 mg, 0.52 mmol) in acetonitrile (4 mL) was heated at 40 °C overnight. After cooling, the solid was filtered through a pad of celite. The filtrate was concentrated *in vacuo*. The residue was purified on silica gel (EtOAc-hexanes, 1:1) to afford the desired product. ¹H NMR (CDCl₃): δ 7.83 (2H, m), 7.23 (1H, s), 7.09 (2H, m), 5.2 (2H, s), 3.64-3.54 (2H, m), 3.3~3.24 (2H, m), 2.99 (3H, s), 2.2-2.1 (2H, m), 1.72-1.64(2H, m), 1.41 (9H, s), 1.36 (3H, s).

### Example 79

### 4-[4-(4-Methanesulfonyl-phenoxymethyl)-5-methyl-thiazol-2-yl]-piperidine-1-carboxylic acid tert-butyl ester

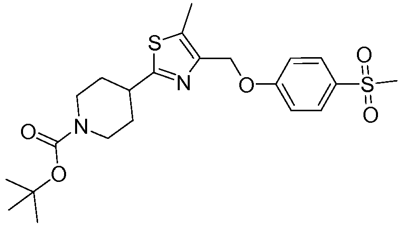

To a solution of 4-(4-hydroxymethyl-5-methyl-thiazol-2-yl)-piperidine-1-carboxylic acid *tert*-butyl ester (0.18g, 0.6 mmol), 4-methanesulfonyl-phenol (0.1 g, 0.6 mmol) and PPh₃ (0.19 g, 0.72mmol) in THF (5 mL) was added diethylazodicarboxylate (DEAD) (0.22 g, 0.72 mmol) at room temperature. The resulting mixture was stirred at room temperature for 30 minutes. The solvent was removed and the residue was purified by flash chromatography on silica gel to afford the desired product. ¹H NMR (CDCl₃): δ 7.9 (2H, d, *J =* 9 Hz), 7.09 (2H, d, *J =* 9 Hz), 5.2 (2H, s), 4.28-4.10 (2H, m), 3.14-3.04 (1H, m), 3.04 (3H, s), 2.9-2.8 (2H, m), 2.44 (3H, s), 2.1∼2 (2H, m), 1.76-1.64 (2H, m), 1.47 (9H, s).

### Example 80

### 4-{4-[1-(4-Methanesulfonyl-phenoxy)-ethyl]-5-methyl-thiazol-2-yl}-piperidine-1-carboxylic acid tert-butyl ester

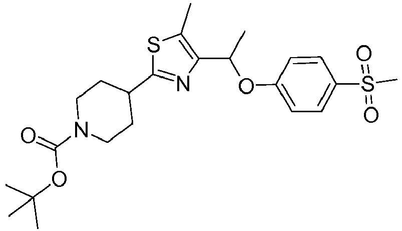

### Step 1: 4-[4-(1-Hydroxy-ethyl)-5-methyl-thiazol-2-yl]-piperidine-1-carboxylic acid tert-butyl ester

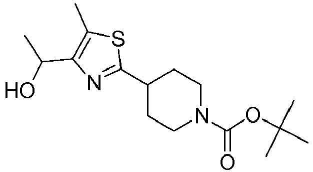

To a solution of 4-(4-formyl-5-methyl-thiazol-2-yl)-piperidine-1-carboxylic acid *tert*-butyl ester (0.31 g, 1 mmol) in THF (10 mL) was added MeMgI (1 mL, 3 mmol) in Et₂O at room temperature. The resulting mixture was stirred at room temperature for 1 hour. The reaction was quenched with saturated aqueous NH₄Cl and extracted with EtOAc. The organic layer was washed with H₂O and brine. After drying over Na₂SO₄, the solvent was removed. The residue was purified by flash chromatography on silica gel to afford the desired product.

### Step 2: 4-{4-[1-(4-Methanesulfbnyl-phenoxy)-ethyl]-5-methyl-thiazol-2-yl}-piperidine-1-carboxylic acid tert-butyl ester

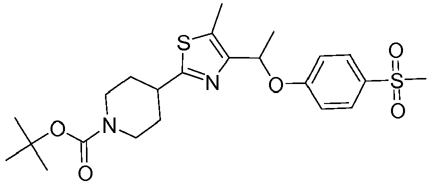

To a solution of 4-[4-(1-Hydroxy-ethyl)-5-methyl-thiazol-2-yl]-piperidine-1-carboxylic acid *tert*-butyl ester (0.15g, 0.46 mmol), 4-methanesulfonyl-phenol (0.08 g, 0.46 mmol) and PPh₃ (0.14 g, 0.55mmol) in THF (5 mL) was added DEAD (0.1 g, 0.55 mmol) at room temperature. The resulting mixture was stirred at room temperature for 30 minutes. The solvent was removed. The residue was purified by flash chromatography on silica gel to afford the desired product. ¹H NMR (CDCl₃): δ 7.79 (2H, m), 6.94 (2H, m), 5.59 (1H, q, J= 6 Hz), ), 4.2-4.04 (2H, m), 3.04-2.94 (1H, m), 2.98 (3H, s), 2.86-2.72 (2H, m), 2.39 (3H, s), 2.04-1.96 (2H, m), 1.67 (3H, *d, J=* 6 Hz), 1.66-1.58 (2H, m), 1.42 (9H, s).

### Example 81

### 4-[3-(4-Methanesulfonyl-phenoxymethyl)-[1,2,4]oxadiazol-5-yl]-piperidine-1-carboxylic acid tert-butyl ester

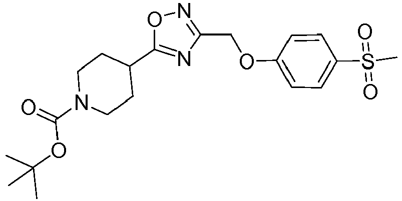

### Step 1: N-Hydroxy-2-(4-methanesulfonyl-phenoxy)-acetamidine

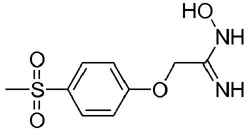

To a mixture of (4-methanesulfonyl-phenoxy)-acetonitrile (2 g, 9.5 mmol), K₂CO₃ (1.3 g, 9.5 mmol) in H₂O (30 mL) and EtOH (15 mL) was added hydroxylamine hydrogenchloride (1.32 g, 19 mmol). The mixture was heated under reflux overnight, cooled and ethanol was removed *in vacuo* and the residue was extracted with EtOAc (150 mL). The organic layer was washed successively with H₂O and brine. After drying (Na₂SO₄), the solvent was removed to afford the desired product.

### Step 2: 4-[3-(4-Methanesulfonyl-phenoxymethyl)-[1,2,4]oxadiazol-5-yl]-piperidine-1-carboxylic acid tert-butyl ester

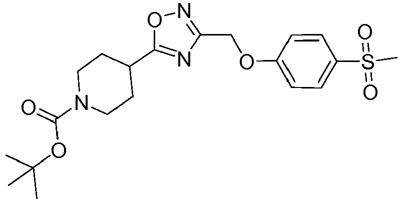

To a solution of piperidine-1,4-dicarboxylic acid mono-*tert*-butyl ester (2.06 g, 9 mmol), NEt₃ ( 1.2 g, 12 mmol) in toluene (150 mL) was added isobutylchloroformate (1.23g, 9 mmol) at 0°C. The mixture was stirred at room temperature for 1.5 hours. N-hydroxy-2-(4-methanesulfonyl-phenoxy)-acetamidine (1.5 g, 6 mmol) was added to the mixture. The mixture was heated under reflux overnight, cooled and the mixture was washed successively with H₂O and brine. After drying (Na₂SO₄), the solvent was removed. The residue was purified by flash chromatography on silica gel to afford the desired product. ¹H NMR (CDCl₃): δ 7.98 (2H, m), 7.14 (2H, m), 5.24 (2H, s), 4.2-4.05 (2H, m), 3.14 (1H, m), 3.03 (3H, s), 2.95 (2H, m), 2.12∼2.04 (2H, m), 1.80 (2H, m), 1.46 (9H, s).

### Example 82

### 4-[5-(4-Methanesulfonyl-phenoxymethyl)-[1,2,4]oxadiazol-3-yl]-piperidine-1-carboxylic acid tert-butyl ester

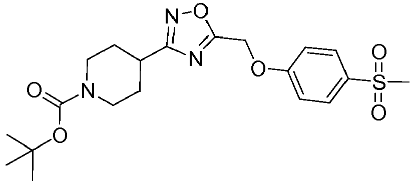

### Step 1: 4-(N-Hydroxycarbamimidoyl)-piperidine-1-carboxylic acid tert-butyl ester

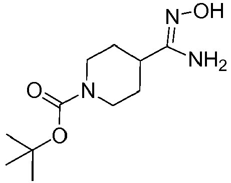

To a mixture of 4-cyano-piperidine-1-carboxylic acid *tert*-butyl ester (6.3 g, 30 mmol), K₂CO₃ (4.2 g, 30 mmol) in H₂O (50 mL) and EtOH (30 mL) was added hydroxylamine hydrogenchloride (4.17 g, 60 mmol). The mixture was heated under reflux overnight, cooled to room temperature and ethanol was removed *in vacuo*. The residue was extracted with EtOAc (300 mL). The organic layer was washed successively with H₂O and brine. After drying (Na₂SO₄), the solvent was removed to afford the desired product.

### Step 2: 4-(5-Hydroxymethyl-[1,2,4]oxadiazol-3-yl)-piperidine-1-carboxylic acid tert-butyl ester

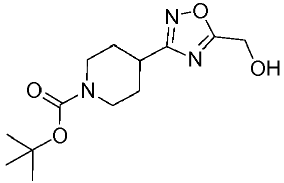

To a solution of hydroxy-acetic acid (1.67 g, 22 mmol), NEt₃ (4.4 g, 44 mmol) in toluene (150 mL) was added isobutylchloroformate (6 g, 44 mmol) at 0°C. The mixture was stirred at room temperature for 1.5 hours. 4-(N-Hydroxycarbamimidoyl)-piperidine-1-carboxylic acid *tert*-butyl ester (5.35 g, 22 mmol) was added to the mixture. The mixture was heated under reflux overnight, and then cooled to room temperature; the mixture was washed successively with H₂O and brine. After drying (Na₂SO₄), the solvent was removed. The residue was dissolved in THF (20 mL), and aqueous NaOH (10 mL, 10 mmol) was added. The mixture was stirred at room temperature for 2 hours and diluted with EtOAc (50 mL). The organic layer was washed with brine, after drying (Na₂SO₄), the solvent was removed *in vacuo*, and the residue was purified by silica column chromatography with EtOAc/hexanes to afford the desired product.

### Step 3: 4-(5-Methanesulfonyloxymethyl-[1,2,4]oxadiazol-3-yl)-piperidine-1-carboxylic acid tert-butyl ester

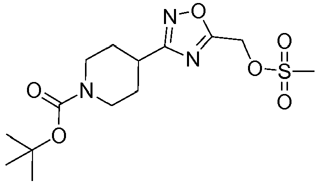

To a solution of 4-(5-hydroxymethyl-[1,2,4]oxadiazol-3-yl)-piperidine-1-carboxylic acid *tert*-butyl ester (0.2 g, 0.7 mmol) in methylene chloride (5 mL) was added methanesulfonyl chloride (0.1 g, 0.9 mmol) and triethyl amine (0.14 g, 1.4 mmol) at 0°C. After stirred at 0°C for 1 hour, the mixture was diluted with EtOAc and washed with H₂O, brine. After drying (Na₂SO₄), the solvent was removed *in vacuo*, and the residue was purified by silica column chromatography with EtOAc/hexanes to afford the desired product

### Step 4: 4-[5-(4-Methanesulfonyl-phenoxymethyl)-[1,2,4]oxadiazol-3-yl]-piperidine-1-carboxylic acid tert-butyl ester

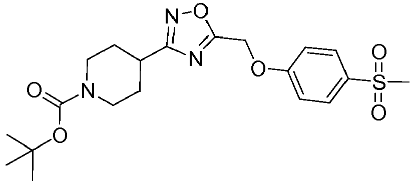

A mixture of 4-(5-methanesulfonyloxymethyl-[1,2,4]oxadiazol-3-yl)-piperidine-1-carboxylic acid *tert*-butyl ester (0.12 g, 0.33 mmol), 4-methanesulfonyl-phenol (86 mg, 0.5 mmol) and Cs₂CO₃ (0.33 g, 1 mmol) in acetonitrile (5 mL) was heated at 50°C for 2 hours. After cooling, the solid was filtered through a pad of celite. The filtrate was concentrated *in vacuo*. The residue was purified on silica gel (EtOAc-hexanes, 1:1) to afford the desired product. ¹H NMR (CDCl₃): δ 7.9 (2H, d, *J* = 8.8 Hz), 7.12 (2H, d, *J* = 8.8 Hz), 5.34 (2H, s), 4.2∼4.05 (2H, m), 3.03 (3H, s), 3.04∼2.85 (3H, m), 2.05∼1.96 (2H, m), 1.8∼1.7 (2H, m), 1.45 (9H, s).

### Example 83

### 4-(5-Benzyloxymethyl-[1,2,4]oxadiazol-3-yl)-piperidine-1-carboxylic acid tert-butyl ester

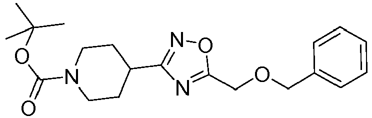

To a solution of benzyloxy-acetic acid (5 g, 30 mmol), NEt₃ (3.6 g, 36 mmol) in toluene (150 mL) was added isobutylchloroformate (4.1 g, 30 mmol) at 0°C. The mixture was stirred at room temperature for 1.5 hours. 4-(N-hydroxycarbamimidoyl)-piperidine-1-carboxylic acid *tert*-butyl ester (7.3 g, 30 mmol) was added to the mixture. The mixture was heated under reflux overnight, cooled and the mixture was washed successively with H₂O and brine. After drying (Na₂SO₄), the solvent was removed. The residue was purified by flash chromatography on silica gel to afford the desired product. ¹H NMR (CDCl₃): δ 7.4∼7.3 (5H, m), 4.7 (2H, s), 4.69 (2H, s), 4.2∼4.04 (2H, m), 3.02∼2.84 (3H, m), 2.04∼1.94 (2H, m), 1.84∼1.7 (2H, m), 1.46 (9H, s).

### Example 84

### 5-Ethyl-2-{4-[3-(4-methanesulfonyl-phenoxymethyl)-[1,2,4]oxadiazol-5-yl]-piperidin-1-yl}-pyrimidine

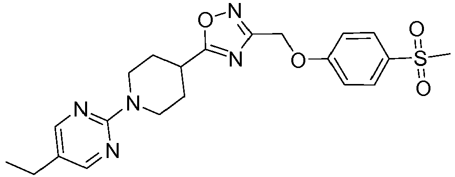

To the crude HCl salt (0.18 g, -0.5 mmol) of 4-[3-(4-methanesulfonyl-phenoxymethyl)-[1,2,4]oxadiazol-5-yl]-piperidine, prepared by treatment of 4-[3-(4-methanesulfonyl-phenoxymethyl)-[1,2,4]oxadiazol-5-yl]-piperidine-1-carboxylic acid *tert-*butyl ester **(Example 81)** in dixoane with 4N HCl, was added 2-propanol (3 mL), followed by DIPEA (0.13 g, 1 mmol) and 2-Chloro-5-ethyl-pyrimidine (0.14 g, 1 mmol). The resulting mixture was stirred at 70 °C overnight. Aafter concentration *in vacuo*, the residue was purified by silica column chromatography with EtOAc/hexanes to afford the desired product. ¹H NMR (CDCl₃): δ 8.18 (2H, s), 7.89 (2H, *d, J=* 8.8 Hz), 7.15 (2H, *d, J=* 8.8 Hz), 5.24 (2H, s), 4.75∼4.65 (2H, m), 3.3∼3.2 (1H, m), 3.2∼3.1 (2H, m), 3.03 (3H, s), 2.47 (2H, *q, J=* 7.6 Hz), 2.22∼2.16 (2H, m), 1.96∼1.84 (2H, m), 1.19 (3H, *t, J=* 7.6 Hz).

### Example 85

### 4-Hydroxy-4-[4-(4-methylsulfanyl-phenoxymethyl)-thiazol-2-yl]-piperidine-1-carboxylic acid tert-butyl ester

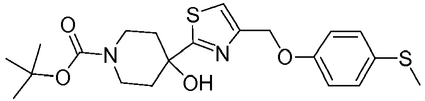

### Step 1: 4-(4-Methylsulfanyl-phenoxymethyl)-thiazole

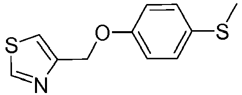

A mixture of 4-chloromethyl thiazole hydrochloride (3.0 g, 17.6 mmol), 4-methylsulfanyl-phenol (2.5 g, 1 eq.) and K₂CO₃ (6.1 g, 2.5 eq.) in acetone (60 mL) was heated to reflux for 48 hours. After cooling, the solid was filtered off. The filtrate was evaporated to dryness *in vacuo*. The crude product was redissolved in diethyl ether. The solution was washed twice with 2N NaOH solution and then with H₂O. After being dried over Na₂SO₄, removal of the solvent afforded the desired product as an off-white solid.

### Step 2: 4-Hydroxy-4-[4-(4-methylsulfanyl-phenoxymethyl)-thiazol-2-yl]-piperidine-1-carboxylic acid tert-butyl ester

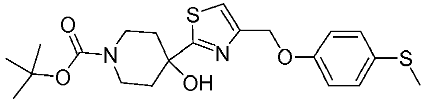

To a stirred solution of 4-(4-methanesulfanyl-phenoxymethyl)-thiazole (3.92 g, 16.5 mmol) in THF (40 mL) at -78 °C was added *n*-BuLi (1.73 mL, 1.05 eq., 10.0 M in hexanes). The resulting solution was stirred at this temperature for 30 minutes. Then a solution of 1-Boc-4-piperidone (3.30 g, 1 eq.) in THF (20 mL) was added in dropwise. The resulting mixture was stirred for 30 minutes. The reaction was quenched by addition of H₂O (5 mL). Most of the THF was removed *in vacuo*. The mixture was extracted with EtOAc. The organic layer was separated, washed with brine and dried over Na₂SO₄. After removal of the solvent, the crude product was purified on silica gel (EtOAc:hexanes = 2:3) to afford the desired product as a foam. ¹H NMR (CDCl₃): δ 7.27 (2H, d, *J* = 8.8 Hz), 7.26 (1H, s), 6.93 (2H, d, *J* = 8.8 Hz), 5.14 (2H, s), 4.02 (2H, br), 3.27 (2H, br), 2.97 (1H, br), 2.45 (3H, s), 2.11 (2H, m), 1.86 (2H, m), 1.48 (9H, s).

### Example 86

### 4-Hydroxy-4-[4-(4-methanesulfonyl-phenoxymethyl)-thiazol-2-yl]-piperidine-1-carboxylic acid tert-butyl ester

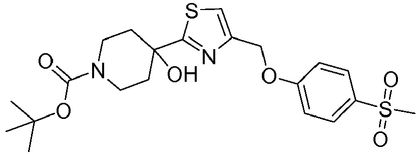

To a solution of 4-hydroxy-4-[4-(4-methylsulfanyl-phenoxymethyl)-thiazol-2-yl]-piperidine-1-carboxylic acid *tert*-butyl ester (**Example 85**, 6.8 g, 15.6 mmol) in CH₂Cl₂ (150 mL) at room temperature was added *m*-CPBA (8.4 g, 2.2 eq.) portionwise. The resulting solution was stirred for 30 minutes, then it was washed with 2 N NaOH solution twice and dried over Na₂SO₄. After removal of the solvent, the crude product was purified on silica gel (EtOAc:hexanes = 3:2) to afford the desired product as a white foam. ¹H NMR (CDCl₃): δ 7.88 (2H, d, *J* = 8.8 Hz), 7.31 (1H, s), 7.12 (2H, d, *J* = 8.8 Hz), 5.24 (2H, s), 4.03 (2H, br), 3.27 (2H, br), 3.04 (3H, s), 2.13 (2H, m), 1.86 (2H, m), 1.48 (9H, s).

### Example 87

### 4-Fluoro-4-[4-(4-methanesulfonyl-phenoxymethyl)-thiazol-2-yl]-piperidine-1-carboxylic acid tert-butyl ester

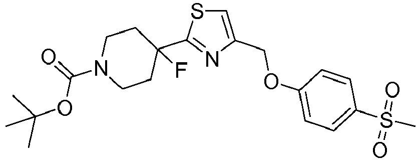

To a solution of 4-hydroxy-4-[4-(4-methanesulfonyl-phenoxymethyl)-thiazol-2-yl]-piperidine-1-carboxylic acid *tert*-butyl ester (**Example 86,** 5.29 g, 11.3 mmol) in CH₂Cl₂ (100 mL) at 0 °C was added DAST (1.8 mL, 1.2 eq.). The reaction mixture was stirred for 30 minutes before it was quenched by addition of saturated NaHCO₃ solution (20 mL). The organic phase was separated and dried over Na₂SO₄. After removal of the solvent, the crude product was purified on silica gel (EtOAc:hexanes = 2:3) to afford the desired product as a white solid. ¹H NMR (CDCl₃): δ 7.86 (2H, d, *J* = 9.2 Hz), 7.35 (1H, s), 7.10 (2H, d, *J* = 9.2 Hz), 5.22 (2H, s), 4.08 (2H, br), 3.19 (2H, br), 3.02 (3H, s), 2.05 ~ 2.32 (4H, m), 1.46 (9H, s).

### Example 88

### 5-Ethyl-2-{4-fluoro-4-[4-(4-methanesulfonyl-phenoxymethyl)-thiazol-2-yl]-piperidin-1-yl}-pyrimidine

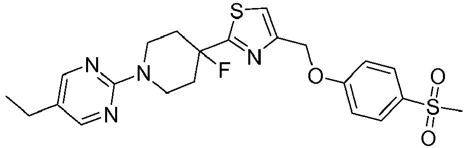

### Step 1: 4-Fluoro-4-[4-(4-methanesulfonyl-phenoxymethyl)-thiazol-2-yl]-piperidine hydrochloride

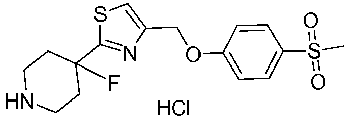

To a solution of 4-fluoro-4-[4-(4-methanesulfonyl-phenoxymethyl)-thiazol-2-yl]-piperidine-1-carboxylic acid *tert*-butyl ester (**Example 87**, 4.24 g, 9.01 mmol) in methanol (50 mL) was added 4 N HCl in dioxane (15 mL). The resulting solution was stirred overnight. The mixture was then evaporated to dryness *in vacuo* to afford the desired product as a white solid.

### Step 2: 5-Ethyl-2-{4-fluoro-4-[4-(4-methanesulfonyl-phenoxymethyl)-thiazol-2-yl]-piperidin-1-yl}-pyrimidine

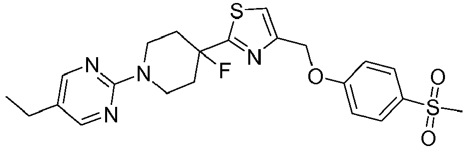

A solution of 4-fluoro-4-[4-(4-methanesulfonyl-phenoxymethyl)-thiazol-2-yl]-piperidine hydrochloride (4.0 g, 9.01 mmol), 2-chloro-5-ethyl-pyrimidine (1.55 g, 1.2 eq.) and DIPEA (4.7 g, 4 eq.) in 2-propanol (30 mL) in a sealed pressure vessel was stirred at 160 °C (oil bath temperature) overnight. After cooling, the solvent was removed *in vacuo*. The residue was partitioned between water and EtOAc. The organic phase was washed with brine and dried over Na₂SO₄. After removal of the solvent, the crude product was purified on silica gel (EtOAc:hexanes = 1:1) to afford the desired product as a white solid. ¹H NMR (CDCl₃): δ 8.19 (2H, s), 7.87 (2H, d, *J* = 9.2 Hz), 7.36 (1H, s), 7.10 (2H, d, *J* = 9.2 Hz), 5.23 (2H, s), 4.69 (2H, m), 3.44 (2H, m), 3.03 (3H, s), 2.48 (2H, q, *J* = 7.6 Hz), 2.15 ~ 2.39 (4H, m), 1.21 (3H, t, *J* = 7.6 Hz).

### Example 89

### 4-Fluoro-4-[5-(4-methanesulfonyl-phenoxymethyl)-thiazol-2-yl]-piperidine-1-carboxylic acid tert-butyl ester

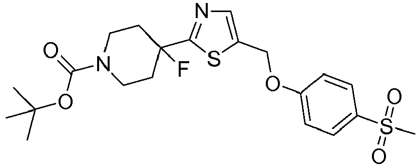

### Step 1: 4-Hydroxy-4-thiazol-2-yl-piperidine-1-carboxylic acid tert-butyl ester

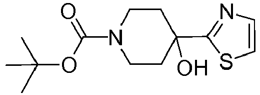

To a cooled (-78°C) and stirred solution of *n*-BuLi (2.6 mL, 1.05 eq., 10.0 M in hexanes) in dry Et₂O (20 mL) was added dropwise a solution of 2-bromothiazole (4.0 g, 24.4 mmol) in THF (10 mL) over a 10 minute period. After the yellow mixture had been stirred at -78°C for 30 minutes, a solution of 1-Boc-4-piperidone (4.9 g, 1 eq.) in THF (20 mL) was added slowly. The mixture was then continued to stir for another 30 minutes before the reaction was quenched by addition of water (5 mL). The mixture was warmed to room temperature and extracted with EtOAc. The organic phase was separated, washed with brine and dried over Na₂SO₄. After removal of the solvent, the crude product was purified on silica gel (45% EtOAc in hexanes) to afford the desired product as a thick oil.

### Step 2: 4-Fluoro-4-thiazol-2-yl-piperidine-1-carboxylic acid tert-butyl ester

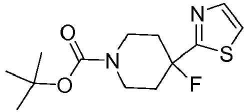

To a solution of 4-hydroxy-4-thiazol-2-yl-piperidine-1-carboxylic acid *tert*-butyl ester (4.36 g, 15.3 mmol) in CH₂Cl₂ (50 mL) at 0 °C was added DAST (2.4 mL, 1.2 eq.). The reaction mixture was stirred for 30 minutes before it was quenched by addition of saturated NaHCO₃ solution (20 mL). The organic phase was separated and dried over Na₂SO₄. After removal of the solvent, the crude product was purified on silica gel (EtOAc:hexanes = 1:4) to afford the desired product as a pale yellow oil.

### Step 3: 4-Fluoro-4-(5-hydroxymethyl-thiazol-2-yl)-piperidine-1-carboxylic acid tert-butyl ester

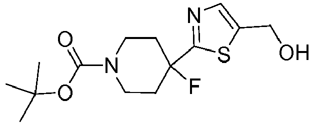

To a cooled (-78 °C) and stirred solution of 4-fluoro-4-thiazol-2-yl-piperidine-1-carboxylic acid *tert*-butyl ester (3.65 g, 12.7 mmol) in THF (20 mL) was added n-BuLi (1.33 mL, 1.05 eq., 10.0 M in hexanes). The mixture was stirred at this temperature for 30 minutes. Then a suspension of paraformaldehyde (383 mg, 1eq.) in THF (10 mL) was added in. The resulting mixture was continued to stir at -78 °C for another 30 minutes and gradually warmed to room temperature overnight. The reaction was quenched by addition of water (10 mL). The mixture was extracted with EtOAc. The organic phase was washed with brine and dried over Na₂SO₄. After removal of the solvent, the crude product was purified on silica gel (60% EtOAc in hexanes) to afford the desired product as a pale yellow solid.

### Step 4: 4-(5-Chloromethyl-thiazol-2-yl)-4-fluoro-piperidine-1-carboxylic acid tert-butyl ester

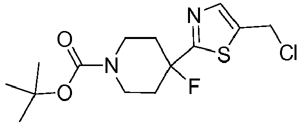

To a mixture of 4-fluoro-4-(5-hydroxymethyl-thiazol-2-yl)-piperidine-1-carboxylic acid *tert-butyl* ester (1.34 g, 4.24 mmol) and pyridine (426 mg, 1.3 eq.) in CH₂Cl₂ (30 mL) at 0 °C was added MsCl (631 mg, 1.3 eq.). The mixture was warmed to room temperature and stirred overnight. The reaction mixture was washed with saturated NaHCO₃ solution and dried over Na₂SO₄. Removal of the solvent afforded the desired product, which was used directly in the following reaction without further purification.

### Step 5: 4-Fluoro-4-[5-(4-methanesulfonyl-phenoxymethyl)-thiazol-2-yl]-piperidine-1-carboxylic acid tert-butyl ester

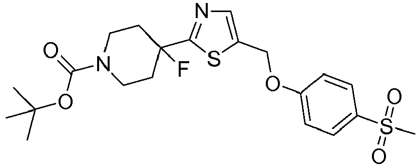

A mixture of 4-(5-Chloromethyl-thiazol-2-yl)-4-fluoro-piperidine-1-carboxylic acid *tert*-butyl ester (1.42 g, 4.24 mmol), 4-methanesulfonyl-phenol (731 mg, 1.0 eq.) and K₂CO₃ (878 mg, 1.5 eq.) in acetone (30 mL) was heated to reflux overnight. After cooling, the solid was filtered off through a pad of celite. The filtrate was concentrated *in vacuo*. The crude product was purified on silica gel (EtOAc:hexanes = 1:1) to afford the desired product as a white solid. ¹H NMR (CDCl₃): δ 7.86 (2H, d, *J* = 9.2 Hz), 7.35 (1H, s), 7.10 (2H, d, *J* = 9.2 Hz), 5.22 (2H, s), 4.08 (2H, br), 3.19 (2H, br), 3.02 (3H, s), 2.05 ~ 2.32 (4H, m), 1.46 (9H, s).

### Example 90

### 5-Ethyl-2-{4-fluoro-4-[5-(4-methanesulfonyl-phenoxymethyl)-thiazol-2-yl]-piperidin-1-yl}-pyrimidine

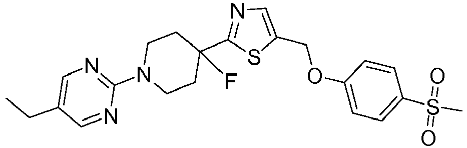

### Step 1: 4-Fluoro-4-[5-(4-methanesulfonyl-phenoxymethyl)-thiazol-2-yl]-piperidine hydrochloride

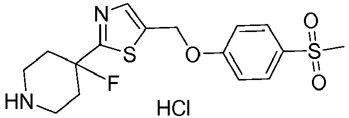

To a solution of 4-fluoro-4-[5-(4-methanesulfonyl-phenoxymethyl)-thiazol-2-yl]-piperidine-1-carboxylic acid *tert*-butyl ester (**Example 89,** 1.30 g, 2.76 mmol) in methanol (5 mL) was added 4 N HCl in dioxane (10 mL). The resulting solution was stirred overnight. The mixture was then evaporated to dryness *in vacuo* to afford the desired product as a white solid.

### Step 2: 5-Ethyl-2-{4-fluoro-4-[5-(4-methanesulfonyl-phenoxymethyl)-thiazol-2-yl]-piperidin-1-yl}-pyrimidine

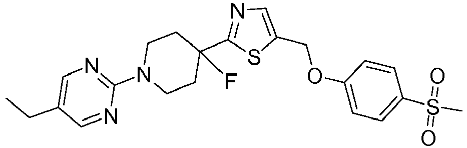

A solution of 4-fluoro-4-[5-(4-methanesulfonyl-phenoxymethyl)-thiazol-2-yl]-piperidine hydrochloride (1.2 g, 2.76 mmol), 2-chloro-5-ethyl-pyrimidine (425 mg, 1.1 eq.) and DIPEA (1.4 g, 4 eq.) in 2-propanol (20 mL) in a sealed pressure vessel was stirred at 160 °C (oil bath temperature) overnight. After cooling, the solvent was removed *in vacuo*. The residue was partitioned between water and EtOAc. The organic phase was washed with brine and dried over Na₂SO₄. After removal of the solvent, the crude product was purified on silica gel (EtOAc:hexanes = 1:1) to afford the desired product as a white solid. ¹H NMR (CDCl₃): δ 8.19 (2H, s), 7.90 (2H, d, *J* = 8.8 Hz), 7.73 (1H, d), 7.10 (2H, d, *J* = 8.8 Hz), 5.31 (2H, s), 4.67 (2H, m), 3.44 (2H, m), 3.04 (3H, s), 2.48 (2H, q, *J* = 7.6 Hz), 2.13 ~ 2.38 (4H, m), 1.20 (3H, t, *J* = 7.6 Hz).

### Example 91

### 4-[4-(4-Methanesulfonyl-phenoxymethyl)-thiazol-2-yl]-piperazine-1-carboxylic acid tert-butyl ester

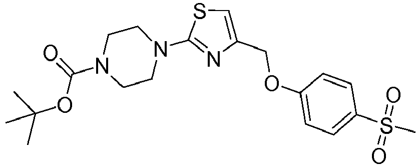

### Step 1: 4-(4-Ethoxycarbonyl-thiazol-2-yl)-piperazine-1-carboxylic acid tert-butyl ester

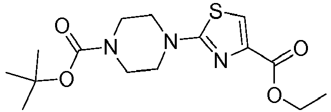

A mixture of 2-bromo-thiazole-4-carboxylic acid ethyl ester (1.4 g, 5.93 mmol), piperazine-1-carboxylic acid tert-butyl ester (1.16 g, 1.05 eq.) and DIPEA (1.15 g, 1.5 eq.) in 1,4-dioxane (20 mL) was heated to reflux overnight. After cooling, the solvent was removed *in vacuo.* The crude product was purified on silica gel (EtOAc:hexanes = 1:4) to afford the desired product as a pale yellow solid.

### Step 2: 4-(4-Hydroxymethyl-thiazol-2-yl)-piperazine-1-carboxylic acid tert-butyl ester

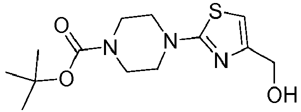

A solution of 4-(4-ethoxycarbonyl-thiazol-2-yl)-piperazine-1-carboxylic acid *tert-*butyl ester (1.15 g, 3.37 mmol) in THF (15 mL) at 0 °C was treated with LiAlH₄ (128 mg, 1 eq.). The mixture was stirred for 1 hour, then the reaction was quenched with 2 N NaOH solution. The solid was filtered off through a pad of celite and washed with EtOAc (100 mL). The filtrate was washed with water and dried over Na₂SO₄. Removal of the solvent afforded the desired product as an oil.

### Step 3: 4-(4-Chloromethyl-thiazol-2-yl)-piperazine-1-carboxylic acid tert-butyl ester

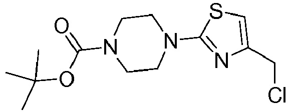

To a solution of 4-(4-hydroxymethyl-thiazol-2-yl)-piperazine-1-carboxylic acid *tert-*butyl ester (848 mg, 2.83 mmol) and DIPEA (550 mg, 1.5 eq.) in CH₂Cl₂ (10 mL) was added MsCl (285 L, 1.3 eq.) dropwise. The resulting mixture was stirred overnight. The reaction solution was then concentrated *in vacuo*. The crude product was purified on silica gel (EtOAc:hexanes = 1:4) to afford the desired product as an oil.

### Step 4: 4-[4-(4-Methanesulfonyl-phenoxymethyl)-thiazol-2-yl]-piperazine-1-carboxylic acid tert-butyl ester

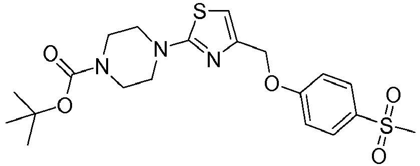

A mixture of 4-(4-Chloromethyl-thiazol-2-yl)-piperazine-1-carboxylic acid *tert-*butyl ester (700 m g, 2.20 mmol), 4-methanesulfonyl-phenol (417 mg, 1.1 eq.) and K₂CO₃ (609 mg, 2 eq.) in acetone (30 mL) was heated to reflux overnight. After cooling, the solid was filtered off through a pad of celite. The filtrate was concentrated *in vacuo*. The crude product was purified on silica gel (EtOAc:hexanes = 1:1) to afford the desired product as an off-white solid. ¹H NMR (CDCl₃): δ 7.87 (2H, d, *J* = 8.8 Hz), 7.12 (2H, d, *J* = 8.8 Hz), 6.59 (1H, s), 5.05 (2H, s), 3.56 (4H, m), 3.48 (4H, m), 3.04 (3H, s), 1.49 (9H, s).

### Example 92

### 1-[4-(4-Methanesulfonyl-phenoxymethyl)-thiazol-2-yl]-4-(2-methyl-propane-1-sulfonyl)-piperazine

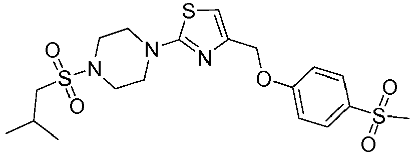

### Step 1: 1-[4-(4-Methanesulfonyl-phenoxymethyl)-thiazol-2-yl]-piperazine hydrochloride

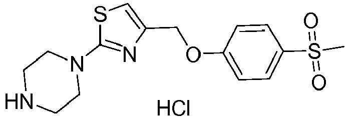

To a solution of 4-[4-(4-Methanesulfonyl-phenoxymethyl)-thiazol-2-yl]-piperazine-1-carboxylic acid *tert*-butyl ester (**Example 91,** 430 mg, 0.95 mmol) in methanol (5 mL) was added 4 N HCl in dioxane (5 mL). The resulting solution was stirred for 30 minutes at room temperature. The mixture was then evaporated to dryness *in vacuo* to afford the desired product as a pale yellow solid.

### Step 2: 1-[4-(4-Methanesulfonyl-phenoxymethyl)-thiazol-2-yl]-4-(2-methyl-propane-1-sulfonyl)-piperazine

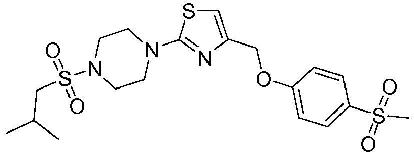

A solution of 1-[4-(4-Methanesulfonyl-phenoxymethyl)-thiazol-2-yl]-piperazine hydrochloride (100 mg, 0.26 mmol) and DIPEA (134 mL, 3 eq.) in CH₂Cl₂ (5 mL) was added isobutanesulfonyl chloride (41 mL, 1.2 eq.). The mixture was stirred for 1 hour, then the reaction solution was directly purified on silica gel (EtOAc:hexanes = 1:1) to afford the desired product as a pale yellow solid. ¹H NMR (CDCl₃): δ 7.87 (2H, d, *J* = 8.8 Hz), 7.12 (2H, d, *J* = 8.8 Hz), 6.62 (1H, s), 5.05 (2H, s), 3.61 (4H, m), 3.39 (4H, m), 3.04 (3H, s), 2.78 (2H, d, *J* = 6.8 Hz), 2.32 (1H, m), 1.12 (6H, d, *J* = 6.8 Hz).

### Example 93

### 4-[4-Methyl-5-(4-tetrazol-1-yl-phenoxymethyl)-thiazol-2-yl]-piperidine-1-carboxylic acid tert-butyl ester

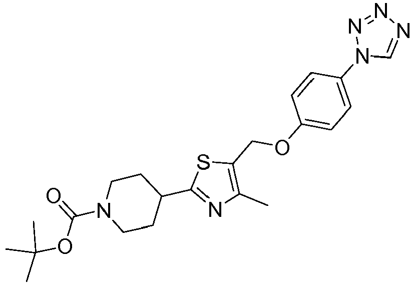

To a solution of 4-(5-Hydroxymethyl-4-methyl-thiazol-2-yl)-piperidine-1-carboxylic acid tert-butyl ester (1.00 g, 3.2 mmol) in THF (6.4 mL) was added, 4-tetrazol-1-yl-phenol (0.52 g, 3.2 mmol), polymer bound triphenylphosphine (3 mmol/g, 1.6 g). To this solution was added ditertierybutylazodicarboxylate (1.1 g, 4.8 mmol), stirred for 4 hours and filtered through a pad of celite. The filtrate was concentrated and purified by silica gel chromatography to provide the desired product. ¹H NMR (CDCl₃): δ 9.01 (1H, s), 7.66 (2H, d), 7.15 (2H, d), 5.21 (2H, s), 4.19 (2H, m), 3.10 (1H, m), 2.86 (2H, m), 2.45 (3H, s), 2.08 (2H, m), 1.72 (2H, m), 1.47 (9H, s).

### Example 94

### 4-{4-[(6-Fluoro-pyridin-3-ylamino)-methyl]-thiazol-2-yl}-piperidine-1-carboxylic acid tert-butyl ester

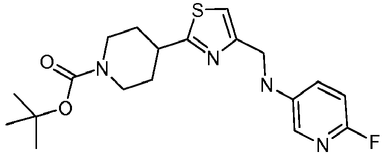

5-amino-2-fluoropyridine (0.476g, 4.2 mmol) was added to 4-(4-Formyl-thiazol-2-yl)-piperidine-1-carboxylic acid tert-butyl ester (0.84g, 2.8 mmol) in dry DCM (10 mL). Sodium triacetoxyborohydride (0.9g, 4.2 mmol) was then added. The reaction was stirred for 3 hours at room temperature under N₂. The organic layer was washed with 2M NaOH solution, water, brine, dried (MgSO₄), and the solvent was removed *in vacuo*. The material was purified by silica gel chromatography (DCM/methanol: 10:1 v/v) to give the desired product. ¹H NMR (CDCl₃): δ 7.59-7.60 (1H, m), 7.06-7.10 (1H, m), 7.02 (1H, s), 6.76 (1H, dd, *J* = 8.8, 3,6 Hz), 4.4 (2H, d), 4.20-4.31 (3H, m), 3.09-3.17 (1H, m), 2.8-2.95 (2H, m), 2.07-2.10 (2H, m), 1.77-1.47, (2H, m), 1.47 (9H, s).

### Example 95

### 1-(3-Isopropyl-[1,2,4]oxadiazol-5-yl)-4-[4-(4-tetrazol-1-yl-phenoxymethyl)-thiazol-2-yl]-piperidine

### Step 1: 4-[4-(4-Tetrazol-1-yl-phenoxymethyl)-thiazol-2-yl]-piperidine-1-carbonitrile

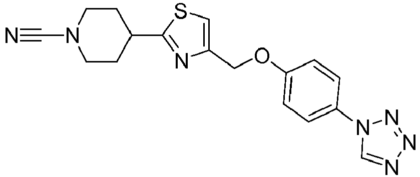

To a mixture of 4-[4-(4-Tetrazol-1-yl-phenoxymethyl)-thiazol-2-yl]-piperidine (1.00 g, 2.92 mmol) and potassium carbonate (1.5 g, 10.9 mmol) in chloroform (25 mL) was added cyanogen bromide (0.371 g, 3.5 mmol). The slurry was refluxed for 48 hours then stirred at room temperature for an additional 48 hours. The reaction was filtered through a pad of celite, concentrated and chromatographed on silica gel (1:1 Hexanes/EtOAc) to afford the desired compound.

### Step 2: 1-(3-Isopropyl-[1,2,4]oxadiazol-5-yl)-4-[4-(4-tetrazol-1-yl-phenoxymethyl)-thiazol-2-yl]-piperidine

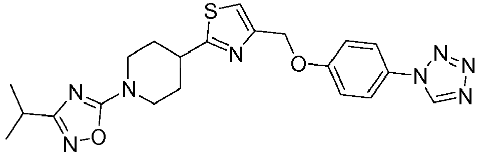

To a solution of 4-[4-(4-Tetrazol-1-yl-phenoxymethyl)-thiazol-2-yl]-piperidine-1-carbonitrile (0.450, 1.22 mmol) and N-hydroxy-isobutyramidine (0.150 g, 1.47 mmol) in dry THF (10 mL) was added a 1 M solution of zinc chloride in THF (1.47 mL, 1.47 mmol) over 15 min. The suspension was left to settle for 15 minutes and the white precipitate was collected by filtration and dissolved in 4N HCl in ethanol and water (1:1). The solution was refluxed for 1 hour, cooled and the solid precipitate was filtered off. The filtrate was neutralized by the addition of excess sodium carbonate. The excess was filtered off and the filtrate was diluted with EtOAc. The solution was washed with water, separated, dried (Na₂SO₄), filtered and concentrated. The residual oil was chromatographed on silica gel (1:1 Hex/EtOAc) to afford the desired compound. ¹H NMR (CDCl₃): δ 8.92 (1H, s), 7.62 (2H, d), 7.28 (1H, s), 7.19 (2H, d), 5.24 (2H, s), 4.26 (2H, m), 3.20 (3H, m), 2.89 (1H, m), 2.26 (2H, m), 1.92 (2H, m), 1.30 (6H, d).

The following three examples were syntheized in similar manner as **Example 95** using the required hydroxy amidine and 4-[4-(4-tetrazol-1-yl-phenoxymethyl)-thiazol-2-yl]-piperidine-1-carbonitrile.

### Example 96

### 1-(3-Ethyl-[1,2,4]oxadiazol-5-yl)-4-[4-(4-tetrazol-1-yl-phenoxymethyl)-thiazol-2-yl]-piperidine

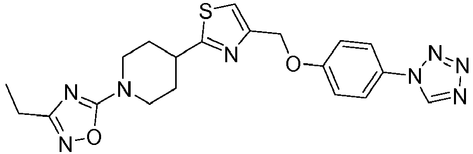

¹H NMR (CDCl₃): δ 8.85 (1H, s), 7.57 (2H, d), 7.28 (1H, s), 7.19 (2H, d), 5.17 (2H, s), 4.22 (2H, m), 3.22 (3H, m), 2.55 (2H, q), 2.17 (2H, m), 1.89 (2H, m), 1.35 (3H, t).

### Example 97

### 1-(3-Cyclopropyl-[1,2,4]oxadiazol-5-yl)-4-[4-(4-tetrazol-1-yl-phenoxymethyl)-thiazol-2-yl]-piperidine

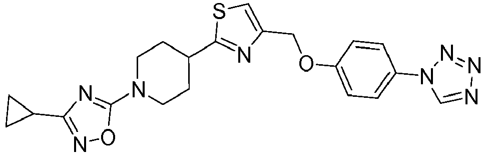

¹H NMR (CDCl₃): δ 8.90 (1H, s), 7.61 (2H, d), 7.27 (1H, s), 7.17 (2H, d), 5.23 (2H, s), 4.22 (2H, m), 3.22 (3H, m), 2.25 (2H, m), 1.88 (3H, m), 0.96 (4H, m).

### Example 98

### 4-[4-(4-Tetrazol-1-yl-phenoxymethyl)-thiazol-2-yl]-1-(3-trifluoromethyl-[1,2,4]oxadiazol-5-yl)-piperidine

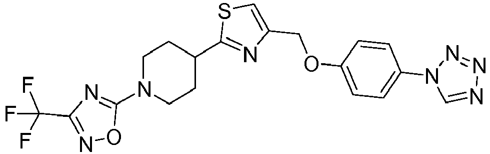

¹H NMR (CDCl₃): δ 8.92 (1H, s), 7.60 (2H, d), 7.23 (1H, s), 7.16 (2H, d), 5.21 (2H, s), 4.25 (2H, m), 4.15 (2H, m), 3.22 (1H, m), 2.90 (2H, m), 2.18 (2H, m).

### Example 99

### 4-[4-(4-Tetrazol-1-yl-phenoxymethyl)-thiazol-2-yl]-piperidine-1-carboxylic acid amide

### Step 1: 4-[4-(4-Tetrazol-1-yl-phenoxymethyl)-thiazol-2-yl]-piperidine-1-carbonitrile

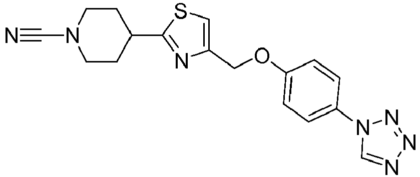

To a mixture of 4-[4-(4-Tetrazol-1-yl-phenoxymethyl)-thiazol-2-yl]-piperidine (1.00 g, 2.92 mmol) and potassium carbonate (1.5 g, 10.9 mmol) in chloroform (25 mL) was added cyanogen bromide (0.371 g, 3.5 mmol). The slurry was refluxed for 48 hours then stirred at room temperature for an additional 48 hours. The reaction was filtered through a pad of celite, concentrated and chromatographed on silica gel (1:1 Hexanes/EtOAc) to afford the desired compound.

### Step 2: 4-[4-(4-Tetrazol-1-yl-phenoxymethyl)-thiazol-2-yl]-piperidine-1-carboxylic acid amide

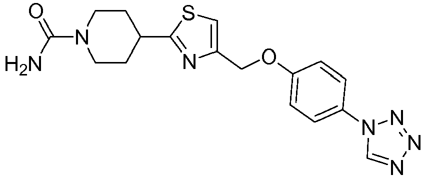

4-[4-(4-Tetrazol-1-yl-phenoxymethyl)-thiazol-2-yl]-piperidine-1-carbonitrile (1.07 g, 2.92 mmol) was dissolved in 4 N HCl in ethanol/water (1:1). The solution was refluxed for 1 hour, cooled and the solid precipitate was filtered off. The filtrate was neutralized by the addition of excess sodium carbonate. The excess sodium carbonate was filtered off and the filtrate was diluted with EtOAc. The solution was washed with water, separated, dried (Na₂SO₄), filtered and concentrated. The residual oil was chromatographed on silica gel (1:1 Hexanes/EtOAc) to afford the desired compound. ¹H NMR (CDCl₃): δ 8.92 (1H, s), 7.60 (2H, d), 7.23 (1H, s), 7.167 (2H, d), 5.21 (2H, s), 4.25 (2H, m), 4.15 (2H, m), 3.22 (1H, m), 2.90 (2H, m), 2.18 (2H, m).

### Example 100

### 4-[4-(4-Tetrazol-1-yl-phenoxymethyl)-thiazol-2-yl]-piperidine-1-carboxamidine

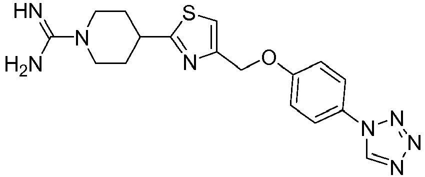

A mixture of 4-[4-(4-tetrazol-1-yl-phenoxymethyl)-thiazol-2-yl]-piperidine (300 mg, 0.876 mmol), pyrazole-1-carboxamidine hydrochloride (0.128 g, 0.876 mmol.) and triethylamine (0.122 mL, 0.876 mmol) in DMF (2 mL) was stirred at rt for 3 hours. The precipitate was collected by filtration and washed with ether to afford the expected product. ¹H NMR (DMSO-*d₆*): δ 10.02 (1H, s), 7.93 (1H, s), 7.82 (2H, m), 7.70 (1H, s), 7.60(2H, br), 7.28 (2H, m), 5.20 (2H, s), 3.95 (2H, m), 3.38 (1H, m), 3.15 (2H, m), 2.09 (2H, m), 1.66 (2H, m).

### Example 101

### 3-[4-(4-tetrazol-1-yl-phenoxymethyl)-thiazol-2-yl]-azetidine-1-carboxylic acid tert-butyl ester

### Step 1: 3-(4-chloromethyl-thiazol-2-yl)-azetidine-1-carboxylic acid tert-butyl ester

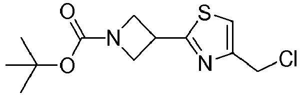

To a solution of 3-Thiocarbamoyl-azetidine-1-carboxylic acid tert-butyl ester (0.800 g, 3.7 mmol) in acetone (15 mL) was added 1,3-dichloroacetone (0.611 g, 4.81 mmol), MgSO₄ (0.67 g, 5.6 mmol) and MgCO₃ (3.12 g, 3.7 mmol). The mixture was heated under reflux overnight, cooled and filtered through celite. The solvent was removed *in vacuo* and the residue was redissolved with EtOAc (20 mL). The resulting solution was washed successively with 5% NaHSO₃, saturated NaHCO₃, and brine. After drying (Na₂SO₄), the solvent was removed to afford the desired product which was used without further purification.

### Step 2: 3-[4-(4-tetrazol-1-yl-phenoxymethyl)-thiazol-2-yl]-azetidine-1-carboxylic acid tert-butyl ester

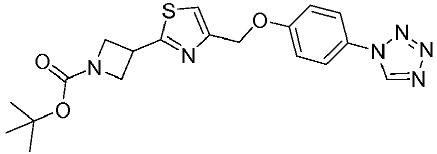

A mixture of 3-(4-chloromethyl-thiazol-2-yl)-azetidine-1-carboxylic acid tert-butyl ester (**From Step 1**) (386 mg, 1.34 mmol), 4-tetrazol-1-yl-phenol (217 mg, 1.34 mmol), Cs₂CO₃ (655 mg, 2.01 mmol) and KI (22 mg, 0.13 mmol) in acetonitrile (5 mL) was heated under reflux for 4 hours. After cooling, the solid was filtered through a pad of celite. The filtrate was concentrated *in vacuo.* The residue was purified on silica gel (EtOAc-hexanes, 1:1) to afford the desired product. ¹H NMR (CDCl₃): δ 8.92 (1H, s), 7.61 (2H, d), 7.32 (1H, s), 7.19 (2H, d), 5.25 (2H, s), 4.39 (2H, m), 4.18 (2H, m), 4.14 (1H, m), 1.46 (9H, s).

### Example 102

### 3-[4-(4-Tetrazol-1-yl-phenoxymethyl)-thiazol-2-yl]-pyrrolidine-1-carboxylic acid tert-butyl ester

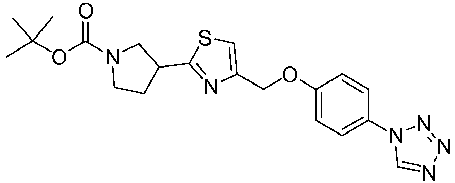

### Step 1: 3-(4-Chloromethyl-thiazol-2-yl)-pyrrolidine-1-carboxylic acid tert-butyl ester

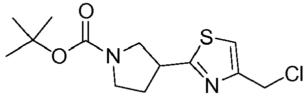

To a solution of 3-thiocarbamoyl-pyrrolidine-1-carboxylic acid tert-butyl ester (1.06 g, 4.60 mmol) in acetone (25 mL) was added 1,3-dichloroacetone (0.76 g, 5.98 mmol), MgSO₄ (0.83 g, 6.1 mmol) and MgCO₃ (3.87 g, 4.6 mmol). The mixture was heated under reflux overnight, cooled and filtered through celite. The solvent was removed *in vacuo* and the residue was redissolved with EtOAc (20 mL). The resulting solution was washed successively with 5% NaHSO₃, saturated NaHCO₃, and brine. After drying (Na₂SO₄), the solvent was removed to afford the desired product which was used without further purification.

### Step 2: 3-[4-(4-Tetrazol-1-yl-phenoxymethyl)-thiazol-2-yl]-pyrrolidine-1-carboxylic acid tert-butyl ester

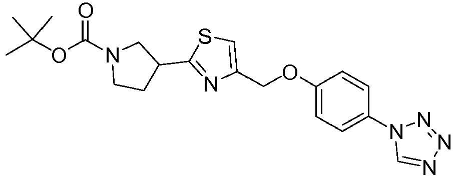

A mixture of 3-(4-Chloromethyl-thiazol-2-yl)-pyrrolidine-1-carboxylic acid tert-butyl ester (**From Step 1**) (775 mg, 2.56 mmol), 4-tetrazol-1-yl-phenol (415 mg, 2.56 mmol), CsCO₃ (1.25 mg, 3.84 mmol) and KI (44 mg, 0.26 mmol) in acetonitrile (20 mL) was heated under reflux overnight. After cooling, the solid was filtered through a pad of celite. The filtrate was concentrated *in vacuo*. The residue was purified on silica gel (EtOAc-hexanes, 1:1) to afford the desired product. ¹H NMR (CDCl₃): δ 8.92 (1H, s), 7.63 (2H, d), 7.27(1H, s), 7.17 (2H, d), 5.24 (2H, s), 3.87 (1H, m), 3.79 (1H, m), 3.65 (2H, m), 3.45 (1H, m), 2.40 (1H, m), 2.23 (1H, m), 1.47 (9H, s).

### Example 103

### 5-Ethyl-2-{3-[4-(4-tetrazol-1-yl-phenoxymethyl)-thiazol-2-yl]-pyrrolidin-1-yl}-pyrimidine Step 1: 1-[4-(2-Pyrrolidin-3-yl-thiazol-4-ylmethoxy)-phenyl]-1H-tetrazole

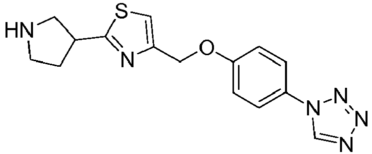

A solution of 3-[4-(4-Tetrazol-1-yl-phenoxymethyl)-thiazol-2-yl]-pyrrolidine-1-carboxylic acid tert-butyl ester (**from Example 102**) (411 mg, 0.959 mmol) in dichloromethane (10 mL) and methanol (2 mL) were treated with 1 mL of 4N HCl in dioxane. The resulting solution was stirred at room temperature for 30 minutes. The solvents were removed *in vacuo* to afford the desired product as an HCl salt.

### Step 2: 5-Ethyl-2- {3-[4-(4-tetrazol-1-yl-phenoxymethyl)-thiazol-2-yl]-pyrrolidin-1-yl}-pyrimidine

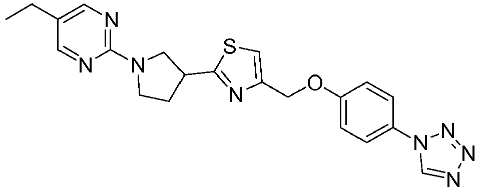

A mixture of 1-[4-(2-Pyrrolidin-3-yl-thiazol-4-ylmethoxy)-phenyl]-1H-tetrazole hydrochloride **(From Step 1)** (350 mg, 0.959 mmol), 2-chloropyrimidine (0.23 mL, 2.0 eq.) and K₂CO₃ (398 mg, 2.88 mmol) in DMF (5 mL) was heated at 90 °C for 4 hours. Water was added and the solution was extracted with ethyl acetate, separated, dried over sodium sulfate, filtered and concentrated. The residue was purified on silica gel (50:50 EtOAc/hexanes) to afford the desired product. ¹H NMR (CDCl₃): δ 8.91 (1H, s), 8.21 (2H, s), 7.62 (2H, d), 7.27(1H, s), 7.17 (2H, d), 5.24 (2H, s), 4.12 (1H, m), 3.98 (1H, m), 3.87 (2H, m), 3.69 (1H, m), 2.56 (1H, m), 2.47 (2H, m), 2.37 (1H, m), 1.21 (3H, t).

### Example 104

### 3-[4-(4-Tetrazol-1-yl-phenoxymethyl)-thiazol-2-yl]-piperidine-1-carboxylic acid tert-butyl ester

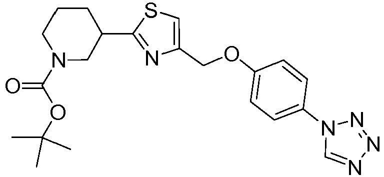

### Step 1: 3-(4-Chloromethyl-thiazol-2-yl)-piperidine-1-carboxylic acid tert-butyl ester

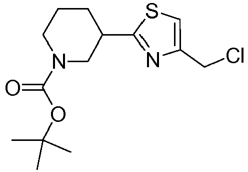

To a solution of 3-Thiocarbamoyl-piperidine-1-carboxylic acid tert-butyl ester (2.2 g, 9.02 mmol) in acetone (45 mL) was added 1,3-dichloroacetone (1.49 g, 11.7 mmol), MgSO₄ (1.63 g, 13.5 mmol) and MgCO₃ (0.76 g, 9.02 mmol). The mixture was heated under reflux overnight, cooled and filtered through celite. The solvent was removed *in vacuo* and the residue was redissolved with EtOAc (20 mL). The resulting solution was washed successively with 5% NaHSO₃, saturated NaHCO₃, and brine. After drying (Na₂SO₄), the solvent was removed to afford the desired product which was used without further purification.

### Step 2: 3-[4-(4-Tetrazol-1-yl-phenoxymethyl)-thiazol-2-yl]-piperidine-1-carboxylic acid tert-butyl ester

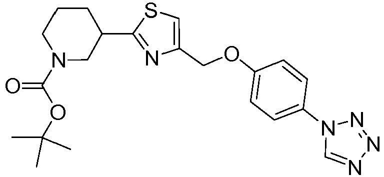

A mixture of 3-(4-Chloromethyl-thiazol-2-yl)-piperidine-1-carboxylic acid tert-butyl ester **(From Step 1)** (300 mg, 0.946 mmol), 4-tetrazol-1-yl-phenol (155 mg, 0.946 mmol), CsCO₃ (467 mg, 1.42 mmol) and KI (16 mg, 0.095 mmol) in acetonitrile (10 mL) was heated under reflux for 4 hours. After cooling, the solid was filtered through a pad of celite. The filtrate was concentrated *in vacuo.* The residue was purified on silica gel (EtOAc-hexanes, 1:1) to afford the desired product. ¹H NMR (CDCl₃): δ 8.91 (1H, s), 7.63 (2H, d), 7.26(1H, s), 7.17 (2H, d), 5.24 (2H, s), 4.30 (1H, br), 4.02 (1H, m), 3.20 (1H, m), 3.10 (1H, br), 2.88 (1H, t), 2.21(1H, m), 1.77 (2H, m), 1.61 (1H, m), 1.47 (9H, s).

### Example 105

### 5-Ethyl-2-{3-[4-(4-tetrazol-1-yl-phenoxymethyl)-thiazol-2-yl]-piperidin-1-yl}-pyrimidine Step 1: 3-[4-(4-Tetrazol-1-yl-phenoxymethyl)-thiazol-2-yl]-piperidine

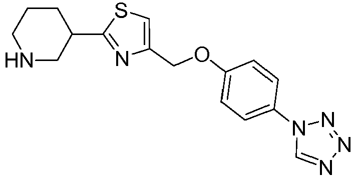

A solution of 3-[4-(4-Tetrazol-1-yl-phenoxymethyl)-thiazol-2-yl]-piperidine-1-carboxylic acid tert-butyl ester (500 mg, 1.13 mmol) in dichloromethane (10 mL) and methanol (2 mL) were treated with 2 mL of 4N HCl in dioxane. The resulting solution was stirred at room temperature for 30 minutes. The solvents were removed *in vacuo* to afford the desired product as an HCl salt.

### Step 2: 5-Ethyl-2- {3-[4-(4-tetrazol-1-yl-phenoxymethyl)-thiazol-2-yl]-piperidin-1-yl}-pyrimidine

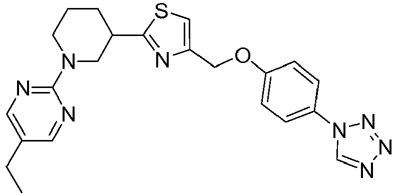

A mixture of 3-[4-(4-Tetrazol-1-yl-phenoxymethyl)-thiazol-2-yl]-piperidine hydrochloride (150 mg, 0.407 mmol), 2-chloropyrimidine (0.074 mL, 2.0 eq.) and NaHCO₃ (171 mg, 2.03 mmol) in DMF (5 mL) was heated at 90 °C for 4 hours. Water was added and the solution was extracted with ethyl acetate, separated, dried over sodium sulfate, filtered and concentrated. The residue was purified on silica gel (50:50 EtOAc/hexanes) to afford the desired product. ¹H NMR (CDCl₃): δ 8.91 (1H, s), 8.19 (2H, s), 7.63 (2H, m), 7.26(1H, s), 7.17 (2H, m), 5.25 (2H, s), 4.97 (1H, m), 4.62 (1H, m), 3.25 (2H, m), 3.07 (1H, m), 2.46 (2H, q), 2.28(1H, m), 1.88 (2H, m), 1.68 (1H, m), 1.20 (3H, t).

### Example 106

### 4-[4-(4-Methanesulfonyl-benzyloxymethyl)-thiazol-2-yl]-piperidine-1-carboxylic acid tert-butyl ester

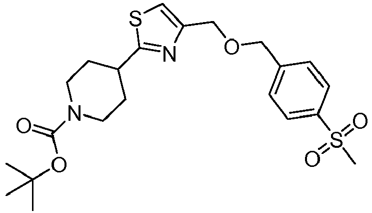

Hydroxybenzyl-4-methylsulfone (1.7eq.) was dissolved in anhydrous DMF (10mL), cooled to 0 °C and NaH (2eq.) was added in one portion. The reaction was allowed to stir at 0 °C for 30 minutes and at room temperature for an additional 30 minutes. 4-(4-Chloromethyl-thiazol-2-yl)-piperidine-1-carboxylic acid *tert*-butyl ester **(Intermediate 1)** (0.632mmol) was added and the reaction was stirred overnight. The reaction was quenched with water and extracted with EtOAc, dried over sodium sulfate, filtered and concentrated under reduced pressure. The residue was purified by silica gel chromatography (EtOAc/hexanes 1:1) to afford the desired product. ¹H NMR (CDCl₃): δ 7.92 (2H, d, *J* = 8.8 Hz), 7.57 (2H, d, *J* = 8.8 Hz), 7.14 (1H, s), 4.71 (2H, s), 4.66 (2H, s), 4.19 (2H, m), 3.13 (1H, m), 3.05 (3H, s), 2.86 (2H, m), 2.09 (2H, m), 1.72 (2H, m), 1.45 (9H, s).

### Example 107

### 2-{4-[4-(4-Tetrazol-1-yl-phenoxymethyl)-thiazol-2-yl]-piperidin-1-yl}-pyrimidin-5-ylamine

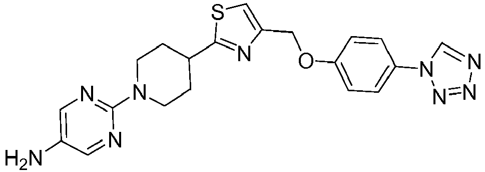

5-Nitro-2-{4-[4-(4-tetrazol-1-yl-phenoxymethyl)-thiazol-2-yl]-piperidin-1-yl}-pyrimidine **(Example 192)** (1.07mmol), ammonium chloride(3eq.) and iron powder(3eq.) were suspended in EtOH:THF:H2O(40:20:10) and heated at100 °C for 5hours. The hot reaction mixture was filtered through a pad of celite and the filtrate was concentrated. The resulting oil was dissolved in DMF and water and extracted with ethylacetate. The organic layer was washed with water, brine and dried over sodium sulfate. The resulting filtrate was concentrated under reduced pressure. Purification using silica gel chromatography (DCM/MeOH 98:2) provided the expected product. ¹H NMR (DMSO-*d₆*): δ 9.96 (1H, s), 7.97 (2H, m), 7.90 (2H, m), 7.63 (1H, s), 5.19 (2H, s), 4.44 (2H, m), 3.73 (1H, m), 2.97 (2H, m), 2.20 (2H, m), 1.95 (2H, m).

### Example 108

### N-(2-{4-[4-(4-Tetrazol-1-yl-phenoxymethyl)-thiazol-2-yl]-piperidin-1-yl}-pyrimidin-5-yl)-acetamide

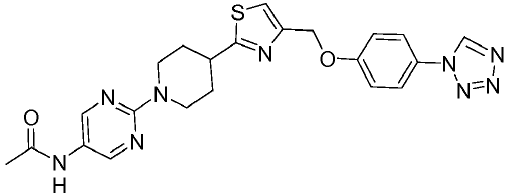

2-{4-[4-(4-Tetrazol-1-yl-phenoxymethyl)-thiazol-2-yl]-piperidin-1-yl}-pyrimidin-5-ylamine **(Example 107)** (0.321mmol) was dissolved in DCM and triethylamine (2eq.) was added. The reaction was cooled to 0 °C, acetylchloride (1eq.) was added dropwise and the reaction was stirred at room temperature overnight. Water was added and the mixture was extracted with ethyl acetate, dried over sodium sulfate, filtered and concentrated under reduced pressure. Silica gel chromatography of the resulting oil (DCM/MeOH) provided the expected product. ¹H NMR (CDCl₃): δ 8.84 (1H, s), 8.36 (2H, s), 7.55 (2H, m), 7.19 (1H, s), 7.11 (2H, m), 6.94 (1H, s), 5.16 (2H, s), 4.77 (2H, m), 3.25 (1H, m), 3.01 (2H, m), 2.16 (2H, m), 2.15 (3H, s), 1.75 (2H, m).

### Example 109

### 4-[4-(4-Tetrazol-1-yl-phenylcarbamoyl)-thiazol-2-yl]-piperidine-1-carboxylic acid tert-butyl ester

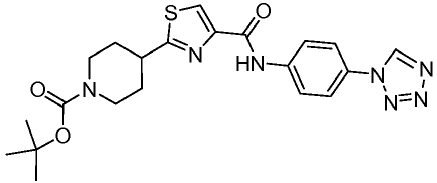

4-(4-Carboxy-thiazol-2-yl)-piperidine-1-carboxylic acid tert-butyl ester (1.28 mmol) was dissolved in anhydrous DMF (20mL). To the solution was added triethylamine (4eq.) and O-(Benzotriazol-1-yl)-*N,N,N',N'*-tetramethyluronium tetrafluoroborate (TBTU) (1.5eq.). The reaction was allowed to stir at room temperature for 5minutes before 4-tetrazol-1-yl-phenylamine (1.2eq.) was added. The reaction was stirred overnight, quenched with water, extracted with ethylacetate, washed with brine, dried over sodium sulfate and filtered. The organic filtrate was concentrated *in vacuo* and the residual oil was purified by column crhromatography (EtOAC/Hex) furnishing the expected product. ¹H NMR (CDCl₃): δ9.37 (1H, s), 9.02 (1H, s), 8.14 (1H, s), 7.96 (2H, d), 7.72 (2H, d), 4.23 (2H, m), 3.20 (1H, m), 2.91 (2H, m), 2.14 (2H, m), 1.79 (2H, m), 1.45 (9H, s).

### Example 110

### 4-[4-(4-Trifluoromethanesulfonyl-phenoxymethyl)-thiazol-2-yl]-piperidine-1-carboxylic acid tert-butyl ester

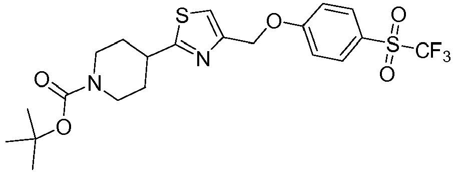

To a solution of [4-(4-Trifluoromethanesulfanyl-phenoxymethyl)-thiazol-2-yl]-piperidine-1-carboxylic acid tert-butyl ester **(Example 134)** (1.12mmol) in DCM (20mL) at room temperature was added 3-chloro-benzenecarboperoxoic acid (2eq.). The reaction was allowed to stir for 1.5 hours and an additional portion of 3-chloro-benzenecarboperoxoic acid (1eq.) was added to the reaction mixture. The reaction was stirred at room temperature for an additional 4 hours. The organic solution was washed with sodium bicarbonate, the organic layer was isolated, dried over sodium sulfate and filtered. The filtrate was concentrated and the crude product was purified by column chromatography to afford both the expected sulfone and sulfoxide products. Sulfone: ¹H NMR (DMSO-*d₆*): δ 8.05 (2H, d, *J =* 8.6 Hz), 7.70 (1H, s), 7.44 (2H, d, *J =* 8.6 Hz), 5.32 (2H, s), 3.98 (2H, m), 3.19 (1H, m), 2.86 (2H, m), 2.02 (2H, m), 1.56 (2H, m), 1.38 (9H, s).

### Example 111

4-[4-(4-Trifluoromethanesulfinyl-phenoxymethyl)-thiazol-2-yl]-piperidine-1-carboxylic acid tert-butyl ester

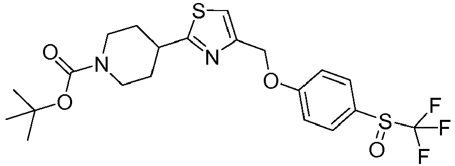

This compound was isolated from the reaction mixture of the previous example. ¹H NMR (DMSO-*d₆*): δ 8.02 (2H, d, *J* = 8.6 Hz), 7.75 (1H, s), 7.32 (2H, *d, J* = 8.6 Hz), 5.31 (2H, s), 3.96 (2H, m), 3.20 (1H, m), 2.85 (2H, m), 2.02 (2H, m), 1.50 (2H, m), 1.38 (9H, s).

**Example 112-145** were synthesized from 4-(4-Chloromethyl-thiazol-2-yl)-piperidine-1-carboxylic acid *tert*-butyl ester **(Intermediate 1),** 2-[4-(4-Chloromethyl-thiazol-2-yl)-piperidin-1-yl]-5-ethyl-pyrimidine **(Intermediate 2)** or 4-(4-Chloromethyl-oxazol-2-yl)-piperidine-1-carboxylic acid tert-butyl ester **(Intermediate 14)** with the corresponding phenol, thiophenol, amine or aniline in a similar manner to that described in **Example 1**. One skilled in the art of organic synthesis will appreciate that conditions such as solvent (such as DMF, CH₃CN); temperature, base (such as NEt₃, K₂CO₃, NaHCO₃, Na₂CO₃, Cs₂CO₃) and concentration can be selected through routine experimentation to optimize yields. Additionally, alternative coupling methods can be used that are well known in the art of organic synthesis.

### Example 112

### 4-[4-(2,6-Difluoro-4-tetrazol-1-yl-phenoxymethyl)-thiazol-2-yl]-piperidine-1-carboxylic acid tert-butyl ester

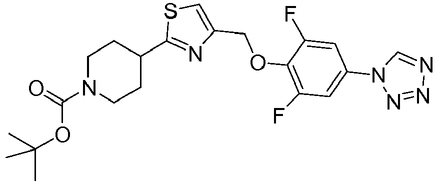

¹H NMR (CDCl₃): δ 8.98 (1H, s), 7.34 (2H, m), 7.30 (1H, s), 5.36 (2H, s), 4.19 (2H, m), 3.15 (1H, m), 2.87 (2H, m), 2.07 (2H, m), 1.70 (2H, m), 1.47 (9H, s).

### Example 113

### 4-[4-(4-Pyrrol-1-yl-phenoxymethyl)-thiazol-2-yl]-piperidine-1-carboxylic acid tert-butyl ester

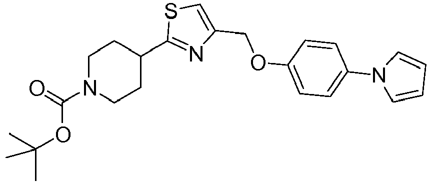

¹H NMR (CDCl₃): δ 7.24 (3H, m), 7.01 (4H, m), 6.31 (2H, m), 5.17 (2H, s), 4.21 (2H, m), 3.14 (1H, m), 2.87 (2H, m), 2.01 (2H, m), 1.74 (2H, m), 1.47 (9H, s).

### Example 114

### 4- {4-[(4-Tetrazol-1-yl-phenylamino)-methyl]-thiazol-2-yl}-piperidine-1-carboxylic acid tert-butyl ester

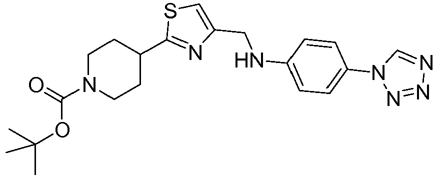

¹H NMR (CDCl₃): δ 8.85 (1H, s), 7.40 (2H, m), 7.01 (1H, s), 6.72 (2H, m), 4.76 (1H, s), 4.44 (2H, s), 4.15 (2H, m), 3.08 (1H, m), 2.83 (2H, m), 2.04 (2H, m), 1.66 (2H, m), 1.43 (9H, s).

### Example 115

### 2- {4-[4-(3-Chloro-4-tetrazol-1-yl-phenoxymethyl)-thiazol-2-yl]-piperidin-1-yl}-5-ethyl-pyrimidine

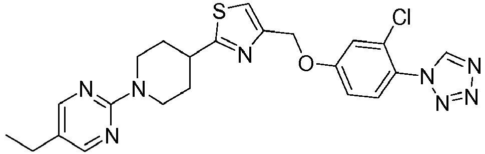

¹H NMR (CDCl₃):δ 8.93 (1H, s), 8.18 (2H, s), 7.48 (1H, m), 7.25 (1H, s), 7.08 (2H, m), 5.22 (2H, s), 4.82 (2H, m), 3.29 (1H, m), 3.04 (2H, m), 2.46 (2H, q), 2.21 (2H, m), 1.80 (2H, m), 1.18 (3H, t).

### Example 116

### N-(4- {2-[1-(5-Ethyl-pyrimidin-2-yl)-piperidin-4-yl]-thiazol-4-ylmethoxy}-phenyl)-formamide

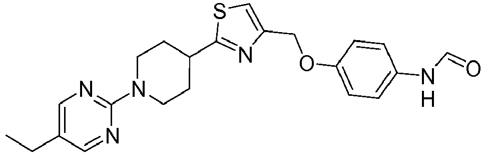

¹H NMR (CDCl₃): δ 8.55-8.30 (1H, m), 8.18 (2H, s), 7.50-6.90 (6H, m), 5.14 (2H, s), 4.83 (2H, m), 3.29 (1H, m), 3.03 (2H, m), 2.46 (2H, q), 2.20 (2H, m), 1.80 (2H, m), 1.19 (3H, t).

### Example 117

### N-(4- {2-[1-(5-Ethyl-pyrimidin-2-yl)-piperidin-4-yl]-thiazol-4-ylmethoxy}-phenyl)-methanesulfonamide

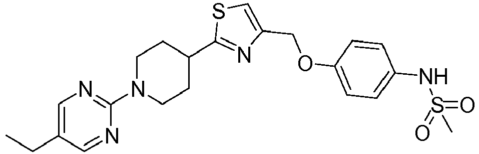

¹H NMR (CDCl₃): δ 8.20 (s, 2H), 7.21 (m, 3H), 6.95 (m, 2H), 5.13 (s, 2H), 4.81 (m, 2H), 3.29 (m, 1H), 3.06 (m, 2H), 2.94 (s, 3H), 2.47 (q, 2H), 2.20 (m, 2H), 1.81 (m, 2H), 1.19 (t, 3H).

### Example 118

### 4-[4-(2-Methyl-4-tetrazol-1-yl-phenoxymethyl)-thiazol-2-yl]-piperidine-1-carboxylic acid tert-butyl ester

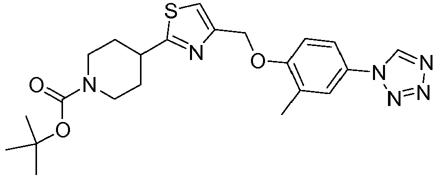

¹H NMR (CDCl₃): δ 8.89 (1H, s), 7.48 (1H, s), 7.43 (1H, m), 7.25 (1H, m), 7.05 (1H, m), 5.27 (2H, s), 4.27 (2H, m), 3.18 (1H, m), 2.89 (2H, m), 2.37 (3H, s), 2.21 (2H, m), 1.74 (2H, m), 1.47 (9H, s).

### Example 119

### 5-Ethyl-2-{4-[4-(4-tetrazol-1-yl-2-trifluoromethyl-phenoxymethyl)-thiazol-2-yl]-piperidin-1-yl}-pyrimidine

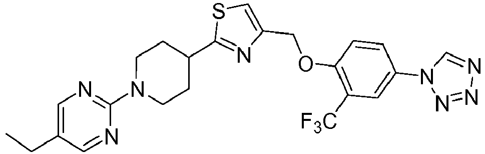

¹H NMR (CDCl₃): δ 8.97 (1H, s), 8.18 (2H, s), 7.92 (1H, m), 7.84 (1H, m), 7.33 (1H, m), 7.26 (1H, s), 5.38 (2H, s), 4.81 (2H, m), 3.27 (1H, m), 3.05 (2H, m), 2.46 (2H, q), 2.19 (2H, m), 1.79 (2H, m), 1.19 (3H, t).

### Example 120

### 2- {4-[4-(2-Chloro-4-tetrazol-1-yl-phenoxymethyl)-thiazol-2-yl]-piperidin-1-yl}-5-ethyl-pyrimidine

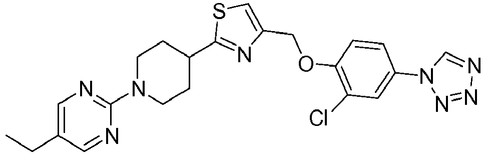

¹H NMR (acetone-*d₆*), δ 9.68 (1H, s), 8.24 (2H, s), 8.01 (1H, s), 7.86 (1H, m), 7.60 (1H, m), 7.59 (1H, s), 5.40 (2H, s), 4.82 (2H, m), 3.36 (1H, m), 3.08 (2H, m), 2.48 (2H, q), 2.17 (2H, m), 1.75 (2H, m), 1.18 (3H, t).

### Example 121

### 4-[4-(4-Tetrazol-1-yl-phenoxymethyl)-oxazol-2-yl]-piperidine-1-carboxylic acid tert-butyl ester

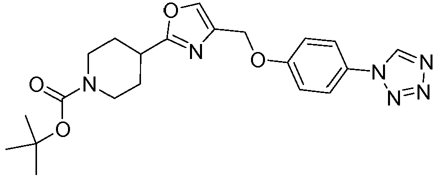

¹H NMR (CDCl₃): δ 8.94 (1H, s), 7.65 (1H, s), 7.60 (2H, m), 7.13 (2H, m), 5.01 (2H, s), 4.08 (2H, m), 2.94 (3H, m), 2.03 (2H, m), 1.75 (2H, m), 1.43 (9H, s).

### Example 122

### 4-[4-(2-Fluoro-4-tetrazol-1-yl-phenoxymethyl)-oxazol-2-yl]-piperidine-1-carboxylic acid tert-butyl ester

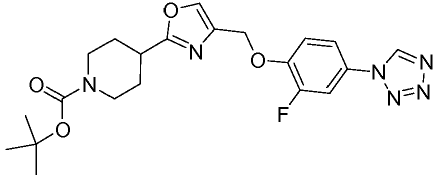

¹H NMR (CDCl₃): δ 8.88 (1H, s), 7.62 (1H, s), 7.45 (1H, m), 7.36 (1H, m), 7.23 (1H, m), 5.05 (2H, s), 4.04 (2H, m), 2.85 (3H, m), 1.97 (2H, m), 1.71 (2H, m), 1.40 (9H, s).

### Example 123

### 5-Ethyl-2- {4-[4-(4-methanesulfonyl-phenoxymethyl)-oxazol-2-yl]-piperidin-1-yl}-pyrimidine

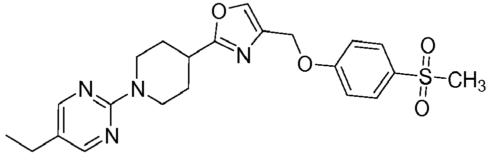

¹H NMR (CDCl₃): δ 8.16 (2H, s), 7.84 (2H, m), 7.63 (1H, s), 7.08 (2H, m), 5.02 (2H, s), 4.67 (2H, m), 3.08 (3H, m), 3.01 (3H, s), 2.44 (2H, q), 2.12 (2H, m), 1.84 (2H, m), 1.17 (3H, t).

### Example 124

### 4-[4-(2,6-Difluoro-4-propionyl-phenoxymethyl)-thiazol-2-yl]-piperidine-1-carboxylic acid tert-butyl ester

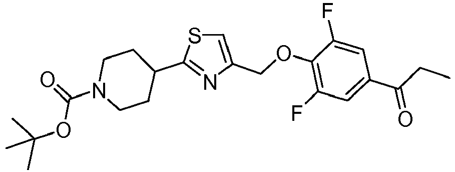

¹H NMR (CDCl₃): δ 7.51 (2H, d), 7.27 (1H, s), 5.37 (2H, s), 4.18 (2H, m), 3.14 (1H, m), 2.92 (2H, q, *J* = 7.4 Hz), 2.88 (2H, m), 2.07 (2H, m), 1.71 (2H, m), 1.47 (9H, s), 1.21 (3H, t, *J* = 7.4 Hz).

### Example 125

### 4-[4-(4-Acetyl-2-fluoro-phenoxymethyl)-thiazol-2-yl]-piperidine-1-carboxylic acid tert-butyl ester

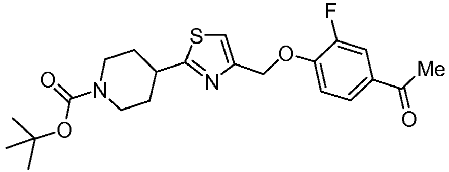

¹H NMR (CDCl₃): δ 7.70~7.72 (2H, m), 7.28 (1H, s), 7.09~7.13 (1H, m), 5.30 (2H, s), 4.20 (2H, m), 3.17 (1H, m), 2.88 (2H, m), 2.55 (3H, s), 2.10 (2H, m), 1.72 (2H, m), 1.47 (9H, s).

### Example 126

### 4-[4-(4-Cyano-2-fluoro-phenoxymethyl)-thiazol-2-yl]-piperidine-1-carboxylic acid tert-butyl ester

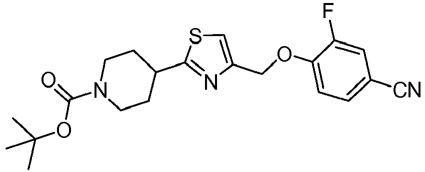

¹H NMR (CDCl₃): δ 7.37∼7.42 (2H, m), 7.27 (1H, s), 7.13∼7.17 (1H, m), 5.28 (2H, s), 4.20 (2H, m), 3.15 (1H, m), 2.89 (2H, m), 2.09 (2H, m), 1.72 (2H, m), 1.47 (9H, s).

### Example 127

### 4-[4-(6-Tetrazol-1-yl-pyridin-3-yloxymethyl)-thiazol-2-yl]-piperidine-1-carboxylic acid tert-butyl ester

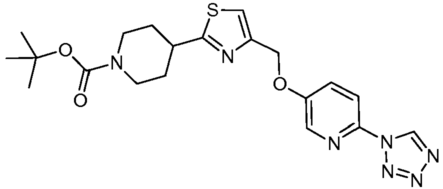

¹H NMR (CDCl₃): δ 9.41 (1H, s), 8.27 (1H, d), 8.01 (1H, d,), 7.58 (1H, dd,), 7.28 (1H, s), 5.27 (2H, s), 4.20 (2H, m), 3.14-3.20 (1H, m), 2.87 (2H, m), 2.09-2.12 (2H, m), 1.68-1.78 (2H, m), 1.46 (9H, s)

### Example 128

### 4-[4-(4-[1,2,3]Triazol-1-yl-phenoxymethyl)-thiazol-2-yl]-piperidine-1-carboxylic acid tert-butyl ester

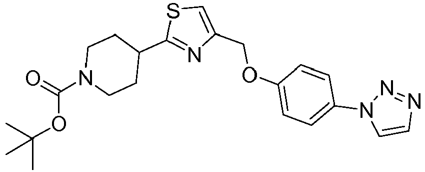

¹H NMR (CDCl₃): δ 7.92 (1H, s), 7.84 (1H, s), 7.65 (2H, d), 7.25 (1H, s), 7.11 (2H, d), 5.22 (2H, s), 4.21 (2H, br), 3.18 (1H, m), 2.88 (2H, br), 2.12 (2H, m), 1.75 (2H, m), 1.47 (9H, s).

### Example 129

### 4-[4-(4-Ethoxycarbonyl-phenoxymethyl)-thiazol-2-yl]-piperidine-1-carboxylic acid tert-butyl ester

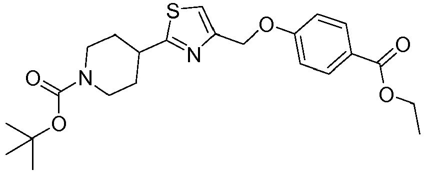

¹H NMR (CDCl₃): δ 8.01 (2H, d), 7.23 (1H, s), 7.01 (2H, d), 5.22 (2H, s), 4.36 (2H, q), 4.22 (2H, br), 3.17 (1H, m), 2.87 (2H, br), 2.12 (2H, m), 1.75 (2H, m), 1.47 (9H, s), 1.39 (2H, t).

### Example 130

### 4-[4-(4-tert-Butoxycarbonylamino-phenoxymethyl)-thiazol-2-yl]-piperidine-1-carboxylic acid tert-butyl ester

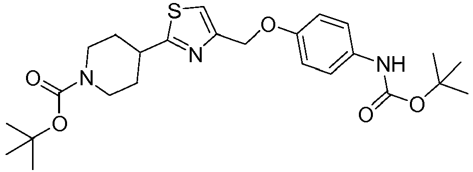

¹H NMR (CDCl₃): δ 7.28 (2H, d), 7.19 (1H, s), 6.92 (2H, d), 6.40 (1H, s), 5.12 (2H, s), 4.22 (2H, br), 3.17 (1H, m), 2.87 (2H, br), 2.12 (2H, m), 1.75 (2H, m), 1.50 (9H, s), 1.47 (9H, s).

### Example 131

### 4-[4-(4-Carboxy-phenoxymethyl)-thiazol-2-yl]-piperidine-1-carboxylic acid tert-butyl ester

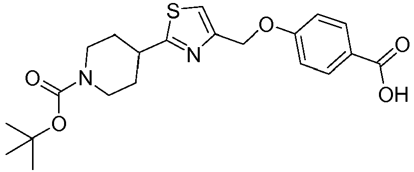

¹H NMR (DMSO-*d₆*): δ 7.86 (2H, d), 7.64 (1H, s), 7.10 (2H, d), 5.17 (2H, s), 3.96 (2H, m), 3.18 (1H, m), 2.87 (2H, br), 1.96 (2H, m), 1.49 (2H, m), 1.38 (9H, s).

### Example 132

### 4-[4-(2,6-Difluoro-4-methanesulfonyl-phenoxymethyl)-thiazol-2-yl]-piperidine-1-carboxylic acid tert-butyl ester

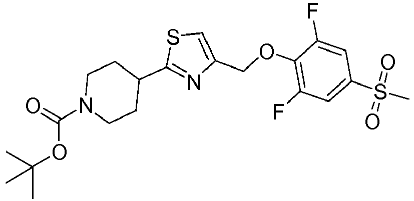

¹H NMR (CDCl₃): δ 7.42 (2H, d), 7.21 (1H, s), 5.25 (2H, s), 4.12 (2H, br), 3.17 (1H, m), 3.00 (3H, s), 2.87 (2H, br), 1.98 (2H, m), 1.71 (2H, m).

### Example 133

### 4-[4-(4-Morpholin-4-yl-phenoxymethyl)-thiazol-2-yl]-piperidine-1-carboxylic acid tert-butyl ester

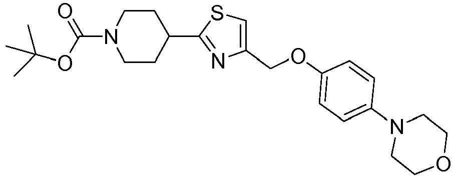

¹H NMR (CDCl₃): δ 7.19 (1H, s), 6.92 (4H, m), 5.12 (2H, s), 4.20 (2H, br), 3.85 (4H, br), 3.16 (1H, m), 3.07 (4H, m), 2.86 (2H, m), 2.10 (2H, m), 1.72 (2H, m), 1.47 (9H, s).

### Example 134

### 4-[4-(4-Trifluoromethylsulfanyl-phenoxymethyl)-thiazol-2-yl]-piperidine-1-carboxylic acid tert-butyl ester

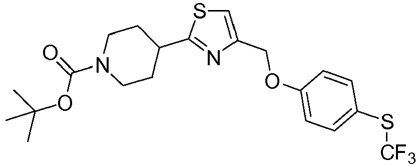

¹H NMR (DMSO-*d₆*): δ 7.64 (1H, s), 7.63 (2H, d, *J* = 8.6 Hz), 7.17 (2H, d, *J* = 8.6 Hz), 5.17 (2H, s), 3.99 (2H, m), 3.18 (1H, m), 2.83 (2H, m), 2.01 (2H, m), 1.52 (2H, m), 1.38 (9H, s).

### Example 135

### 4-[4-(4-Benzyloxy-phenoxymethyl)-thiazol-2-yl]-piperidine-1-carboxylic acid tert-butyl ester

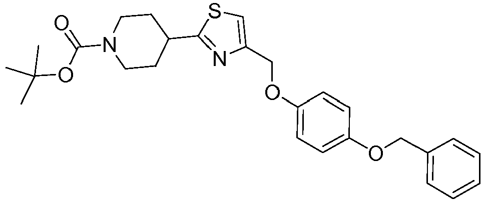

¹H NMR (DMSO-*d₆*): δ 7.55 (1H, s), 7.41 (5H, m), 6.92 (4H, m), 5.12 (4H, s), 3.98 (2H, m), 3.20 (1H, m), 2.84 (2H, m), 2.01 (2H, m), 1.52 (2H, m), 1.38 (9H, s).

### Example 136

### 4-[4-(2-Acetylamino-4-methanesulfonyl-phenoxymethyl)-thiazol-2-yl]-piperidine-1-carboxylic acid tert-butyl ester

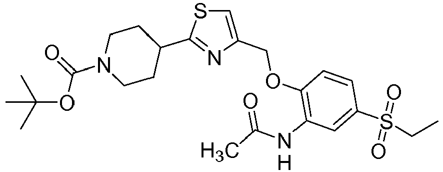

¹H NMR (CDCl₃): δ 8.81 (1H, s), 7.97 (1H, s), 7.53 (1H, d), 7.25 (1H, s), 7.09 (1H, d), 5.24 (2H, s), 4.16 (2H, m), 3.10 (3H, m), 2.83 (2H, m), 2.16 (3H, s), 2.04 (2H, d), 1.66 (2H, m), 1.40 (9H, s), 1.19(3H, t).

### Example 137

### 4-(4-Phenoxymethyl-thiazol-2-yl)-piperidine-1-carboxylic acid tert-butyl ester

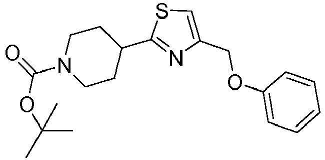

¹H NMR (CDCl₃): δ 7.28 (2H, m), 7.19 (1H, s), 6.93 (3H, m), 5.14 (2H, s), 4.19 (2H, s), 3.15 (1H, m), 2.85 (2H, m), 2.07 (2H, d), 1.67 (2H, m), 1.45 (9H, s).

### Example 138

### 4-{4-[(4-Methanesulfonyl-phenylamino)-methyl]-thiazol-2-yl}-piperidine-1-carboxylic acid tert-butyl ester

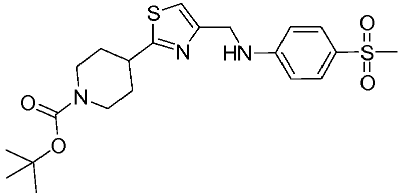

¹H NMR (CDCl₃): δ 7.67 (2H, d, *J* = 8.8 Hz), 6.99 (1H, s), 6.67 (2H, d, *J* = 8.8 Hz), 5.07 (1H, m), 4.45 (2H, d), 4.18 (2H, s), 3.13 (1H, m), 2.97 (3H, s), 2.85 (2H, m), 2.04 (2H, d), 1.68 (2H, m), 1.44 (9H, s).

### Example 139

### 4-{4-[(2-Fluoro-4-methanesulfonyl-phenylamino)-methyl]-thiazol-2-yl}-piperidine-1-carboxylic acid isopropyl ester

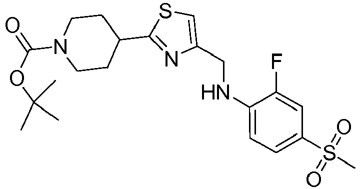

¹H NMR (CDCl₃): δ 7.55 (2H, m), 7.05 (1H, s), 6.76 (1H, m), 5.12 (1H, m), 4.52 (2H, d), 4.19 (2H, m), 3.13 (1H, m), 3.05 (3H, s), 2.86 (2H, m), 2.10 (2H, m), 1.76 (2H, m), 1.46 (9H, s).

### Example 140

### 4-[4-(4-Bromo-phenoxymethyl)-thiazol-2-yl]-piperidine-1-carboxylic acid tert-butyl ester

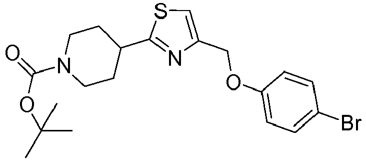

¹H NMR (CDCl₃): δ 7.36 (2H, m), 7.17 (1H, s), 6.82 (2H, m), 5.10 (2H, s), 4.18 (2H, s), 3.13 (1H, m), 2.85 (2H, m), 2.09 (2H, d), 1.75 (2H, m), 1.43 (9H, s).

### Example 141

### {2-[1-(5-Ethyl-pyrimidin-2-yl)-piperidin-4-yl]-thiazol-4-ylmethyl}-(2-fluoro-4-methanesulfonyl-phenyl)-amine

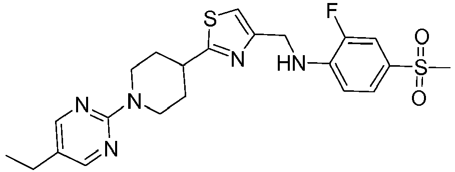

¹H NMR (CDCl₃):δ 8.16 (2H, s), 7.52 (2H, m), 7.01 (1H, s), 6.74 (1H, m), 5.15 (1H, m), 4.83 (2H, m), 4.51 (2H, d), 3.26 (1H, m), 3.02 (5H, m), 2.46 (2H, m), 2.19 (2H, m), 1.78 (2H, m), 1.19 (3H, t).

### Example 142

### 4-{4-[(4-Methanesulfonyl-benzylamino)-methyl]-thiazol-2-yl}-piperidine-1-carboxylic acid tert-butyl ester

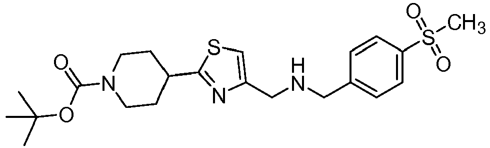

¹H NMR (CDCl₃): δ 7.85 (2H, d, *J* = 8.8 Hz), 7.53 (2H, d, *J* = 8.8 Hz), 6.95 (1H, s), 4.14 (2H, s), 3.87 (2H, s), 3.83 (2H, s), 3.11 (1H, m), 3.04 (3H, s), 2.86 (2H, m), 2.07 (3H, m), 1.67 (2H, m), 1.42 (9H, s).

### Example 143

### 4-(4-{[1-(4-Methanesulfonyl-phenyl)-ethylamino]-methyl}-thiazol-2-yl)-piperidine-1-carboxylic acid tert-butyl ester

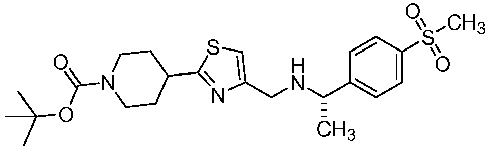

¹H NMR (CDCl₃): δ 7.87 (2H, d, *J* = 8.8 Hz), 7.56 (2H, d, *J* = 8.8 Hz), 6.87 (1H, s), 4.22 (2H, m), 3.90 (1H, s), 3.66 (2H, m), 3.09 (1H, m), 3.04 (3H, s), 2.82 (3H, m), 2.02 (2H, m), 1.71 (2H, m), 1.40 (9H, s), 1.29 (3H, d).

### Example 144

### 3-Methyl-4-[4-(4-tetrazol-1-yl-phenoxymethyl)-thiazol-2-yl]-piperidine-1-carboxylic acid tert-butyl ester

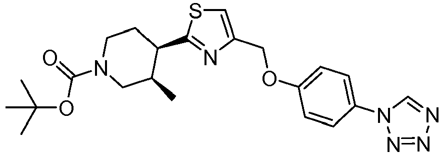

¹H NMR (CDCl₃): δ 8.93 (1H, s), 7.61 (2H, m), 7.25 (1H, m), 7.12 (2H, m), 5.22 (2H, m), 4.2 (1H, m), 3.95 (1H, m), 3.33 (1H, m), 3.13 (1H, m), 2.8 (1H, m), 2.34 (1H, m), 2.04 (1H, m), 1.89 (1H, m), 1.45 (9H, s), 0.85 (3H, m).

### Example 145

### 4-[4-(2-Fluoro-4-tetrazol-1-yl-phenoxymethyl)-thiazol-2-yl]-3-methyl-piperidine-1-carboxylic acid tert-butyl ester

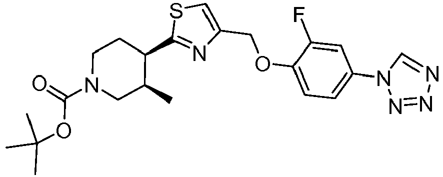

¹H NMR (CDCl₃): δ 9.07 (1H, s), 7.51 (1H, m), 7.41 (1H, m), 7.23 (2H, m), 5.25 (2H, s), 4.16 (1H,m), 3.88 (1H, m), 3.34 (1H, m), 3.09 (1H, m), 2.8 (1H, m), 2.26 (1H, m), 1.96 (1H, m), 1.83 (1H, m), 1.39 (9H, s), 0.76 (3H, m).

**Examples 146-157** were synthesized from one of **Intermediates 3-13** or **Intermediates 15-25** with the corresponding sulfonyl chloride, alkyl chloride, alkyl bromide, chloroformate, acid chloride, carbamyl chloride or isocyanate in a manner similar to that described in **Example 22**. One skilled in the art of organic synthesis will appreciate that conditions such as solvent (*e.g.,* DMF, CH₃CN); temperature, base (*e.g.,* NEt₃, K₂CO₃, NaHCO₃, Na₂CO₃, Cs₂CO₃) and concentration can be selected through routine experimentation to optimize yields. Additionally, alternative coupling methods can be used that are well known in the art of organic synthesis.

### Example 146

### 4-[4-(2-Fluoro-4-tetrazol-1-yl-phenoxymethyl)-thiazol-2-yl]-piperidine-1-carboxylic acid allyl ester

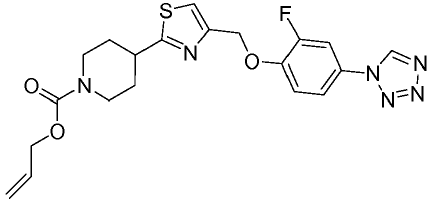

¹H NMR (CDCl₃), δ 9.00 (1H, s), 7.54 (1H, m), 7.45 (1H, m), 7.29 (2H, m), 5.95 (1H, m), 5.30 (3H, m), 5.22 (1H, m), 4.61 (2H, m), 4.28 (2H, m), 3.20 (1H, m), 2.98 (2H, m), 2.14 (2H, m), 1.78 (2H, m).

### Example 147

### 4-[4-(4-Tetrazol-1-yl-phenoxymethyl)-thiazol-2-yl]-piperidine-1-carboxylic acid cyclohexyl ester

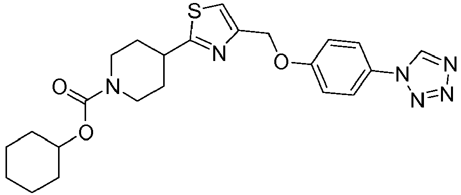

¹H NMR (CDCl₃): δ 8.91 (1H, s), 7.60 (2H, m), 7.25 (1H, s), 7.16 (2H, m), 5.22 (2H, s), 4.68 (1H, m), 4.36 (2H, m), 3.19 (1H, m), 2.91 (2H, m), 2.12 (2H, m), 1.88 (6H, m), 1.40 (6H, m).

### Example 148

### 4-[4-(2-Fluoro-4-methanesulfonyl-phenoxymethyl)-thiazol-2-yl]-piperidine-1-carboxylic acid isopropyl ester

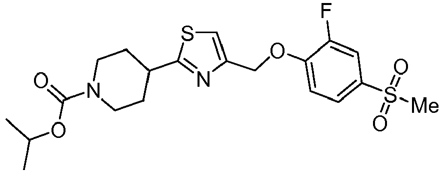

¹H NMR (CDCl₃): δ 7.64∼7.70 (2H, m), 7.20∼7.26 (2H, m), 5.29 (2H, s), 4.89∼4.95 (1H, m), 4.24 (2H, m), 3.13∼3.19 (1H, m), 3.03 (3H, s), 2.86∼2.93 (2H, m), 2.11 (2H, m), 1.69∼1.78 (2H, m), 1.23 (6H, *d, J=* 6.4 Hz).

### Example 149

### 1-Isopropyl-4-[4-(4-tetrazol-1-yl-phenoxymethyl)-thiazol-2-yl]-piperidine

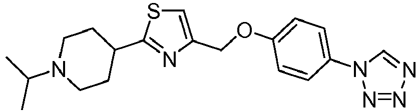

¹H NMR (DMSO-*d₆*): δ 9.98 (1H, s), 7.79 (2H, d, *J* = 8.8 Hz), 7.63 (1H, s), 7.28 (2H, d, *J* = 8.8 Hz), 5.19 (2H, s), 2.91 (1H, m), 2.82 (2H, m), 2.68 (1H, m), 2.20 (2H, m), 2.01 (2H, m), 1.63 (2H, m), 0.94 (6H, *d, J=* 6.4 Hz).

### Example 150

### 1-Propyl-4-[4-(4-tetrazol-1-yl-phenoxymethyl)-thiazol-2-yl]-piperidine

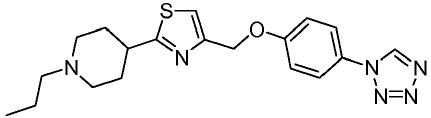

¹H NMR (DMSO-*d₆*): δ 9.97 (1H, s), 7.80 (2H, d, *J* = 8.8 Hz), 7.64 (1H, s), 7.28 (2H, d, *J* = 8.8 Hz), 5.20 (2H, s), 2.94 (1H, m), 2.88 (2H, m), 2.22 (2H, t, *J* = 7.2 Hz), 1.99 (4H, m), 1.64 (2H, m), 1.41 (2H, m), 0.83 (3H, t, *J* = 7.2 Hz).

### Example 151

### 3,3-Dimethyl-1-{4-[4-(4-tetrazol-1-yl-phenoxymethyl)-thiazol-2-yl]-piperidin-1-yl}-butan-2-one

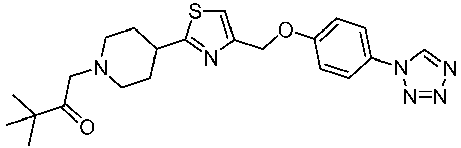

¹H NMR (DMSO-*d₆*): δ 9.98 (1H, s), 7.80 (2H, d, *J* = 8.8 Hz), 7.64 (1H, s), 7.28 (2H, d, *J* = 8.8 Hz), 5.20 (2H, s), 3.41 (2H, s), 2.95 (1H, m), 2.82 (2H, m), 2.18 (2H, m), 1.98 (2H, m), 1.69 (2H, m), 1.07 (9H, s).

### Example 152

### 1-Butyl-4-[4-(4-tetrazol-1-yl-phenoxymethyl)-thiazol-2-yl]-piperidine

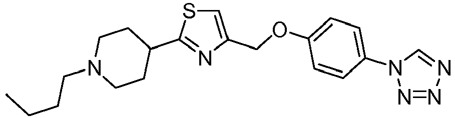

¹H NMR (DMSO-*d₆*): δ 9.97 (1H, s), 7.80 (2H, d, *J* = 8.8 Hz), 7.64 (1H, s), 7.28 (2H, d, *J* = 8.8 Hz), 5.20 (2H, s), 2.94 (1H, m), 2.88 (2H, m), 2.26 (2H, t, *J* = 6.8 Hz), 1.98 (4H, m), 1.66 (2H, m), 1.39 (2H, m), 1.26 (2H, m), 0.86 (3H, t, *J* = 7.2 Hz).

### Example 153

### 2-{4-[4-(4-Tetrazol-1-yl-phenoxymethyl)-thiazol-2-yl]-piperidin-1-yl}-1-(4-trifluoromethoxy-phenyl)-ethanone

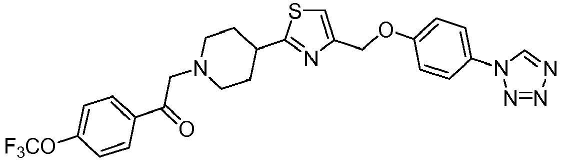

¹H NMR (DMSO-*d₆*): δ 9.97 (1H, s), 8.14 (2H, d, *J* = 6.4 Hz), 8.02 (2H, d, *J* = 6.4 Hz), 7.80 (2H, d, *J* = 8.8 Hz), 7.64 (1H, s), 7.28 (2H, d, *J* = 8.8 Hz), 5.20 (2H, s), 3.84 (2H, s), 2.98 (1H, m), 2.93 (2H, m), 2.38 (2H, m), 2.00 (2H, m), 1.68 (2H, m).

### Example 154

### 1-Methanesulfonyl-4-[4-(4-tetrazol-1-yl-phenoxymethyl)-thiazol-2-yl]-piperidine

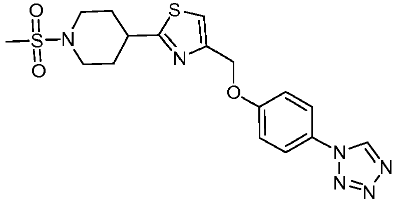

¹H NMR (DMSO-*d₆*): δ 9.98 (1H, s), 7.81 (2H, d, *J* = 8.8 Hz), 7.69 (1H, s), 7.29 (2H, d, *J* = 8.8 Hz), 5.21 (2H, s), 3.60-3.63 (2H, m), 3.32 (3H, s), 3.12-3.18 (1H, m), 2.83-2.90 (2H, m), 2.14-2.17 (2H, m), 1.71 (2H, m).

### Example 155

### 4-[4-(4-Tetrazol-1-yl-phenoxymethyl)-thiazol-2-yl]-piperidine-1-carboxylic acid heptyl ester

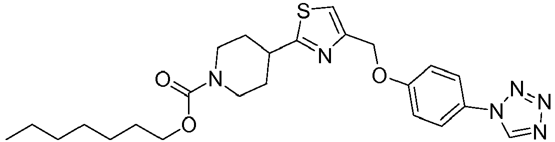

¹H NMR (CDCl₃): δ 8.91 (1H, s), 7.60 (2H, d), 7.25 (1H, s), 7.19 (2H, d), 5.24 (2H, s), 4.26 (2H, br), 4.09 (2H, t), 3.20 (1H, m), 2.94 (2H, m), 2.16 (2H, m), 1.77 (2H, m), 1.60 (2H, m), 1.32 (8H, m), 0.90 (3H, t).

### Example 156

### 4-[4-(4-Tetrazol-1-yl-phenoxymethyl)-thiazol-2-yl]-1-(toluene-4-sulfonyl)-piperidine

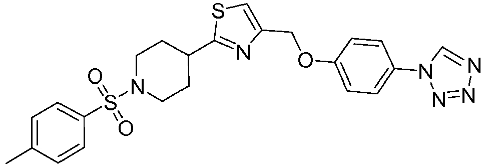

¹H NMR (CDCl₃): δ 8.91 (1H, s), 7.67 (2H, d, *J* = 8.8 Hz), 7.59 (2H, d, *J* = 8.8 Hz), 7.35 (2H, d, *J* = 8.8 Hz), 7.25 (1H, s), 7.15 (2H, m), 5.19 (2H, s), 3.91 (2H, d), 2.95 (1H, m), 2.44 (3H, s), 2.37 (2H, m), 2.17 (2H, d), 1.94 (2H, m).

### Example 157

### 2-tert-Butoxy-1-{4-[4-(4-tetrazol-1-yl-phenoxymethyl)-thiazol-2-yl]-piperidin-1-yl}-ethanone

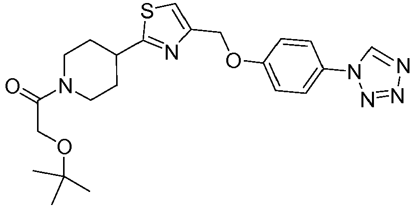

¹H NMR (DMSO-*d₆*): δ 9.99 (1H, s), 7.81 (2H, m), 7.26 (2H, m), 5.20 (2H, s), 4.36 (1H, m), 3.97 (3H, m), 3.28 (1H, m), 3.12 (1H, m), 2.71 (1H, m), 2.04 (2H, m), 1.67 (1H, m), 1.46(1H, m), 1.13(9H, s).

**Examples 158-205** were synthesized from one **of Intermediates 3-13** or **Intermediates 15-25** with the corresponding 2-chloropyrimidine, 2-iodopyrimidine, 2-chloropyridine, 2-fluoropyridine, 2-methanesulfonyl-pyrimidine, 2-chloropyrazine, 2-chloropyridazine or other suitable heterocycles in a manner similar to that described in **Example 47**. One skilled in the art of organic synthesis will appreciate that conditions such as solvent (such as DMF, CH₃CN); temperature, base (such as NEt₃, K₂CO₃, NaHCO₃, Na₂CO₃, Cs₂CO₃) and concentration can be selected through routine experimentation to optimize yields. Additionally, alternative coupling methods can be used that are well known in the art of organic synthesis.

### Example 158

### 5-Ethyl-2- {4-[4-(3-fluoro-4-tetrazol-1-yl-phenoxymethyl)-thiazol-2-yl]-piperidin-1-yl}-pyrimidine

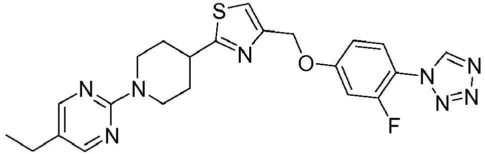

¹H NMR (CDCl₃): δ 9.04 (1H, s), 8.19 (2H, s), 7.78 (1H, m), 7.28 (1H, s), 6.70 (2H, m), 5.23 (2H, s), 4.83 (2H, m), 3.31 (1H, m), 3.05 (2H, m), 2.47 (2H, q), 2.21 (2H, m), 1.81 (2H, m), 1.20 (3H, t).

### Example 159

### 2- {4-[4-(2,6-Difluoro-4-tetrazol-1-yl-phenoxymethyl)-thiazol-2-yl]-piperidin-1-yl}-5-ethyl-pyrimidine

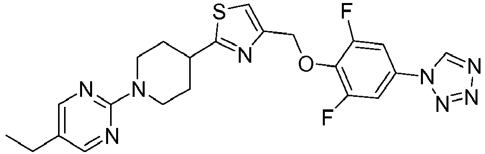

¹H NMR (CDCl₃): δ 8.95 (1H, s), 8.17 (2H, s), 7.34 (2H, m), 7.28 (1H, s), 5.35 (2H, s), 4.76 (2H, m), 3.27 (1H, m), 3.04 (2H, m), 2.46 (2H, q), 2.16 (2H, m), 1.76 (2H, m), 1.19 (3H, t).

### Example 160

### 5-Ethyl-2- {4-[4-(4-pyrrol-1-yl-phenoxymethyl)-thiazol-2-yl]-piperidin-1-yl}-pyrimidine

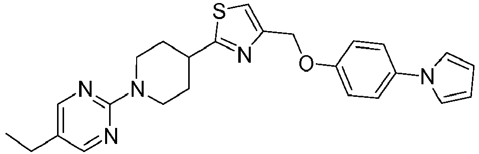

¹H NMR (CDCl₃): δ 8.18 (2H, s), 7.29 (2H, m), 7.20 (1H, s), 6.99 (4H, m), 6.31 (2H, m), 5.17 (2H, s), 4.84 (2H, m), 3.28 (1H, m), 3.03 (2H, m), 2.46 (2H, q), 2.21 (2H, m), 1.81 (2H, m), 1.19 (3H, t).

### Example 161

### {2-[1-(5-Ethyl-pyrimidin-2-yl)-piperidin-4-yl]-thiazol-4-ylmethyl}-(4-tetrazol-1-yl-phenyl)-amine

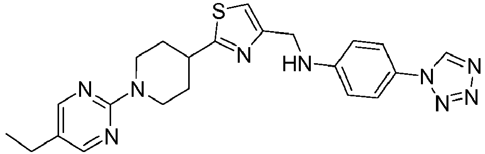

¹H NMR (CDCl₃): δ 8.83 (1H, s), 8.16 (2H, s), 7.41 (2H, m), 7.02 (1H, s), 6.74 (2H, m), 4.82 (1H, s), 4.79 2H, s), 4.45 (2H, m), 3.25 (1H, m), 3.01 (2H, m), 2.44 (2H, q), 2.17 (2H, m), 1.77 (2H, m), 1.11 (3H, t).

### Example 162

### 2-{4-[4-(2-Fluoro-4-tetrazol-1-yl-phenoxymethyl)-thiazol-2-yl]-piperidin-1-yl}-5-isopropyl-pyrimidine

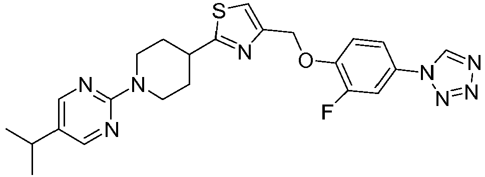

¹H NMR (CDCl₃): δ 8.92 (1H, s), 8.21 (2H, s), 7.51 (1H, m), 7.40 (1H, m), 7.29 (1H, s), 7.26 (1H, m), 5.30 (2H, s), 4.82 (2H, m), 3.28 (1H, m), 3.04 (2H, m), 2.77 (1H, m), 2.20 (2H, m), 1.80 (2H, m), 1.23 (6H, d).

### Example 163

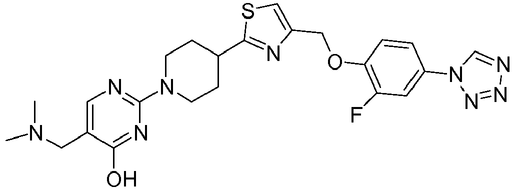

¹H NMR (CDCl₃): δ 8.97 (1H, s), 7.80 (1H, s), 7.50 (1H, m), 7.40 (1H, m), 7.27 (1H, s), 7.24 (1H, m), 5.27 (2H, s), 4.42 (4H, m), 3.24 (1H, m), 3.04 (9H, m), 2.16 (2H, m), 1.88 (2H, m).

### Example 164

### 5-Ethyl-2- {4-[4-(2-methyl-4-tetrazol-1-yl-phenoxymethyl)-thiazol-2-yl]-piperidin-1-yl} - pyrimidine

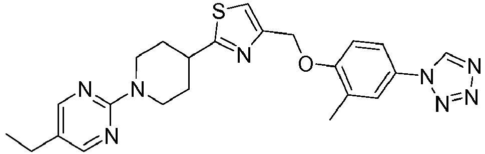

¹H NMR (CDCl₃): δ 8.88 (1H, s), 8.19 (2H, s), 7.48 (1H, s), 7.44 (1H, m), 7.24 (1H, m), 7.05 (1H, m), 5.26 (2H, s), 4.83 (2H, m), 3.27 (1H, m), 3.05 (2H, m), 2.47 (2H, q), 2.37 (3H, s), 2.22 (2H, m), 1.81 (2H, m), 1.19 (3H, t).

### Example 165

### 5-Chloro-2- {4-[4-(2-chloro-4-tetrazol-1-yl-phenoxymethyl)-thiazol-2-yl]-piperidin-1-yl}-pyrimidine

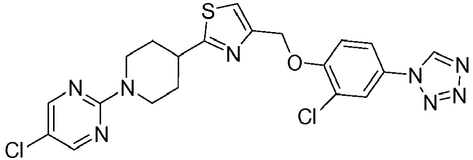

¹H NMR (acetone-*d*₆), δ 9.68 (1H, s), 8.33 (2H, s), 8.01 (1H, s), 7.86 (1H, m), 7.60 (1H, m), 7.59 (1H, s), 5.40 (2H, s), 4.78 (2H, m), 3.40 (1H, m), 3.16 (2H, m), 2.20 (2H, m), 1.77 (2H, m).

### Example 166

### 2- {4-[4-(2-Chloro-4-tetrazol-1-yl-phenoxymethyl)-thiazol-2-yl]-piperidin-1-yl}-5-trifluoromethyl-pyrimidine

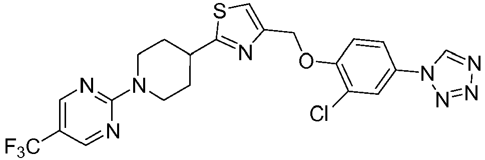

¹H NMR (acetone-*d*₆), δ 9.68 (1H, s), 8.62 (2H, s), 8.01 (1H, s), 7.86 (1H, m), 7.61 (1H, s), 7.60 (1H, m), 5.41 (2H, s), 4.92 (2H, m), 3.46 (1H, m), 3.27 (2H, m), 2.25 (2H, m), 1.80 (2H, m).

### Example 167

### 2-{4-[4-(2-Isopropyl-5-methyl-4-tetrazol-1-yl-phenoxymethyl)-thiazol-2-yl]-piperidin-1-yl} -5-trifluoromethyl-pyrimidine

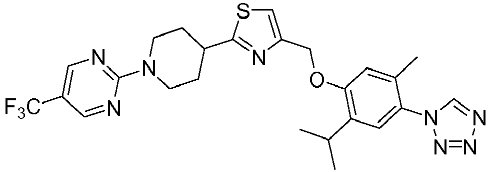

¹H NMR (CDCl₃): δ 8.73 (1H, s), 8.46 (2H, s), 7.22 (1H, s), 7.10 (1H, s), 6.90 (1H, s), 5.24 (2H, s), 4.93 (2H, m), 3.35 (2H, m), 3.17 (2H, m), 2.23 (2H, m), 2.09 (3H, s), 1.82 (2H, m), 1.20 (6H, d).

### Example 168

### 5-Chloro-2- {4-[4-(2-isopropyl-5-methyl-4-tetrazol-1-yl-phenoxymethyl)-thiazol-2-yl]-piperidin-1-yl}-pyrimidine

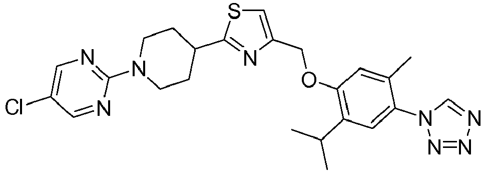

¹H NMR (CDCl₃): δ 8.73 (1H, s), 8.20 (2H, s), 7.21 (1H, s), 7.09 (1H, s), 6.90 (1H, s), 5.24 (2H, s), 4.78 (2H, m), 3.35 (1H, m), 3.28 (1H, m), 3.07 (2H, m), 2.19 (2H, m), 2.09 (3H, s), 1.79 (2H, m), 1.20 (6H, d).

### Example 169

### 5-Ethyl-2- {4-[4-(4-tetrazol-1-yl-phenoxymethyl)-oxazol-2-yl]-piperidin-1-yl}-pyrimidine

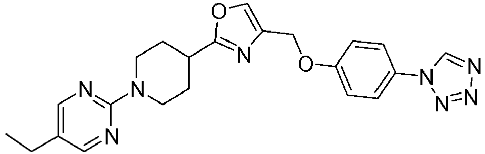

¹H NMR (CDCl₃): δ 8.91 (1H, s), 8.18 (2H, s), 7.65 (1H, s), 7.60 (2H, m), 7.15 (2H, m), 5.03 (2H, s), 4.69 (2H, m), 3.10 (3H, m), 2.44 (2H, q), 2.14 (2H, m), 1.86 (2H, m), 1.19 (3H, t).

### Example 170

### 5-Ethyl-2- {4-[4-(2-fluoro-4-tetrazol-1-yl-phenoxymethyl)-oxazol-2-yl]-piperidin-1-yl}-pyrimidine

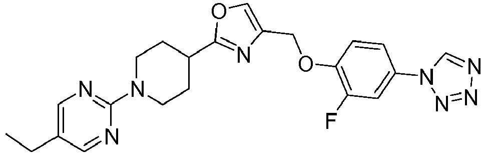

¹H NMR (CDCl₃): δ 8.93 (1H, s), 8.17 (2H, s), 7.67 (1H, s), 7.50 (1H, m), 7.41 (1H, m), 7.29 (1H, m), 5.11 (2H, s), 4.67 (2H, m), 3.08 (3H, m), 2.45 (2H, q), 2.12 (2H, m), 1.84 (2H, m), 1.18 (3H, t).

### Example 171

### 2-{4-[4-(2-Fluoro-4-tetrazol-1-yl-phenoxymethyl)-thiazol-2-yl]-piperidin-1-yl}-5-trifluoromethyl-pyrimidine

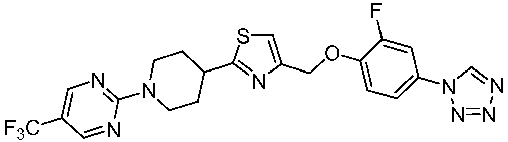

¹H NMR (CDCl₃): δ 8.91 (1H, s), 8.49 (2H, s), 7.52 (1H, d, *J* = 7.6 Hz), 7.41 (1H, d, *J* = 7.6 Hz), 7.32 (1H, s), 7.29 (1H, m), 5.32 (2H, s), 4.95 (2H, m), 3.37 (1H, m), 3.15 (2H, m), 2.24 (2H, m), 1.81 (2H, m).

### Example 172

### 5-Decyl-2- {4-[4-(4-tetrazol-1-yl-phenoxymethyl)-thiazol-2-yl]-piperidin-1-yl}-pyrimidine

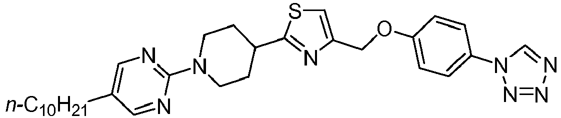

¹H NMR (DMSO-d₆): δ 9.97 (1H, s), 8.21 (2H, s), 7.80 (2H, d, *J* = 8.8 Hz), 7.65 (1H, s), 7.28 (2H, d, *J* = 8.8 Hz), 5.20 (2H, s), 4.66 (2H, m), 3.32 (1H, m), 3.01 (2H, m), 2.37 (2H, m), 2.09 (2H, m), 1.60 (2H, m), 1.45 (2H, m), 1.21 (14H, m), 0.82 (3H, m).

### Example 173

### 6-Methyl-2-{4-[4-(4-tetrazol-1-yl-phenoxymethyl)-thiazol-2-yl]-piperidin-1-yl}-pyrimidine-4-carboxylic acid methyl ester

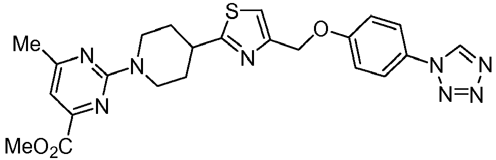

¹H NMR (DMSO-*d₆*): δ 9.97 (1H, s), 7.80 (2H, d, *J* = 8.8 Hz), 7.66 (1H, s), 7.28 (2H, d, *J* = 8.8 Hz), 7.01 (1H, s), 5.21 (2H, s), 4.76 (2H, m), 3.84 (3H, s), 3.33 (1H, m), 3.06 (2H, m), 2.36 (3H, s), 2.14 (2H, m), 1.61 (2H, m).

### Example 174

### 4-Chloro-2- {4-[4-(4-tetrazol-1-yl-phenoxymethyl)-thiazol-2-yl]-piperidin-1-yl}-pyrimidine

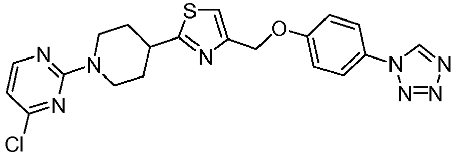

¹H NMR (CDCl₃): δ 8.91 (1H, s), 8.15 (1H, d, *J* = 5.2 Hz), 7.60 (2H, d, *J* = 8.8 Hz), 7.25 (1H, s), 7.16 (2H, d, *J* = 8.8 Hz), 6.49 (1H, d, *J* = 5.2 Hz), 5.22 (2H, s), 4.85 (2H, m), 3.30 (1H, m), 3.07 (2H, m), 2.21 (2H, m), 1.80 (2H, m).

### Example 175

### 2-Chloro-4- {4-[4-(4-tetrazol-1-yl-phenoxymethyl)-thiazol-2-yl]-piperidin-1-yl}-pyrimidine

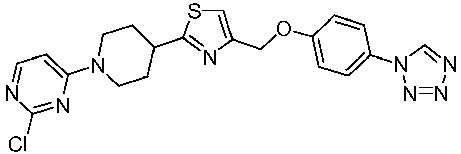

¹H NMR (CDCl₃): δ 8.90 (1H, s), 8.05 (1H, d, *J* = 6.4 Hz), 7.61 (2H, d, *J* = 8.8 Hz), 7.28 (1H, s), 7.17 (2H, d, *J* = 8.8 Hz), 6.46 (1H, d, *J* = 6.4 Hz), 5.23 (2H, s), 4.45 (2H, m), 3.35 (1H, m), 3.15 (2H, m), 2.27 (2H, m), 1.85 (2H, m).

### Example 176

### 6-Methyl-2-{4-[4-(4-tetrazol-1-yl-phenoxymethyl)-thiazol-2-yl]-piperidin-1-yl}-pyrimidine-4-carboxylic acid

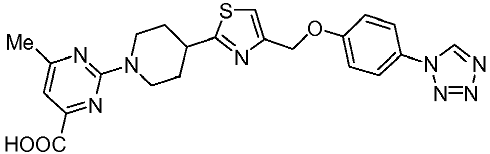

¹H NMR (DMSO-*d₆*): δ 13.3 (1H, br), 9.97 (1H, s), 7.80 (2H, d, *J* = 8.8 Hz), 7.66 (1H, s), 7.28 (2H, d, *J* = 8.8 Hz), 6.98 (1H, s), 5.21 (2H, s), 4.79 (2H, m), 3.34 (1H, m), 3.05 (2H, m), 2.35 (3H, s), 2.13 (2H, m), 1.62 (2H, m).

### Example 177

### 5-Chloro-4,6-difluoro-2-{4-[4-(4-tetrazol-1-yl-phenoxymethyl)-thiazol-2-yl]-piperidin-1-yl}-pyrimidine

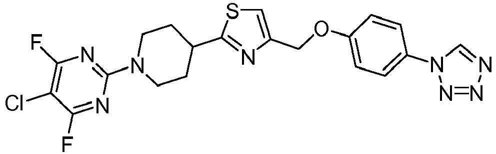

¹H NMR (CDCl₃): δ 8.91 (1H, s), 7.61 (2H, d, *J* = 8.8 Hz), 7.27 (1H, s), 7.16 (2H, d, *J* = 8.8 Hz), 5.23 (2H, s), 4.69 (2H, m), 3.32 (1H, m), 3.10 (2H, m), 2.23 (2H, m), 1.80 (2H, m).

### Example 178

### 4-Fluoro-2-{4-[4-(4-tetrazol-1-yl-phenoxymethyl)-thiazol-2-yl]-pipendin-1-yl}-pyrimidine

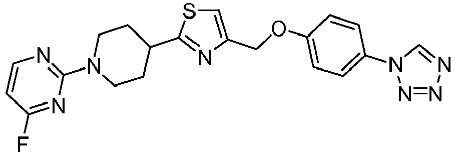

¹H NMR (DMSO-*d₆*): δ 9.97 (1H, s), 8.41 (1H, m), 7.80 (2H, d, *J* = 8.0 Hz), 7.66 (1H, s), 7.28 (2H, d, *J* = 8.0 Hz), 6.34 (1H, m), 5.20 (2H, s), 4.60 (2H, m), 3.32 (1H, m), 3.10 (2H, m), 2.11 (2H, m), 1.61 (2H, m).

### Example 179

### 2-Fluoro-4-{4-[4-(4-tetrazol-1-yl-phenoxymethyl)-thiazol-2-yl]-piperidin-1-yl}-pyrimidine

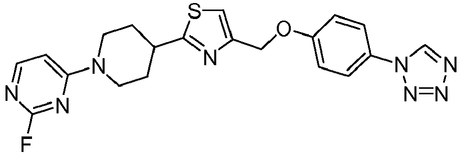

¹H NMR (DMSO-*d₆*): δ 9.98 (1H, s), 8.08 (1H, m), 7.80 (2H, d, *J* = 9.2 Hz), 7.67 (1H, s), 7.28 (2H, d, *J* = 9.2 Hz), 6.84 (1H, m), 5.20 (2H, s), 4.40 (2H, m), 3.40 (1H, m), 3.14 (2H, m), 2.13 (2H, m), 1.63 (2H, m).

### Example 180

### 2-{4-[4-(4-Tetrazol-1-yl-phenoxymethyl)-thiazol-2-yl]-piperidin-1-yl}-thiazole-5-carboxylic acid ethyl ester

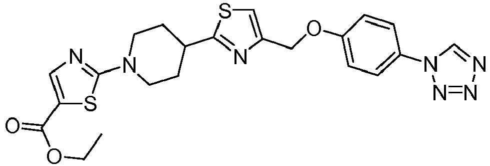

¹H NMR (DMSO-d₆): δ 9.97 (1H, s), 7.84 (1H, m), 7.80 (2H, d, *J* = 9.0 Hz), 7.68 (1H, s), 7.28 (2H, d, *J* = 9.0 Hz), 5.21 (2H, s), 4.19 (2H, t, *J* = 7.20 Hz), 4.03 (2H, m), 3.35 (3H, m), 2.15 (2H, m), 1.75 (2H, m), 1.23 (3H, t, *J* = 7.20 Hz).

### Example 181

### 4-Imidazol-1-yl-6-{4-[4-(4-tetrazol-1-yl-phenoxymethyl)-thiazol-2-yl]-piperidin-1-yl}-pyrimidine

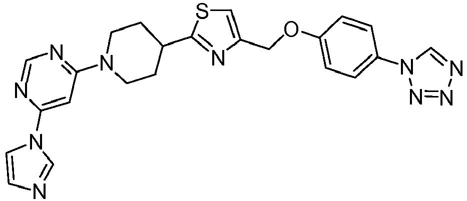

¹H NMR (DMSO-*d₆*): δ 9.98 (1H, s), 8.59 (1H, s), 8.43 (1H, s), 8.01 (1H, d, *J* = 1.2 Hz), 7.81 (2H, d, *J* = 8.8 Hz), 7.67 (1H, s), 7.27 (2H, d, *J* = 8.8 Hz), 7.14 (1H, s), 7.10 (1H, d, *J* = 1.2 Hz), 5.20 (2H, s), 4.61 (2H, m), 3.40 (1H, m), 3.15 (2H, m), 2.15 (2H, m), 1.66 (2H, m).

### Example 182

### 5-Ethyl-2- {4-[4-(6-tetrazol-1-yl-pyridin-3-yloxymethyl)-thiazol-2-yl]-piperidin-1-yl}-pyrimidine.

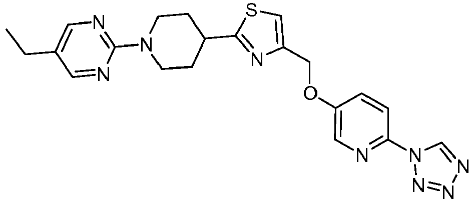

¹H NMR (CDCl₃): δ 9.44 (1H, s), 8.28 (1H, d, *J* = 3.0 Hz), 8.2 (2H, s), 8.02, (1H, d, *J* = 8.8 Hz), 7.58 (1H, dd, *J* = 8.8 Hz, 3.0 Hz), 7.27 (1H, s), 5.27 (2H, s), 4.82-4.85 (2H,m), 3.22-3.35 (1H,m), 3.0-3.1, (2H, m), 2.47 (2H, q, *J* = 7.2 Hz), 2.2-2.23 (2H, m), 1.76-1.86 (2H, m), 1.19 (3H, t, *J* = *7.2* Hz).

### Example 183

### 5-Methyl-2-{4-[4-(6-tetrazol-1-yl-pyridin-3-yloxymethyl)-thiazol-2-yl]-piperidin-1-yl}-pyrimidine

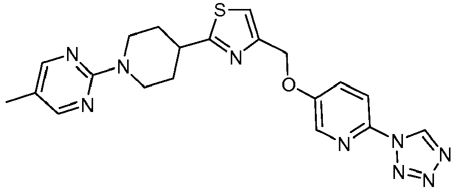

¹H NMR (DMSO-*d₆*): δ 10.07 (1H, s), 8.42 (1H, d, *J* = 3.0 Hz), 8.21 (2H, s), 7.99 (1H, d, *J* = 9.2 Hz), 7.86 (1H, dd, *J* = 9.2 Hz, 3.0 Hz), 7.70 (1H, s), 5.30 (2H, s), 4.62 (2H, m), 3.56-3.60 (1H, m), 2.98-3.04 (2H, m), 2.06 (3H, s), 1.72-1.76 (2H, m), 1.59 (2H, m).

### Example 184

### 5-Chloro-2-{4-[4-(6-tetrazol-1-yl-pyridin-3-yloxymethyl)-thiazol-2-yl]-piperidin-1-yl}-pyrimidine.

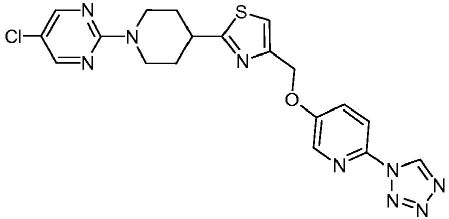

¹H NMR (CDCl₃) δ 9.44 (1H, s), 8.28 (1H, d, *J* = 3.0 Hz), 8.23 (2H, s), 8.02 (1H, d, *J* = 9.0 Hz), 7.58 (1H, dd, *J* = 9.0 Hz, 3.0 Hz), 7.28 (1H, s), 5.27 (2H, s), 4.8-4.83 (2H, m), 3.22-3.38 (1H, m), 3.04-3.11 (2H, m), 2.20-2.23 (2H, m), 1.80 (2H, m)

### Example 185

### 2- {4-[4-(6-Tetrazol-1-yl-pyridin-3-yloxymethyl)-thiazol-2-yl]-piperidin-1-yl}-5-trifluoromethyl-pyrimidine.

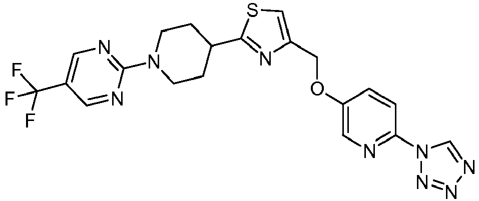

¹H NMR (DMSO-*d₆*): δ 10.07 (1H, s), 8.68 (2H, s), 8.42 (1H, d, *J* = 3.0 Hz), 7.99 (1H, d, *J* = 9.2 Hz), 7.86 (1H, dd, *J* = 9.2 Hz, 3.0 Hz), 7.72 (1H, s), 5.73 (2H, s), 4.74-4.77 (2H, m), 3.37-3.43 (1H, m), 3.15-3.21 (2H, m), 2.12-2.16 (2H, m), 1.59-1.68 (2H, m).

### Example 186

### 3-Chloro-6-{4-[4-(4-tetrazol-1-yl-phenoxymethyl)-thiazol-2-yl]-piperidin-1-yl}-pyridazine

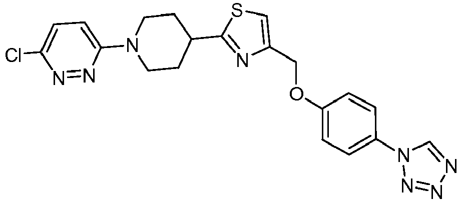

¹H NMR (CDCl₃): δ 8.91 (1H, s), 7.61 (2H, d, *J* = 9.0 Hz), 7.26 (1H, s), 7.22 (1H, d, *J* = 9.6 Hz), 7.17 (2H, *d, J* = 9.0 Hz), 6.95 (1H, *d, J* = 9.6 Hz), 5.23 (2H, s), 4.43-4.47 (2H, m), 3.31-3.37 (1H, m), 3.12-3.19 (2H, m), 2.25-2.28 (2H, m), 1.90 (2H, m).

### Example 187

### 2-Tetrazol-1-yl-5-{4-[4-(4-tetrazol-1-yl-phenoxymethyl)-thiazol-2-yl]-piperidin-1-yl}-pyrazine

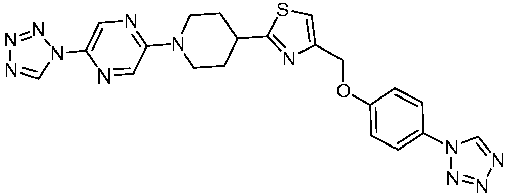

¹H NMR (DMSO-*d₆*): δ 9.97 (2H, s), 8.67 (1H, s), 8.37 (1H, s), 7.80 (2H, d, *J* = 8.8 Hz), 7.67 (1H, s), 7.28 (2H, d, *J* = 8.8 Hz), 5.21 (2H, s), 4.50-4.53 (2H, m), 3.38-3.44 (1H, m), 3.17-3.23 (2H, m), 2.15-2.18 (2H, m), 1.69-1.77 (2H, m).

### Example 188

### {2-[1-(5-Ethyl-pyrimidin-2-yl)-piperidin-4-yl]-thiazol-4-ylmethyl}-(6-fluoro-pyridin-3-yl)-amine

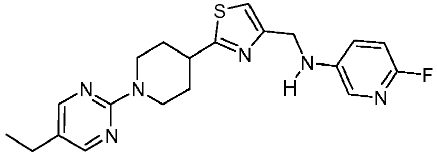

¹H NMR (CDCl₃): δ 8.19 (2H, s), 7.58-7.62 (1H, m), 7.05-7.10 (1H, m), 7.01 (1H, s), 6.75 (1H, dd, *J* = 8.4 Hz, 2.8 Hz), 4.81-4.85 (2H, m), 4.40 (2H, d, *J* = 5.2 Hz), 4.29 (1H, br s), 3.23-3.29 (1H, m), 3.00-3.06 (2H, m), 2.47 (2H, q, *J* = 7.6 Hz), 2.18-2.20 (2H, m), 1.79 (2H, m), 1.20 (3H, t, *J* = 7.6 Hz).

### Example 189

### 2- {4-[4-(2,6-Difluoro-4-methanesulfonyl-phenoxymethyl)-thiazol-2-yl]-piperidin-1-yl}-5-ethyl-pyrimidine

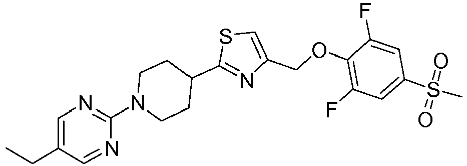

¹H NMR (CDCl₃): δ 8.19 (2H, s,), 7.51 (2H, d), 7.25 (1H, s), 5.40 (2H, s), 4.82 (2H, m), 3.30 (1H, m), 3.06 (3H, s), 3.03 (2H, m), 2.48 (2H, q), 2.15 (2H, m), 1.74 (2H, m), 1.20 (3H, t).

### Example 190

### 5-Butyl-2- {4-[4-(4-tetrazol-1-yl-phenoxymethyl)-thiazol-2-yl]-piperidin-1-yl}-pyrimidine

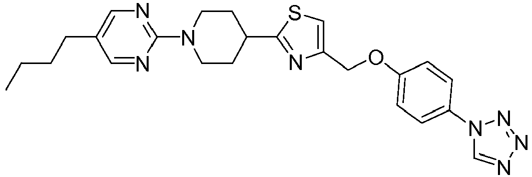

¹H NMR (CDCl₃): δ 8.92 (1H, s), 8.17 (2H, s), 7.62 (2H, m), 7.25 (1H, s), 7.17 (2H, m), 5.24 (2H, s), 4.83 (2H, m), 3.30 (1H, m), 3.04 (2H, m), 2.42 (2H, t), 2.23 (2H, m), 1.84 (2H, m), 1.52 (2H, m), 1.34 (2H, m), 0.92 (3H, m).

### Example 191

### 4-(4-{2-[1-(5-Ethyl-pyrimidin-2-yl)-piperidin-4-yl]-thiazol-4-ylmethoxy}-phenyl)-morpholine

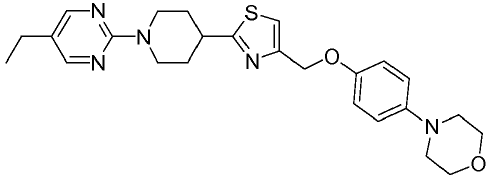

¹H NMR (CDCl₃): δ 8.18 (2H, s), 7.19 (1H, s), 6.92 (4H, m), 5.12 (2H, s), 4.84 (2H, m), 3.86 (4H, br), 3.30 (1H, m), 3.05 (6H, m), 2.46 (2H, q), 2.21 (2H, m), 1.78 (2H, m), 1.19 (3H, t).

### Example 192

### 5-Nitro-2-{4-[4-(4-tetrazol-1-yl-phenoxymethyl)-thiazol-2-yl]-piperidin-1-yl}-pyrimidine

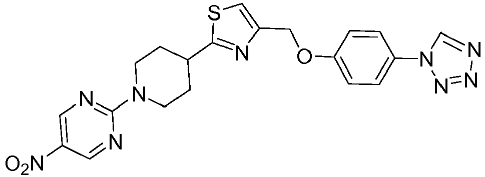

¹H NMR (DMSO-*d₆*): δ 9.91 (1H, s), 9.11 (2H, s), 7.83 (2H, d, *J* = 8.8 Hz), 7.68 (1H, s), 7.25 (2H, d, *J* = 8.8 Hz), 5.22 (2H, s), 4.81 (2H, m), 3.39 (1H, m), 3.31 (2H, m), 2.23 (2H, s), 1.68 (2H, m).

### Example 193

### 3'-Chloro-4-[4-(4-tetrazol-1-yl-phenoxymethyl)-thiazol-2-yl]-5'-trifluoromethyl-3,4,5,6-tetrahydro-2H-[1,2']bipyridinyl

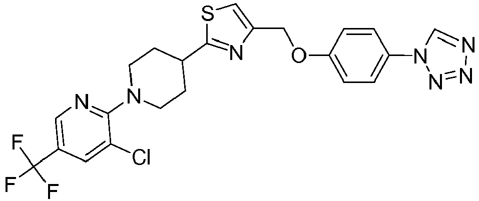

¹H NMR (CDCl₃): δ 8.91 (1H, s), 8.39 (1H, s), 7.76 (1H, s), 7.61 (2H, m), 7.25 (1H, s), 7.18 (2H, m), 5.24 (2H, s), 4.16 (2H, m), 3.26 (1H, m), 3.06 (2H, m), 2.25 (2H, m), 2.01 (2H, m).

### Example 194

### 3'-Chloro-4-[4-(2-fluoro-4-tetrazol-1-yl-phenoxymethyl)-thiazol-2-yl]-5'-trifluoromethyl-3,4,5,6-tetrahydro-2H-[1,2']bipyridinyl

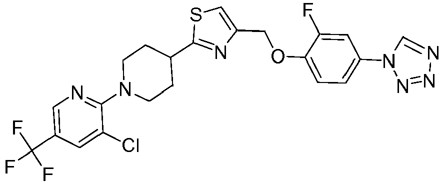

¹H NMR (CDCl₃):δ 8.94 (1H, s), 8.38 (1H, s), 7.75 (1H, s), 7.53 (1H, m), 7.40 (1H, m), 7.31 (1H, s), 7.25 (1H, m), 5.31 (2H, s), 4.15 (2H, d), 3.25 (1H, m), 3.09 (2H, m), 2.23 (2H, d), 1.99 (2H, m).

### Example 195

### 5-Chloro-2- {4-[4-(2-fluoro-4-tetrazol-1-yl-phenoxymethyl)-thiazol-2-yl]-piperidin-1-yl}-pyrimidine

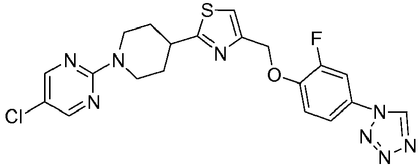

¹H NMR (CDCl₃): δ 8.96 (1H, s), 8.20 (2H, s), 7.52 (1H, m), 7.40 (1H, m), 7.28 (1H, s), 7.25 (1H, m), 5.28 (2H, s), 4.78 (2H, m), 3.30 (1H, m), 3.07 (2H, m), 2.20 (2H, m), 1.79 (2H, m).

### Example 196

### 3',5'-Dichloro-4-[4-(4-tetrazol-1-yl-phenoxymethyl)-thiazol-2-yl]-3,4,5,6-tetrahydro-2H-[1,2']bipyridinyl

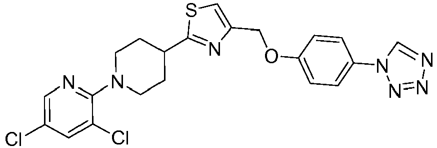

¹H NMR (DMSO-*d₆*): δ 9.98 (1H, s), 8.26 (1H, s), 8.03 (1H, s), 7.81 (2H, d), 7.67 (1H, s), 7.29 (2H, d), 5.21 (2H, s), 3.79 (2H, m), 3.24 (1H, m), 2.97 (2H, m), 2.14 (2H, m), 1.84 (2H, m).

### Example 197

### 3'-Chloro-4-[4-(4-tetrazol-1-yl-phenoxymethyl)-thiazol-2-yl]-3,4,5,6-tetrahydro-2H-[1,2']bipyridinyl-5'-carboxylic acid ethyl ester

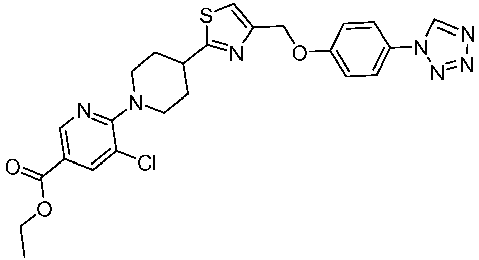

¹H NMR (CDCl₃): δ 8.92 (1H, s), 8.74 (1H, s), 8.11 (1H, s), 7.61 (2H, d), 7.25 (1H, s), 7.17 (2H, d), 5.23 (2H, s), 4.37 (2H, m), 4.22 (2H, m), 3.31 (1H, m), 3.08 (2H, m) 2.26 (2H, m), 1.98 (2H, m), 1.38 (3H, m).

### Example 198

### 5'-Chloro-4-[4-(4-tetrazol-1-yl-phenoxymethyl)-thiazol-2-yl]-3,4,5,6-tetrahydro-2H-[1,2']bipyridinyl-3'-carboxylic acid methyl ester

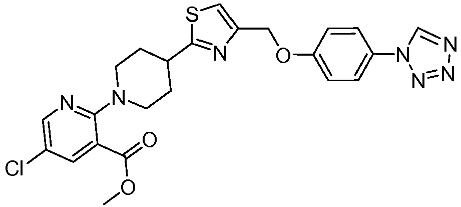

**¹H** NMR (CDCl₃): δ 8.91 (1H, s), 8.20 (1H, s), 7.99 (1H, s), 7.61 (2H, d), 7.25 (1H, s), 7.16 (2H, d), 5.21 (2H, s), 3.91 (2H, m), 3.88 (3H, s), 3.28 (1H, m), 3.08 (2H, m), 2.20 (2H, m), 1.93 (2H, m).

### Example 199

### 5-Ethyl-2-{3-methyl-4-[4-(4-tetrazol-1-yl-phenoxymethyl)-thiazol-2-yl]-piperidin-1-yl}-pyrimidine

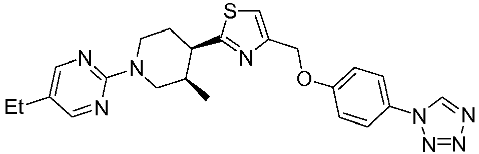

¹H NMR (CDCl₃): δ 8.90 (1H, s), 8.18 (2H), 7.60 (2H, m), 7.25 (1H, s), 7.17 (2H, m), 5.26 (2H), 4.89-4.51 (2H, m), 3.49-3.20 (2H, m), 2.92 (1H, m), 2.65-2.45 (1H, m), 2.45 (2H, m), 2.17-1.81 (2H, m), 1.20 (3H, m), 0.82-0.92 (3H).

### Example 200

### 5-Ethyl-2- {4-[4-(2-fluoro-4-tetrazol-1-yl-phenoxymethyl)-thiazol-2-yl]-3-methylpiperidin-1-yl}-pyrimidine

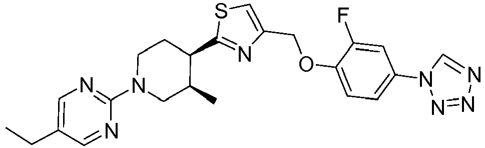

¹H NMR (CDCl₃): δ 8.93 (1H, s), 8.17 (2H), 7.52-7.25 (4H, m), 5.32 (2H), 4.84-4.46 (2H, m), 3.47-3.22 (2H, m), 2.91 (1H, m), 2.62-2.43 (1H, m), 2.42 (2H, m), 2.07 (2H, m), 1.18 (3H, m), 0.90-0.79 (3H, m).

### Example 201

### 5-Chloro-2-4-[4-(2-fluoro-4-tetrazol-1-yl-phenoxymethyl)-thiazol-2-yl]-3-methylpiperidin-1-yl}-pyrimidine

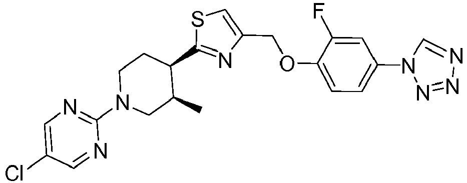

¹H NMR (CDCl₃): δ 8.93 (1H, s), 8.19 (2H), 7.52-7.25 (4H, m), 5.29 (2H), 4.82-4.51 (2H, m), 3.46-3.21 (2H, m), 2.95 (1H, m), 2.64-2.42 (1H, m), 2.02 (2H, m), 0.90-0.78 (3H, m).

### Example 202

### 2-{4-[4-(2-Fluoro-4-tetrazol-1-yl-phenoxymethyl)-thiazol-2-yl]-3-methyl-piperidin-1-yl}-5-trifluoromethyl-pyrimidine

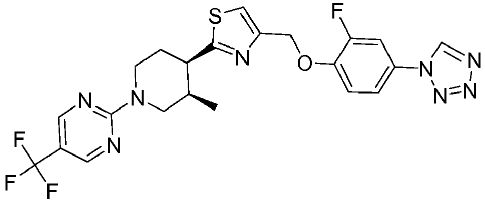

¹H NMR (CDCl₃): δ 8.94 (1H, s), 8.47 (2H), 7.53-7.27 (4H, m), 5.34 (2H), 5.02-4.62 (2H, m), 3.52-2.97 (3H, m), 2.73-2.47 (1H, m), 2.17-2.01 (2H, m), 0.94-0.78 (3H, m).

### Example 203

### 5-Ethyl-2-{4-[4-(4-methanesulfonyl-benzyloxymethyl)-thiazol-2-yl]-piperidin-1-yl}-pyrimidine

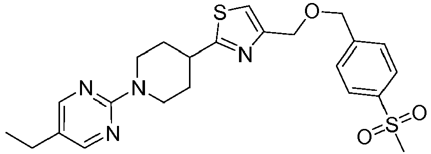

¹H NMR (CDCl₃): δ 8.17 (2H, s), 7.92 (2H, d, *J=* 8.8 Hz), 7.58 (2H, *d, J=* 8.8 Hz), 7.13 (1H, s), 4.83 (2H, m), 4.71 (2H, s), 4.66 (2H, s), 3.27 (1H, m), 3.03 (3H, s), 2.98 (2H, m), 2.46 (2H, m), 2.19 (2H, m), 1.76 (2H, m), 1.19 (3H, m).

### Example 204

### 5-Fluoro-2-{4-[4-(2-fluoro-4-tetrazol-1-yl-phenoxymethyl)-thiazol-2-yl]-piperidin-1-yl}-pyrimidine

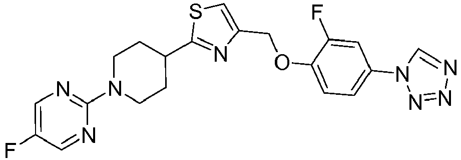

¹H NMR (CDCl₃): δ 8.91 (1H, s), 8.21 (2H, s), 7.52 (1H, m), 7.41 (1H, m), 7.27 (1H, m), 7.25 (1H, s), 5.31 (2H, s), 4.76 (2H, m), 3.28 (1H, m), 3.06 (2H, m), 2.20 (2H, m), 1.81 (2H, m).

### Example 205

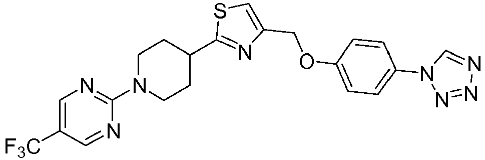

¹H NMR (CDCl₃): δ 8.91 (1H, s), 8.49 (2H, s), 7.61 (2H, d), 7.27 (1H, s), 7.17 (2H, d), 5.24 (2H, s), 4.96 (2H, m), 3.38 (1H, m), 3.14 (2H, m), 2.26 (2H, m), 1.82(2H, m).

### Example 206

### 4-(4-{[(4-Methanesulfonyl-phenyl)-methyl-amino]-methyl}-thiazol-2-yl)-piperidine-1-carboxylic acid tert-butyl ester

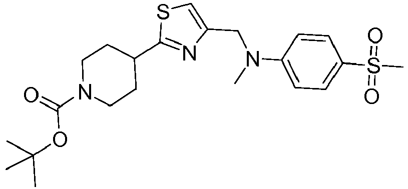

4-{4-[(4-Methanesulfonyl-phenylamino)-methyl]-thiazol-2-yl}-piperidine-1-carboxylic acid tert-butyl ester **(Example 138)** (0.10mmol) was dissolved in DMF (2mL) and NaH (2eq.) was added in a single portion at room temperature. The reaction was stirred for 30 minutes and methyliodide (10eq.) was added. After stirring for 3 hours, the reaction was quenched with water and extracted with EtOAc. The organic layer was washed with brine, dried over sodium sulfate, filtered and concentrated *in vacuo*. Purification of the residue by silica gel chromatography (Hexanes/EtOAc 1:1) provided the expected product. ¹H NMR (CDCl₃): δ 7.73 (2H, m), 6.78 (2H, m), 6.76 (1H, s), 4.70 (2H, s), 4.20 (2H, br), 3.19 (3H, s), 3.12 (1H, m), 3.01 (3H, s), 2.87 (2H, m), 2.07 (2H, m), 1.80 (2H, m), 1.47 (9H, s).

### Example 207

### {2-[1-(5-Ethyl-pyrimidin-2-yl)-piperidin-4-yl]-thiazol-4-ylmethyl}-(2-fluoro-4-methanesulfonyl-phenyl)-methyl-amine

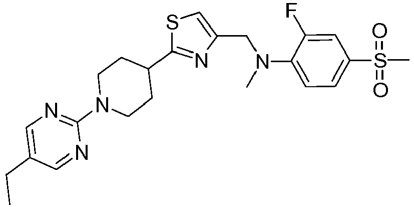

**Example 207** was synthesized in a manner analogous to **Example 206** utilizing {2-[1-(5-Ethyl-pyrimidin-2-yl)-piperidin-4-yl]-thiazol-4-ylmethyl}-(2-fluoro-4-methanesulfonyl-phenyl)-amine **(Example 141)** as the starting material. ¹H NMR (CDCl₃): δ 8.19 (2H, s), 7.47-7.57 (2H, m), 6.94 (1H, s), 6.91 (1H, m), 4.80 (2H, m), 4.62 (2H, s), 3.24 (1H, m), 3.09 (3H, s), 3.03 (3H, s), 3.00 (2H, m), 2.47 (2H, m), 2.17 (2H, m), 1.74 (2H, m), 1.19 (3H, t).

### Example 208

### 4-[4-(2-Methylsulfanyl-pyrimidin-5-yloxymethyl)-thiazol-2-yl]-piperidine-1-carboxylic acid tert-butyl ester

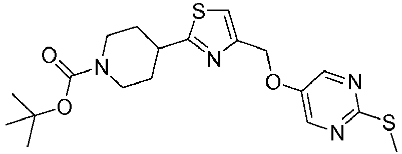

**Example 208** was prepared from 4-(4-Chloromethyl-thiazol-2-yl)-piperidine-1-carboxylic acid *tert*-butyl ester (**Intermediate 1**) and 2-Methylsulfanyl-pyrimidin-5-ol in a manner similar to that described in **Example 1**. ¹H NMR (CDCl₃): δ 8.35 (2H, s), 7.23 (1H, s), 5.19 (2H, s), 4.22 (2H, m), 3.16 (1H, m), 2.87 (2H, m), 2.55 (3H, s), 2.10 (2H, m), 1.71 (2H, m), 1.46 (9H, s).

### Example 209

### 4-[4-(4-Tetrazol-1-yl-phenoxymethyl)-thiazol-2-yl]-piperidine-1-carboxylic acid allyl ester

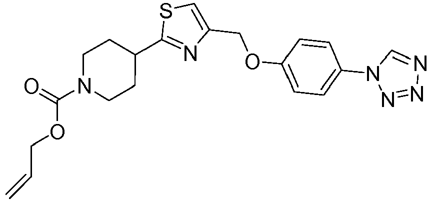

**Example 209** was prepared from 4-[4-(4-Tetrazol-1-yl-phenoxymethyl)-thiazol-2-yl]-piperidine (**Intermediate 4**) and allyl chloroformate in a manner similar to that described in **Example 22.** ¹H NMR (CDCl₃): δ 8.96 (1H, s), 7.63 (2H, m), 7.20 (1H, s), 7.18 (2H, m), 5.96 (1H, m), 5.31 (1H, m), 5.22 (3H, m), 4.61 (2H, m), 4.29 (2H, m), 3.21 (1H, m), 2.97 (2H, m), 2.15 (2H, m), 1.78 (2H, m).

### Example 210

### 2- {4-[4-Methyl-5-(4-tetrazol-1-yl-phenoxymethyl)-thiazol-2-yl]-piperidin-1-yl}-5-trifluoromethyl-pyrimidine

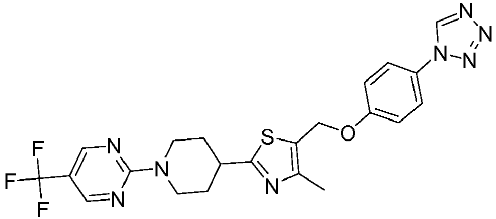

### Step 1: 4-[4-Methyl-5-(4-tetrazol-1-yl-phenoxymethyl)-thiazol-2-yl]-piperidine

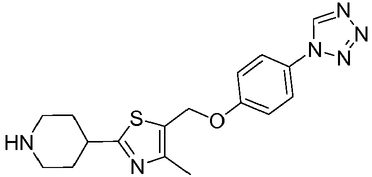

A solution of 4-[4-Methyl-5-(4-tetrazol-1-yl-phenoxymethyl)-thiazol-2-yl]-piperidine-1-carboxylic acid tert-butyl ester (**Example 93**) (500 mg, 1.10 mmol) in dichloromethane (5 mL) was treated with 1.5 mL of 4N HCl in dioxane. The resulting solution was stirred at room temperature for 5 hours and all the solvent were removed *in vacuo* to afford the desired product as an HCl salt.

### Step 2: 2-{4-[4-Methyl-5-(4-tetrazol-1-yl-phenoxymethyl)-thiazol-2-yl]-piperidin-1-yl}-5-trifluoromethyl-pyrimidine

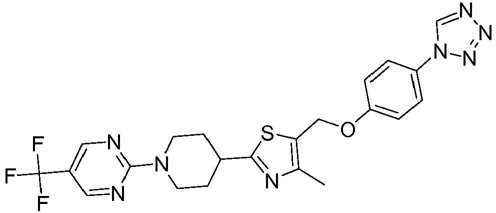

This compound was prepared from 4-[4-Methyl-5-(4-tetrazol-1-yl-phenoxymethyl)-thiazol-2-yl]-piperidine hydrochloride in a similar manner as described in **Example 47**. ¹H NMR (CDCl₃): δ 8.94 (1H, s), 8.49 (2H, s), 7.64 (2H, m), 7.14 (2H, m), 5.20 (2H, s), 4.95 (2H, m), 3.27 (1H, m), 3.13 (2H, m), 2.46 (3H, s), 2.21 (2H, m), 1.77 (2H, m).

### Biological Example 1

### Oral Glucose Tolerance Test of 5-Ethyl-2-{4-[4-(4-tetrazol-1-yl-phenoxymethyl)-thiazol-2-yl]-piperidin-1-yl}-pyrimidine and sitagliptin

This example shows that in mice, co-administration of 5-Ethyl-2-{4-[4-(4-tetrazol-1-yl-phenoxymethyl)-thiazol-2-yl]-piperidin-1-yl}-pyrimidine and sitagliptin improves glucose excursion than treatment with either compound alone.

C57B1/6J mice were fasted for 6 hours prior to drug administration. Blood glucose was measured after the 6 hour fast (T30 min), and animals were sorted into groups evenly matched for fasting glucose levels. At T30, drug suspension was administered to the mice by oral gavage. Glucose was administered at T0 min at 2 g/kg. The administration volume was 5 mL/kg of body weight. Blood was sampled at T0, prior to glucose administration, then at 15, 30, 60, 90 and 120 min after glucose administration for measurement of glucose by glucometer. 10 mice were used for each dose group. The formulation of 5-Ethyl-2-{4-[4-(4-tetrazol-1-yl-phenoxymethyl)-thiazol-2-yl]-piperidin-1-yl}-pyrimidine and sitagliptin was 1% carboxymethylcellulose, 2% Tween 80 prepared in a manner essentially as described in Example 2. The formulation was a suspension and was continuously stirred during dosing of the animals.

Glucose levels were plotted against time and the incremental area under the curve (AUC) of the glucose excursion was determined from T0 to T120 using GraphPad Prism 5.1. Statistical significance of differences in AUC between compound treatment and vehicle was determined by non-parametric Kruskal-Wallis test with Dunn's post test. Differences with a p-value ≤0.05 were considered significant. Statistical differences in the glucose levels at each time point during OGTT were determined by two way ANOVA with Bonferroni's post test. Differences with a p-value ≤0.05 were considered significant.

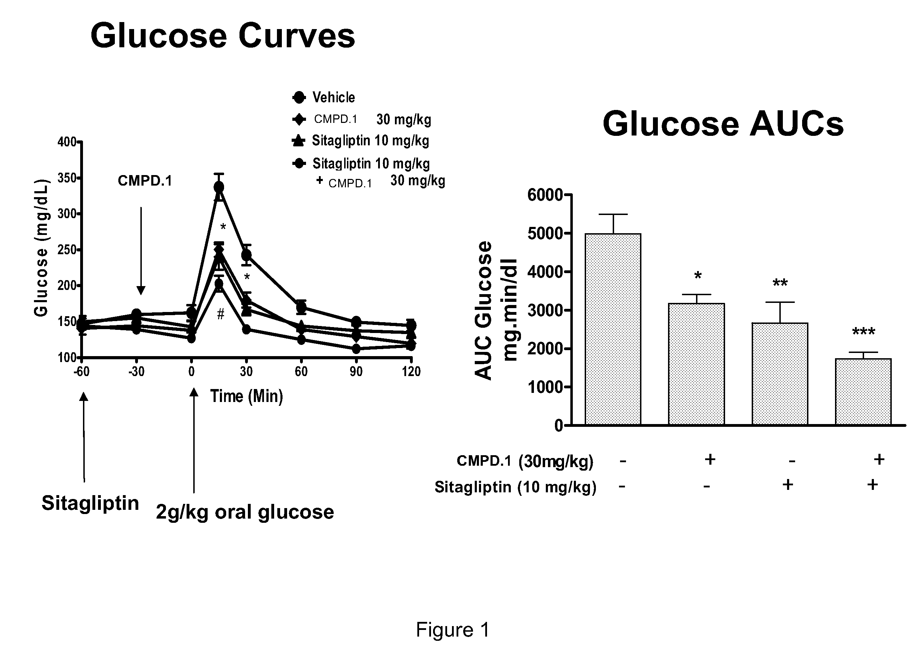

The data as provide in Figure 1 shows that the AUC for mice treated with 5-Ethyl-2-{4-[4-(4-tetrazol-1-yl-phenoxymethyl)-thiazol-2-yl]-piperidin-1-yl}-pyrimidine only at 30 mg/kg was approximately 3200 mg.min/dl. The AUC for mice treated with sitagliption alone at 10 mg/kg was approxmiately 2800mg.min/dl. The AUC for mice treated with both 5-Ethyl-2-{4-[4-(4-tetrazol-1-yl-phenoxymethyl)-thiazol-2-yl]-piperidin-1-yl}-pyrimidine at 30 mg/kg and sitagliptin at 10 mg/kg was approximately was 1900 mg.min/dl.

### Biological Example 2

### Oral Glucose Tolerance Test of 5-Ethyl-2-{4-[4-(4-tetrazol-1-yl-phenoxymethyl)-thiazol-2-yl]-piperidin-1-yl}-pyrimidine and vildagliptin

This example shows that in diet induced obesity (DIO) rats, co-administration of 5-Ethyl-2- {4-[4-(4-tetrazol-1-yl-phenoxymethyl)-thiazol-2-yl]-piperidin-1-yl}-pyrimidine and sitagliptin improves glucose excursion than treatment with either compound alone.

A total of 40 DIO rats were included in the study. At the start of the experiment, the animals were 23 weeks old (19 weeks on high-fat diet). The rats were housed under a controlled light cycle (light from 6:00-18:00 h) at controlled temperature and humidity conditions. They are offered an energy-dense high-fat diet (#12266B; Research Diets) and water *ad libitum,* up until 16h before the OGTT, when fasting is initiated by withdrawal of the food.

HE-diet: High energy diet (4.41 kcal/g - Energy %: Carbohydrate 51.4 kcal %, Fat 31.8 kcal %, Protein 16.8 kcal %; diet #12266B; Research Diets, New Jersey, USA).

One week before the OGTT, the animals were transferred to single housing (1 rat/cage). The OGTT was preceded by a 3-day run-in period with daily handling and to make the animals accustomed to the PO injection procedure.

The animals were stratified according to body weight and fasting blood glucose concentration on day 0 and were assigned to one of the following treatment groups. After stratification, there were no statistical differences between the average body weights or the fasting blood glucose concentrations of the different treatment groups.
Group 1. Vehicle (n=10)
Group 2. 5-Ethyl-2-{4-[4-(4-tetrazol-1-yl-phenoxymethyl)-thiazol-2-yl]-piperidin-1-yl}-pyrimidine (300 mg/kg, n=10)
Group 3. vildagliptin (5 mg/kg, n=10)
Group 4. 5-Ethyl-2-{4-[4-(4-tetrazol-1-yl-phenoxymethyl)-thiazol-2-yl]-piperidin-1-yl}-pyrimidine (300 mg/kg) + vildagliptin (5 mg/kg) (n=10)

### VEHICLE AND COMPOUND PREPARATION

The vehicle was 1 % CMC (w/v), 2 % TWEEN80 (w/v) (CMC/T80). In a glass beaker, 4 grams of Tween 80 (polysorbate 80) was added to 194 ml of DI water and stirred. To the stirring solution 2 grams of Carboxymethylcellulose (CMC, sodium salt, medium viscosity) was gradually added. The solution was stirred overnight until a clear uniform solution formed

5-Ethyl-2- {4-[4-(4-tetrazol-1-yl-phenoxymethyl)-thiazol-2-yl]-piperidin-1-yl}-pyrimidine dosing suspension preparation were prepared. For dosing at 300 mg/kg in a dosing volume of 5 ml/kg, the dosing suspensions were prepared in 60 mg/ml suspensions in the vehicle. 1980 mg (for 60 mg/ml) of 5-Ethyl-2-{4-[4-(4-tetrazol-1-yl-phenoxymethyl)-thiazol-2-yl]-piperidin-1-yl}-pyrimidine were added to a glass beaker. To the beaker was added about 15 ml of vehicle to the beaker to wet the compound completely (by gentle swirl) and then q.s. to 33 ml with CMC/T80 vehicle.

Next, the beakers were covered put into a bath sonicator and sonicated for about 30 min or longer until no lumps were visible. The beaker was covered to prevent evaporation and stirred with a magnetic stir bar overnight. The suspension will settle to the bottom of the container once stirring is stopped, so the beakers must be kept on the stirrer plates throughout the dosing procedure.

### Vildagliptin Solution Preparation

For dosing at 5 mg/kg in a dosing volume of 5 ml/kg, the dosing solutions were prepared as 1 mg/ml solutions. For experiments using vildagliptin alone, 33 mg Vildagliptin was dissolved in 33 ml of the CMC/T80 vehicle.

For the animals treated with both vildagliptin and 5-Ethyl-2-{4-[4-(4-tetrazol-1-yl-phenoxymethyl)-thiazol-2-yl]-piperidin-1-yl}-pyrimidine, 33 mg Vildagliptin was added to a 33 ml batch of 5-Ethyl-2-{4-[4-(4-tetrazol-1-yl-phenoxymethyl)-thiazol-2-yl]-piperidin-1-yl}-pyrimidine suspension as described above.

### EXPERIMENTAL PROTOCOL

The DIO rats were handled for three days before the OGTT, to make them accustomed to the oral gavage procedure. The day before the OGTT (day -1) the animals are changed into clean cages and fully fasted from 18:30 hours.

On day 0, the body weight and fasting blood glucose concentration of each individual animal were determined and used for stratification.

The animals were dosed by oral gavage at 60 minutes (t = -60) before the oral glucose load was administered. To prevent the 5-Ethyl-2-{4-[4-(4-tetrazol-1-yl-phenoxymethyl)-thiazol-2-yl]-piperidin-1-yl}-pyrimidine compound from settling, the suspension was stirred throughout the entire dosing procedure.

At t = 0 minutes, the animals were given an oral glucose load of 2 mg/kg via a gastric tube attached to a syringe to ensure accurate dosing. For the monitoring of plasma glucose levels, a 100 µl blood sample was taken in heparinized tubes at t = -60, 0, 2, 15, 30, 60, 120, and 240 minutes. Additionally, a 200 µl blood sample was collected at each of these time points for determination of plasma insulin and active GLP-1 concentrations. After sampling in EDTA coated tubes, 2 µl vildagliptin was added to inhibit any DPP IVactivity.

After the OGTT, the animals were sacrificed by CO₂ anesthesia followed by decapitation.

Blood and plasma glucose levels were measured on a Biosen analyzer (Biosen s_line apparatus, EKF diagnostics). Plasma levels of insulin and active GLP-1 were determined on a Luminex System, in duplicate.

The results are presented as mean ± SEM (standard error of the mean), unless otherwise stated. Statistical evaluation of the data is carried out using one-way analysis of variance (ANOVA) with appropriate post-hoc analysis between vehicle and treatment groups in the cases where statistical significance is established (*p*<0.05; Fisher's PLSD).

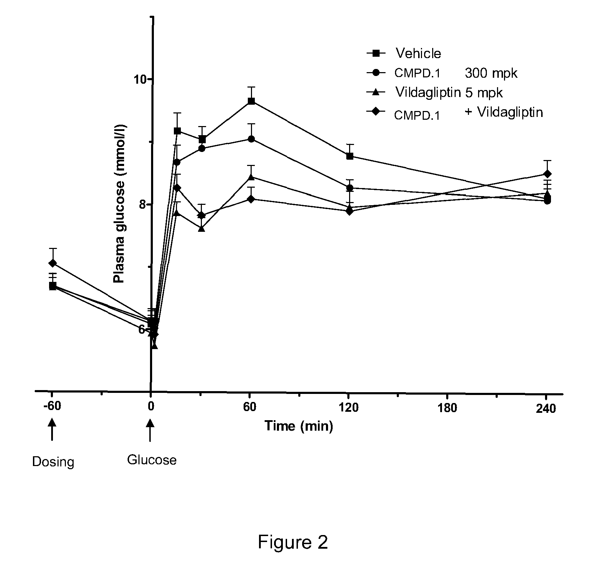

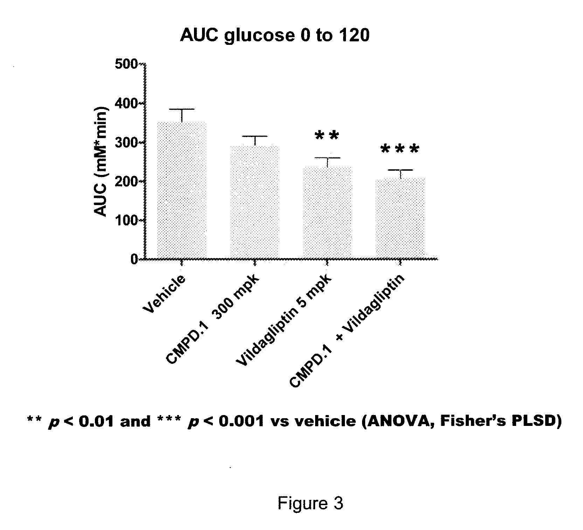

Figures 2 and 3 show the data obtained from an OGTT experiment. Figure 2 shows the time course of an OGTT experiment. The combination of 5-Ethyl-2-{4-[4-(4-tetrazol-1-yl-phenoxymethyl)-thiazol-2-yl]-piperidin-1-yl}-pyrimidine and vildagliptin was more effective at lowering plasma glucose levels than either compound alone.

Figure 3 shows the AUC of plasma glucose levels from T0 to T120 minutes. The combination of 5-Ethyl-2- {4-[4-(4-tetrazol-1-yl-phenoxymethyl)-thiazol-2-yl]-piperidin-1-yl}-pyrimidine and vildagliptin was more effective at lowering plasma glucose levels than either compound alone.

### Biological Example 3

Plasma insulin levels of DIO rats when treated with 5-Ethyl-2-{4-[4-(4-tetrazol-1-yl-phenoxymethyl)-thiazol-2-yl]-piperidin-1-yl}-pyrimidine and vildagliptin The plasma insulin level of DIO rats as described in Example 2 were tested. 5-Ethyl-2-{4-[4-(4-tetrazol-1-yl-phenoxymethyl)-thiazol-2-yl]-piperidin-1-yl}-pyrimidine, and vildagliptin were prepared as in Example 2.

Figure 4 shows a time course of plasma insulin levels fromT0 to T240 minutes. The combination of 5-Ethyl-2-{4-[4-(4-tetrazol-1-yl-phenoxymethyl)-thiazol-2-yl]-piperidin-1-yl}-pyrimidine and vildagliptin was more effective at lowering plasma insulin levels than either compound alone.

### Biological Example 4

Incretin secretion of mice treated with 5-Ethyl-2-{4-[4-(4-tetrazol-1-yl-phenoxymethyl)-thiazol-2-yl]-piperidin-1-yl}-pyrimidine and sitagliptin This example shows that in mice, co-administration of 5-Ethyl-2-{4-[4-(4-tetrazol-1-yl-phenoxymethyl)-thiazol-2-yl]-piperidin-1-yl}-pyrimidine (Compound 1), and sitagliptin stimulated incretin secretion than treatment with either compound alone. 5-Ethyl-2-{4-[4-(4-tetrazol-1-yl-phenoxymethyl)-thiazol-2-yl]-piperidin-1-yl}-pyrimidine, sitagliptin, and vildagliptin were prepared as in Examples 1 and 2.

Mice were fasted for 6 h prior to drug administration. Blood glucose was measured after the 6 h fast (T30 min), and animals were sorted into 6 groups evenly matched for fasting glucose levels and body weight. At T30 min, drug suspension as prepared in Example 1 herein, was administered to the mice by oral gavage. All groups were given sitagliptin at 100 mg/kg, and Compound 1 was given at 1, 3, 10, 30 and 300 mg/kg. 100 mg/kg sitagliptin was chosen since this dose is greater than that required to achieve 80% DPP-IV inhibition in mice over the time period evaluated in this study. (Kim et al, 2005). Glucose (2 g/kg) was administered by oral gavage at T0. Animals were anesthetized and blood drawn by terminal cardiac puncture 10 minutes after glucose administration. To address the effects on incretin production, total GIP levels were measured. Since sufficient amounts of plasma could not be obtained to measure total (active + inactive) GLP-1, only active GLP-1 was measured. The DPP-IV inhibitor sitagliptin was co-administered to prevent breakdown of active GLP-1 by DPP-IV (Kim et al; 2005). Statistical significance of differences in GLP-1, GIP and glucose levels between compound + sitagliptin treatment and sitagliptin alone was determined by one way ANOVA with Dunnet's post test. Differences with a p value less than 0.05 were considered significant. (Kim D, Wang L, Beconi M, et al., (2R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine: a potent, orally active dipeptidyl peptidase IV inhibitor for the treatment of type 2 diabetes. J Med Chem. 48(1):141-51. 2005.)

Figure 5a shows the data obtained from the experiment. Increasing dosages of 5-Ethyl-2-{4-[4-(4-tetrazol-1-yl-phenoxymethyl)-thiazol-2-yl]-piperidin-1-yl}-pyrimidine given in addition to sitapliptin at 100 mg/kg stimulated secretion of active GLP-1.

### Biological Example 5

### Incretin secretion of DIO rats treated with 5-Ethyl-2-{4-[4-(4-tetrazol-1-yl-phenoxymethyl)-thiazol-2-yl]-piperidin-1-yl}-pyrimidine and sitagliptin

The plasma levels of active GLP-1 of DIO rats as described in Example 2 were determined. 5-Ethyl-2-{4-[4-(4-tetrazol-1-yl-phenoxymethyl)-thiazol-2-yl]-piperidin-1-yl}-pyrimidine and vildagliptin were prepared as in Example 2.

Figure 6 shows that the combination of 5-Ethyl-2-{4-[4-(4-tetrazol-1-yl-phenoxymethyl)-thiazol-2-yl]-piperidin-1-yl}-pyrimidine and sitagliptin was more effective at increasing plasma levels at active GLP-1 than either compound alone. Figure 5b shows that the combination of 5-Ethyl-2-{4-[4-(4-tetrazol-1-yl-phenoxymethyl)-thiazol-2-yl]-piperidin-1-yl}-pyrimidine and vildagliptin was more effective at increasing plasma levels at active GLP-1 than either compound alone.

### Biological Example 6

### Incretin secretion of C57BL/6J mice and DIO rats treated with 5-Ethyl-2-{4-[4-(4-tetrazol-1-yl-phenoxymethyl)-thiazol-2-yl]-piperidin-1-yl}-pyrimidine and sitagliptin

This example shows that in C57BL/6J mice, co-administration of 5-Ethyl-2-{4-[4-(4-tetrazol-1-yl-phenoxymethyl)-thiazol-2-yl]-piperidin-1-yl} -pyrimidine and sitagliptin stimulated incretin secretion than treatment with either compound alone. 5-Ethyl-2-{4-[4-(4-tetrazol-1-yl-phenoxymethyl)-thiazol-2-yl]-piperidin-1-yl} -pyrimidine, and sitagliptin were prepared as in Example 1.

Mice were fasted for 6 h prior to drug administration. 5-Ethyl-2-{4-[4-(4-tetrazol-1-yl-phenoxymethyl)-thiazol-2-yl]-piperidin-1-yl}-pyrimidine and sitagliptin were prepared as in Example 1. Blood glucose was measured after the 6 h fast (T30 min), and animals sorted into 6 groups evenly matched for fasting glucose levels and body weight. At T30 min, drug suspension was administered to the mice by oral gavage. Groups were given either vehicle, or 5-Ethyl-2-{4-[4-(4-tetrazol-1-yl-phenoxymethyl)-thiazol-2-yl]-piperidin-1-yl}-pyrimidine at 30mg/kg, or sitagliptin at 1 mg/kg or a combination of sitagliptin (1mg/kg) and 5-Ethyl-2- {4-[4-(4-tetrazol-1-yl-phenoxymethyl)-thiazol-2-yl]-piperidin-1-yl}-pyrimidine (30mg/kg). Glucose (2 g/kg) was administered by oral gavage at T0. Animals were anesthetized and blood drawn by terminal cardiac puncture 2 minutes after glucose administration. Since sufficient amounts of plasma could not be obtained to measure total (active + inactive) GLP-1, only active GLP-1 was measured. Statistical significance of differences in GLP-1 levels between compound treatment and vehicle was determined by non-parametric Kruskal-Wallis test with Dunn's post test. Differences with a p-value ≤ 0.05 were considered significant.

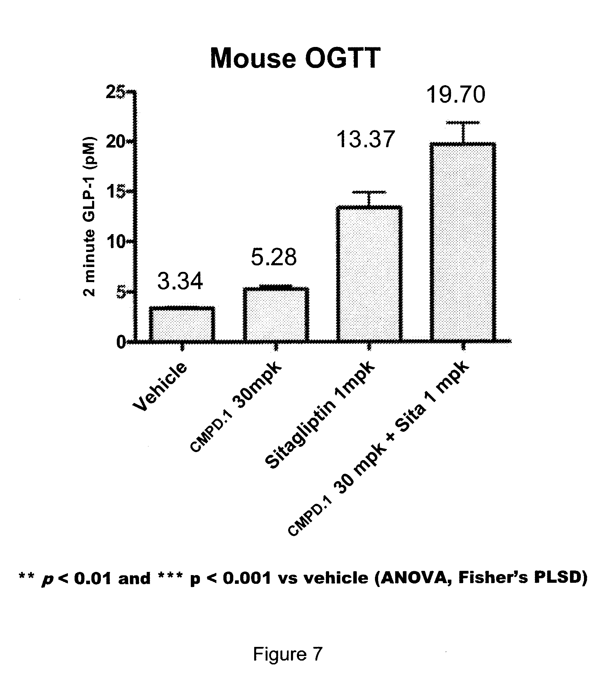

Figure 7 shows that the combination of 5-Ethyl-2-{4-[4-(4-tetrazol-1-yl-phenoxymethyl)-thiazol-2-yl]-piperidin-1-yl}-pyrimidine and sitagliptin was more effective at increasing plasma levels at active GLP-1 at 2 minutes than either compound alone.

Any conflict between any reference cited herein and the teaching of this specification is to be resolved in favor of the latter. Similarly, any conflict between an art-recognized definition of a word or phrase and a definition of the word or phrase as provided in this specification is to be resolved in favor of the latter.

## Claims

1. A compound selected from the group consisting of: 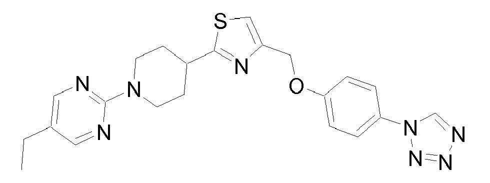 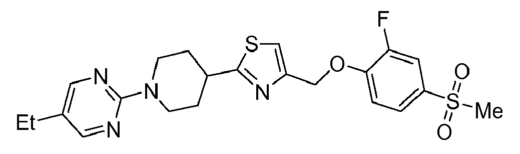 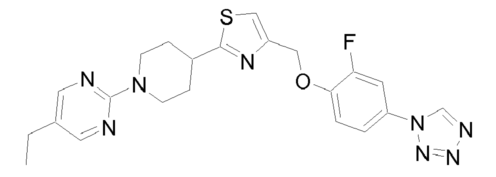 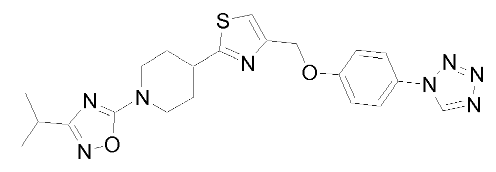 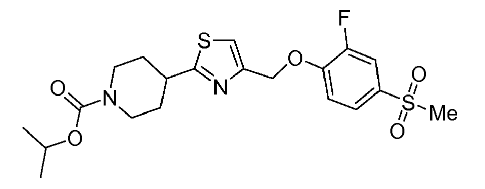 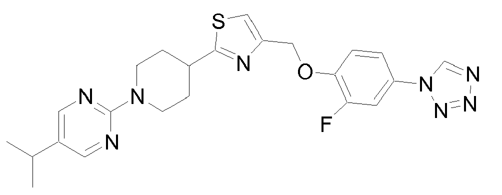 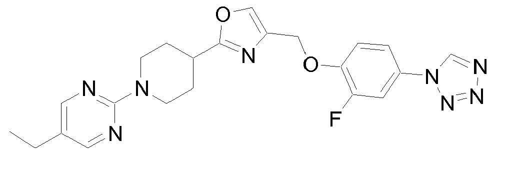 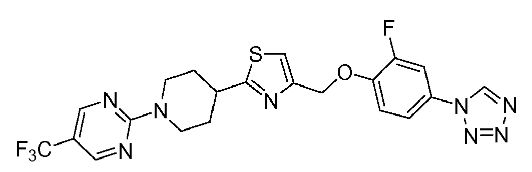 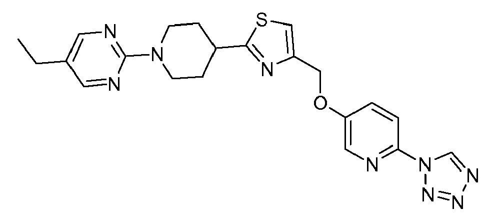 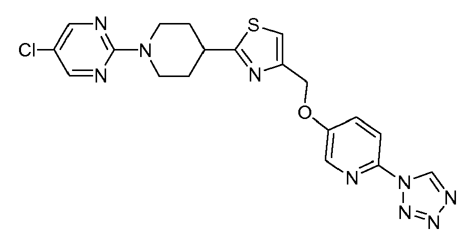 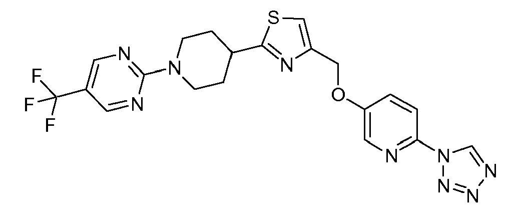 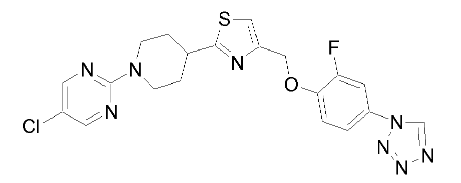 or a pharmaceutically acceptable salt or ester thereof; and a DPP IV inhibitor, for use in a method of
(i) treating diabetes,
(ii) lowering blood levels of glucose,
(iii) lowering blood levels of insulin,
(iv) increasing blood levels of incretins,
(v) lowering blood levels of triglycerides, or
(vi) increasing glucose dependent insulin production.

2. The compound and DPP IV inhibitor for use as claimed in claim 1, wherein said compound is 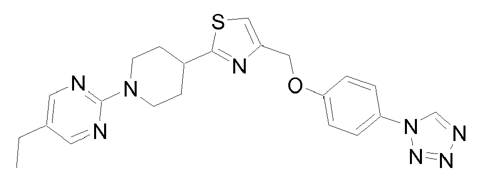 or a pharmaceutically acceptable salt thereof.

3. The compound and DPP IV inhibitor for use as claimed in claim 1, wherein said DPP-IV inhibitor is selected from the group consisting of sitagliptin, vildagliptin, Denagliptin, saxagliptin, and alogliptin.

4. The compound and DPP IV inhibitor for use as claimed in claim 1, wherein said DPP-IV inhibitor is sitagliptin.

5. The compound and DPP IV inhibitor for use as claimed in claim 1, wherein said DPP-IV inhibitor is vildagliptin.

6. The compound and DPP IV inhibitor for use as claimed in claim 1, wherein said incretin is selected from the group consisting of GLP-1 and GIP.

7. A compound as defined in claim 1, and a DPP IV inhibitor, as a combined preparation for simultaneous separate or sequential use in: treating diabetes; lowering blood levels of glucose; lowering blood levels of insulin; increasing blood levels of incretins; lowering blood levels oftriglycerides; and/or increasing glucose dependent insulin production.

8. A compound as defined in claim 1, and a DPP IV inhibitor, for combined use in:
treating diabetes; lowering blood levels of glucose; lowering blood levels of insulin;
increasing blood levels of incretins; lowering blood levels of triglycerides; and/or
increasing glucose dependent insulin production.

9. A compound as defined in claim 1, for use in combination with a DPP IV inhibitor, in: treating diabetes; lowering blood levels of glucose; lowering blood levels of insulin; increasing blood levels of incretins; lowering blood levels of triglycerides; and/or increasing glucose dependent insulin production.

10. A DPP IV inhibitor for use, in combination with a compound as defined in claim 1, in: treating diabetes; lowering blood levels of glucose; lowering blood levels of insulin; increasing blood levels of incretins; lowering blood levels of triglycerides; and/or increasing glucose dependent insulin production.

11. A compound as defined in claim 1, for use in: treating diabetes; lowering blood levels of glucose; lowering blood levels of insulin; increasing blood levels of incretins; lowering blood levels of triglycerides; and/or increasing glucose dependent insulin production, in a patient who has previously been treated with a DPP IV inhibitor.

12. A DPP IV inhibitor in the manufacture of a medicament for: treating diabetes; lowering blood levels of glucose; lowering blood levels of insulin; increasing blood levels of incretins; lowering blood levels of triglycerides; and/or increasing glucose dependent insulin production, in a patient who has previously been treated with a compound as defined in claim 1.

13. A compound as defined in claim 1, for use in: treating diabetes; lowering blood levels of glucose; lowering blood levels of insulin; increasing blood levels of incretins; lowering blood levels of triglycerides; and/or increasing glucose dependent insulin production, wherein the compound is administered, or is prepared for administration, with a DPP IV inhibitor.

14. A DPP IV inhibitor for use in: treating diabetes; lowering blood levels of glucose; lowering blood levels of insulin; increasing blood levels of incretins; lowering blood levels of triglycerides; and/or increasing glucose dependent insulin production, wherein the DPP IV inhibitor is administered, or is prepared for administration, with a compound as defined in claim 1.

15. Use of (a) a compound as defined in claim 1, and (b) a DPP IV inhibitor, in the manufacture of a medicament for: treating diabetes; lowering blood levels of glucose; lowering blood levels of insulin; increasing blood levels of incretins; lowering blood levels of triglycerides; and/or increasing glucose dependent insulin production.

16. Use of a compound as defined in claim 1, in the manufacture of a medicament for:
treating diabetes; lowering blood levels of glucose; lowering blood levels of insulin;
increasing blood levels of incretins; lowering blood levels oftriglycerides; and/or
increasing glucose dependent insulin production, wherein the medicament is administered, or is prepared for administration, with a DPP IV inhibitor.

17. Use of a DPP IV inhibitor in the manufacture of a medicament for: treating diabetes; lowering blood levels of glucose; lowering blood levels of insulin; increasing blood levels of incretins; lowering blood levels of triglycerides; and/or increasing glucose dependent insulin production, wherein the medicament is administered, or is prepared for administration, with a compound as defined in claim 1.

18. Use of a compound as defined in claim 1, in the manufacture of a medicament for:
treating diabetes; lowering blood levels of glucose; lowering blood levels of insulin;
increasing blood levels of incretins; lowering blood levels oftriglycerides; and/or
increasing glucose dependent insulin production, in a patient who has previously been treated with a DPP IV inhibitor.

19. Use of a DPP IV inhibitor in the manufacture of a medicament for: treating diabetes; lowering blood levels of glucose; lowering blood levels of insulin; increasing blood levels of incretins; lowering blood levels of triglycerides; and/or increasing glucose dependent insulin production, in a patient who has previously been treated with a compound as defined in claim 1.

## Patentansprüche

1. Verbindung, ausgewählt aus der Gruppe bestehend aus: 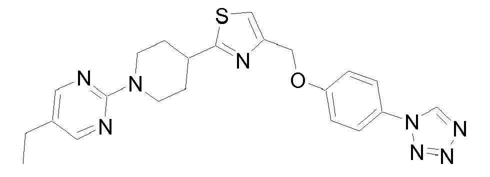 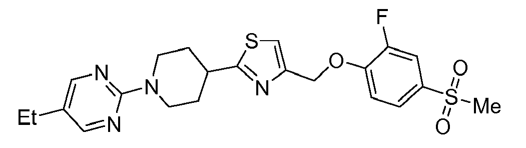 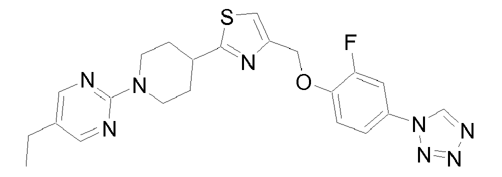 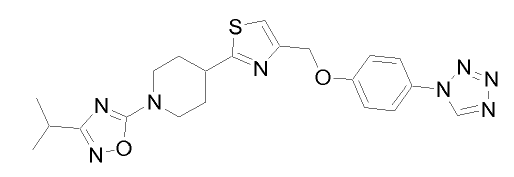 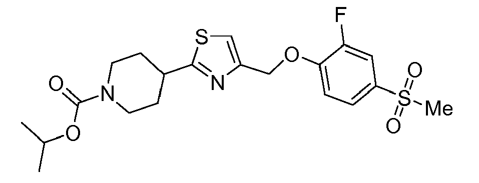 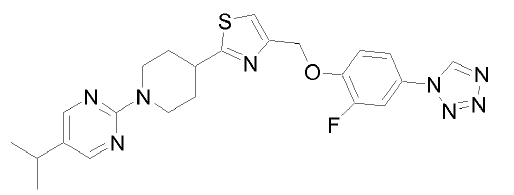 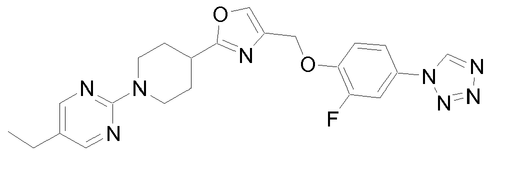 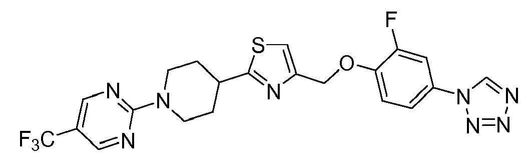 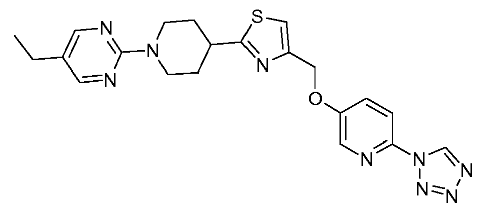 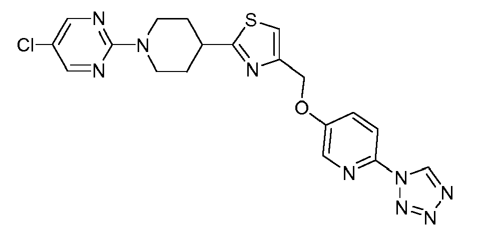 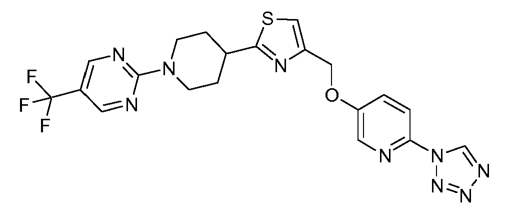 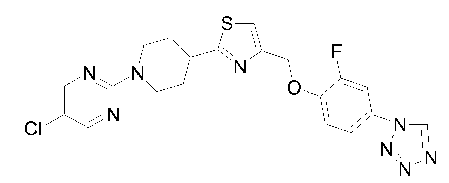 oder ein(en) pharmazeutisch akzeptables/n Salz oder Ester davon; und ein DPP-IV Inhibitor, zur Verwendung in einem Verfahren zum:
(i) Behandeln von Diabetes,
(ii) Senken von Blutglukosespiegeln,
(iii) Senken von Blutinsulinspiegeln,
(iv) Erhöhen von Blutinkretinspiegeln,
(v) Senken von Bluttriglyceridspiegeln, oder
(vi) Erhöhen von glukoseabhängiger Insulinproduktion.

2. Verbindung und DPP-IV Inhibitor zur Verwendung nach Anspruch 1, wobei die genannte Verbindung 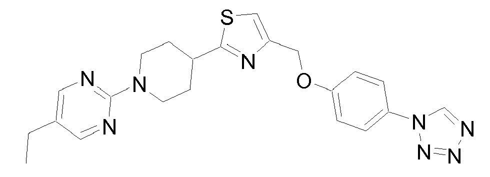 oder ein pharmazeutisch akzeptables Salz davon ist.

3. Verbindung und DPP-IV Inhibitor zur Verwendung nach Anspruch 1, wobei der genannte DPP-IV Inhibitor aus der Gruppe bestehend aus Sitagliptin, Vildagliptin, Denagliptin, Saxagliptin und Alogliptin ausgewählt ist.

4. Verbindung und DPP-IV Inhibitor zur Verwendung nach Anspruch 1, wobei der genannte DPP-IV Inhibitor Sitagliptin ist.

5. Verbindung und DPP-IV Inhibitor zur Verwendung nach Anspruch 1, wobei der genannte DPP-IV Inhibitor Vildagliptin ist.

6. Verbindung und DPP-IV Inhibitor zur Verwendung nach Anspruch 1, wobei das genannte Inkretin aus der Gruppe bestehend aus GLP-1 und GIP ausgewählt ist.

7. Verbindung nach Anspruch 1 und DPP-IV Inhibitor als kombiniertes Präparat zur gleichzeitigen, separaten oder sequentiellen Verwendung bei: der Behandlung von Diabetes; der Senkung von Blutglukosespiegeln; der Senkung von Blutinsulinspiegeln; der Erhöhung von Blutinkretinspiegeln; der Senkung von Bluttriglyceridspiegeln; und/oder der Erhöhung von glukoseabhängiger Insulinproduktion.

8. Verbindung nach Anspruch 1 und DPP-IV Inhibitor zur kombinierten Verwendung bei: der Behandlung von Diabetes; der Senkung von Blutglukosespiegeln; der Senkung von Blutinsulinspiegeln; der Erhöhung von Blutinkretinspiegeln; der Senkung von Bluttriglyceridspiegeln; und/oder der Erhöhung von glukoseabhängiger Insulinproduktion.

9. Verbindung nach Anspruch 1 zur Verwendung in Kombination mit einem DPP-IV Inhibitor bei: der Behandlung von Diabetes; der Senkung von Blutglukosespiegeln; der Senkung von Blutinsulinspiegeln; der Erhöhung von Blutinkretinspiegeln; der Senkung von Bluttriglyceridspiegeln; und/oder der Erhöhung von glukoseabhängiger Insulinproduktion.

10. DPP-IV Inhibitor zur Verwendung, in Kombination mit einer Verbindung nach Anspruch 1, bei: der Behandlung von Diabetes; der Senkung von Blutglukosespiegeln; der Senkung von Blutinsulinspiegeln; der Erhöhung von Blutinkretinspiegeln; der Senkung von Bluttriglyceridspiegeln; und/oder der Erhöhung von glukoseabhängiger Insulinproduktion.

11. Verbindung nach Anspruch 1 zur Verwendung bei: der Behandlung von Diabetes; der Senkung von Blutglukosespiegeln; der Senkung von Blutinsulinspiegeln; der Erhöhung von Blutinkretinspiegeln; der Senkung von Bluttriglyceridspiegeln; und/oder der Erhöhung von glukoseabhängiger Insulinproduktion, in einem Patienten, der zuvor mit einem DPP-IV Inhibitor behandelt wurde.

12. DPP-IV Inhibitor bei der Herstellung eines Medikaments zum: Behandeln von Diabetes; Senken von Blutglukosespiegeln; Senken von Blutinsulinspiegeln; Erhöhen von Blutinkretinspiegeln; Senken von Bluttriglyceridspiegeln; und/oder Erhöhen von glukoseabhängiger Insulinproduktion, in einem Patienten, der zuvor mit einer Verbindung nach Anspruch 1 behandelt wurde.

13. Verbindung nach Anspruch 1 zur Verwendung bei: der Behandlung von Diabetes; der Senkung von Blutglukosespiegeln; der Senkung von Blutinsulinspiegeln; der Erhöhung von Blutinkretinspiegeln; der Senkung von Bluttriglyceridspiegeln; und/oder der Erhöhung von glukoseabhängiger Insulinproduktion, wobei die Verbindung mit einem DPP-IV Inhibitor verabreicht oder zur Verabreichung damit hergestellt wird.

14. DPP-IV Inhibitor zur Verwendung bei: der Behandlung von Diabetes; der Senkung von Blutglukosespiegeln; der Senkung von Blutinsulinspiegeln; der Erhöhung von Blutinkretinspiegeln; der Senkung von Bluttriglyceridspiegeln; und/oder der Erhöhung von glukoseabhängiger Insulinproduktion, wobei der DPP-IV Inhibitor mit einer Verbindung nach Anspruch 1 verabreicht oder zur Verabreichung damit hergestellt wird.

15. Verwendung (a) einer Verbindung nach Anspruch 1, und (b) eines DPP-IV Inhibitors bei der Herstellung eines Medikaments zum: Behandeln von Diabetes; Senken von Blutglukosespiegeln; Senken von Blutinsulinspiegeln; Erhöhen von Blutinkretinspiegeln; Senken von Bluttriglyceridspiegeln; und/oder Erhöhen von glukoseabhängiger Insulinproduktion.

16. Verwendung einer Verbindung nach Anspruch 1 bei der Herstellung eines Medikaments zum: Behandeln von Diabetes; Senken von Blutglukosespiegeln; Senken von Blutinsulinspiegeln; Erhöhen von Blutinkretinspiegeln; Senken von Bluttriglyceridspiegeln; und/oder Erhöhen von glukoseabhängiger Insulinproduktion, wobei das Medikament mit einem DPP-IV Inhibitor verabreicht oder zur Verabreichung damit hergestellt wird.

17. Verwendung eines DPP-IV Inhibitors bei der Herstellung eines Medikaments zum:
Behandeln von Diabetes; Senken von Blutglukosespiegeln; Senken von Blutinsulinspiegeln; Erhöhen von Blutinkretinspiegeln; Senken von Bluttriglyceridspiegeln; und/oder Erhöhen von glukoseabhängiger Insulinproduktion, wobei das Medikament mit einer Verbindung nach Anspruch 1 verabreicht oder zur Verabreichung damit hergestellt wird.

18. Verwendung einer Verbindung nach Anspruch 1 bei der Herstellung eines Medikaments zum: Behandeln von Diabetes; Senken von Blutglukosespiegeln; Senken von Blutinsulinspiegeln; Erhöhen von Blutinkretinspiegeln; Senken von Bluttriglyceridspiegeln; und/oder Erhöhen von glukoseabhängiger Insulinproduktion, in einem Patienten, der zuvor mit einem DPP-IV Inhibitor behandelt wurde.

19. Verwendung eines DPP-IV Inhibitors bei der Herstellung eines Medikaments zum:
Behandeln von Diabetes; Senken von Blutglukosespiegeln; Senken von Blutinsulinspiegeln; Erhöhen von Blutinkretinspiegeln; Senken von Bluttriglyceridspiegeln; und/oder Erhöhen von glukoseabhängiger Insulinproduktion, in einem Patienten, der zuvor mit einer Verbindung nach Anspruch 1 behandelt wurde.

## Revendications

1. Composé sélectionné dans le groupe consistant en les suivants : 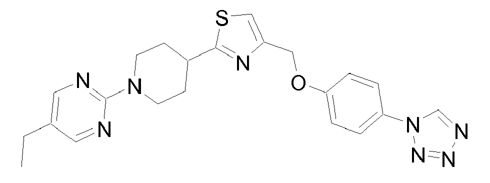 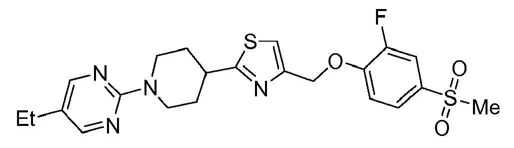 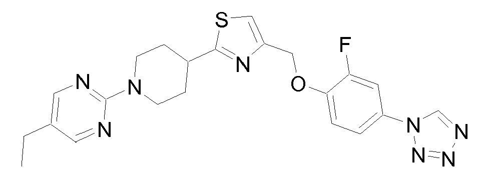 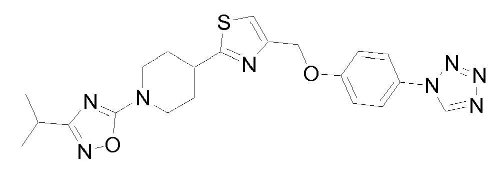 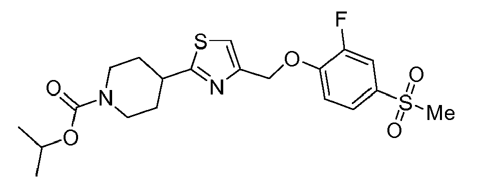 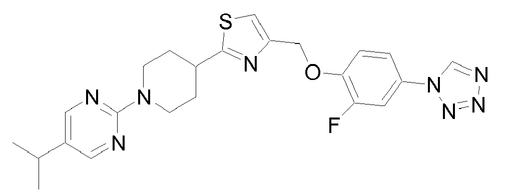 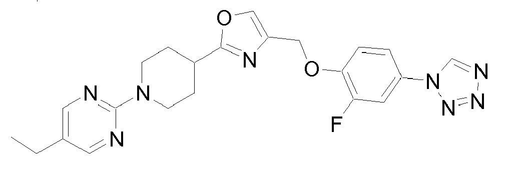 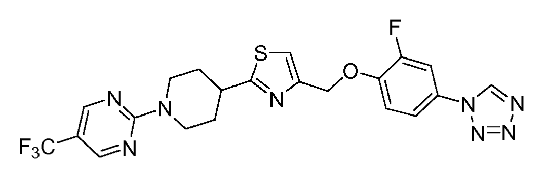 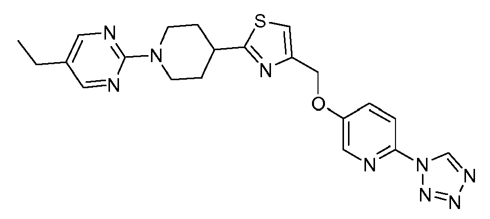 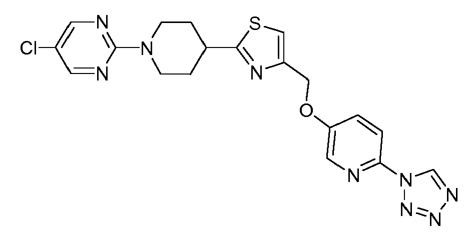 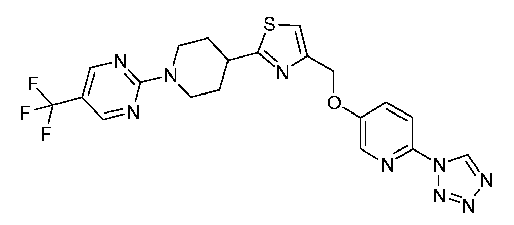 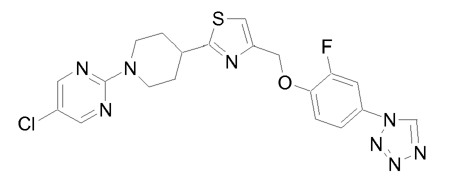 ou sel ou ester pharmaceutiquement acceptable de celui-ci ; et inhibiteur de la DPP-IV, pour une utilisation dans une méthode
(i) de traitement du diabète,
(ii) de réduction des taux sanguins de glucose,
(iii) de réduction des taux sanguins d'insuline,
(iv) d'augmentation des taux sanguins d'incrétines,
(v) de réduction des taux sanguins de triglycérides ou
(vi) d'augmentation de la production d'insuline dépendante du glucose.

2. Composé et inhibiteur de la DPP-IV pour une utilisation selon la revendication 1, où ledit composé est le suivant : 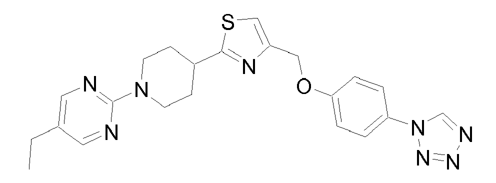 ou sel pharmaceutiquement acceptable de celui-ci.

3. Composé et inhibiteur de la DPP-IV pour une utilisation selon la revendication 1, où ledit inhibiteur de la DPP-IV est sélectionné dans le groupe consistant en la sitagliptine, la vildagliptine, la denagliptine, la saxagliptine et l'alogliptine.

4. Composé et inhibiteur de la DPP-IV pour une utilisation selon la revendication 1, où ledit inhibiteur de la DPP-IV est la sitagliptine.

5. Composé et inhibiteur de la DPP-IV pour une utilisation selon la revendication 1, où ledit inhibiteur de la DPP-IV est la vildagliptine.

6. Composé et inhibiteur de la DPP-IV pour une utilisation selon la revendication 1, où ladite incrétine est sélectionnée dans le groupe consistant en le GLP-1 et le GIP.

7. Composé selon la revendication 1 et inhibiteur de la DPP-IV sous forme d'une préparation combinée pour une utilisation simultanée, séparée ou en succession dans : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose.

8. Composé selon la revendication 1 et inhibiteur de la DPP-IV pour une utilisation en combinaison dans : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose.

9. Composé selon la revendication 1 pour une utilisation, en combinaison à un inhibiteur de la DPP-IV, dans : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose.

10. Inhibiteur de la DPP-IV pour une utilisation, en combinaison à un composé selon la revendication 1, dans : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose.

11. Composé selon la revendication 1 pour une utilisation dans : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose chez un patient qui a été traité antérieurement par un inhibiteur de la DPP-IV.

12. Inhibiteur de la DPP-IV utilisé dans la fabrication d'un médicament pour : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose chez un patient qui a été traité antérieurement par un composé selon la revendication 1.

13. Composé selon la revendication 1 pour une utilisation dans : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose, où le composé est administré ou est préparé pour être administré avec un inhibiteur de la DPP-IV.

14. Inhibiteur de la DPP-IV pour une utilisation dans : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose, où l'inhibiteur de la DPP-IV est administré ou est préparé pour être administré avec un composé selon la revendication 1.

15. Utilisation (a) d'un composé selon la revendication 1 et (b) d'un inhibiteur de la DPP-IV dans la fabrication d'un médicament pour : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose.

16. Utilisation d'un composé selon la revendication 1 dans la fabrication d'un médicament pour : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose, où le médicament est administré ou est préparé pour être administré avec un inhibiteur de la DPP-IV.

17. Utilisation d'un inhibiteur de la DPP-IV dans la fabrication d'un médicament pour :
le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose, où le médicament est administré ou est préparé pour être administré avec un composé selon la revendication 1.

18. Utilisation d'un composé selon la revendication 1 dans la fabrication d'un médicament pour : le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose chez un patient qui a été traité antérieurement par un inhibiteur de la DPP-IV.

19. Utilisation d'un inhibiteur de la DPP-IV dans la fabrication d'un médicament pour :
le traitement du diabète ; la réduction des taux sanguins de glucose ; la réduction des taux sanguins d'insuline ; l'augmentation des taux sanguins d'incrétines ; la réduction des taux sanguins de triglycérides ; et/ou l'augmentation de la production d'insuline dépendante du glucose chez un patient qui a été traité antérieurement par un composé selon la revendication 1.
